# EUROPEAN PATENT SPECIFICATION

(11) **EP 2 648 625 B1**
(45) Date of publication and mention of the grant of the patent: **13.11.2024**
(21) Application number: 10736095.0
(22) Date of filing: 29.01.2010
(51) Int. Cl.: A61F 5/00

(54) **STOMACH INSTRUMENT**
MAGENINSTRUMENT
INSTRUMENT STOMACAL

(30) Priority: 29.01.2009 WO PCT/SE2009/000044; 29.01.2009 WO PCT/SE2009/000043; 29.01.2009 WO PCT/SE2009/000042; 29.01.2009 WO PCT/SE2009/000045; 29.01.2009 WO PCT/SE2009/000046; 29.01.2009 WO PCT/SE2009/000047; 29.01.2009 WO PCT/SE2009/000048; 29.01.2009 WO PCT/SE2009/000049; 29.01.2009 WO PCT/SE2009/000050; 29.01.2009 WO PCT/SE2009/000051; 29.01.2009 WO PCT/SE2009/000052; 29.01.2009 WO PCT/SE2009/000053; 29.01.2009 WO PCT/SE2009/000054; 29.01.2009 WO PCT/SE2009/000055; 29.01.2009 WO PCT/SE2009/000056; 29.01.2009 WO PCT/SE2009/000057; 29.01.2009 WO PCT/SE2009/000058; 29.01.2009 WO PCT/SE2009/000059; 12.10.2009 WO PCT/SE2009/000452; 12.10.2009 WO PCT/SE2009/051154; 12.10.2009 WO PCT/SE2009/051155; 12.10.2009 WO PCT/SE2009/051156; 17.07.2009 US 213803 P; 17.07.2009 US 213813 P; 17.07.2009 US 213817 P; 17.07.2009 SE 0900997; 17.07.2009 SE 0901007; 17.07.2009 SE 0901009; 24.07.2009 SE 0901027; 27.07.2009 US 228633 P
(43) Date of publication of application: 16.10.2013
(73) Proprietor: Implantica Patent Ltd., 223 70 Lund (SE)
(72) Inventor: FORSELL, Peter, 6300 Zug (CH)
(74) Representative: AWA Sweden AB
(86) International application number: PCT/SE2010/000024
(87) International publication number: WO 2010/087756

(56) References cited:
- EP-A1- 1 859 746
- WO-A1-00/53102
- WO-A1-2005/092210
- WO-A2-01/67964
- WO-A2-2008/016776
- US-A1- 2003 216 754
- US-A1- 2004 193 190
- US-A1- 2004 225 305
- US-A1- 2005 261 712
- US-A1- 2007 167 960
- US-A1- 2007 282 356
- US-B1- 6 494 888
- US-B1- 6 506 196

## Description

### Technical field

The present invention relates generally to an instrument for treating Gastro Esophageal Reflux Disease (GERD) and/or Obesity in a human patient.

### Background

Gastro Esophageal Reflux Disease (GERD), or acid reflux disease, is a chronic condition resulting in mucosal damage in the esophagus produced by the recurring occurrence of acid reflux in the esophagus. This is commonly due to transient or permanent changes in the barrier between the esophagus and the stomach. This can be due to incompetence of the lower esophageal sphincter (LES), transient LED relaxation, impaired expulsion of gastric reflux from the esophagus, or a hiatal hernia.

Gastro Esophageal Reflux Disease can be treated in a number of ways, where surgical treatments are sometimes preferred over longtime medication. A standard surgical treatment is Nissen funduplication where the upper portion of the stomach is wrapped around LES to strengthen the sphincter and prevent acid reflux and to repair hiatal hernia. This procedure often done laparoscopically and is hard to perform transorally.

Another surgical treatment is to create an anti-reflux valve of fundus tissue. This treatment can be performed transorally, by using an endoscope to visualize the Z-line and attract tissue creating a suitable valve which is then fastened. Such treatment is hard and requires a skilled endoscopist. It includes numerous steps in each procedure, typically manipulating tissue more than two times and applying a plurality of individual fasteners to create an anti-reflux valve. Some patients also suffer from hiatal hernia which is an additional step to treat. This both increases time consumption of the endoscopist and each added step increase the risk of mistakes which can potentially be hard to undo.

From experience with implantation of medical devices, it is known that sutures between an implanted device and human tissue will not hold in the long term. For long term implantation of a device, there are two possibilities to keep the device in place. A first solution has been to suture human tissue to human tissue, to thereby keep the device in place. A second approach has been to provide sutures holding a device in place in the short term and to allow in-growth of human tissue into the device for holding the device in place over the long term.

A problem with providing an implantable device associated with the esophagus is that the outer surface of the esophagus is only comprised of esophagus muscle tissue, which is very easy to damage or migrate through. This is probably one reason why the Anglechik prosthesis described above has resulted in many complications, such as migration.

The stomach, on the other hand, has a serosa on its outside, thereby providing a much stronger membrane for suturing. Thus, suturing a device directly to the stomach wall provides a better result than suturing an implanted device to the esophagus.

Today, there exists a need for a long term treatment of GERD that is more effective than prior treatments and which does not result in any severe complications.

Surgical treatments in the upper gastrointestinal tract are today generally complicated to do in a minimal or non-invasive manner. The treatment's level of invasiveness depends on the arrangement of surgical instruments and the handling thereof. Thus, there exists a need for an instrument for performing surgical operations in a more effective way than prior instruments for surgical treatments in the stomach cavity.

The present invention also relates to an apparatus, a system, for treating obesity.

Obesity has been treated by gastric banding a band placed around the stomach to create a stoma, a restricted opening, to restrict the flow of food down to below the band. There has also been tried to use electrical stimulation of the stomach wall to cause the patient to feel satiety.

When the stomach gets distended the patient gets a feeling that the stomach is full.

Another prior art way of treating obesity is to insert a balloon-like object into the stomach of the patient. In this way, the patient is given the feeling of satiety much more quickly when eating, preventing excessive intake of food. However, these prior art balloon-like objects are subject to stomach acids, leading to their destruction within a couple of months of use.

An example of a prior art inflatable gastric device for treating obesity is disclosed in US patent No. 4,246,893 to Berson. In this document, it is disclosed an abdominal method wherein an inflatable balloon is surgically implanted in the abdominal cavity of the patient adjacent to the stomach. An adjusting port is provided subcutaneously and the balloon is subsequently inflated by means of inserting a hypodermic needle through the skin of the patient into the adjusting port and introducing a fluid under pressure into the port for passage into the balloon to distend the upper abdomen, compressing the stomach and thereby producing a sense of satiety. Patent document EP1859746 discloses features falling under the preamble of claim 1.

The treatment's level of invasiveness depends on the arrangement of surgical instruments and the handling thereof. Thus, there exists a need for an instrument for performing surgical operations in a more effective way than prior instruments for surgical treatments in the stomach cavity.

### Summary of the invention

It is an object of the present invention to overcome, or at least reduce, some of the problems associated with existing surgical treatments of Gastro Esophageal Reflux Disease (GERD). It is another object of the present invention to provide an apparatus, instrument for treating Gastro Esophageal Reflux Disease. Furthermore, it is another object of the present invention to provide an apparatus, instrument for treating obesity. These objects and others are obtained by appended claims.

Another object of the present invention to provide obesity treatment apparatus, system with improved long term properties.

This object and others are obtained by an apparatus described in the appended claims. In our first embodiment we provide: The invention is defined in claim 1. Further embodiments of the invention are defined in the dependent claims.

An endoscopic instrument adapted to engage the stomach tissue and/or the esophagus tissue of a patient from the inside and/or outside thereof is provided, the esophagus being a substantially tube shaped tissue leading to the stomach comprising stomach tissue, the esophagus having an esophagus center axis, being substantially aligned with a cranial-caudal axis of the patient and having a substantially circular circumference substantially aligned with a horizontal plane of the patient, the esophagus further having an inner and outer substantially cylindrical surface extending radially in relation to the esophagus center axis, wherein the instrument is adapted to deliver at least one fixating member, such that the at least one fixating member engages at least the stomach tissue. Such endoscopic instrument may help the endoscopist to a greater extent accurately perform attachment of stomach tissue correctly and more efficiently. Thus, enabling the endoscopist to perform the treatment in a minimal invasive way. The method may also enable the endoscopist to perform the treatment in a less time consuming manner.

According to another embodiment, the instrument comprises at least one unit adapted to deliver fixating members, and wherein the fixating members are adapted to penetrate at least one layer of stomach tissue and one layer of esophagus or stomach tissue.

According to yet another embodiment, the instrument comprises at least one unit adapted to deliver fixating members, and wherein the fixating members are adapted to penetrate at least two layers of stomach tissue and one layer of esophagus tissue.

The instrument according to another embodiment comprises at least one unit adapted to deliver a fixating member in form of a staple or a suture.

According to yet another embodiment, the at least one unit is adapted to deliver a fixating member comprising a first and second state, wherein the fixating member is adapted to, in the first state, penetrate tissue, and in the second state, hinder the fixating member from penetrating tissue.

According to another embodiment, the instrument comprises a fixation member insertion device and a fixation member altering device, wherein the fixation member insertion device is adapted to insert the fixation member through the tissue, the fixation device being in the first state, and wherein the fixation member altering device is adapted to alter the state of the fixation member such that the fixation member is placed in the second state.

According to yet another embodiment, the fixation member insertion device and/or the insertion member altering device comprise an elongated member adapted to house the fixation member.

The instrument according to another embodiment further comprises at least one anvil member, and wherein the unit is adapted to deliver the fixation member such that the fixation member penetrates the tissue of the esophagus and the tissue of the stomach and engages the anvil member.

According to another embodiment, the anvil member is adapted to be positioned within the esophagus.

In yet another embodiment, the anvil member is adapted to be positioned within the stomach.

According to yet another embodiment, the instrument is a gastroscopic instrument adapted to be inserted into the esophagus and/or stomach through the throat of the patient for operating on the esophagus and/or stomach.

According to yet another embodiment, the instrument is a laparoscopic instrument adapted to be inserted into the patient from the outside of the patient into the abdominal cavity and further adapted to be inserted into the esophagus and/or stomach through an incision opening in the stomach and/or esophagus wall of the patient and operate on the esophagus and/or stomach from the inside thereof.

The instrument according to another embodiment, wherein the instrument is a laparoscopic instrument adapted to be inserted into the patient through a trocar from the outside of the patient into the abdominal cavity and operate on the esophagus and/or stomach from the outside thereof.

The instrument according to yet another embodiment, wherein the at least one unit is adapted to deliver fixation members penetrating three layers of stomach tissue and one layer of esophagus tissue, for fixating the three layers of stomach tissue and one layer of esophagus tissue to each other.

According to another embodiment, wherein the instrument is adapted to create a pouch of stomach tissue, through the fixation of two layers of stomach tissue.

According to yet another embodiment, wherein the instrument is adapted to create the pouch around a volume filling device placed in contact with the stomach tissue.

The medical instrument according another embodiment, wherein the instrument further comprises a cutting member adapted to create an opening in the pouch from the inside of the stomach.

The instrument according to yet another embodiment, wherein the unit is adapted to deliver fixation members penetrating two layers of stomach tissue and one layer of esophagus tissue, for fixating at least one of the two layers of stomach tissue and one layer of esophagus tissue to each other.

According to another embodiment, wherein the unit is adapted to deliver at least one fixation member penetrating four layers of stomach tissue and one layer of esophagus tissue, for fixating at least three layers of stomach tissue and one layer of esophagus tissue to each other.

According to yet another embodiment, wherein the instrument is adapted to create a pouch of stomach tissue, through the fixation of two of the three layers of stomach tissue.

The medical instrument according to another embodiment, wherein the instrument is adapted to create the pouch around a volume filling device placed in contact with the stomach tissue.

The medical instrument according to yet another embodiment, wherein the instrument further comprises a cutting member adapted to create an opening in the pouch from the outside of the stomach.

According to another embodiment, wherein the instrument is further adapted to insert a volume filling device in the pouch, after the creation of the pouch.

According to yet another embodiment, wherein the instrument is adapted to insert a volume filling device comprising a solidifying liquid.

The instrument according to another embodiment, wherein the instrument comprises a conduit adapted to be inserted into the pouch created in the stomach tissue, wherein the instrument is adapted to deliver the solidifying fluid into the pouch through the conduit.

The instrument according to yet another embodiment, wherein the instrument comprises at least one operable joint, for enabling the instrument to deliver the solidifying liquid to the pouch, when the pouch is at least partly created of fundus stomach tissue.

According to another embodiment, wherein the instrument is adapted to insert a volume filling device comprising a plurality of volume filling devices.

According to yet another embodiment, wherein the plurality of volume filling devices are adapted to be connected to each other to form an interconnected volume filling device, after the plurality of volume filling devices have been inserted into the pouch.

The instrument according to another embodiment, wherein the instrument further comprises a tubular member adapted to be inserted into the pouch created of the stomach tissue, wherein the instrument is adapted to deliver the plurality of volume filling devices into the pouch created of the stomach tissue through the tubular member.

The instrument according to yet another embodiment, wherein the instrument comprises at least one operable joint, for enabling the instrument to deliver the plurality of volume filling devices to the pouch, when the pouch is created of fundus stomach tissue or stomach tissue in general.

According to another embodiment, wherein the instrument is adapted to insert a volume filling device comprising an expandable volume filling device.

According to yet another embodiment, wherein the instrument is adapted to fill the expandable volume filling device with a solidifying liquid.

The instrument according to another embodiment, wherein the instrument is adapted to fill the expandable volume filling device with a plurality of volume filling devices.

The instrument according to yet another embodiment, wherein the instrument is adapted to insert a volume filling device comprising a combination of: a plurality of volume filling devices, and a solidifying liquid.

According to another embodiment, wherein the instrument further comprises an elongated tubular member adapted to enable the volume filling device to pass through the tubular member and into the pouch.

According to yet another embodiment, wherein the tubular member is adapted to be inserted into the pouch created by the stomach tissue, for enabling the insertion of the volume filling device through the tubular member.

The instrument according to another embodiment, wherein the instrument further comprises a tissue closing unit adapted to close the opening created in the pouch.

The instrument according to yet another embodiment, wherein the instrument further comprises a stomach tissue pulling or pushing device adapted to hold and pull or push a portion of the stomach tissue.

The instrument according to yet another embodiment, wherein the stomach tissue pulling or pushing device is adapted to engage the stomach tissue using a stomach tissue penetrating member or a stomach clamping member.

An endoscopic instrument according to another embodiment, wherein the stomach tissue pulling or pushing device is adapted to engage the stomach tissue using suction.

According to another embodiment, wherein the stomach tissue pulling device is adapted to pull the stomach tissue into an opening of a luminal space inside of the instrument.

According to yet another embodiment, wherein the area of a first cross-section of the luminal space is larger than an area of a second parallel cross-section placed in the opening of the luminal space, placed more distally in the luminal space or distally in the instrument.

The instrument according to another embodiment, wherein the unit is adapted to be placed more distal in the instrument than the luminal space, such that the unit creates a pouch when the at least one fixating member is delivered.

The instrument according to yet another embodiment, wherein a retractable portion is placed more distal than the first and second unit, thus enabling the removal of the instrument after the fixating members have been delivered.

According to another embodiment, wherein the instrument comprises at least one operable joint adapted to enable a first portion of the instrument to be placed inside of the stomach in a position such that the layer of stomach tissue and the layer of esophagus tissue are placed between the first portion of the instrument and a second portion of the instrument.

According to yet another embodiment, wherein unit is adapted to place the fixating members above the gastroesophageal junction.

The instrument according to another embodiment, wherein the instrument is adapted to place the fixating members above the gastroesophageal junction for creating a tunnel between the esophagus and stomach above the junction.

The instrument according to yet another embodiment, wherein the instrument further comprises an operable joint for placing a portion of the instrument inside of the stomach above the gastroesophageal junction in relation to the cranial-caudal axis of the patient.

According to another embodiment, wherein the instrument is adapted to create a pouch and place at least one first fixating member for creating the pouch, and wherein the instrument is adapted to place at least one second fixating member for creating the pouch, such that the at least one second fixating member intersects the at least one first fixating member.

According to yet another embodiment, wherein the instrument is adapted to place the at least one second fixating member perpendicularly to the at least one first fixating member.

An endoscopic instrument according to another embodiment, wherein the instrument is an instrument for treating reflux disease, wherein the instrument comprises: a first unit adapted to deliver a first fixating member for fixating the esophagus tissue to the stomach tissue, and a second unit adapted to deliver a second fixating member, wherein the first and second units are adapted to be arranged such that the first unit can deliver the first fixating member at a first point along a first esophagus surface length axis substantially parallel to the esophagus center axis, and wherein the second unit is adapted to be arranged such that the second unit can deliver the second fixating member at a second point along a second esophagus surface length axis substantially parallel to the first esophagus surface length axis and the esophagus center axis center, wherein the first esophagus surface length axis is positioned at a distance from the second esophagus surface length axis, radially in relation to the esophagus center axis. An endoscopic instrument enabling the endoscopist to fixate esophagus tissue to stomach tissue at a first and a second point at a distance from the first point may result in a more durable fixation. Such fixation may further enable serosa-to-serosa to be displaced in contact which may enable the process if tissue of esophagus and serosa tissue of the stomach to grow together which may even further increase the durability of the fixation. Such fixation may be advantageous in achieving long-term fixation of two tissues.

According to another embodiment, wherein the first and second units are adapted to deliver the first and second fixating member substantially simultaneously or in a sequence.

According to yet another embodiment, wherein the first and/or second units are adapted to deliver a fixating member in form of a staple or suture.

The instrument according to another embodiment, wherein the first and/or second unit is adapted to deliver a fixating member comprising a first and second state, wherein the fixating member is adapted to in the first state penetrate tissue, and in the second state hinder the fixating member from penetrating tissue.

The instrument according to yet another embodiment, wherein the instrument comprises a fixation member insertion device and a fixation member altering device, wherein the fixation member insertion device is adapted to insert the fixation member through the tissue, the fixation device being in the first state, and wherein the fixation member altering device is adapted to alter the state of the fixation member such that the fixation member is placed in the second state.

According to another embodiment, wherein the fixation member insertion device and/or the insertion member altering device comprises an elongated member adapted to house the fixation member.

According to yet another embodiment, wherein the instrument further comprises at least one anvil member, and wherein the first and second unit is adapted to deliver the first and second fixation member such that the first and second fixation member penetrates the tissue of the esophagus and the tissue of the stomach and engages the anvil member.

The instrument according to another embodiment, wherein the anvil member is adapted to be positioned within the esophagus.

The instrument according to yet another embodiment, wherein the anvil member is adapted to be positioned within the stomach.

According to another embodiment, wherein the instrument is a gastroscopic instrument adapted to be inserted into the esophagus and/or stomach through the throat of the patient.

According to yet another embodiment, wherein the instrument is a laparoscopic instrument adapted to be to be inserted into the patient from the outside of the body into the abdominal cavity and further adapted to be inserted into the esophagus and/or stomach through an incision if the stomach and/or esophagus wall of the patient for operating on the esophagus and/or stomach from the inside thereof.

The instrument according to another embodiment, wherein the instrument is a laparoscopic instrument adapted to be inserted into the patient through a trocar from the outside of the patient into the abdominal cavity and operate on the esophagus and/or stomach from the outside thereof.

The instrument according to yet another embodiment, wherein the first and second positions are located at a distance, radially, larger than 2 mm.

According to another embodiment, wherein the first and second positions are located at a distance, radially, larger than 4 mm.

According to yet another embodiment, wherein the first and second units are adapted to deliver fixation members penetrating three layers of stomach tissue and one layer of esophagus tissue, for fixating the three layers of stomach tissue and one layer of esophagus tissue to each other.

An endoscopic instrument according to another embodiment, wherein the instrument is adapted to create a pouch of stomach tissue, through the fixation of two of the layers of stomach tissue.

An endoscopic instrument according to yet another embodiment, wherein the instrument is adapted to create the pouch around a volume filling device placed in contact with the stomach tissue.

An instrument according to another embodiment, wherein the instrument further comprises a cutting member adapted to create an opening in the pouch from the inside of the stomach.

An instrument according to yet another embodiment, wherein the first and second units are adapted to deliver fixation members penetrating two layers of stomach tissue and one layer of esophagus tissue, for fixating at least one of the two layers of stomach tissue and one layer of esophagus tissue to each other.

According to another embodiment, wherein the first and second units are adapted to deliver fixation members penetrating four layers of stomach tissue and one layer of esophagus tissue, for fixating at least three layers of stomach tissue and one layer of esophagus tissue to each other.

According to yet another embodiment, wherein the instrument is adapted to create a pouch of stomach tissue, through the fixation of two of the three layers of stomach tissue.

An instrument according to another embodiment, wherein the instrument is adapted to create the pouch around a volume filling device placed in contact with the stomach tissue.

According to another embodiment, wherein the instrument further comprises a cutting member adapted to create an opening in the pouch from the outside of the stomach.

According to another embodiment, wherein the instrument is further adapted to insert a volume filling device in the pouch, after the creation of the pouch.

According to yet another embodiment, wherein the instrument is adapted to insert a volume filling device comprising a solidifying liquid.

An instrument according to another embodiment, wherein the instrument comprises a conduit adapted to be inserted into the pouch created in the stomach tissue, wherein the instrument is adapted to deliver the solidifying fluid into the pouch through the conduit.

An instrument according to yet another embodiment, wherein the instrument comprises at least one operable joint, for enabling the instrument to deliver the solidifying liquid to the pouch, when the pouch is at least partly created of fundus stomach tissue.

According to another embodiment, wherein the instrument is adapted to insert a volume filling device comprising a plurality of volume filling devices.

According to yet another embodiment, wherein the plurality of volume filling devices are adapted to be connected to each other to form an interconnected volume filling device, after the plurality of volume filling devices have been inserted into the pouch.

An instrument according to another embodiment, wherein the instrument further comprises a tubular member adapted to be inserted into the pouch created of the stomach tissue, wherein the instrument is adapted to deliver the plurality of volume filling devices into the pouch created of the stomach tissue through the tubular member.

An instrument according to yet another embodiment, wherein the instrument comprises at least one operable joint, for enabling the instrument to deliver the plurality of volume filling devices to the pouch, wherein the pouch is created of fundus stomach tissue or stomach tissue in general.

An instrument according to yet another embodiment, wherein the instrument is adapted to insert a volume filling device comprising an expandable volume filling device.

An instrument according to yet another embodiment, wherein the instrument is adapted to fill the expandable volume filling device with a solidifying liquid.

According to another embodiment, wherein the instrument is adapted to fill the expandable volume filling device with a plurality of volume filling devices.

According to yet another embodiment, wherein the instrument is adapted to insert a volume filling device comprising a combination of: a plurality of volume filling devices, and a solidifying liquid.

According to yet another embodiment, wherein the instrument further comprises an elongated tubular member adapted to enable the volume filling device to pass through the tubular member and into the pouch.

According to yet another embodiment, wherein the tubular member is adapted to be inserted into the pouch created by the stomach tissue, for enabling the insertion of the volume filling device through the tubular member.

An instrument according to another embodiment, wherein the instrument further comprises a tissue closing unit adapted to close the opening created in the pouch.

An instrument according to yet another embodiment, wherein the instrument further comprises a stomach tissue pulling or pushing device adapted to hold and pull or push a portion of the stomach tissue.

According to another embodiment, wherein the stomach tissue pulling or pushing device is adapted to engage the stomach tissue using a stomach tissue penetrating member or stomach clamping member.

According to another embodiment, wherein the stomach tissue pulling or pushing device is adapted to engage the stomach tissue using suction.

According to another embodiment, wherein the stomach tissue pulling device is adapted to pull the stomach tissue into an opening of a luminal space inside of the instrument.

According to yet another embodiment, wherein the area of a first cross-section of the luminal space is larger than an area of a second parallel cross-section placed in the opening of the luminal space, placed more distally in the instrument or more distally in the luminal space.

An endoscopic instrument according to another embodiment, wherein the first and second units are adapted to be placed more distal in the instrument than the luminal space, such that the units creates a pouch when the fixating members are delivered.

An endoscopic instrument according to yet another embodiment, wherein a retractable portion is placed more distal than the first and second unit, thus enabling the removal of the instrument after the fixating members have been delivered.

According to another embodiment, wherein the instrument comprises at least one operable joint adapted to enable a first portion of the instrument to be placed inside of the stomach in a position such that the layer of stomach tissue and the layer of esophagus tissue are placed between the first portion of the instrument and a second portion of the instrument.

According to yet another embodiment, wherein the first and second units are adapted to place the first and second fixating members above the gastroesophageal junction.

According to yet another embodiment, wherein the instrument further comprises an operable joint for placing a portion of the instrument inside of the stomach above the gastroesophageal junction in relation to the cranial-caudal axis of the patient.

An instrument according to another embodiment, wherein the instrument is adapted to place the fixating members above the gastro-esophageal junction for creating a tunnel between the esophagus and stomach above the junction.

An instrument according to yet another embodiment, wherein the instrument is adapted to place at least one first fixating member for creating the pouch, and wherein the instrument is adapted to place at least one second fixating member for creating the pouch, such that the at least one second fixating member intersects the at least one first fixating member.

According to yet another embodiment, wherein the instrument comprises a tubular member adapted to be inserted into a pouch created in the stomach tissue, wherein the instrument is adapted to deliver a plurality of volume filling devices into the pouch created in the stomach tissue through the tubular member. One possible advantage with inserting a plurality of volume filling devices into the pouch may be the ability to avoid ileus in the case if the pouch ruptures. Ileus may be both harmful and bring great discomfort to the patient. The plurality of volume filling object may satisfy the property of avoiding ileus, due to each volume filling device's individual size, and as a plurality satisfy the property of filling the volume required by of the treatment method.

According to another embodiment, wherein the instrument comprises at least one operable joint, for enabling the instrument to deliver the plurality of volume filling devices to a pouch created of the fundus tissue, when the instrument is inserted in the stomach or abdomen.

According to yet another embodiment, wherein the instrument comprises a conduit adapted to be inserted into a pouch created in the stomach tissue, and wherein the instrument is adapted to deliver a solidifying fluid into the pouch created in the stomach tissue through the conduit.

An instrument according to another embodiment, wherein the instrument comprises at least one operable joint, for enabling the instrument to deliver the plurality of volume filling devices to a pouch created of the fundus tissue, when the instrument is inserted in the stomach or abdomen.

An instrument according to yet another embodiment, wherein the instrument further comprises a conduit for delivering a healing agent from outside of the human patient to an area of the stomach and/or esophagus.

An endoscopic instrument according to another embodiment, wherein the instrument comprises at least one unit adapted to deliver fixating members, and wherein the fixating members are adapted to penetrate at least one layer of stomach tissue and one layer of esophagus tissue, wherein the instrument is adapted to place the fixating members above the gastro-esophageal junction for creating a tunnel between the esophagus and stomach above the junction, wherein the at least one unit comprising an esophagus part adapted to be placed in the esophagus and a stomach part adapted to be placed in the stomach, wherein the stomach and esophagus part is adapted to place the fixating member substantially between themselves, wherein the instrument is adapted to have a part of the instrument including the esophagus part of the unit inserted into the esophagus via the stomach cavity and a stomach part of the instrument including the stomach part of the unit inserted into the stomach cavity from the abdominal cavity and further from the outside of the body, wherein the instrument has a shape to allow it to be directed to reach a position both in the esophagus and the stomach such that at least one fixating member will attach the outer stomach wall to the outer esophagus wall above the gastro-esophageal junction for creating the tunnel, wherein all of the following distances of the instrument is adapted for enabling the placement of such a fixation member: a distance between the end of the instrument and the stomach part of the unit and a distance between the esophagus part and stomach part of the unit. This instrument may in some cases decrease the treatment complexity and potentially increase the time consumed and/or the invasiveness of the treatment.

According to another embodiment, wherein the instrument comprises at least one unit adapted to deliver fixating members, and wherein the fixating members are adapted to penetrate at least one layer of stomach tissue and one layer of esophagus tissue, wherein the instrument is adapted to place the fixating members above the gastro-esophageal junction for creating a tunnel between the esophagus and stomach above the junction, wherein the at least one unit comprising an esophagus part adapted to be placed in the esophagus and a stomach part adapted to be placed in the stomach, wherein the stomach and esophagus part is adapted to place the fixating member substantially between themselves, wherein the instrument is adapted to be inserted into the main stomach cavity below the junction and adapted to direct the instrument in cranial direction to reach a position of the unit above the junction, wherein the tunnel will allow a substantially unrestricted contraction and release of the cardia closing sphincter muscle placed in the junction, when such a tunnel has been created, wherein all of the following distances of the instrument is adapted for enabling the creation of such a tunnel: a distance between the unit and the end of the instrument and a distance between the esophagus part and stomach part of the unit. This instrument may in some cases decrease the treatment complexity and potentially increase the time consumed and/or the invasiveness of the treatment.

According to yet another embodiment, wherein the instrument comprises at least one unit adapted to deliver fixating members, and wherein the fixating members are adapted to penetrate at least one layer of stomach tissue and one layer of esophagus tissue, wherein the instrument is adapted to place the fixating members above the gastro-esophageal junction for creating a tunnel between the esophagus and stomach above the junction, wherein the at least one unit comprising an esophagus part adapted to be placed in the esophagus and a stomach part adapted to be placed in the stomach, wherein the parts is adapted to place the fixating member substantially between themselves, wherein the instrument comprises at least one operable joint, for enabling the instrument to be inserted into the main stomach cavity above the junction and wherein the joint is adapted to bend and direct the instrument in a direction to reach a position of the part of the unit in the esophagus above the junction, wherein the tunnel will allow substantially unrestricted contraction and release of the cardia closing sphincter muscle placed in the junction, when such tunnel has been created, wherein all of the following distances of the instrument is adapted for enabling the creation of such a tunnel: a distance between the operable joint and the unit delivering the fixating member, a distance between the unit and the end of the instrument and a distance between the esophagus part and stomach part of the unit. This instrument may in some cases decrease the treatment complexity and potentially increase the time consumed and/or the invasiveness of the treatment.

According to another embodiment, wherein the at least one unit is adapted to deliver a fixating member in form of a staple or suture.

According to yet another embodiment, wherein the at least one unit is adapted to deliver a fixating member comprising a first and second state, wherein the fixating member is adapted to, in the first state, penetrate tissue, and in the second state, hinder the fixating member from penetrating tissue.

An instrument according to another embodiment, wherein the instrument comprises a fixation member insertion device and a fixation member altering device, wherein the fixation member insertion device is adapted to insert the fixation member through the tissue, the fixation device being in the first state, and wherein the fixation member altering device is adapted to alter the state of the fixation member such that the fixation member is placed in the second state.

An instrument according to yet another embodiment, wherein the fixation member insertion device and/or the insertion member altering device comprises an elongated member adapted to house the fixation member.

According to another embodiment, wherein the instrument further comprises at least one anvil member, and wherein the unit is adapted to deliver the fixation member such that the fixation member penetrates the tissue of the esophagus and the tissue of the stomach and engages the anvil member.

According to yet another embodiment, wherein the anvil member is adapted to be positioned within the esophagus or within the stomach.

An instrument according to another embodiment, wherein the instrument is a gastroscopic instrument adapted to be inserted into the esophagus and/or stomach through the throat of the patient and operate on the esophagus and/or stomach from the inside thereof.

An instrument according to yet another embodiment, wherein the instrument is a laparoscopic instrument adapted to be inserted into the patient from the outside of the patient into the abdominal cavity and further adapted to be inserted into the esophagus and/or stomach through an opening in the stomach and/or esophagus wall of the patient and operate on the esophagus and/or stomach from the inside thereof.

An instrument according to yet another embodiment, wherein the instrument is a laparoscopic instrument adapted to be inserted into the patient through a trocar from the outside of the patient into the abdominal cavity and operate on the esophagus and/or stomach from the outside thereof.

An instrument according to yet another embodiment, wherein the at least one unit is adapted to deliver fixation members penetrating three layers of stomach tissue and one layer of esophagus tissue, for fixating the three layers of stomach tissue and one layer of esophagus tissue to each other.

According to another embodiment, wherein the instrument is adapted to create a pouch of stomach tissue, through the fixation of two layers of stomach tissue.

According to yet another embodiment, wherein the instrument is adapted to create the pouch around a volume filling device placed in contact with the stomach tissue.

According to yet another embodiment, wherein the instrument further comprises a cutting member adapted to create an opening in the pouch from the inside of the stomach.

According to yet another embodiment, wherein the unit is adapted to deliver fixation members penetrating two layers of stomach tissue and one layer of esophagus tissue, for fixating at least one of the two layers of stomach tissue and one layer of esophagus tissue to each other.

According to yet another embodiment, wherein the unit is adapted to deliver at least one fixation member penetrating four layers of stomach tissue and one layer of esophagus tissue, for fixating at least three layers of stomach tissue and one layer of esophagus tissue to each other.

An instrument according to another embodiment, wherein the instrument is adapted to create a pouch of stomach tissue, through the fixation of two of the three layers of stomach tissue.

An instrument according to yet another embodiment, wherein the instrument is adapted to create the pouch around a volume filling device placed in contact with the stomach tissue.

An instrument according to yet another embodiment, wherein the instrument further comprises a cutting member adapted to create an opening in the pouch from the outside of the stomach.

According to another embodiment, wherein the instrument is further adapted to insert a volume filling device in the pouch, after the creation of the pouch.

According to yet another embodiment, wherein the instrument is adapted to insert a volume filling device comprising a solidifying liquid.

According to yet another embodiment, wherein the instrument comprises a conduit adapted to be inserted into the pouch created in the stomach tissue, wherein the instrument is adapted to deliver the solidifying fluid into the pouch through the conduit.

An instrument according to another embodiment, wherein the instrument comprises at least one operable joint, for enabling the instrument to deliver the solidifying liquid to the pouch, when the pouch is at least partly created by fundus stomach tissue.

An instrument according to yet another embodiment, wherein the instrument is adapted to insert a volume filling device comprising a plurality of volume filling devices.

An instrument according to yet another embodiment, wherein the plurality of volume filling devices are adapted to be connected to each other to form an interconnected volume filling device, after the plurality of volume filling devices have been inserted into the pouch.

An instrument according to yet another embodiment, wherein the instrument further comprises a tubular member adapted to be inserted into the pouch created of the stomach tissue, wherein the instrument is adapted to deliver the plurality of volume filling devices into the pouch created of the stomach tissue through the tubular member.

According to another embodiment, wherein the instrument comprises at least one operable joint, for enabling the instrument to deliver the plurality of volume filling devices to the pouch, when the pouch is created of fundus stomach tissue or stomach tissue in general.

According to yet another embodiment, wherein the instrument is adapted to insert a volume filling device comprising an expandable volume filling device.

According to yet another embodiment, wherein the instrument is adapted to fill the expandable volume filling device with a solidifying liquid.

An instrument according to another embodiment, wherein the instrument is adapted to fill the expandable volume filling device with a plurality of volume filling devices.

An instrument according to yet another embodiment, wherein the instrument is adapted to insert a volume filling device comprising a combination of: a plurality of volume filling devices, and a solidifying liquid.

An instrument according to yet another embodiment, wherein the instrument further comprises an elongated tubular member adapted to enable the volume filling device to pass through the tubular member and into the pouch.

An instrument according to yet another embodiment, wherein the tubular member is adapted to be inserted into the pouch created by the stomach tissue, for enabling the insertion of the volume filling device through the tubular member.

An instrument according to yet another embodiment, wherein the instrument further comprises a tissue closing unit adapted to close the opening created in the pouch.

According to another embodiment, wherein the instrument further comprises a stomach tissue pulling or pushing device adapted to hold and pull or push a portion of the stomach tissue.

According to yet another embodiment, wherein the stomach tissue pulling or pushing device is adapted to engage the stomach tissue using a stomach tissue penetrating member or a stomach clamping member.

According to yet another embodiment, wherein the stomach tissue pulling or pushing device is adapted to engage the stomach tissue using suction.

According to yet another embodiment, wherein the stomach tissue pulling device is adapted to pull the stomach tissue into an opening of a luminal space inside of the instrument.

According to yet another embodiment, wherein the area of a first cross-section of the luminal space is larger than an area of a second parallel cross-section placed in the opening of the luminal space, placed more distally in the luminal space or distally in the instrument.

An instrument according to another embodiment, wherein the unit is adapted to be placed more distal in the instrument than the luminal space, such that the unit creates a pouch when the at least one fixating member is delivered.

An instrument according to yet another embodiment, wherein a retractable portion is placed more distal than the first and second unit, thus enabling the removal of the instrument after the fixating members have been delivered.

An instrument according to yet another embodiment, wherein the instrument comprises at least one operable joint adapted to enable a first portion of the instrument to be placed inside of the stomach in a position such that the layer of stomach tissue and the layer of esophagus tissue are placed between the first portion of the instrument and a second portion of the instrument.

An instrument according to yet another embodiment, wherein the first and second unit are adapted to place the fixating members above the gastroesophageal junction.

According to another embodiment, wherein the instrument is adapted to place the fixating members above the gastroesophageal junction for creating a tunnel between the esophagus and stomach above the junction.

According to yet another embodiment, wherein the instrument further comprises an operable joint for placing a portion of the instrument inside of the stomach above the gastroesophageal junction in relation to the cranial-caudal axis of the patient.

According to yet another embodiment, wherein the instrument is adapted to place the at least one second fixating member perpendicularly to the at least one first fixating member.

An instrument according to another embodiment, wherein the instrument is adapted to create a pouch and place at least one first fixating member for creating the pouch, and wherein the instrument is adapted to place at least one second fixating member for creating the pouch, such that the at least one second fixating member intersects the at least one first fixating member.

An instrument according to yet another embodiment, wherein the instrument is adapted to place the at least one second fixating member perpendicularly to the at least one first fixating member.

An endoscopic instrument according to another embodiment, for inserting a volume filling device into a pouch created of stomach tissue, in the stomach area of a patient, the instrument comprising an elongated member adapted to transport the volume filling device to the pouch.

According to another embodiment, wherein the instrument comprises a tubular member adapted to be inserted into a pouch created in the stomach tissue, wherein the instrument is adapted to deliver a plurality of volume filling devices into the pouch created in the stomach tissue through the tubular member.

One possible advantage with inserting a plurality of volume filling devices into the pouch may be the ability to avoid ileus in the case if the pouch ruptures. Ileus may be both harmful and bring great discomfort to the patient. The plurality of volume filling object may satisfy the property of avoiding ileus, due to each volume filling device's individual size, and as a plurality satisfy the property of filling the volume required by of the treatment. Such treatment may require an endoscope instrument with significant adoptions in order to enable efficient and secure transportation of the volume filling devices into the pouch.

According to yet another embodiment, wherein the instrument comprises a tubular member adapted to deliver a plurality of spherical volume filling devices.

According to yet another embodiment, wherein the tubular member has a cylindrical cross-section.

According to yet another embodiment, wherein the instrument comprises a tubular member adapted to deliver a plurality of polyedrical volume filling devices.

According to another embodiment, wherein the tubular member has a polygonal cross-section.

According to another yet embodiment, wherein the instrument comprises a conduit adapted to be inserted into a pouch created in the stomach tissue, and wherein the instrument is adapted to deliver a solidifying fluid into the pouch created in the stomach tissue through the conduit.

An instrument according to another embodiment, wherein the instrument further comprises a tubular member adapted to insert a plurality of volume filling devices and a solidifying fluid into the pouch.

An instrument according to yet another embodiment, wherein the elongated member comprises a tubular member adapted to be inserted into a pouch created in the stomach tissue, and wherein the instrument is adapted to deliver an expandable volume filling device into the pouch created in the stomach tissue through the tubular member.

An instrument according to yet another embodiment, wherein the instrument further comprises a conduit adapted to inject a solidifying fluid into the expandable volume filling device.

An instrument according to yet another embodiment, wherein the instrument further comprises a tubular member adapted to insert a plurality of volume filling devices into the expandable volume filling device.

An instrument according to yet another embodiment, wherein the instrument comprises a tubular member adapted to deliver a plurality of spherical volume filling devices.

According to another embodiment, wherein the tubular member has a cylindrical cross-section.

According to yet another embodiment, wherein the instrument comprises a tubular member adapted to deliver a plurality of polyhedral volume filling devices.

According to yet another embodiment, wherein the tubular member has a polygonal cross-section.

According to yet another embodiment, wherein the instrument further comprises a tubular member adapted to deliver a plurality of volume filling devices and a solidifying fluid into the expandable volume filling device.

An instrument according to another embodiment, wherein the plurality of volume filling devices are adapted to be connected to each other to form an interconnected volume filling device, after the plurality of volume filling devices have been delivered into the pouch.

An instrument according to yet another embodiment, wherein the instrument further comprises at least one unit adapted to deliver fixating members, and wherein the fixating members are adapted to penetrate at least one layer of stomach tissue and one layer of esophagus or stomach tissue.

An instrument according to yet another embodiment, wherein the instrument comprises at least one unit adapted to deliver fixating members, and wherein the fixating members are adapted to penetrate at least two layers of stomach tissue and one layer of esophagus tissue.

An instrument according to yet another embodiment, wherein the at least one unit is adapted to deliver a fixating member in form of a staple or a suture.

According to another embodiment, wherein the at least one unit is adapted to deliver a fixating member comprising a first and second state, wherein the fixating member is adapted to, in the first state, penetrate tissue, and in the second state, hinder the fixating member from penetrating tissue.

According to yet another embodiment, wherein the instrument comprises a fixation member insertion device and a fixation member altering device, wherein the fixation member insertion device is adapted to insert the fixation member through the tissue, the fixation device being in the first state, and wherein the fixation member altering device is adapted to alter the state of the fixation member such that the fixation member is placed in the second state.

According to yet another embodiment, wherein the fixation member insertion device and/or the insertion member altering device comprise an elongated member adapted to house the fixation member.

According to yet another embodiment, wherein the instrument further comprises at least one anvil member, and wherein the unit is adapted to deliver the fixation member such that the fixation member penetrates the tissue of the esophagus and the tissue of the stomach and engages the anvil member.

According to yet another embodiment, wherein the anvil member is adapted to be positioned within the esophagus.

An instrument according to another embodiment, wherein the anvil member is adapted to be positioned within the stomach.

An instrument according to yet another embodiment, wherein the instrument is a gastroscopic instrument adapted to be inserted into the esophagus and/or stomach through the throat of the patient for operating on the esophagus and/or stomach.

An instrument according to yet another embodiment, wherein the instrument is a laparoscopic instrument adapted to be inserted into the patient from the outside of the patient into the abdominal cavity and further adapted to be inserted into the esophagus and/or stomach through an opening in the stomach and/or esophagus wall of the patient and operate on the esophagus and/or stomach from the inside thereof.

An instrument according to yet another embodiment, wherein the instrument is a laparoscopic instrument adapted to be inserted into the patient through a trocar from the outside of the patient into the abdominal cavity and operate on the esophagus and/or stomach from the outside thereof.

An instrument according to yet another embodiment, wherein the at least one unit is adapted to deliver fixation members penetrating three layers of stomach tissue and one layer of esophagus tissue, for fixating the three layers of stomach tissue and one layer of esophagus tissue to each other.

An instrument according to yet another embodiment, wherein the instrument is adapted to create the pouch of stomach tissue, through the fixation of two layers of stomach tissue to each other.

According to another embodiment, wherein the instrument further comprises a cutting member adapted to create an opening in the pouch from the inside of the stomach.

According to yet another embodiment, wherein the unit is adapted to deliver fixation members penetrating two layers of stomach tissue and one layer of esophagus tissue, for fixating at least one of the two layers of stomach tissue and one layer of esophagus tissue to each other.

According to yet another embodiment, wherein the unit is adapted to deliver at least one fixation member penetrating four layers of stomach tissue and one layer of esophagus tissue, for fixating at least three layers of stomach tissue and one layer of esophagus tissue to each other.

According to yet another embodiment, wherein the instrument is adapted to create the pouch of stomach tissue through the fixation of two of the three layers of stomach tissue.

According to yet another embodiment, wherein the instrument comprises at least one operable joint, for enabling the instrument to deliver the volume filling device to the pouch, when the pouch is at least partly created of fundus stomach tissue.

According to yet another embodiment, wherein the instrument comprises at least two operable joints, for enabling the instrument to deliver the volume filling device to the pouch, when the pouch is at least partly created of fundus stomach tissue.

According to yet another embodiment, wherein the second joint is placed at a distance from the first operable joint.

According to yet another embodiment, comprising a unit delivering the fixating members, wherein the unit is placed at a distance from the first operable joint.

An instrument according to another embodiment, wherein the unit is placed at a distance from the second operable joint.

An instrument according to yet another embodiment, wherein the instrument further comprises a tissue closing unit adapted to close an opening created in the pouch.

An instrument according to yet another embodiment, wherein the instrument further comprises a stomach tissue pulling or pushing device adapted to hold and pull or push a portion of the stomach tissue.

An instrument according to yet another embodiment, wherein the stomach tissue pulling or pushing device is adapted to engage the stomach tissue using a stomach tissue penetrating member or a stomach clamping member.

An instrument according to yet another embodiment, wherein the stomach tissue pulling or pushing device is adapted to engage the stomach tissue using suction.

An instrument according to yet another embodiment, wherein the stomach tissue pulling or pushing device is adapted to engage the stomach tissue using a pressurized fluid.

An instrument according to yet another embodiment, wherein the stomach tissue pulling device is adapted to pull the stomach tissue into an opening of a luminal space inside of the instrument.

An instrument according to yet another embodiment, wherein the area of a first cross-section of the luminal space is larger than an area of a second parallel cross-section placed in the opening of the luminal space, placed more distally in the luminal space or distally in the instrument.

An instrument according to yet another embodiment, wherein the unit is adapted to be placed more distal in the instrument than the luminal space, such that the luminal space creates a pouch when the at least one fixating member is delivered.

An instrument according to yet another embodiment, wherein a retractable portion is placed more proximal than the unit, thus enabling the removal of the instrument after the fixating members have been delivered.

An instrument according to yet another embodiment, wherein the instrument comprises at least one operable joint adapted to enable a first portion of the instrument to be placed inside of the stomach in a position such that the layer of stomach tissue and the. layer of esophagus tissue are placed between the first portion of the instrument and a second portion of the instrument.

An instrument according to yet another embodiment, wherein the unit is adapted to place the at least one fixating member above the gastroesophageal junction.

An instrument according to yet another embodiment, wherein the instrument is adapted to place the at least one fixating member above the gastroesophageal junction for creating a tunnel between the esophagus and stomach above the junction.

According to another embodiment, wherein the instrument further comprises an operable joint for placing a portion of the instrument inside of the stomach above the gastroesophageal junction in relation to the cranial-caudal axis of the patient.

According to yet another embodiment, wherein the instrument is adapted to create a pouch and place at least one first fixating member for creating the pouch, and wherein the instrument is adapted to place at least one second fixating member for creating the pouch, such that the at least one second fixating member intersects the at least one first fixating member.

According to yet another embodiment, wherein the instrument is adapted to place the at least one second fixating member perpendicularly to the at least one first fixating member.

According to yet another embodiment, wherein the instrument further comprises a second unit adapted to deliver a second fixating member, wherein the first and second units are adapted to be arranged such that the first unit can deliver the first fixating member at a first point along a first esophagus surface length axis substantially parallel to the esophagus center axis, and wherein the second unit is adapted to be arranged such that the second unit can deliver the second fixating member at a second point along a second esophagus surface length axis substantially parallel to the first esophagus surface length axis and the esophagus center axis center, wherein the first esophagus surface length axis is positioned at a distance from the second esophagus surface length axis, radially in relation to the esophagus center axis.

According to yet another embodiment, wherein the first and second units are adapted to deliver the first and second fixating member substantially simultaneously or in a sequence.

According to yet another embodiment, wherein the first and/or second units are adapted to deliver a fixating member in form of a staple or suture.

An endoscopic instrument according to another embodiment, wherein the instrument comprises at least one unit adapted to deliver fixating members for penetrating at least two layers of stomach tissue, wherein the instrument is adapted to place the fixating members for creating a substantially closed space surrounded by stomach wall, wherein the instrument has a shape to allow it to place the fixating members in the stomach wall creating a pouch of stomach wall including the closed space.

Creating a closed space surrounded by stomach wall may be less invasive compared to other methods of creating a hollow pouch from stomach tissue.

According to another embodiment, wherein the instrument is adapted to fixate a pouch formed as a tobacco pouch presenting a neck part of the pouch being wrinkled back and forth to create the closed space, when the instrument being used on stomach tissue.

Further, a pouch having the form of a tobacco pouch may enable sequenced method and procedure for creating the pouch substantially having the intended size. Further, the sequenced method may be more efficient to the endoscopist and lessen the risk of human error in the treatment.

According to yet another embodiment, wherein the instrument is adapted to fixate a pouch formed as a flat folded pouch with the fixating members placed in a curve to create the closed space, when the instrument being used on stomach tissue.

According to yet another embodiment, wherein the instrument is adapted to predefine the pouch using vacuum suction for sucking the pouch into a predefined shape, when placing the fixating members and the instrument being used on stomach tissue.

According to yet another embodiment, wherein the instrument is adapted to predefine the pouch by clamping and pulling or pushing the stomach wall into a pre fixating pouch shape, when placing the fixating members and the instrument being used on stomach tissue.

According to yet another embodiment, wherein the instrument is adapted to deliver a fixating member in form of a staple or suture.

According to yet another embodiment, comprising at least one unit is adapted to deliver a fixating member comprising a first and second state, wherein the fixating member is adapted to, in the first state, penetrate tissue, and in the second state, hinder the fixating member from penetrating tissue.

An instrument according to another embodiment, wherein the instrument comprises a fixation member insertion device and a fixation member altering device, wherein the fixation member insertion device is adapted to insert the fixation member through the tissue, the fixation device being in the first state, and wherein the fixation member altering device is adapted to alter the state of the fixation member such that the fixation member is placed in the second state.

An instrument according to yet another embodiment, wherein the fixation member insertion device and/or the insertion member altering device comprises an elongated member adapted to house the fixation member.

An instrument according to yet another embodiment, wherein the instrument further comprises at least one anvil member, and wherein the unit is adapted to deliver the fixation member such that the fixation member penetrates the tissue of the stomach and engages the anvil member.

An instrument according to yet another embodiment, wherein the anvil member is adapted to be positioned within or outside the stomach.

An instrument according to yet another embodiment, wherein the instrument is a gastroscopic instrument adapted to be inserted into the stomach via the throat and esophagus of the patient and operate on the stomach passing the inside thereof.

An instrument according to yet another embodiment, wherein the instrument is a laparoscopic instrument adapted to be inserted into the patient from the outside of the patient into the abdominal cavity and further adapted to be inserted into stomach through an incision opening in the stomach wall of the patient and operate on the stomach from the inside thereof.

An instrument according to yet another embodiment, wherein the instrument is a laparoscopic instrument adapted to be inserted into the patient through a trocar from the outside of the patient into the abdominal cavity and operate on the stomach from the outside thereof.

According to another embodiment, wherein the instrument is adapted to deliver fixation members penetrating three layers of stomach tissue and one layer of esophagus tissue, for fixating the three layers of stomach tissue and one layer of esophagus tissue to each other.

According to yet another embodiment, wherein the instrument is adapted to create a pouch of stomach tissue, through the fixation of two layers of stomach tissue.

According to yet another embodiment, wherein the instrument is adapted to create the pouch around a volume filling device placed in contact with the stomach tissue.

According to yet another embodiment, wherein the instrument further comprises a cutting member adapted to create an opening in the pouch from the inside of the stomach.

According to yet another embodiment, wherein the unit is adapted to deliver fixation members penetrating two layers of stomach tissue and one layer of esophagus tissue, for fixating at least one of the two layers of stomach tissue and one layer of esophagus tissue to each other.

According to yet another embodiment, wherein the unit is adapted to deliver at least one fixation member penetrating four layers of stomach tissue and one layer of esophagus tissue, for fixating at least three layers of stomach tissue and one layer of esophagus tissue to each other.

An instrument according to another embodiment, wherein the instrument is adapted to create a pouch of stomach tissue, through the fixation of two of the three layers of stomach tissue.

An instrument according to yet another embodiment, wherein the instrument is adapted to create the pouch around a volume filling device placed in contact with the stomach tissue.

An instrument according to yet another embodiment, wherein the instrument further comprises a cutting member adapted to create an opening in the pouch from the outside of the stomach.

An instrument according to yet another embodiment, wherein the instrument is further adapted to insert a volume filling device in the pouch, after the creation of the pouch.

An instrument according to yet another embodiment, wherein the instrument is adapted to insert a volume filling device comprising a solidifying liquid.

According to another embodiment, wherein the instrument comprises a conduit adapted to be inserted into the pouch created in the stomach tissue, wherein the instrument is adapted to deliver the solidifying fluid into the pouch through the conduit.

According to yet another embodiment, wherein the instrument comprises at least one operable joint, for enabling the instrument to deliver the solidifying liquid to the pouch.

The According to yet another embodiment, wherein the instrument is adapted to insert a volume filling device comprising a plurality of volume filling devices.

According to yet another embodiment, wherein the plurality of volume filling devices are adapted to be connected to each other to form an interconnected volume filling device, after the plurality of volume filling devices have been inserted into the pouch.

According to yet another embodiment, wherein the instrument further comprises a tubular member adapted to be inserted into the pouch created of the stomach tissue, wherein the instrument is adapted to deliver the plurality of volume filling devices into the pouch created of the stomach tissue through the tubular member.

An instrument according to another embodiment, wherein the instrument comprises at least one operable joint, for enabling the instrument to deliver the plurality of volume filling devices to the pouch, when the pouch is created of fundus stomach tissue or stomach tissue in general.

An instrument according to yet another embodiment, wherein the instrument is adapted to insert a volume filling device comprising an expandable volume filling device.

An instrument according to yet another embodiment, wherein the instrument is adapted to fill the expandable volume filling device with a solidifying liquid.

An instrument according to yet another embodiment, wherein the instrument is adapted to fill the expandable volume filling device with a plurality of volume filling devices.

According to another embodiment, wherein the instrument is adapted to insert a volume filling device comprising a combination of: a plurality of volume filling devices, and a solidifying liquid.

According to yet another embodiment, wherein the instrument further comprises an elongated tubular member adapted to enable the volume filling device to pass through the tubular member and into the pouch.

According to yet another embodiment, wherein the tubular member is adapted to be inserted into the pouch created by the stomach tissue, for enabling the insertion of the volume filling device through the tubular member.

An instrument according to another embodiment, wherein the instrument further comprises a tissue closing unit adapted to close the opening created in the pouch.

An instrument according to yet another embodiment, wherein the instrument further comprises a stomach tissue pulling or pushing device adapted to hold and pull or push a portion of the stomach tissue.

An instrument according to yet another embodiment, wherein the stomach tissue pulling or pushing device is adapted to engage the stomach tissue using a stomach tissue penetrating member or a stomach clamping member.

According to another embodiment, wherein the stomach tissue pulling or pushing device is adapted to engage the stomach tissue using suction.

According to yet another embodiment, wherein the stomach tissue pulling device is adapted to pull the stomach tissue into an opening of a luminal space inside of the instrument.

According to yet another embodiment, wherein the area of a first cross-section of the luminal space is larger than an area of a second parallel cross-section placed in the opening of the luminal space, placed more distally in the luminal space or distally in the instrument.

According to yet another embodiment, wherein the luminal space is placed more distal in the instrument than a unit delivering the fixating member, such that the luminal space creates a pouch when the at least one fixating member is delivered.

According to yet another embodiment, comprising two portions of the instrument, wherein the instrument comprises at least one operable joint adapted to enable a first portion of the instrument to be placed inside of the stomach and a second portion to be placed outside the stomach in a position such that the two layers of stomach tissue are placed between the first portion of the instrument and the second portion of the instrument.

An instrument according to another embodiment, wherein the first and second portions are adapted to place the fixating members.

An instrument according to yet another embodiment, wherein the instrument is adapted to place the fixating members above the gastroesophageal junction for creating a tunnel between the esophagus and stomach above the junction.

An instrument according to yet another embodiment, wherein the instrument further comprises an operable joint for placing the portion of the instrument inside of the stomach.

An instrument according to yet another embodiment, wherein the instrument is adapted to place the at least one second fixating member perpendicularly to the at least one first fixating member.

An instrument according to yet another embodiment, wherein the instrument is adapted to create a pouch and place at least one first fixating member for creating the pouch, and wherein the instrument is adapted to place at least one second fixating member for creating the pouch, such that the at least one second fixating member intersects the at least one first fixating member.

An instrument according to yet another embodiment, wherein the instrument is adapted to place the at least one second fixating member perpendicularly to the at least one first fixating member.

According to another embodiment, wherein a retractable portion is . placed more proximal than the unit, thus enabling the removal of the instrument after the fixating members have been delivered.

According to yet another embodiment, wherein the instrument comprises at least one operable joint, for enabling the instrument to create the pouch.

According to yet another embodiment, wherein the instrument comprises at least one further second operable joint, for enabling the instrument to creating the pouch.

According to yet another embodiment, wherein the second joint is placed at a distance from the first operable joint.

According to yet another embodiment, comprising a unit delivering the fixating members, wherein the unit is placed at a distance from the first operable joint.

According to yet another embodiment, wherein the unit is placed at a distance from the second operable joint.

An instrument, according to one embodiment, for attaching at least two layers of human tissue, wherein the instrument comprises at least one unit adapted to deliver fixating members in a row for penetrating at least part of the at least two layers of tissue and adapted to having different fixating members penetrating into the tissue in the row with at least two different depths. This method configuration may be less invasive and may further decrease the risk of post-treatment problems to the patient. Human tissue is of varying thickness. Thus, fixating elements of varying depths may be more suited for its purpose in some cases.

According to another embodiment, wherein the at least one instrument is adapted to deliver fixating members for attaching at least two layers of human tissue during a first part of the row penetrating with a first depth into the tissue and attaching three or more layers of tissue during a second part of the row with the fixating members penetrating the second part or more parts of the row with a second and larger depth into the tissue to be able to attach the three or more layers of tissue.

According to yet another embodiment, wherein the at least one instrument is adapted to attach four or five layers of tissue during a second part or more parts of the row.

According to yet another embodiment, wherein the at least one instrument is adapted to attach at least two layers of stomach tissue during a first part of the row penetrating the fixating members with a first depth into the tissue and further adapted to attach one layer of esophageal tissue to the at least two layers of stomach tissue during a second part or more parts of the row with the fixating members penetrating the second part or more parts of the row with a second and larger depth into the tissue to be able to attach the one layer esophageal tissue to the at least two layers of stomach tissue.

An instrument according to another embodiment, wherein the at least one instrument is adapted to attach the one layer esophageal tissue to three layers of stomach tissue.

An instrument according to yet another embodiment, wherein the at least one instrument is adapted to attach the one layer esophageal tissue to four layers of stomach tissue.

An instrument according to yet another embodiment, wherein the at least one instrument is adapted to attach the one layer esophageal tissue to at least two layers of stomach tissue above the gastroesophageal junction and above the cardia sphincter.

An instrument according to yet another embodiment, wherein the at least one instrument comprising a unit adapted to deliver fixating members for attaching four layers of stomach tissue to each other, wherein the instrument is adapted to attach two layers of the stomach serosa and two plus two layers of stomach mucosa to each other.

An instrument, according to another embodiment, for attaching stomach tissue, comprising a unit adapted to deliver fixating members for attaching a first two layers of stomach tissue to each other facing serosa against serosa, wherein the instrument is adapted to clamp totally four layers of stomach tissue to each other including the first two layers, wherein the instrument is adapted to, when attaching the two layers serosa to serosa, clamping the other two layers of stomach tissue one layer on each side of the first two layers thereby facing stomach mucosa to mucosa on each side of the first two layers, wherein the fixating members only attach the first inner stomach layers serosa to serosa. One possible effect of attaching tissue such two layers is facing each other serosa against serosa could be the increased durability of the tissue attachment. Thus, this instrument may be less invasive.

According to yet another embodiment, wherein the instrument comprises at least one unit adapted to deliver fixating members, and wherein the fixating members are adapted to penetrate three layers of stomach tissue and one layer of esophagus tissue, wherein the instrument is adapted to place the fixating members above the gastro-esophageal junction for attaching the esophagus and stomach above the junction, wherein the at least one unit comprising an esophagus part adapted to be placed in the esophagus and a stomach part adapted to be placed in the stomach, wherein the stomach and esophagus part is adapted to place the fixating member substantially between themselves with a row of fixating members, wherein the instrument is adapted to have a part of the instrument including the esophagus part of the unit inserted into the esophagus via the stomach cavity and a stomach part of the instrument including the stomach part of the unit inserted into the stomach cavity from the throat, wherein the instrument has a shape and comprising a joint to allow it to be directed to reach a position both in the esophagus and the stomach such that at least one fixating member will attach the outer serosa of the stomach wall to the outer esophagus wall above the gastro-esophageal junction, wherein the instrument is adapted to place, at the two ends of the row of fixating members, fixating members with a shorter depth going into the stomach tissue consisting of two layers of stomach tissue therein. An instrument adopted to perform such procedure may lessen the invasiveness and the risk of human error related to the procedure. An instrument adopted to perform such procedure may further increase the speed and precision of the endoscopist.

An instrument according to another embodiment, wherein the esophageal part of the unit is located at a distance from the joint being larger than a distance between the corresponding inner folded most caudal part of the stomach in the region of the angle of His and the cranial edge of the cardia sphincter, and wherein the stomach part of the instrument has a distance from the stomach part of the unit to the end of the instrument being smaller than the distance between the cranial edge of the cardia sphincter and the inner cranial roof of the stomach fundus cavity, at the position of the instrument when placed in the stomach. This embodiment of the instrument may enable a treatment where on possible effect is to decrease the risks of patient discomfort and related problems in emptying the stomach of content through vomiting or eructation. Thus this method may restore the function of the cardia closing sphincter muscle to a greater extent.

An instrument according to yet another embodiment, wherein the instrument is an instrument for treating reflux disease, wherein the instrument comprises: a first unit adapted to deliver a first fixating member for fixating the esophagus tissue to the stomach tissue, and a second unit adapted to deliver a second fixating member, wherein the first and second units are adapted to be arranged such that the first unit can deliver the first fixating member at a first point along a first esophagus surface length axis substantially parallel to the esophagus center axis, and wherein the second unit is adapted to be arranged such that the second unit can deliver the second fixating member at a second point along a second esophagus surface length axis substantially parallel to the first esophagus surface length axis and the esophagus center axis center, wherein the first esophagus surface length axis is positioned at a distance from the second esophagus surface length axis, radially in relation to the esophagus center axis.

An instrument according to yet another embodiment, wherein the instrument is a laparoscopic instrument adapted to be inserted into the patient from the outside of the patient into the abdominal cavity and further adapted to be inserted into stomach through an incision opening in the stomach wall of the patient and operate on the stomach from the inside thereof.

An instrument according to yet another embodiment, wherein the instrument is a laparoscopic instrument adapted to be inserted into the patient from the outside of the patient into the abdominal cavity and operate on the stomach from the outside thereof.

An instrument according to yet another embodiment, wherein the laparoscopic instrument is adapted to be inserted into the patient through a trocar from the outside of the patient into the abdominal cavity.

An instrument according to yet another embodiment, wherein the instrument is a gastroscopic instrument adapted to be inserted into the patient from the throat of the patient into the stomach cavity and operate on the stomach from the inside thereof.

An instrument according to yet another embodiment, wherein the instrument is a gastroscopic instrument adapted to be inserted into the patient from the throat of the patient into the stomach cavity and further penetrating the stomach wall entering into the abdominal cavity and operating on the stomach from the outside thereof.

An instrument according to yet another embodiment, wherein the unit is adapted to deliver at least one fixating members for fixating the stomach tissue without penetrating the mucosa layer of the stomach wall.

An instrument according to yet another embodiment, wherein the instrument is an instrument for treating obesity adapted to substantially reduce the volume of the stomach cavity.

An instrument according to yet another embodiment, wherein the unit is adapted to clamp four layers of stomach for creating a pouch of stomach wall from the outside of the stomach with a closed space within the pouch, and adapted to deliver at least one fixating members for fixating the stomach tissue penetrating only two muscular layers of the stomach wall.

An instrument according to yet another embodiment, wherein the instrument comprises at least one unit adapted to deliver fixating members for penetrating at least two layers of stomach tissue, wherein the instrument is adapted to place the fixating members for creating a substantially closed space surrounded by stomach wall, wherein the instrument has a shape to allow it to place the fixating members in the stomach wall creating a pouch of stomach wall including the closed space.

An instrument according to yet another embodiment, wherein the instrument is adapted to fixate a pouch formed as a tobacco pouch presenting a neck part of the pouch being wrinkled back and forth to create the closed space, when the instrument is being used on stomach tissue.

According to another embodiment, wherein the instrument is adapted to fixate a pouch formed as a flat folded pouch with the fixating members placed in a curve to create the closed space, when the instrument being used on stomach tissue.

According to yet another embodiment, wherein the instrument is adapted to predefine the pouch using vacuum suction for sucking the pouch into a predefined shape, when placing the fixating members and the instrument being used on stomach tissue.

According to yet another embodiment, wherein the instrument is adapted to predefine the pouch by clamping and pulling or pushing the stomach wall into a pre fixating pouch shape, when placing the fixating members and the instrument being used on stomach tissue.

According to yet another embodiment, wherein the instrument is adapted to deliver a fixating member in form of a staple or suture.

According to yet another embodiment, comprising at least one unit is adapted to deliver a fixating member comprising a first and second state, wherein the fixating member is adapted to, in the first state, penetrate tissue, and in the second state, hinder the fixating member from penetrating tissue.

According to yet another embodiment, wherein the instrument comprises a fixation member insertion device and a fixation member altering device, wherein the fixation member insertion device is adapted to insert the fixation member through the tissue, the fixation device being in the first state, and wherein the fixation member altering device is adapted to alter the state of the fixation member such that the fixation member is placed in the second state.

According to yet another embodiment, wherein the fixation member insertion device and/or the insertion member altering device comprise an elongated member adapted to house the fixation member.

An instrument according to another embodiment, wherein the instrument further comprises at least one anvil member, and wherein the unit is adapted to deliver the fixation member such that the fixation member penetrates the tissue of the stomach and engages the anvil member.

An instrument according to yet another embodiment, wherein the anvil member is adapted to be positioned within or outside the stomach.

An instrument according to yet another embodiment, wherein the instrument is a gastroscopic instrument adapted to be inserted into the stomach via the throat and esophagus of the patient and operate on the stomach passing the inside thereof.

An instrument according to yet another embodiment, wherein the instrument is a laparoscopic instrument adapted to be inserted into the patient from the outside of the patient into the abdominal cavity and further adapted to be inserted into stomach through an incision opening in the stomach wall of the patient and operate on the stomach from the inside thereof.

An instrument according to yet another embodiment, wherein the instrument is a laparoscopic instrument adapted to be inserted into the patient through a trocar from the outside of the patient into the abdominal cavity and operate on the stomach from the outside thereof.

An instrument according to yet another embodiment, wherein the instrument is adapted to deliver fixation members penetrating three layers of stomach tissue and one layer of esophagus tissue, for fixating the three layers of stomach tissue and one layer of esophagus tissue to each other.

An instrument according to yet another embodiment, wherein the instrument is adapted to create a pouch of stomach tissue, through the fixation of two layers of stomach tissue.

According to another embodiment, wherein the instrument is adapted to create the pouch around a volume filling device placed in contact with the stomach tissue.

According to yet another embodiment, wherein the instrument further comprises a cutting member adapted to create an opening in the pouch from the inside of the stomach.

According to yet another embodiment, wherein the unit is adapted to deliver fixation members penetrating two layers of stomach tissue and one layer of esophagus tissue, for fixating at least one of the two layers of stomach tissue and one layer of esophagus tissue to each other.

According to yet another embodiment, wherein the unit is adapted to deliver at least one fixation member penetrating four layers of stomach tissue and one layer of esophagus tissue, for fixating at least three layers of stomach tissue and one layer of esophagus tissue to each other.

According to yet another embodiment, wherein the instrument is adapted to create a pouch of stomach tissue, through the fixation of two of the three layers of stomach tissue.

According to yet another embodiment, wherein the instrument is adapted to create the pouch around a volume filling device placed in contact with the stomach tissue.

According to yet another embodiment, wherein the instrument further comprises a cutting member adapted to create an opening in the pouch from the outside of the stomach.

According to yet another embodiment, wherein the instrument is further adapted to insert a volume filling device in the pouch, after the creation of the pouch.

According to yet another embodiment, wherein the instrument is adapted to insert a volume filling device comprising a solidifying liquid.

The instrument according to claim 45, wherein the instrument comprises a conduit adapted to be inserted into the pouch created in the stomach tissue, wherein the instrument is adapted to deliver the solidifying fluid into the pouch through the conduit.

An instrument according to another embodiment, wherein the instrument comprises at least one operable joint, for enabling the instrument to deliver the solidifying liquid to the pouch.

An instrument according to yet another embodiment, wherein the instrument is adapted to insert a volume filling device comprising a plurality of volume filling devices.

An instrument according to yet another embodiment, wherein the plurality of volume filling devices are adapted to be connected to each other to form an interconnected volume filling device, after the plurality of volume filling devices have been inserted into the pouch.

An instrument according to yet another embodiment, wherein the instrument further comprises a tubular member adapted to be inserted into the pouch created of the stomach tissue, wherein the instrument is adapted to deliver the plurality of volume filling devices into the pouch created of the stomach tissue through the tubular member.

An instrument according to yet another embodiment, wherein the instrument comprises at least one operable joint, for enabling the instrument to deliver the plurality of volume filling devices to the pouch, when the pouch is created of fundus stomach tissue or stomach tissue in general.

An instrument according to yet another embodiment, wherein the instrument is adapted to insert a volume filling device comprising an expandable volume filling device.

According to another embodiment, wherein the instrument is adapted to fill the expandable volume filling device with a solidifying liquid.

According to yet another embodiment, wherein the instrument is adapted to fill the expandable volume filling device with a plurality of volume filling devices.

According to yet another embodiment, wherein the instrument is adapted to insert a volume filling device comprising a combination of: a plurality of volume filling devices, and a solidifying liquid.

According to yet another embodiment, wherein the instrument further comprises an elongated tubular member adapted to enable the volume filling device to pass through the tubular member and into the pouch.

According to yet another embodiment, wherein the tubular member is adapted to be inserted into the pouch created by the stomach tissue, for enabling the insertion of the volume filling device through the tubular member.

The According to yet another embodiment, wherein the instrument further comprises a tissue closing unit adapted to close the opening created in the pouch.

According to yet another embodiment, wherein the instrument further comprises a stomach tissue pulling or pushing device adapted to hold and pull or push a portion of the stomach tissue.

According to yet another embodiment, wherein the stomach tissue pulling or pushing device is adapted to engage the stomach tissue using a stomach tissue penetrating member or α stomach clamping member.

According to yet another embodiment, wherein the stomach tissue pulling or pushing device is adapted to engage the stomach tissue using suction.

An instrument according to another embodiment, wherein the stomach tissue pulling device is adapted to pull the stomach tissue into an opening of a luminal space inside of the instrument.

An instrument according to yet another embodiment, wherein the area of a first cross-section of the luminal space is larger than an area of a second parallel cross-section placed in the opening of the luminal space, placed more distally in the luminal space or distally in the instrument.

An instrument according to yet another embodiment, wherein the luminal space is placed more distal in the instrument than a unit delivering the fixating member, such that the luminal space creates a pouch when the at least one fixating member is delivered.

An instrument according to yet another embodiment, comprising two portions of the instrument, wherein the instrument comprises at least one operable joint adapted to enable a first portion of the instrument to be placed inside of the stomach and a second portion to be placed outside the stomach in a position such that the two layers of stomach tissue are placed between the first portion of the instrument and the second portion of the instrument.

An instrument according to yet another embodiment, wherein the first and second portions are adapted to place the fixating members.

An instrument according to yet another embodiment, wherein the instrument is adapted to place the fixating members above the gastroesophageal junction for creating a tunnel between the esophagus and stomach above the junction.

An instrument according to yet another embodiment, wherein the instrument further comprises an operable joint for placing the portion of the instrument inside of the stomach.

An instrument according to yet another embodiment, wherein the instrument is adapted to place the at least one second fixating member perpendicularly to the at least one first fixating member.

An instrument according to yet another embodiment, wherein the instrument is adapted to create a pouch and place at least one first fixating member for creating the pouch, and wherein the instrument is adapted to place at least one second fixating member for creating the pouch, such that the at least one second fixating member intersects the at least one first fixating member.

An instrument according to yet another embodiment, wherein the instrument is adapted to place the at least one second fixating member perpendicularly to the at least one first fixating member.

An instrument according to yet another embodiment, wherein a retractable portion is placed more proximal than the unit, thus enabling the removal of the instrument after the fixating members have been delivered.

An instrument according to yet another embodiment, wherein the instrument comprises at least one operable joint, for enabling the instrument to create the pouch.

An instrument according to yet another embodiment, wherein the instrument comprises at least one further second operable joint, for enabling the instrument to creating the pouch.

An instrument according to yet another embodiment, wherein the second joint is placed at a distance from the first operable joint.

An instrument according to yet another embodiment, comprising a unit delivering the fixating members, wherein the unit is placed at a distance from the first operable joint.

An instrument according to yet another embodiment, wherein the unit is placed at a distance from the second operable joint.

An instrument according to yet another embodiment, wherein the instrument further comprises at least one anvil member or a unit for delivering fixation members placed on; a separate gastro-esophageal tube or esophageal tube or a gastroscope adapted to be placed in the esophagus with the unit above the cardia, wherein the instrument is adapted to be introduced into the abdominal cavity for clamping on both sides of the esophagus at the position of the unit and wherein the unit is adapted to be involved in the fixation of stomach tissue to the right wall of the esophageal tissue, when the instrument deliver the fixation member such that the fixation member penetrates the tissue of the stomach and the tissue of the right wall of the esophagus and engages with the unit for attaching stomach and esophageal tissue above the cardia sphincter.

According to another embodiment, wherein the instrument is an instrument for treating reflux disease, wherein the instrument comprises:
a first elongated member comprising a first part of a unit mounted on the elongated member for engaging in placement of fixating members, wherein the elongated member and first part of the unit is adapted to be placed in the esophagus for engaging in the fixating member fixating the esophagus tissue to the stomach tissue, and a second elongated member spaced apart from the first elongated member comprising a second part of the unit for engaging in placement of fixating members, wherein the second elongated member is adapted to be placed in the abdominal cavity outside of the stomach and further adapted to clamp the esophagus from the outside thereof including stomach tissue from the outside thereof, at the position of the first part of the unit placed above the cardia sphincter and below the diaphragm muscle, wherein the unit, having the second and the first part in a predefined position in relation to each other, is adapted to engage with and deliver the fixating members.

According to yet another embodiment, wherein the instrument comprises: a first elongated member comprising a first part of a unit mounted on the first elongated member and a second elongated member comprising a second part of a unit mounted on the second elongated member, for engaging in placement of one or more fixating members, wherein the elongated members is adapted to be placed in the abdominal cavity outside of the stomach and further adapted to clamp the esophagus from the outside thereof including clamping the stomach fundus tissue from the outside thereof, at the position of the esophagus above the cardia sphincter and below the diaphragm muscle, wherein the unit, when having the second and the first part in a predefined position in relation to each other, is adapted to engage with and deliver the fixating members for attaching and fixating the folded stomach fundus wall including two layers of stomach fundus wall to one layer of esophagus wall, wherein the instrument is adapted to deliver the one or more fixating member with a depth attaching only one layer of the two clamped layers of esophageal tissue.

An instrument, according to another embodiment, for attaching the esophagus to the stomach, wherein the instrument further comprises at least one anvil member or a unit for delivering fixation members placed on a separate gastro-esophageal tube or esophageal tube or a gastroscope adapted to be placed in the esophagus with the unit above the cardia, wherein the instrument is adapted to be introduced into the abdominal cavity for clamping on both sides of the esophagus at the position of the unit and wherein the unit is adapted to be involved in the fixation of stomach tissue to the right wall of the esophageal tissue, when the instrument deliver the fixation member such that the fixation member penetrates the tissue of the stomach and the tissue of the right wall of the esophagus and engages with the unit for attaching stomach and esophageal tissue above the cardia sphincter. Such instrument may enable the endoscopist to perform a treatment comprising a plurality of steps in a more efficient manner. Such instrument may further enable the endoscopist to decrease risk of human error and may further enable the endoscopist, to deliver the fixation members more accurately at the intended positions-

An instrument, according to another embodiment, for attaching esophagus to stomach tissue, wherein the instrument is an instrument for treating reflux disease, wherein the instrument comprises:
a first elongated member comprising a first part of a unit mounted on the elongated member for engaging in placement of fixating members, wherein the elongated member and first part of the unit is adapted to be placed in the esophagus for engaging in the fixating member fixating the esophagus tissue to the stomach tissue, and
a second elongated member spaced apart from the first elongated member comprising a second part of the unit for engaging in placement of fixating members, wherein the second elongated member is adapted to be placed in the abdominal cavity outside of the stomach and further adapted to clamp the esophagus from the outside thereof including stomach tissue from the outside thereof, at the position of the first part of the unit placed above the cardia sphincter and below the diaphragm muscle, wherein the unit, having the second and the first part in a predefined position in relation to each other, is adapted to engage with and deliver the fixating members.

An instrument, according to yet another embodiment, for treating reflux disease, wherein the instrument comprises: α first elongated member comprising a first part of a unit mounted on the first elongated member and a second elongated member comprising a second part of a unit mounted on the second elongated member, for engaging in placement of one or more fixating members, wherein the elongated members is adapted to be placed in the abdominal cavity outside of the stomach and further adapted to clamp the esophagus from the outside thereof including clamping the stomach fundus tissue from the outside thereof, at the position of the esophagus above the cardia sphincter and below the diaphragm muscle, wherein the unit, when having the second and the first part in a predefined position in relation to each other, is adapted to engage with and deliver the fixating members for attaching and fixating the folded stomach fundus wall including two layers of stomach fundus wall to one layer of esophagus wall, wherein the instrument is adapted to deliver the one or more fixating member with a depth attaching only one layer of the two clamped layers of esophageal tissue.

An instrument, according to another embodiment, for creating a pouch in the stomach from the outside thereof, comprising at least one pulling member for pulling the instrument into the stomach wall from the outside thereof pre shaping a pouch or impression in the stomach wall, wherein the instrument further comprising a clamping member to clamp the stomach wall to complete the pouch or pre shaping a closure of the impression, wherein the instrument further comprising one or more fixating members adapted to fixate a closure of the pouch or stomach impression to fulfill the pouch creation when the instrument is used in the abdominal cavity.

An instrument according to yet another embodiment, wherein when the pulling member comprises a vacuum suction to hold the pouch or impression in place.

According to another embodiment, wherein the laparoscopic instrument is adapted to be inserted into the patient through a trocar from the outside of the patient into the abdominal cavity.

According to yet another embodiment, wherein the unit is adapted to deliver at least one fixating members for fixating the stomach tissue without penetrating the mucosa layer of the stomach wall.

According to yet another embodiment, wherein the instrument is an instrument for treating obesity adapted to substantially reduce the volume of the stomach cavity.

According to yet another embodiment, wherein the unit is adapted to clamp four layers of stomach for creating a pouch of stomach wall from the outside of the stomach with a closed space within the pouch, and adapted to deliver at least one fixating members for fixating the stomach tissue penetrating only two muscular layers of the stomach wall.

According to yet another embodiment, wherein the instrument comprises at least one unit adapted to deliver fixating members for penetrating at least two layers of stomach tissue, wherein the instrument is adapted to place the fixating members for creating a substantially closed space surrounded by stomach wall, wherein the instrument has a shape to allow it to place the fixating members in the stomach wall creating a pouch of stomach wall including the closed space.

According to yet another embodiment, wherein the instrument is adapted to fixate a pouch formed as a tobacco pouch presenting a neck part of the pouch being wrinkled back and forth to create the closed space, when the instrument is being used on stomach tissue.

According to yet another embodiment, wherein the instrument is adapted to fixate a pouch formed as a flat folded pouch with the fixating members placed in a curve to create the closed space, when the instrument being used on stomach tissue.

The instrument according to another embodiment, wherein the instrument is adapted to predefine the pouch using vacuum suction for sucking the pouch into a predefined shape, when placing the fixating members and the instrument being used on stomach tissue.

The instrument according to yet another embodiment, wherein the instrument is adapted to predefine the pouch by clamping and pulling or pushing the stomach wall into a pre fixating pouch shape, when placing the fixating members and the instrument being used on stomach tissue.

The instrument according to yet another embodiment, wherein the instrument is adapted to deliver a fixating member in form of a staple or suture.

The instrument according to yet another embodiment, wherein the instrument is adapted to create a pouch of stomach tissue, through the fixation of two layers of stomach tissue.

The instrument according to yet another embodiment, wherein the instrument is adapted to create the pouch around a volume filling device placed in contact with the stomach tissue.

The instrument according to yet another embodiment, wherein the unit is adapted to deliver fixation members penetrating two layers of stomach tissue and one layer of esophagus tissue, for fixating at least one of the two layers of stomach tissue and one layer of esophagus tissue to each other.

The instrument according to yet another embodiment, wherein the unit is adapted to deliver at least one fixation member penetrating four layers of stomach tissue and one layer of esophagus tissue, for fixating at least three layers of stomach tissue and one layer of esophagus tissue to each other.

The instrument according to yet another embodiment, wherein the instrument is further adapted to insert a volume filling device in the pouch, after the creation of the pouch.

The instrument according to yet another embodiment, wherein the instrument is adapted to insert a volume filling device comprising a solidifying liquid.

The instrument according to yet another embodiment, wherein the instrument comprises a conduit adapted to be inserted into the pouch created in the stomach tissue, wherein the instrument is adapted to deliver the solidifying fluid into the pouch through the conduit.

The instrument according to yet another embodiment, wherein the instrument comprises at least one operable joint, for enabling the instrument to deliver the solidifying liquid to the pouch.

The instrument according to yet another embodiment, wherein the instrument is adapted to insert a volume filling device comprising a plurality of volume filling devices.

According to another embodiment, wherein the plurality of volume filling devices are adapted to be connected to each other to form an interconnected volume filling device, after the plurality of volume filling devices have been inserted into the pouch.

According to yet another embodiment, wherein the instrument further comprises a tubular member adapted to be inserted into the pouch created of the stomach tissue, wherein the instrument is adapted to deliver the plurality of volume filling devices into the pouch created of the stomach tissue through the tubular member.

According to yet another embodiment, wherein the instrument comprises at least one operable joint, for enabling the instrument to deliver the volume filling devices to the pouch, when the pouch is created of fundus stomach tissue or stomach tissue in general.

According to yet another embodiment, wherein the instrument is adapted to insert a volume filling device comprising an expandable volume filling device.

According to yet another embodiment, wherein the instrument is adapted to fill the expandable volume filling device with a solidifying liquid.

According to yet another embodiment, wherein the instrument is adapted to fill the expandable volume filling device with a plurality of volume filling devices.

According to yet another embodiment, wherein the instrument is adapted to insert a volume filling device comprising a combination of: a plurality of volume filling devices, and a solidifying liquid.

According to yet another embodiment, wherein the instrument further comprises an elongated tubular member adapted to enable the volume filling device to pass through the tubular member and into the pouch.

According to yet another embodiment, wherein the tubular member is adapted to be inserted into the pouch created by the stomach tissue, for enabling the insertion of the volume filling device through the tubular member.

According to yet another embodiment, wherein the instrument further comprises a tissue closing unit adapted to close the opening created in the pouch.

According to yet another embodiment, wherein the instrument further comprises a stomach tissue pulling or pushing device adapted to hold and pull or push a portion of the stomach tissue.

The instrument according to yet another embodiment, wherein the stomach tissue pulling or pushing device is adapted to engage the stomach tissue using a stomach tissue penetrating member or a stomach clamping member.

The instrument according to yet another embodiment, wherein the stomach tissue pulling or pushing device is adapted to engage the stomach tissue using suction.

### METHOD

A method of treating reflux disease in a patient using an endoscopic instrument adapted to engage the stomach tissue and/or the esophagus tissue of a patient from the inside and/or outside thereof, the esophagus being a substantially tube shaped tissue leading to the stomach comprising stomach tissue, the esophagus having an esophagus center axis, being substantially aligned with a cranial-caudal axis of the patient and having a substantially circular circumference substantially aligned with a horizontal plane of the patient, the esophagus further having an inner and outer substantially cylindrical surface extending radially in relation to the esophagus center axis, wherein the method comprises the steps of: positioning the instrument such that the instrument engages the stomach and/or esophagus tissue and delivering at least one fixating member using the instrument, such that the, at least one, fixating member engages at least the stomach tissue. This method may help the endoscopist to more accurately perform attachment of stomach tissue correctly and more efficiently. Thus, enabling the endoscopist to perform the treatment in a minimal invasive way. The method may also enable the endoscopist to perform the treatment in a less time consuming manner.

According to one embodiment of the method wherein the step of delivering the at least one fixating member comprises the step of delivering the at least one fixating member such that the fixating member penetrates at least one layer of stomach tissue and one layer of esophagus or stomach tissue.

According to yet another embodiment wherein the step of delivering the at least one fixating member comprises the step of delivering at least one fixating member such that fixating member penetrates at least two layers of stomach tissue and one layer of esophagus tissue.

The method according another embodiment, wherein the step of delivering the at least one fixating member comprises the step of delivering a fixating member in form of a staple or a suture.

According to another embodiment wherein the step of delivering the at least one fixating member comprises the step of delivering a fixating member in form of a fixating member comprising a first and second state, wherein the method further comprises the steps of delivering the fixating member in the first state such that the fixating member penetrates tissue, and altering the fixation device to a second state in which the fixating member hinders the fixating member from penetrating tissue. The same embodiment of the method may further comprise the step of delivering the at least one fixating member comprises the step of delivering the fixating members using an instrument comprising a fixation member insertion device and a fixation member altering device, wherein the method further comprises the steps of: inserting the fixation member through the tissue, the fixation device being in the first state, and altering the state of the fixation member using the fixation member altering device such that the fixation member is placed in the second state. This method may hinder the endoscopic operator to commit mistakes in the treatment method. Thus this embodiment may also decrease the invasiveness of the treatment method.

According to another embodiment of the method wherein the step of delivering the at least one fixation member, comprises the step of delivering the at least one fixation member housed in an elongated member adapted to house the fixation member.

The method according another embodiment wherein the instrument further comprises at least one anvil member, and wherein the step of delivering the fixation member comprises the step of delivering the fixation member such that the fixation member penetrates the tissue of the esophagus and the tissue of the stomach and engages the anvil member. Wherein the method further comprises the step of positioning the anvil member within the esophagus or wherein the method further comprises the step of positioning the anvil member within the stomach.

According to one embodiment of the method for treating reflux disease wherein the instrument is a gastroscopic instrument, and wherein the method further comprises the step of inserting the instrument into the esophagus and/or stomach through the throat of the patient for operating on the esophagus and/or stomach.

The method according to any another embodiment wherein the instrument is a laparoscopic instrument, and wherein the method further comprises the step if inserting the instrument into the patient from the outside of the patient into the abdominal cavity, further inserting the instrument into the esophagus and/or stomach through an incision opening in the stomach and/or esophagus wall of the patient, and operating on the esophagus and/or stomach from the inside thereof.

According another embodiment of the method wherein the instrument is a laparoscopic instrument adapted to be inserted into the patient through a trocar, and wherein the method comprises the steps of inserting the instrument through a trocar from the outside of the patient into the abdominal cavity, and operating on the esophagus and/or stomach from the outside thereof.

The method according one embodiment wherein the at least one unit is adapted to deliver fixation members penetrating three layers of stomach tissue and one layer of esophagus tissue, and wherein the step of delivering fixating members comprises the step of: fixating three layers of stomach tissue and one layer of esophagus tissue to each other.

According to another embodiment of the method of treating reflux disease wherein the method further comprises the step of creating a pouch of stomach tissue, through the fixation of two layers of stomach tissue. Wherein the step of creating the pouch comprises the step of creating the pouch around a volume filling device placed in contact with the stomach tissue. Wherein the method further comprises the step of creating an opening in the pouch from the inside of the stomach using a cutting member.

The method according to any another embodiment wherein the step of delivering fixating members comprises the step of delivering fixation members penetrating two layers of stomach tissue and one layer of esophagus tissue, for fixating at least one of the two layers of stomach tissue and one layer of esophagus tissue to each other.

According another embodiment of the method wherein the step if delivering fixating members comprises the step of delivering at least one fixation member penetrating four layers of stomach tissue and one layer of esophagus tissue, for fixating at least three layers of stomach tissue and one layer of esophagus tissue to each other. Wherein the method further may comprise the steps of creating a pouch of stomach tissue, through the fixation of two of the three layers of stomach tissue. Wherein the step of creating a pouch may comprise the steps of creating a pouch around a volume filling device placed in contact with the stomach tissue. Wherein the method further may comprise the step of creating an opening in the pouch from the outside of the stomach using a cutting member.

According to another embodiment the method further comprises the step of inserting a volume filling device into the pouch, after the creation of the pouch. Wherein the step of inserting a volume filling device may comprise the step of inserting a solidifying liquid.

According to yet another embodiment, wherein the instrument may further comprise a conduit, and wherein the step of inserting the solidifying liquid may comprise the step of inserting the conduit into the pouch created in the stomach tissue, and delivering the solidifying fluid into the pouch through the conduit.

The method according to another embodiment wherein the instrument comprises at least one operable joint and wherein the step of delivering the solidifying liquid to the pouch comprises delivering the solidifying liquid into a pouch created in the fundus area of the stomach.

According to another embodiment of the method wherein the step if inserting a volume filling device comprises the step of inserting a plurality of volume filling devices.

The method according another embodiment wherein the step of inserting a volume filling device comprising a plurality of volume filling devices, further comprises the step of connecting the plurality of volume filling devices to each other to form an interconnected volume filling device, after the plurality of volume filling devices have been inserted into the pouch.

According to another embodiment of the method wherein the instrument further comprises a tubular member adapted to be inserted into the pouch created of the stomach tissue, and wherein the step of delivering the plurality of volume filling devices into the pouch comprises the step of delivering the plurality of volume filling devices through the tubular member.

According to yet another embodiment of the method wherein the instrument comprises at least one operable joint and wherein the step of delivering the plurality of volume filling devices through the tubular member comprises the step of delivering the plurality of volume filling devices to the pouch created in the fundus area of the stomach.

The method according to another embodiment wherein the step of inserting a volume filling device comprises the step of inserting an expandable volume filling device.

According to another embodiment of the method wherein the method further comprises the step of filling the expandable volume filling device with a solidifying liquid.

The method according to anther embodiment wherein the method further comprises the step of filling the expandable volume filling device with a plurality of volume filling devices.

According to another embodiment of the method wherein the step of inserting a volume filling device comprises the step of inserting a combination of: a plurality of volume filling devices, and a solidifying liquid. Wherein the step of inserting the volume filling device may further comprise the step of inserting the volume filling device through an elongated tubular member adapted to enable the volume filling device to pass through the tubular member and into the pouch. Wherein the step of inserting the volume filling device may comprise the step of inserting the tubular member into the pouch created by the stomach tissue, for enabling the insertion of the volume filling device through the tubular member.

According to another embodiment of the method of treating reflux disease wherein the method further comprises the step of closing the opening created in the pouch.

The method according to any yet another embodiment wherein the method further comprises the step of pulling, pushing and/or holding the stomach tissue. Wherein the step pulling, pushing and/or holding the stomach tissue may comprise the steps of pulling, pushing and/or holding using a device adapted to engage the stomach tissue by penetrating and/or clamping the stomach tissue. Wherein the step of pulling, pushing and/or holding the stomach tissue also may comprise the step of pulling, pushing and/or holding the stomach tissue using suction.

The method according to another embodiment wherein the step of pulling or pushing the stomach tissue comprises the step of pulling or pushing the stomach tissue into an opening of a luminal space inside of the instrument. Wherein the step of pulling or pushing the stomach tissue into an opening of a luminal space inside of the instrument comprises the step of pushing or pulling the stomach tissue into a luminar space having a first cross-section being larger than an area of a second parallel cross-section placed in the opening of the luminal space, placed more distally in the luminal space or distally in the instrument.

According to another embodiment of the method wherein the unit is adapted to be placed more distal in the instrument than the luminal space, and wherein the method further comprises the step of creating a pouch when the at least one fixating member is delivered.

The method according to anther embodiment, wherein a holding portion is placed more distal than the unit, and wherein the method further comprises the step of removing the instrument after the at least one fixating member has been delivered.

According to yet another embodiment of the method wherein the instrument comprises at least one operable joint, and wherein the method comprises the step of placing a first portion of the instrument inside of the stomach in a position such that the layer of stomach tissue and the layer of esophagus tissue are placed between the first portion of the instrument and a second portion of the instrument.

The method according to- another embodiment wherein the method comprises the step of placing the at least one fixating member above the gastroesophageal junction. Wherein the method may further comprise the step of placing the at least one fixating member above the gastroesophageal junction for creating a tunnel between the esophagus and stomach above the junction.

According to another embodiment of the method, wherein the instrument further comprises an operable joint, and wherein the method further comprises the step of placing a portion of the instrument inside of the stomach above the gastroesophageal junction in relation to the cranial-caudal axis of the patient.

According to yet another embodiment of the method, wherein the method further comprises the step of: creating a pouch, and placing at least one first fixating member for fixating the pouch, and placing at least one second fixating member for fixating the pouch, such that the at least one second fixating member intersects the at least one first fixating member.

The method according another embodiment wherein the step of placing the at least one second fixating member, comprises the step of placing the at least one second fixating member perpendicularly to the at least one first fixating member.

A method according one embodiment, wherein the method comprises the steps of: fixating the esophagus tissue to the stomach tissue at a first point along α first esophagus surface length axis, substantially parallel to the esophagus center axis, using the first fixating member delivered using the instrument, and fixating the esophagus tissue to the stomach tissue at a second point along a second esophagus surface length axis, substantially parallel to the esophagus center axis, at a distance from the first esophagus surface length axis, radially in relation to the esophagus center axis, using a second fixating member delivered using the instrument.

A method embodiment enabling the endoscopist to fixate esophagus tissue to stomach tissue at a first and a second point at a distance from the first point may result in a more durable fixation. Such fixation may further enable serosa-to-serosa to be displaced in contact which may enable the process if tissue of esophagus and serosa tissue the stomach to grow together which may even further increase the durability of the fixation. Such fixation may be advantageous in achieving long-term fixation of two tissues.

The method according to another embodiment, wherein the step of delivering the first and second fixating member, comprises the step of delivering the first and second fixating member substantially simultaneously or in a sequence. Wherein the step of delivering the first and/or second fixating member may comprise the step of delivering a staple or suture. Wherein the step of delivering the first and second fixating member may comprise the step of delivering a fixating member in form of a fixating member comprising a first and second state, wherein the method further comprises the steps of delivering the fixating member in the first state such that the fixating member penetrates tissue, and altering the fixation device to a second state in which the fixating member hinders the fixating member from penetrating tissue.

According to another embodiment wherein the step of delivering the first and second fixating member comprises the step of delivering the first and second fixating members using an instrument comprising a fixation member insertion device and a fixation member altering device, wherein the method further comprises the steps of: inserting the fixation member through the tissue, the fixation device being in the first state, and altering the state of the fixation member using the fixation member altering device such that the fixation member is placed in the second state. Wherein the step of delivering the fixation member, may comprise the step of delivering the fixation member housed in an elongated member adapted to house the fixation member.

The method according to another embodiment, wherein the instrument further comprises at least one anvil member, and wherein the step of delivering the first and second fixation member comprises the step of delivering the first and second fixation member such that the first and second fixation member penetrates the tissue of the esophagus and the tissue of the stomach and engages the anvil member. Wherein the method further comprises the step of positioning the anvil member within the esophagus. Wherein the method may further comprise the step of positioning the anvil member within the stomach.

The method according to another embodiment, wherein the instrument is a gastroscopic instrument, and wherein the method further comprises the step of inserting the instrument into the esophagus and/or stomach through the throat of the patient for operating on the esophagus and/or stomach.

According to another embodiment wherein the instrument is a laparoscopic instrument, and wherein the method further comprises the step if inserting the instrument into the patient from the outside of the patient into the abdominal cavity, further inserting the instrument into the esophagus and/or stomach through an incision opening in the stomach and/or esophagus wall of the patient, and operating on the esophagus and/or stomach from the inside thereof.

According to another embodiment wherein the instrument is a laparoscopic instrument adapted to be inserted into the patient through a trocar, and wherein the method comprises the steps of inserting the instrument through a trocar from the outside of the patient into the abdominal cavity, and operating on the esophagus and/or stomach from the outside thereof.

The method according to another embodiment wherein the step of delivering the first and second fixating members comprises delivering the first and second fixating members at positions located at a distance, radially, larger than 2 mm.

According to another embodiment, wherein the step of delivering the first and second fixating members comprises delivering the first and second fixating members at positions located at a distance, radially, larger than 4 mm.

The method according to another embodiment, wherein the first and second unit is adapted to deliver fixation members penetrating three layers of stomach tissue and one layer of esophagus tissue, and wherein the step of delivering fixating members comprises the step of: fixating three layers of stomach tissue and one layer of esophagus tissue to each other. Wherein the method may further comprise the step of creating a pouch of stomach tissue, through the fixation of two layers of stomach tissue. Wherein the step of creating the pouch may comprise the step of creating the pouch around a volume filling device placed in contact with the stomach tissue. Wherein the method further may comprise the step of creating an opening in the pouch from the inside of the stomach using a cutting member.

According to another embodiment of the method, wherein the step if delivering fixating members comprises the step of delivering fixation members penetrating two layers of stomach tissue and one layer of esophagus tissue, for fixating at least one of the two layers of stomach tissue and one layer of esophagus tissue to each other.

According to yet another embodiment of the method, wherein the step if delivering fixating members may comprise the step of delivering at least one fixation member penetrating four layers of stomach tissue and one layer of esophagus tissue, for fixating at least three layers of stomach tissue and one layer of esophagus tissue to each other.

The method according to yet another embodiment wherein the method further comprises the step of creating a pouch of stomach tissue, through the fixation of two of the three layers of stomach tissue. Wherein the method further may comprise the step of creating an opening in the pouch from the outside of the stomach using a cutting member. Wherein the step of creating a pouch may comprises the step of creating a pouch around a volume filling device placed in contact with the stomach tissue.

According to another embodiment of the method, wherein method further comprises the step of inserting a volume filling device into the pouch, after the creation of the pouch. Wherein the step of inserting a volume filling device may comprises the step of inserting a solidifying liquid. Wherein the instrument may comprise a conduit, and wherein the step of inserting the solidifying liquid may comprise the step of inserting the conduit into the pouch created in the stomach tissue, and delivering the solidifying fluid into the pouch through the conduit.

According to another embodiment of the method, wherein the instrument comprises at least one operable joint, and wherein the step of delivering the solidifying liquid to the pouch comprises delivering the solidifying liquid into a pouch created in the fundus area of the stomach.

The method according to yet another embodiment wherein the step if inserting a volume filling device comprises the step of inserting a plurality of volume filling devices. Wherein the step of inserting a volume filling device further may comprise a plurality of volume filling devices, further comprises the step of connecting the plurality of volume filling devices to each other to form an interconnected volume filling device, after the plurality of volume filling devices have been inserted into the pouch.

According to yet another embodiment of the method wherein the instrument further comprises α tubular member adapted to be inserted into the pouch created of the stomach tissue, and wherein the step of delivering the plurality of volume filling devices into the pouch comprises the step of delivering the plurality of volume filling devices through the tubular member. Wherein the instrument may comprise at least one operable joint, and wherein the step of delivering the plurality of volume filling devices through the tubular member comprises the step of delivering the plurality of volume filling devices to the pouch created in the fundus area of the stomach.

The method according to another embodiment of the method, wherein the step of inserting a volume filling device comprises the step of inserting an expandable volume filling device. Wherein the method may further comprise the step of filling the expandable volume filling device with a solidifying liquid. Wherein the method further may comprise the step of filling the expandable volume filling device with a plurality of volume filling devices.

The method according to another embodiment, wherein the step of inserting a volume filling device comprises the step of inserting a combination of: a plurality of volume filling devices, and a solidifying liquid.

According to another embodiment of the method wherein the step of inserting the volume filling device may further comprises the step of inserting the volume filling device through an elongated tubular member adapted to enable the volume filling device to pass through the tubular member and into the pouch. Wherein the step of inserting the volume filling device may further comprise the step of inserting the tubular member into the pouch created by the stomach tissue, for enabling the insertion of the volume filling device through the tubular member.

According to yet another embodiment, wherein the method further comprises the step of closing the opening created in the pouch.

The method according to another embodiment wherein the method further comprises the step of pulling, pushing and/or holding the stomach tissue. Wherein the stomach tissue pulling or pushing device is adapted to engage the stomach tissue using a stomach tissue penetrating member or stomach clamping member. Wherein the step of pulling, pushing and/or holding the stomach tissue may comprise the step of pulling, pushing and/or holding the stomach tissue using suction. Wherein the step of pulling or pushing the stomach tissue may comprise the step of pulling or pushing the stomach tissue into an opening of a luminal space inside of the instrument.

According to yet another embodiment of the method, wherein the step of pulling or pushing the stomach tissue into an opening of a luminal space inside of the instrument may further comprise the step of pushing or pulling the stomach tissue into a luminal space having a first cross-section being larger than an area of a second parallel cross-section placed in the opening of the luminal space, placed more distally in the luminal space or distally in the instrument. Wherein the unit is adapted to be placed more distal in the instrument than the luminal space, and wherein the method further may comprise the step of creating a pouch when the at least one fixating member is delivered.

According to yet another embodiment of the method, wherein a holding portion is placed more distal than the unit, and wherein the method further comprises the step of removing the instrument after the at least one fixating member has been delivered.

The method according to another embodiment, wherein the instrument comprises at least one operable joint, and wherein the method comprises the step of placing a first portion of the instrument inside of the stomach in a position such that the layer of stomach tissue and the layer of esophagus tissue are placed between the first portion of the instrument and a second portion of the instrument.

According to another embodiment, wherein the method comprises the step of placing at least one of the first and second fixating members above the gastroesophageal junction. Wherein the instrument further may comprises an operable joint, and wherein the method further comprises the step of placing a portion of the instrument inside of the stomach above the gastroesophageal junction in relation to the cranial-caudal axis of the patient. Wherein the method may comprise the step of placing the first and second fixating member above the gastroesophageal junction for creating a tunnel between the esophagus and stomach above the junction.

The method according to yet another embodiment, wherein the method further comprises the step of: creating a pouch, and placing at least one first fixating member for fixating the pouch, and placing at least one second fixating member for fixating the pouch, such that the at least one second fixating member intersects the at least one first fixating member.

According to another embodiment of the method, wherein the method further may comprises the step of inserting a plurality of volume filling device through a tubular member adapted to be inserted into a pouch created in the stomach tissue., Wherein the instrument comprises at least one operable joint, and wherein the step of inserting the plurality of volume filling devices comprises the step of inserting the plurality of volume filling devices into a pouch created in the fundus area of the stomach.

According to another embodiment of the method of treating reflux disease, wherein the instrument comprises a conduit, and wherein the method comprises the step of inserting a solidifying fluid into the pouch created in the stomach tissue through the conduit. Wherein the instrument may further comprise at least one operable joint, and wherein the step of inserting the solidifying fluid comprises the step of inserting the solidifying fluid into a pouch created in the fundus area of the stomach.

In one embodiment according to any of the previous embodiments wherein the method further comprises the step if delivering a healing agent from outside of the human patient to an area of the stomach and/or esophagus.

The method to another embodiment, wherein the method comprises the steps of: placing a first part of the unit in the esophagus via the stomach cavity, placing a second part of the unit in the stomach via the abdominal cavity, placing fixating members between the first and second part, above the gastro-esophageal junction for creating a tunnel between the esophagus and stomach. By employing these steps the endoscopist may reach better accuracy in delivering the fixating member at the intended point, which may in turn result in a better treatment.

According to another embodiment of the method, wherein the method comprises the steps of: placing a first part of the unit in the esophagus via the stomach cavity, placing a second part of the unit in the stomach via the abdominal cavity, placing fixating members between the first and second part penetrating at least one layer of stomach tissue and at least one layer of esophagus tissue, above the gastro-esophageal junction, for creating a tunnel between the esophagus and stomach, such that the tunnel will allow a substantially unrestricted contraction and release of the cardia closing sphincter muscle placed in the junction. This embodiment of the method may decrease the risks of patient discomfort and related problems in emptying the stomach of content through vomiting or eructation. Thus this method may restore the function of the cardia closing sphincter muscle to a greater extent.

The method according another embodiment, wherein the method comprises the steps of: placing a first part of the unit in the esophagus via the stomach cavity, placing a second part of the unit in the stomach via the abdominal cavity, bending and directing the instrument such that a fixating member can be placed between the first and second part, placing fixating members between the first and second part penetrating at least one layer of stomach tissue and at least one layer of esophagus tissue, above the gastro-esophageal junction, for creating a tunnel between the esophagus and stomach, such that the tunnel will allow a substantially unrestricted contraction and release of the cardia closing sphincter muscle placed in the junction. This embodiment of the method may decrease the risks of patient discomfort and related problems in emptying the stomach of content through vomiting or eructation. The positioning of a fixating member may be important to reach intended result. Thus this method may restore the function of the cardia closing sphincter muscle to a greater extent by delivering the fixating members more accurately in the method comprising steps of creating the tunnel between esophagus and stomach,

According to another embodiment, wherein the step of delivering the at least one fixating member comprises the step of delivering a fixating member in form of α staple or a suture.

According to yet another embodiment, wherein the step of delivering the at least one fixating member comprises the step of delivering a fixating member in form of a fixating member comprising a first and second state, wherein the method further comprises the steps of delivering the fixating member in the first state such that said fixating member penetrates tissue, and altering the fixation device to a second state in which the fixating member hinders said fixating member from penetrating tissue.

The method according another embodiment, wherein the step of delivering the at least one fixating member comprises the step of delivering the fixating members using an instrument comprising a fixation member insertion device and a fixation member altering device, wherein the method further comprises the steps of: inserting the fixation member through the tissue, the fixation device being in the first state, and altering the state of the fixation member using the fixation member altering device such that the fixation member is placed in the second state.

According to another embodiment, wherein the step of delivering the at least one fixation member, comprises the step of delivering the at least one fixation member housed in an elongated member adapted to house the fixation member.

The method according another embodiment, wherein the instrument further comprises at least one anvil member, and wherein the step of delivering the fixation member comprises the step of delivering the fixation member such that the fixation member penetrates the tissue of the esophagus and the tissue of the stomach and engages the anvil member.

According to another embodiment, wherein the method further comprises the step of positioning the anvil member within the esophagus or stomach.

According to another yet embodiment, wherein the instrument is a gastroscopic instrument, and wherein the method further comprises the step of inserting the instrument into the esophagus and/or stomach through the throat of the patient for operating on the esophagus and/or stomach.

The method according another embodiment, wherein the instrument is a laparoscopic instrument, and wherein the method further comprises the step if inserting the instrument into the patient from the outside of the patient into the abdominal cavity, further inserting the instrument into the esophagus and/or stomach through an incision opening in the stomach and/or esophagus wall of the patient, and operating on the esophagus and/or stomach from the inside thereof.

According to another embodiment, wherein the instrument is a laparoscopic instrument adapted to be inserted into the patient through a trocar, and wherein the method comprises the steps of inserting the instrument through a trocar from the outside of the patient into the abdominal cavity, and operating on the esophagus and/or stomach from the outside thereof.

The method according another embodiment, wherein the at least one unit is adapted to deliver fixation members penetrating three layers of stomach tissue and one layer of esophagus tissue, and wherein the step of delivering fixating members comprises the step of: fixating three layers of stomach tissue and one layer of esophagus tissue to each other.

According to yet another embodiment, wherein the method further comprises the step of creating a pouch of stomach tissue, through the fixation of two layers of stomach tissue.

According to another embodiment, wherein the step of creating the pouch comprises the step of creating the pouch around a volume filling device placed in contact with the stomach tissue.

The method according another embodiment, wherein the method further comprises the step of creating an opening in the pouch from the inside of the stomach using a cutting member.

According to yet another embodiment, , wherein the step if delivering fixating members comprises the step of delivering fixation members penetrating two layers of stomach tissue and one layer of esophagus tissue, for fixating at least one of the two layers of stomach tissue and one layer of esophagus tissue to each other.

According to another embodiment, wherein the step if delivering fixating members comprises the step of delivering at least one fixation member penetrating four layers of stomach tissue and one layer of esophagus tissue, for fixating at least three layers of stomach tissue and one layer of esophagus tissue to each other.

According to yet another embodiment, wherein the method further comprises the step of creating a pouch of stomach tissue, through the fixation of two of the three layers of stomach tissue.

According to another embodiment, wherein the step of creating α pouch comprises the step of creating a pouch around a volume filling device placed in contact with the stomach tissue.

According to yet another embodiment, wherein the method further comprises the step of creating an opening in the pouch from the outside of the stomach using a cutting member.

The method according another embodiment, wherein method further comprises the step of inserting a volume filling device into the pouch, after the creation of the pouch.

According to another embodiment, wherein the step of inserting a volume filling device comprises the step of inserting a solidifying liquid. Wherein the instrument may further comprise a conduit, and wherein the step of inserting the solidifying liquid comprises the step of inserting the conduit into the pouch created in the stomach tissue, and delivering the solidifying fluid into the pouch through the conduit.

According to another embodiment, wherein the instrument comprises at least one operable joint, and wherein the step of delivering the solidifying liquid to the pouch comprises delivering the solidifying liquid into a pouch created in the fundus area of the stomach.

The method according another embodiment, wherein the step if inserting a volume filling device comprises the step of inserting a plurality of volume filling devices.

The method according another embodiment, wherein the step of inserting a volume filling device comprising a plurality of volume filling devices, further comprises the step of connecting the plurality of volume filling devices to each other to form an interconnected volume filling device, after the plurality of volume filling devices have been inserted into the pouch.

According to another embodiment, wherein the instrument further comprises a tubular member adapted to be inserted into the pouch created of the stomach tissue, and wherein the step of delivering the plurality of volume filling devices into the pouch comprises the step of delivering the plurality of volume filling devices through the tubular member.

According to yet another embodiment, wherein the instrument comprises at least one operable joint, and wherein the step of delivering the plurality of volume filling devices through the tubular member comprises the step of delivering the plurality of volume filling devices to the pouch created in the fundus area of the stomach.

The method of treating reflux disease according another embodiment, wherein the step of inserting a volume filling device comprises the step of inserting an expandable volume filling device.

The method according another embodiment, wherein the method further comprises the step of filling the expandable volume filling device with a solidifying liquid.

According to yet another embodiment, wherein the method further comprises the step of filling the expandable volume filling device with a plurality of volume filling devices.

According to yet another embodiment, wherein the step of inserting a volume filling device comprises the step of inserting a combination of: a plurality of volume filling devices, and a solidifying liquid.

The method according another embodiment, wherein the step of inserting the volume filling device comprises the step of inserting the volume filling device through an elongated tubular member adapted to enable the volume filling device to pass through the tubular member and into the pouch.

According to yet another embodiment, wherein the step of inserting the volume filling device comprises the step of inserting the tubular member into the pouch created by the stomach tissue, for enabling the insertion of the volume filling device through the tubular member.

According to yet another embodiment, wherein the method further comprises the step of closing the opening created in the pouch.

According to another embodiment, wherein the method further comprises the step of pulling, pushing and/or holding the stomach tissue.

According to yet another embodiment, wherein the step pulling, pushing and/or holding the stomach tissue comprises the step of pulling, pushing and/or holding using a device adapted to engage the stomach tissue by penetrating and/or clamping the stomach tissue.

The method of treating reflux disease according another embodiment, wherein the step of pulling, pushing and/or holding the stomach tissue comprises the step of pulling, pushing and/or holding the stomach tissue using suction.

According to yet another embodiment, wherein the step of pulling or pushing the stomach tissue comprises the step of pulling or pushing the stomach tissue into an opening of a luminal space inside of the instrument.

The method according to another embodiment, wherein the step of pulling or pushing the stomach tissue into an opening of a luminal space inside of the instrument comprises the step of pushing or pulling the stomach tissue into a luminar space having a first cross-section being larger than an area of a second parallel cross-section placed in the opening of the luminal space, placed more distally in the luminal space or distally in the instrument.

According to yet another embodiment" wherein the unit is adapted to be placed more distal in the instrument than the luminal space, and wherein the method further comprises the step of creating a pouch when the at least one fixating member is delivered.

According to yet another embodiment, wherein a holding portion is placed more distal than the unit, and wherein the method further comprises the step of removing the instrument after the at least one fixating member has been delivered.

According to yet another embodiment, wherein the instrument comprises at least one operable joint, and wherein the method comprises the step of placing a first portion of the instrument inside of the stomach in a position such that the layer of stomach tissue and the layer of esophagus tissue are placed between the first portion of the instrument and a second portion of the instrument.

The method according to another embodiment, wherein the method comprises the step of placing the at least one fixating member above the gastroesophageal junction.

The method according to another embodiment, wherein the method comprises the step of placing the at least one fixating member above the gastroesophageal junction for creating a tunnel between the esophagus and stomach above the junction.

According to yet another embodiment, wherein the instrument further comprises an operable joint, and wherein the method further comprises the step of placing a portion of the instrument inside of the stomach above the gastroesophageal junction in relation to the cranial-caudal axis of the patient.

According to yet another embodiment, wherein the step of placing the at least one second fixating member, comprises the step of placing the at least one second fixating member perpendicularly to the at least one first fixating member.

The method according to another embodiment, wherein the method further comprises the step of: creating a pouch, and placing at least one first fixating member for fixating the pouch, and placing at least one second fixating member for fixating the pouch, such that the at least one second fixating member intersects the at least one first fixating member.

According to yet another embodiment, wherein the step of placing the at least one second fixating member, comprises the step of placing the at least one second fixating member perpendicularly to the at least one first fixating member.

A method, according to one embodiment, of inserting a plurality of volume filling device to an area of the stomach of a patient, the method comprising the steps: placing in elongated member inside of the patient, transporting the plurality of volume filling devices through the elongated member, and inserting the plurality of volume filling device into a pouch created of stomach tissue, in the stomach area of a patient.

One possible advantage with inserting a plurality of volume filling devices into the pouch may be the ability to avoid ileus in the case if the pouch ruptures. Ileus may be both harmful and bring great discomfort to the patient. The plurality of volume filling object may satisfy the property of avoiding ileus, due to each volume filling device's individual size, and as a plurality satisfy the property of filling the volume required by of the treatment method.

The method according to another embodiment, wherein the step of transporting the plurality of volume filling devices through the elongated member comprises transporting the plurality of volume filling devices through a tubular member adapted to be inserted into a pouch created in the stomach tissue.

The method according to yet another embodiment, wherein the step of transporting the plurality of volume filling devices through α tubular member comprises the step of transporting a plurality of spherical volume filling devices through the tubular member.

The method according to another embodiment, wherein the step of transporting the plurality of spherical volume filling devices through a tubular member comprises the step of transporting a plurality of spherical volume filling devices through a tubular member having a circular cross-section.

According to another embodiment, wherein the step of transporting the plurality of volume filling devices through a tubular member comprises the step of transporting a plurality of polyhedral volume filling devices through the tubular member.

According to another embodiment, wherein the step of transporting the plurality of polyhedral volume filling devices through a tubular member comprises the step of transporting a plurality of polyhedral volume filling devices through a tubular member having a polygonal cross-section.

According to yet another embodiment, wherein the instrument comprises a conduit adapted to be inserted into a pouch created in the stomach tissue, and wherein the method comprises the step of delivering a solidifying fluid into the pouch created in the stomach tissue through the conduit.

According to another embodiment, wherein the step of inserting a volume filling device comprises the step of inserting a plurality of volume filling devices and a solidifying fluid into the pouch.

According to another embodiment, wherein the step of inserting a volume filling device into the pouch comprises the step of inserting an expandable volume filling device into the pouch created in the stomach tissue through the tubular member.

The method according to another embodiment, wherein the instrument comprises a conduit, and wherein the method further comprises the step of injecting a solidifying fluid into the expandable volume filling device through the conduit.

According to another embodiment, wherein the instrument further comprises a tubular member, and wherein the method further comprises the step of inserting a plurality of volume filling devices into the expandable volume filling device.

According to another embodiment, wherein the step of inserting a plurality of volume filling devices comprises the step of inserting a plurality of spherical volume filling devices.

The method according to another embodiment, wherein the step of inserting a plurality of spherical volume filling device comprises the step of inserting a plurality of spherical volume filling device through a tubular member having a circular cross-section.

According to another embodiment, wherein the step of inserting a plurality of volume filling devices comprises the step of inserting a plurality of polyhedral volume filling devices.

According to another embodiment, wherein the step of inserting a plurality of polyhedral volume filling devices comprises the step of delivering the plurality of polyhedral volume filling devices through a tubular member having a polygonal cross-section.

The method according to another embodiment, wherein the step of inserting a volume filling device comprises the step of inserting a plurality of volume filling devices and a solidifying fluid into the expandable volume filling device.

The method of treating reflux disease according another embodiment, wherein the method further comprises the step of connecting the plurality of volume filling devices to each other to form an interconnected volume filling device, after the plurality of volume filling devices have been delivered into the pouch.

According to another embodiment, wherein the method further comprises the step of delivering at least one fixating members penetrating at least one layer of stomach tissue and one layer of esophagus or stomach tissue.

According to another embodiment, wherein the method comprises the step of delivering fixating members penetrating at least two layers of stomach tissue and one layer of esophagus tissue.

The method according to another embodiment, wherein the step of delivering the at least one fixating member comprises the step of delivering a fixating member in form of a staple or a suture.

According to another embodiment, wherein the step of delivering the at least one fixating member comprises the step of delivering a fixating member in form of a fixating member comprising a first and second state, wherein the method further comprises the steps of delivering the fixating member in the first state such that the fixating member penetrates tissue, and altering the fixation device to a second state in which the fixating member hinders the fixating member from penetrating tissue.

According to yet another embodiment, wherein the step of delivering the at least one fixating member comprises the step of delivering the fixating members using an instrument comprising a fixation member insertion device and a fixation member altering device, wherein the method further comprises the steps of: inserting the fixation member through the tissue, the fixation device being in the first state, and altering the state of the fixation member using the fixation member altering device such that the fixation member is placed in the second state.

According to yet another embodiment, wherein the step of delivering the at least one fixation member, comprises the step of delivering the at least one fixation member housed in an elongated member adapted to house the fixation member.

The method according to another embodiment, wherein the instrument further comprises at least one anvil member, and wherein the step of delivering the fixation member comprises the step of delivering the fixation member such that the fixation member penetrates the tissue of the esophagus and the tissue of the stomach and engages the anvil member.

According to another embodiment, wherein the method further comprises the step of positioning the anvil member within the esophagus.

According to yet another embodiment, wherein the method further comprises the step of positioning the anvil member within the stomach.

The method according to another embodiment, wherein the instrument is a gastroscopic instrument, and wherein the method further comprises the step of inserting the instrument into the esophagus and/or stomach through the throat of the patient for operating on the esophagus and/or stomach.

According to yet another embodiment, wherein the instrument is a laparoscopic instrument, and wherein the method further comprises the step if inserting the instrument into the patient from the outside of the patient into the abdominal cavity, further inserting the instrument into the esophagus and/or stomach through an incision opening in the stomach and/or esophagus wall of the patient, and operating on the esophagus and/or stomach from the inside thereof.

The method according to another embodiment, wherein the instrument is a laparoscopic instrument adapted to be inserted into the patient through a trocar, and wherein the method comprises the steps of inserting the instrument through a trocar from the outside of the patient into the abdominal cavity, and operating on the esophagus and/or stomach from the outside thereof.

According to yet another embodiment, wherein the at least one unit is adapted to deliver fixation members penetrating three layers of stomach tissue and one layer of esophagus tissue, and wherein the step of delivering fixating members comprises the step of: fixating three layers of stomach tissue and one layer of esophagus tissue to each other.

The method according to another embodiment, wherein the method further comprises the step of creating a pouch of stomach tissue, through the fixation of two layers of stomach tissue.

According to yet another embodiment, wherein the method further comprises the step of creating an opening in the pouch from the inside of the stomach using a cutting member.

The method according to another embodiment, wherein the step if delivering fixating members comprises the step of delivering fixation members penetrating two layers of stomach tissue and one layer of esophagus tissue, for fixating at least one of the two layers of stomach tissue and one layer of esophagus tissue to each other.

According to yet another embodiment, wherein the step if delivering fixating members comprises the step of delivering fixation members penetrating two layers of stomach tissue and one layer of esophagus tissue, for fixating at least one of the two layers of stomach tissue and one layer of esophagus tissue to each other.

According to yet another embodiment, wherein the step if delivering fixating members comprises the step of delivering at least one fixation member penetrating four layers of stomach tissue and one layer of esophagus tissue, for fixating at least three layers of stomach tissue and one layer of esophagus tissue to each other.

The method according to another embodiment, wherein the method further comprises the step of creating a pouch of stomach tissue, through the fixation of two of the three layers of stomach tissue.

The method of treating reflux disease according another embodiment, wherein the instrument comprises at least one operable joint, and wherein the method comprises the step of delivering the volume filling device to a pouch at least partly created of fundus stomach tissue.

The method according to another embodiment, wherein the instrument comprises at least two operable joints, wherein the method comprises the step of delivering the volume filling device to a pouch at least partly created of fundus stomach tissue.

According to yet another embodiment, wherein the instrument further comprises α tissue closing unit adapted to close an opening created in the pouch.

The method according to another embodiment, wherein the method further comprises the step of pulling, pushing and/or holding the stomach tissue.

According to yet another embodiment, wherein the step pulling, pushing and/or holding the stomach tissue comprises the step of pulling, pushing and/or holding using a device adapted to engage the stomach tissue by penetrating and/or clamping the stomach tissue.

The method according to another embodiment, wherein the step of pulling, pushing and/or holding the stomach tissue comprises the step of pulling, pushing and/or holding the stomach tissue using suction.

According to yet another embodiment, wherein the step of pulling, pushing and/or holding the stomach tissue comprises the step of pulling, pushing and/or holding the stomach tissue using a pressurized fluid.

According to yet another embodiment, wherein the step of pulling or pushing the stomach tissue comprises the step of pulling or pushing the stomach tissue into an opening of a luminal space inside of the instrument.

According to yet another embodiment, wherein the step of pulling or pushing the stomach tissue into an opening of a luminal space inside of the instrument comprises the step of pushing or pulling the stomach tissue into a luminar space having a first cross-section being larger than an area of a second parallel cross-section placed in the opening of the luminal space, placed more distally in the luminal space or distally in the instrument.

According to yet another embodiment, wherein the unit is adapted to be placed more distal in the instrument than the luminal space, and wherein the method further comprises the step of creating a pouch when the at least one fixating member is delivered.

The method according to another embodiment, wherein a holding portion is placed more distal than the unit, and wherein the method further comprises the step of removing the instrument after the at least one fixating member has been delivered.

According to yet another embodiment, wherein the instrument comprises at least one operable joint, and wherein the method comprises the step of placing a first portion of the instrument inside of the stomach in a position such that the layer of stomach tissue and the layer of esophagus tissue are placed between the first portion of the instrument and a second portion of the instrument.

The method according to yet another embodiment, wherein the method comprises the step of placing the at least one fixating member above the gastroesophageal junction.

The method according to another embodiment, wherein the method comprises the step of placing the at least one fixating member above the gastroesophageal junction for creating a tunnel between the esophagus and stomach above the junction.

According to yet another embodiment, wherein the instrument further comprises an operable joint, and wherein the method further comprises the step of placing a portion of the instrument inside of the stomach above the gastroesophageal junction in relation to the cranial-caudal axis of the patient.

The method according to yet another embodiment, wherein the method further comprises the step of: creating a pouch, and placing at least one first fixating member for fixating the pouch, and placing at least one second fixating member for fixating the pouch, such that the at least one second fixating member intersects the at least one first fixating member.

According to yet another embodiment, wherein the step of placing the at least one second fixating member, comprises the step of placing the at least one second fixating member perpendicularly to the at least one first fixating member.

According to one embodiment, wherein the method comprises the steps of: delivering fixating members penetrating at least two layers of stomach tissue, for creating α pouch including a substantially closed space surrounded by stomach wall tissue. Wherein the step of creating α pouch may further comprise the step of creating a pouch formed as a tobacco pouch having a neck part of the pouch being wrinkled back and forth to create the closed space, when the instrument is being used on stomach tissue.

Creating α closed space surrounded by stomach wall may be less invasive than other methods of creating a hollow pouch from stomach tissue. Further, a pouch having the form of a tobacco pouch may enable sequenced method and procedure for creating the pouch substantially having the intended size. Further, the sequenced method may be more efficient to the endoscopist and decrease the risk of human error in the treatment.

According to another embodiment, wherein the step of creating a pouch comprises the step of creating a pouch formed as a flat folded pouch by placing the fixating members in a curve to create the closed space.

According to yet another embodiment, wherein the method further comprises the step of predefining the pouch using vacuum suction for sucking the pouch into a predefined shape.

According to another embodiment, wherein the method further comprises the step of predefining the pouch by clamping and pulling or pushing the stomach wall into a pre fixating pouch shape.

The method according to another embodiment, wherein the step of delivering a fixating member comprises the step of delivering a staple or suture.

The method according to yet another embodiment, wherein the step of delivering the at least one fixating member comprises the step of delivering a fixating member in form of a fixating member comprising a first and second state, wherein the method further comprises the steps of delivering the fixating member in the first state such that the fixating member penetrates tissue, and altering the fixation device to a second state in which the fixating member hinders the fixating member from penetrating tissue.

According to another embodiment, wherein the step of delivering the at least one fixating member comprises the step of delivering the fixating members using an instrument comprising a fixation member insertion device and a fixation member altering device, wherein the method further comprises the steps of: inserting the fixation member through the tissue, the fixation device being in the first state, and altering the state of the fixation member using the fixation member altering device such that the fixation member is placed in the second state.

According to yet another embodiment, wherein the step of delivering the at least one fixation member, comprises the step of delivering the at least one fixation member housed in an elongated member adapted to house the fixation member.

The method according to another embodiment, wherein the instrument further comprises at least one anvil member, and wherein the step of delivering the fixation member comprises the step of delivering the fixation member such that the fixation member penetrates the tissue of the esophagus and the tissue of the stomach and engages the anvil member.

The method according to yet another embodiment, wherein the method further comprises the step of positioning the anvil member within the esophagus or stomach.

According to another embodiment, wherein the instrument is a gastroscopic instrument, and wherein the method further comprises the step of inserting the instrument into the esophagus and/or stomach through the throat of the patient for operating on the esophagus and/or stomach.

According to yet another embodiment, wherein the instrument is a laparoscopic instrument, and wherein the method further comprises the step if inserting the instrument into the patient from the outside of the patient into the abdominal cavity, further inserting the instrument into the esophagus and/or stomach through an incision opening in the stomach and/or esophagus wall of the patient, and operating on the esophagus and/or stomach from the inside thereof.

The method according to another embodiment, wherein the instrument is a laparoscopic instrument adapted to be inserted into the patient through a trocar, and wherein the method comprises the steps of inserting the instrument through a trocar from the outside of the patient into the abdominal cavity, and operating on the esophagus and/or stomach from the outside thereof.

The method according to yet another embodiment, wherein the at least one unit is adapted to deliver fixation members penetrating three layers of stomach tissue and one layer of esophagus tissue, and wherein the step of delivering fixating members comprises the step of: fixating three layers of stomach tissue and one layer of esophagus tissue to each other.

According to another embodiment, wherein the method further comprises the step of creating a pouch of stomach tissue, through the fixation of two layers of stomach tissue.

According to yet another embodiment, wherein the step of creating the pouch comprises the step of creating the pouch around a volume filling device placed in contact with the stomach tissue.

The method according to another embodiment, wherein the method further comprises the step of creating an opening in the pouch from the inside of the stomach using a cutting member.

The method according to another embodiment, wherein the step if delivering fixating members comprises the step of delivering fixation members penetrating two layers of stomach tissue and one layer of esophagus tissue, for fixating at least one of the two layers of stomach tissue and one layer of esophagus tissue to each other.

The method according to yet another embodiment, wherein the step if delivering fixating members comprises the step of delivering at least one fixation member penetrating four layers of stomach tissue and one layer of esophagus tissue, for fixating at least three layers of stomach tissue and one layer of esophagus tissue to each other.

According to another embodiment, wherein the method further comprises the step of creating a pouch of stomach tissue, through the fixation of two of the three layers of stomach tissue.

According to yet another embodiment, wherein the step of creating a pouch comprises the step of creating a pouch around a volume filling device placed in contact with the stomach tissue.

The method according to another embodiment, wherein the method further comprises the step of creating an opening in the pouch from the outside of the stomach using a cutting member.

The method according to yet another embodiment, wherein method further comprises the step of inserting a volume filling device into the pouch, after the creation of the pouch.

The method of treating reflux disease according another embodiment, wherein the step of inserting a volume filling device comprises the step of inserting a solidifying liquid.

According to another embodiment, wherein the instrument comprises a conduit, and wherein the step of inserting the solidifying liquid comprises the step of inserting the conduit into the pouch created in the stomach tissue, and delivering the solidifying fluid into the pouch through the conduit.

The method according to another embodiment, wherein the instrument comprises at least one operable joint, and wherein the step of delivering the solidifying liquid to the pouch comprises delivering the solidifying liquid into a pouch created in the fundus area of the stomach.

The method of treating reflux disease according another embodiment, wherein the step if inserting a volume filling device comprises the step of inserting a plurality of volume filling devices.

The method according to another embodiment, wherein the step of inserting a volume filling device comprising a plurality of volume filling devices, further comprises the step of connecting the plurality of volume filling devices to each other to form an interconnected volume filling device, after the plurality of volume filling devices have been inserted into the pouch.

The method according to yet another embodiment, wherein the instrument further comprises a tubular member adapted to be inserted into the pouch created of the stomach tissue, and wherein the step of delivering the plurality of volume filling devices into the pouch comprises the step of delivering the plurality of volume filling devices through the tubular member.

According to another embodiment, wherein the instrument comprises at least one operable joint, and wherein the step of delivering the plurality of volume filling devices through the tubular member comprises the step of delivering the plurality of volume filling devices to the pouch created in the fundus area of the stomach.

According to yet another embodiment, wherein the step of inserting a volume filling device comprises the step of inserting an expandable volume filling device.

According to yet another embodiment, wherein the method further comprises the step of filling the expandable volume filling device with a solidifying liquid.

According to yet another embodiment, wherein the method further comprises the step of filling the expandable volume filling device with a plurality of volume filling devices.

The method according to another embodiment, wherein the step of inserting a volume filling device comprises the step of inserting a combination of: a plurality of volume filling devices, and a solidifying liquid.

The method according to yet another embodiment, wherein the step of inserting the volume filling device comprises the step of inserting the volume filling device through an elongated tubular member adapted to enable the volume filling device to pass through the tubular member and into the pouch.

According to another embodiment, wherein the step of inserting the volume filling device comprises the step of inserting the tubular member into the pouch created by the stomach tissue, for enabling the insertion of the volume filling device through the tubular member.

According to yet another embodiment, wherein the method further comprises the step of closing the opening created in the pouch.

The method according to another embodiment, wherein the method further comprises the step of pulling, pushing and/or holding the stomach tissue.

The method according to yet another embodiment, wherein the step pulling, pushing and/or holding the stomach tissue comprises the step of pulling, pushing and/or holding using a device adapted to engage the stomach tissue by penetrating and/or clamping the stomach tissue.

The method according to another embodiment, wherein the step of pulling, pushing and/or holding the stomach tissue comprises the step of pulling, pushing and/or holding the stomach tissue using suction.

The method according to another embodiment, wherein the step of pulling or pushing the stomach tissue comprises the step of pulling or pushing the stomach tissue into an opening of a luminal space inside of the instrument.

The method according to yet another embodiment, wherein the step of pulling or pushing the stomach tissue into an opening of a luminal space inside of the instrument comprises the step of pushing or pulling the stomach tissue into a luminar space having a first cross-section being larger than an area of a second parallel cross-section placed in the opening of the luminal space, placed more distally in the luminal space or distally in the instrument.

According to another embodiment, wherein the unit is adapted to be placed more distal in the instrument than the luminal space, and wherein the method further comprises the step of creating a pouch when the at least one fixating member is delivered.

According to yet another embodiment, wherein the instrument comprises at least one operable joint, and wherein the method comprises the step of placing a first portion of the instrument inside of the stomach in a position such that the layer of stomach tissue and the layer of esophagus tissue are placed between the first portion of the instrument and a second portion of the instrument.

The method according to another embodiment, wherein the method comprises the step of placing the at least one fixating member above the gastroesophageal junction.

The method according to yet another embodiment, wherein the method further comprises the step of: creating a pouch, and placing at least one first fixating member for fixating the pouch, and placing at least one second fixating member for fixating the pouch, such that the at least one second fixating member intersects the at least one first fixating member.

According to another embodiment, wherein the step of placing the at least one second fixating member, comprises the step of placing the at least one second fixating member perpendicularly to the at least one first fixating member.

According to yet another embodiment, wherein a holding portion is placed more distal than the unit, and wherein the method further comprises the step. of removing the instrument after the at least one fixating member has been delivered.

The method according to another embodiment, wherein the method comprises the steps of: deliver fixating members penetrating at least part of two layers of stomach tissue, and placing the fixating members for attaching in a first position of the stomach outer wall, and in a second position of the stomach outer wall, wherein the at least one unit comprises two parts adapted to be placed in one of the following places; in the stomach and outside the stomach and one first part outside and the second part inside the stomach, wherein the unit is adapted to place the fixating member substantially between the first and second part, wherein the instrument is adapted to have the unit inserted into the abdominal cavity from the outside of the body, wherein the instrument comprising a joint and has a shape to allow it to be directed to reach a position such that at least one fixating member will attach the outer stomach wall to the outer stomach wall. Abdominal treatment methods may have lower post-treatment risks. Thus, creating a fixation between positions of a stomach outer wall may be preferable in some cases.

The method according to another embodiment, wherein the instrument comprises at least one unit adapted to deliver fixating members, and wherein the method comprises the steps of: placing an esophagus part of the unit in the esophagus via the stomach cavity, placing a stomach part of the unit in the stomach via the abdominal cavity, delivering the at least one fixating member between the esophagus part and the stomach part, penetrating at least one layer of stomach tissue and one layer of esophagus tissue above the gastro-esophageal junction, for attaching the esophagus and stomach above the junction,

According to another yet embodiment, wherein the step of placing the at least one fixating member comprises the step of delivering the at least one fixating member in a cranial position in relation to the angle of His, at a distance from the angle of His.

According to yet another embodiment, wherein the method further comprises the steps of: delivering a second fixating member using a second unit, such that the first fixating member is placed at a first point along a first esophagus surface length axis, substantially parallel to the esophagus center axis, and the second fixating member is placed at a second point along a second esophagus surface length axis substantially parallel to the first esophagus surface length axis, wherein the first esophagus surface length axis is positioned at a distance from the second esophagus surface length axis, radially in relation to the esophagus center axis.

According to yet another embodiment, wherein the instrument is a laparoscopic instrument, and wherein the method further comprises the steps of: inserting the instrument into the abdominal cavity and further into the stomach through an incision opening in the stomach wall, and operating on the stomach from the inside thereof.

According to yet another embodiment, wherein the instrument is a laparoscopic instrument, and wherein the method further comprises the steps of inserting the instrument into the abdominal cavity, and operating on the stomach from the outside thereof.

According to yet another embodiment, wherein the method further comprises the step of inserting the instrument into the patient through a trocar from the outside of the patient into the abdominal cavity.

The method according to another embodiment, wherein the method further comprises the step of delivering at least one fixating member for fixating the stomach tissue, without penetrating the mucosa layer of the stomach wall.

The method according to yet another embodiment, wherein the method is a method for treating obesity and wherein the method comprises the step of substantially reducing the volume of the stomach cavity.

The method according to yet another embodiment, wherein the method further comprises the step of clamping four layers of stomach for creating a pouch of stomach wall from the outside of the stomach with a closed space within the pouch, and delivering at least one fixating member for fixating the stomach tissue, penetrating only two muscular layers of the stomach wall.

The method according to yet another embodiment, wherein the method comprises the step of delivering fixating members for penetrating at least two layers of stomach tissue, wherein the step of delivering fixating members for penetrating at least two layers of stomach tissue comprises placing the fixating members such that a substantially closed space surrounded by stomach wall is created, such that the stomach wall creates a pouch of stomach wall including the closed space.

The method according to yet another embodiment, wherein the method comprises the step of fixating a pouch formed as a tobacco pouch presenting a neck part of the pouch being wrinkled back and forth to create the closed space.

The method according to yet another embodiment, wherein the method comprises the step of fixating a pouch formed as a flat folded pouch with the fixating members placed in a curve to create the closed space.

The method according to yet another embodiment, wherein the method further comprises the step of predefining the pouch using vacuum suction for sucking the pouch into a predefined shape.

According to another embodiment, wherein the method comprises the step of predefining the pouch by clamping and pulling or pushing the stomach wall into a pre fixating pouch shape.

According to yet another embodiment, wherein the step of delivering a fixating member comprises the step of delivering a staple or suture.

According to yet another embodiment, wherein the step of delivering the at least one fixating member comprises the step of delivering a fixating member in form of a fixating member comprising a first and second state, wherein the method further comprises the steps of delivering the fixating member in the first state such that the fixating member penetrates tissue, and altering the fixation device to a second state in which the fixating member hinders the fixating member from penetrating tissue.

According to yet another embodiment, wherein the step of delivering the at least one fixating member comprises the step of delivering the fixating members using an instrument comprising a fixation member insertion device and a fixation member altering device, wherein the method further comprises the steps of: inserting the fixation member through the tissue, the fixation device being in the first state, and altering the state of the fixation member using the fixation member altering device such that the fixation member is placed in the second state.

According to yet another embodiment, wherein the step of delivering the at least one fixation member, comprises the step of delivering the at least one fixation member housed in an elongated member adapted to house the fixation member.

According to yet another embodiment, wherein the instrument further comprises at least one anvil member, and wherein the step of delivering the fixation member comprises the step of delivering the fixation member such that the fixation member penetrates the tissue of the esophagus and the tissue of the stomach and engages the anvil member.

According to yet another embodiment, wherein the method further comprises the step of positioning the anvil member within or outside of the stomach.

According to yet another embodiment, wherein the instrument is a gastroscopic instrument, and wherein the method further comprises the step of inserting the instrument into the esophagus and/or stomach through the throat of the patient for operating on the esophagus and/or stomach.

According to yet another embodiment, wherein the instrument is a laparoscopic instrument, and wherein the method further comprises the step if inserting the instrument into the patient from the outside of the patient into the abdominal cavity, further inserting the instrument into the esophagus and/or stomach through an incision opening in the stomach and/or esophagus wall of the patient, and operating on the esophagus and/or stomach from the inside thereof.

According to yet another embodiment, wherein the instrument is a laparoscopic instrument adapted to be inserted into the patient through a trocar, and wherein the method comprises the steps of inserting the instrument through a trocar from the outside of the patient into the abdominal cavity, and operating on the esophagus and/or stomach from the outside thereof.

According to yet another embodiment, wherein the at least one unit is adapted to deliver fixation members penetrating three layers of stomach tissue and one layer of esophagus tissue, and wherein the step of delivering fixating members comprises the step of: fixating three layers of stomach tissue and one layer of esophagus tissue to each other.

According to another embodiment, wherein the method further comprises the step of creating a pouch of stomach tissue, through the fixation of two layers of stomach tissue.

According to yet another embodiment, wherein the step of creating the pouch comprises the step of creating the pouch around a volume filling device placed in contact with the stomach tissue.

According to yet another embodiment, wherein the method further comprises the step of creating an opening in the pouch from the inside of the stomach using a cutting member.

According to yet another embodiment, wherein the step if delivering fixating members comprises the step of delivering fixation members penetrating two layers of stomach tissue and one layer of esophagus tissue, for fixating at least one of the two layers of stomach tissue and one layer of esophagus tissue to each other.

According to yet another embodiment, wherein the step if delivering fixating members comprises the step of delivering at least one fixation member penetrating four layers of stomach tissue and one layer of esophagus tissue, for fixating at least three layers of stomach tissue and one layer of esophagus tissue to each other.

According to yet another embodiment, wherein the method further comprises the step of creating a pouch of stomach tissue, through the fixation of two of the three layers of stomach tissue.

According to yet another embodiment, wherein the step of creating a pouch comprises the step of creating a pouch around a volume filling device placed in contact with the stomach tissue.

According to yet another embodiment, wherein the method further comprises the step of creating an opening in the pouch from the outside of the stomach using a cutting member.

According to yet another embodiment, wherein method further comprises the step of inserting a volume filling device into the pouch, after the creation of the pouch.

According to yet another embodiment, wherein the step of inserting a volume filling device comprises the step of inserting a solidifying liquid.

The method according to another embodiment, wherein the instrument comprises a conduit, and wherein the step of inserting the solidifying liquid comprises the step of inserting the conduit into the pouch created in the stomach tissue, and delivering the solidifying fluid into the pouch through the conduit.

The method according to yet another embodiment, wherein the instrument comprises at least one operable joint, and wherein the step of delivering the solidifying liquid to the pouch comprises delivering the solidifying liquid into a pouch created in the fundus area of the stomach.

The method according to yet another embodiment, wherein the step if inserting a volume filling device comprises the step of inserting a plurality of volume filling devices.

The method according to yet another embodiment, wherein the step of inserting a volume filling device comprising a plurality of volume filling devices, further comprises the step of connecting the plurality of volume filling devices to each other to form an interconnected volume filling device, after the plurality of volume filling devices have been inserted into the pouch.

The method according to yet another embodiment, wherein the instrument further comprises a tubular member adapted to be inserted into the pouch created of the stomach tissue, and wherein the step of delivering the plurality of volume filling devices into the pouch comprises the step of delivering the plurality of volume filling devices through the tubular member.

The method according to yet another embodiment, wherein the instrument comprises at least one operable joint, and wherein the step of delivering the plurality of volume filling devices through the tubular member comprises the step of delivering the plurality of volume filling devices to the pouch created in the fundus area of the stomach.

The method according to yet another embodiment, wherein the step of inserting a volume filling device comprises the step of inserting an expandable volume filling device.

The method according to claim 299, wherein the method further comprises the step of filling the expandable volume filling device with a solidifying liquid.

The method according to yet another embodiment, wherein the method further comprises the step of filling the expandable volume filling device with a plurality of volume filling devices.

According to another embodiment, wherein the step of inserting a volume filling device comprises the step of inserting a combination of: a plurality of volume filling devices, and a solidifying liquid.

According to another embodiment, wherein the step of inserting the volume filling device comprises the step of inserting the volume filling device through an elongated tubular member adapted to enable the volume filling device to pass through the tubular member and into the pouch.

According to another embodiment, wherein the step of inserting the volume filling device comprises the step of inserting the tubular member into the pouch created by the stomach tissue, for enabling the insertion of the volume filling device through the tubular member.

According to yet another embodiment, wherein the method further comprises the step of closing the opening created in the pouch.

According to yet another embodiment, wherein the method further comprises the step of pulling, pushing and/or holding the stomach tissue.

According to yet another embodiment, wherein the step pulling, pushing and/or holding the stomach tissue comprises the step of pulling, pushing and/or holding using a device adapted to engage the stomach tissue by penetrating and/or clamping the stomach tissue.

According to yet another embodiment, wherein the step of pulling, pushing and/or holding the stomach tissue comprises the step of pulling, pushing and/or holding the stomach tissue using suction.

According to yet another embodiment, wherein the step of pulling or pushing the stomach tissue comprises the step of pulling or pushing the stomach tissue into an opening of a luminal space inside of the instrument.

According to yet another embodiment, wherein the step of pulling or pushing the stomach tissue into an opening of a luminal space inside of the instrument comprises the step of pushing or pulling the stomach tissue into a luminal space having a first cross-section being larger than an area of a second parallel cross-section placed in the opening of the luminal space, placed more distally in the luminal space or distally in the instrument.

The method according to another embodiment, wherein the unit is adapted to be placed more distal in the instrument than the luminal space, and wherein the method further comprises the step of creating a pouch when the at least one fixating member is delivered.

The method according to yet another embodiment, wherein the method further comprises the steps of: placing a first portion of the instrument inside of the stomach, placing a second portion of the instrument outside the stomach, such that the two layers of stomach tissue are placed between the first portion of the instrument and the second portion of the instrument.

The method according to another embodiment, wherein the step of placing the fixating members comprises the step of placing the fixating members above the gastroesophageal junction, for creating a tunnel between the esophagus and stomach above the junction.

The method according to yet another embodiment, wherein the method further comprises the step of: creating a pouch, and placing at least one first fixating member for fixating the pouch, and placing at least one second fixating member for fixating the pouch, such that the at least one second fixating member intersects the at least one first fixating member.

The method according to yet another embodiment, wherein the step of placing the at least one second fixating member, comprises the step of placing the at least one second fixating member perpendicularly to the at least one first fixating member.

The method according to yet another embodiment, wherein a holding portion is placed more distal than the unit, and wherein the method further comprises the step of removing the instrument after the at least one fixating member has been delivered.

A method for treating reflux disease according to another embodiment, wherein the method comprise the steps of delivering fixating members in a row for penetrating at least a part of the at least two layers of tissue, wherein the step of delivering fixating members comprises delivering fixating members having at least two different depths. This method configuration may be less invasive and may further decrease the risk of post-treatment problems to the patient. Human tissue is of varying thickness. Thus, fixating elements of varying depths may be more suited for its purpose in some cases.

The method according to another embodiment, wherein the step of delivering fixating members comprises the steps of: attaching at least two layers of human tissue using a first part of the row penetrating with a first depth into the tissue, and attaching three or more layers of tissue using a second part of the row penetrating with a second larger depth.

The method according to yet another embodiment, wherein the method further comprises the step of attaching four or five layers of tissue using the second part or a third part of the row.

The method according to yet another embodiment, wherein the method further comprises the steps of attaching at least two layers of stomach tissue using a first part of the row having fixating members with a first depth, and attaching one layer of esophageal tissue to the at least two layers of stomach tissue using a second part of the row with having fixating members penetrating the second part with a second and larger depth into the tissue, for attaching the one layer of esophageal tissue to the at least two layers of stomach tissue.

According to another embodiment, wherein the method further comprises the step of attaching one layer of esophageal tissue to three layers of stomach tissue.

According to yet another embodiment, wherein the method further comprises the step of attaching one layer esophageal tissue to four layers of stomach tissue.

According to yet another embodiment, wherein the method further comprises the step of attaching one layer esophageal tissue to at least two layers of stomach tissue above the gastroesophageal junction and above the cardia sphincter.

A method, according to another embodiment, of attaching stomach tissue, wherein the method comprises the step of delivering fixating members for attaching four layers of stomach tissue to each other, wherein the step of attaching four layers of stomach tissue to each other comprises the step of attaching two layers of the stomach serosa and two plus two layers of stomach mucosa to each other. Attaching four layers of stomach tissue together can in some cases be demanding to the endoscopist or surgeon. Further, attaching four layers in such manner may in some cases contribute to a more durable fixation better suited for its purpose.

A method, according to another embodiment, of attaching stomach tissue, wherein the method comprises the step of delivering fixating members for attaching a first two layers of stomach tissue to each other, serosa against serosa, and wherein the method further comprises the step of clamping four layers of stomach tissue to each other, including the first two layers, and when attaching the two layers serosa to serosa, clamping the other two layers of stomach tissue one layer on each side of the first two stomach layers mucosa to mucosa on each side of the first two layers, such that the fixating members only attaches the first inner stomach layers serosa to serosa. This method may be less invasive than other methods of stomach tissue attachment. A decreased invasiveness may lead to decreased risk of post-treatment problems, such as for example infection. Further, attaching inner stomach layers serosa to serosa may in some cases bring a fixation better suited for its purpose.

According to another embodiment, wherein the instrument comprises at least one unit adapted to deliver fixating members, the unit having a first and second part, and wherein the method either comprises the steps of: a. placing an esophagus part of the unit in the esophagus via the stomach cavity, and placing a stomach part of the unit in the stomach via the abdominal cavity, and b. placing an esophagus part of the unit in the esophagus via the throat, and placing a stomach part of the unit in the stomach via the throat, wherein the method further comprises the steps of: delivering the at least one fixating member between the esophagus part and the stomach part, penetrating at least three layers of stomach tissue and one layer of esophagus tissue above the gastro-esophageal junction, for attaching the esophagus and stomach above the junction, the placing of at least one fixating member comprises placing at least one fixating member attaching the outer serosa of the stomach wall to the outer esophagus wall above the gastro-esophageal junction, wherein the row of fixating members comprises two ends, and wherein the delivering of fixating members comprises delivering fixating members with a shorter depth going into the stomach tissue consisting of two layers of stomach tissue.

According to yet another embodiment, wherein the step of delivering the fixation members comprises the step of delivering the fixating members at a distance from the angle of His.

According to yet another embodiment, wherein the method comprises the steps of: delivering a first fixating member at a first point, for fixating the esophagus tissue to the stomach tissue, and delivering a second fixating member at a second point, wherein the first point is a point along a first esophagus surface length axis substantially parallel to the esophagus center axis, and wherein the second point is a point along a second esophagus surface length axis, substantially parallel to the first esophagus surface length axis and the esophagus center axis, wherein the first esophagus surface length axis is positioned at a distance from the second esophagus surface length axis, radially in relation to the esophagus center axis.

According to another embodiment, wherein the instrument is a laparoscopic instrument, and wherein the method comprises the step of inserting the instrument into the patient from the outside of the patient into the abdominal cavity and further into the stomach through an incision opening in the stomach wall, and operating on the stomach from the inside thereof.

According to yet another embodiment, wherein the instrument is a laparoscopic instrument, and wherein the method comprises the step of inserting the instrument into the patient from the outside of the patient into the abdominal cavity and operate on the stomach from the outside thereof.

According to yet another embodiment, wherein the step of inserting the laparoscopic instrument comprises the step of inserting the laparoscopic instrument through a trocar from the outside of the patient into the abdominal cavity.

According to yet another embodiment, wherein the instrument is a gastroscopic instrument, and wherein the method comprises the steps of inserting the instrument into the patient from the throat of the patient into the stomach cavity, and operating on the stomach from the inside thereof.

According to yet another embodiment, wherein the instrument is a gastroscopic instrument, and wherein the method comprises the steps of inserting the instrument into the patient from the throat of the patient, into the stomach cavity and further penetrating the stomach wall entering into the abdominal cavity, and operating on the stomach from the outside thereof.

According to another embodiment, wherein the step of delivering at least one fixating member comprises the step of delivering at least one fixating member for fixating the stomach tissue, without penetrating the mucosa layer of the stomach wall.

According to yet another embodiment, wherein the method further comprises the step of substantially reducing the volume of the stomach cavity.

According to yet another embodiment, wherein the method further comprises the step of clamping four layers of stomach for creating a pouch of stomach wall from the outside of the stomach with a closed space within the pouch, and wherein the method further comprises the step of delivering at least one fixating member for fixating the stomach tissue penetrating only two muscular layers of the stomach wall.

According to yet another embodiment, wherein the method further comprises the step of delivering fixating members penetrating at least two layers of stomach tissue, and wherein the method further comprises the step of placing the fixating members for creating a substantially closed space surrounded by stomach wall, and creating a pouch of stomach wall including the closed space.

According to yet another embodiment, wherein the method comprises the step of fixating a pouch formed as a tobacco pouch presenting a neck part of the pouch being wrinkled back and forth to create the closed space.

According to yet another embodiment, wherein the method comprises the step of fixating a pouch formed as a flat folded pouch with the fixating members placed in a curve to create the closed space.

The method according to another embodiment, wherein the method further comprises the step of predefining the pouch using vacuum suction for sucking the pouch into a predefined shape.

The method according to yet another embodiment, wherein the method comprises the step of predefining the pouch by clamping and pulling or pushing the stomach wall into a pre fixating pouch shape.

The method according to yet another embodiment, wherein the step of delivering a fixating member comprises the step of delivering a staple or suture.

The method according to yet another embodiment, wherein the step of delivering the at least one fixating member comprises the step of delivering a fixating member in form of a fixating member comprising a first and second state, wherein the method further comprises the steps of delivering the fixating member in the first state such that the fixating member penetrates tissue, and altering the fixation device to a second state in which the fixating member hinders the fixating member from penetrating tissue.

The method according to yet another embodiment, wherein the step of delivering the at least one fixating member comprises the step of delivering the fixating members using an instrument comprising a fixation member insertion device and a fixation member altering device, wherein the method further comprises the steps of: inserting the fixation member through the tissue, the fixation device being in the first state, and altering the state of the fixation member using the fixation member altering device such that the fixation member is placed in the second state.

The method according to yet another embodiment, wherein the step of delivering the at least one fixation member, comprises the step of delivering the at least one fixation member housed in an elongated member adapted to house the fixation member.

The method according to yet another embodiment, wherein the instrument further comprises at least one anvil member, and wherein the step of delivering the fixation member comprises the step of delivering the fixation member such that the fixation member penetrates the tissue of the esophagus and the tissue of the stomach and engages the anvil member.

The method according to yet another embodiment, wherein the method further comprises the step of positioning the anvil member within or outside of the stomach.

The method according to yet another embodiment, wherein the instrument is a gastroscopic instrument, and wherein the method further comprises the step of inserting the instrument into the esophagus and/or stomach through the throat of the patient for operating on the esophagus and/or stomach.

According to another embodiment, wherein the instrument is a laparoscopic instrument, and wherein the method further comprises the step if inserting the instrument into the patient from the outside of the patient into the abdominal cavity, further inserting the instrument into the esophagus and/or stomach through an incision opening in the stomach and/or esophagus wall of the patient, and operating on the esophagus and/or stomach from the inside thereof.

According to another embodiment, wherein the instrument is a laparoscopic instrument adapted to be inserted into the patient through a trocar, and wherein the method comprises the steps of inserting the instrument through a trocar from the outside of the patient into the abdominal cavity, and operating on the esophagus and/or stomach from the outside thereof.

According to another embodiment, wherein the at least one unit is adapted to deliver fixation members penetrating three layers of stomach tissue and one layer of esophagus tissue, and wherein the step of delivering fixating members comprises the step of: fixating three layers of stomach tissue and one layer of esophagus tissue to each other.

According to yet another embodiment, wherein the method further comprises the step of creating a pouch of stomach tissue, through the fixation of two layers of stomach tissue.

According to yet another embodiment, wherein the step of creating the pouch comprises the step of creating the pouch around a volume filling device placed in contact with the stomach tissue.

According to yet another embodiment, wherein the method further comprises the step of creating an opening in the pouch from the inside of the stomach using a cutting member.

The method according to another embodiment, wherein the step if delivering fixating members comprises the step of delivering fixation members penetrating two layers of stomach tissue and one layer of esophagus tissue, for fixating at least one of the two layers of stomach tissue and one layer of esophagus tissue to each other.

The method according to another embodiment, wherein the step if delivering fixating members comprises the step of delivering at least one fixation member penetrating four layers of stomach tissue and one layer of esophagus tissue, for fixating at least three layers of stomach tissue and one layer of esophagus tissue to each other.

The method according to yet another embodiment, wherein the method further comprises the step of creating a pouch of stomach tissue, through the fixation of two of the three layers of stomach tissue.

The method according to yet another embodiment, wherein the step of creating a pouch comprises the step of creating a pouch around a volume filling device placed in contact with the stomach tissue.

The method according to yet another embodiment, wherein the method further comprises the step of creating an opening in the pouch from the outside of the stomach using a cutting member.

The method according to yet another embodiment, wherein method further comprises the step of inserting a volume filling device into the pouch, after the creation of the pouch.

The method according to yet another embodiment, wherein the step of inserting a volume filling device comprises the step of inserting a solidifying liquid.

The method according to yet another embodiment, wherein the instrument comprises a conduit, and wherein the step of inserting the solidifying liquid comprises the step of inserting the conduit into the pouch created in the stomach tissue, and delivering the solidifying fluid into the pouch through the conduit.

The method according to yet another embodiment, wherein the instrument comprises at least one operable joint, and wherein the step of delivering the solidifying liquid to the pouch comprises delivering the solidifying liquid into a pouch created in the fundus area of the stomach.

The method according to yet another embodiment, wherein the step if inserting a volume filling device comprises the step of inserting a plurality of volume filling devices.

The method according to yet another embodiment, wherein the step of inserting a volume filling device comprising a plurality of volume filling devices, further comprises the step of connecting the plurality of volume filling devices to each other to form an interconnected volume filling device, after the plurality of volume filling devices have been inserted into the pouch.

The method according to yet another embodiment, wherein the instrument further comprises a tubular member adapted to be inserted into the pouch created of the stomach tissue, and wherein the step of delivering the plurality of volume filling devices into the pouch comprises the step of delivering the plurality of volume filling devices through the tubular member.

The method according to yet another embodiment, wherein the instrument comprises at least one operable joint, and wherein the step of delivering the plurality of volume filling devices through the tubular member comprises the step of delivering the plurality of volume filling devices to the pouch created in the fundus area of the stomach.

According to another embodiment, wherein the step of inserting a volume filling device comprises the step of inserting an expandable volume filling device.

According to another embodiment, wherein the method further comprises the step of filling the expandable volume filling device with a solidifying liquid.

According to yet another embodiment, wherein the method further comprises the step of filling the expandable volume filling device with a plurality of volume filling devices.

According to yet another embodiment, wherein the step of inserting a volume filling device comprises the step of inserting a combination of: a plurality of volume filling devices, and a solidifying liquid.

The method according to another embodiment, wherein the step of inserting the volume filling device comprises the step of inserting the volume filling device through an elongated tubular member adapted to enable the volume filling device to pass through the tubular member and into the pouch.

The method according to yet another embodiment, wherein the step of inserting the volume filling device comprises the step of inserting the tubular member into the pouch created by the stomach tissue, for enabling the insertion of the volume filling device through the tubular member.

The method according to yet another embodiment, wherein the method further comprises the step of closing the opening created in the pouch.

The method according to yet another embodiment, wherein the method further comprises the step of pulling, pushing and/or holding the stomach tissue.

The method according to yet another embodiment, wherein the step pulling, pushing and/or holding the stomach tissue comprises the step of pulling, pushing and/or holding using a device adapted to engage the stomach tissue by penetrating and/or clamping the stomach tissue.

The method according to yet another embodiment, wherein the step of pulling, pushing and/or holding the stomach tissue comprises the step of pulling, pushing and/or holding the stomach tissue using suction.

The method according to yet another embodiment, wherein the step of pulling or pushing the stomach tissue comprises the step of pulling or pushing the stomach tissue into an opening of a luminal space inside of the instrument.

The method according to yet another embodiment, wherein the step of pulling or pushing the stomach tissue into an opening of a luminal space inside of the instrument comprises the step of pushing or pulling the stomach tissue into a luminal space having a first cross-section being larger than an area of a second parallel cross-section placed in the opening of the luminal space, placed more distally in the luminal space or distally in the instrument.

The method according to yet another embodiment, wherein the unit is adapted to be placed more distal in the instrument than the luminal space, and wherein the method further comprises the step of creating a pouch when the at least one fixating member is delivered.

The method according to yet another embodiment, wherein the method further comprises the steps of: placing a first portion of the instrument inside of the stomach, placing a second portion of the instrument outside the stomach, such that the two layers of stomach tissue are placed between the first portion of the instrument and the second portion of the instrument.

The method according to yet another embodiment, wherein the step of placing the fixating members comprises the step of placing the fixating members above the gastroesophageal junction, for creating a tunnel between the esophagus and stomach above the junction.

The method according to yet another embodiment, wherein the method further comprises the step of: creating a pouch, and placing at least one first fixating member for fixating the pouch, and placing at least one second fixating member for fixating the pouch, such that the at least one second fixating member intersects the at least one first fixating member.

The method according to yet another embodiment, wherein the step of placing the at least one second fixating member, comprises the step of placing the at least one second fixating member perpendicularly to the at least one first fixating member.

The method according to yet another embodiment, wherein a holding portion is placed more distal than the unit, and wherein the method further comprises the step of removing the instrument after the at least one fixating member has been delivered.

According to another embodiment, the method further comprises the steps of: placing at least one anvil member or a unit for delivering fixation members placed on a separate gastro-esophageal tube or esophageal tube or a gastroscope adapted to be placed in the esophagus with the unit above the cardia, and introducing the instrument into the abdominal cavity for clamping on both sides of the esophagus at the position of the unit, and involving the unit in the fixation of stomach tissue to the right wall of the esophageal tissue, and delivering fixation members such that the fixation members penetrates the tissue of the stomach and the tissue of the wall of the esophagus and engages with the unit for attaching stomach and esophageal tissue above the cardia sphincter. This method may be less time consuming than some other methods of fastening stomach tissue to esophageal tissue above the cardia sphincter. Further, by placing the anvil or unit for delivering fixation members on a separate gastro-esophageal tube or esophageal tube or a gastroscope adapted to be placed in the esophagus may in some cases increase the accuracy of deliverance of fixation members.

The method according to another embodiment, wherein the method is a method of treating reflux disease, wherein the method comprises the steps of: placing an elongated member in the esophagus, placing at least one fixating member engaging the elongated member for fixating the esophagus tissue to the stomach tissue, and placing a second elongated member spaced apart from the first elongated member, such that the first and second elongated member clamps the esophagus from the outside thereof including stomach tissue from the outside thereof, at a position above the cardia sphincter and below the diaphragm muscle. This method may help the surgeon to perform the method of treating reflux disease in a manner that may lead to less human error and a better treatment result. Erroneous treatment of reflux may bring great discomfort to the patient and induce cost to correct. Accurate positioned fixation members may, for some methods, be of importance.

The method for treating reflux disease according to another embodiment, wherein the method comprises the steps of: placing a first elongated member comprising a first part of a unit in the abdominal cavity outside of the stomach and further adapted to clamp the esophagus from the outside thereof including clamping the stomach fundus tissue from the outside thereof, placing a second elongated member comprising a second part of the unit, placing one or more fixating members, at the position of the esophagus above the cardia sphincter and below the diaphragm muscle, delivering fixating members for attaching and fixating the folded stomach fundus wall including two layers of stomach fundus wall to one layer of esophagus wall, such that the one or more fixating member, with a depth attaching only one layer of the two clamped layers of esophageal tissue, attaches only one layer of the two clamped layers of esophageal tissue. A method where one or more fixation members attaches two layers of clamped esophagus wall may in some cases expose the patient risks and discomfort. This method can in some cases decrease the risk of attaching two layers of clamped esophagus and thus in some cases for treating reflux disease bring a less invasive, securer and faster treatment method.

A method, according to another embodiment, of attaching the esophagus to the stomach, wherein the method comprises the steps of: placing a separate gastro-esophageal tube or esophageal tube or a gastroscope comprising at least one anvil member or a unit for delivering fixation members, introducing an instrument into the abdominal cavity for clamping on both sides of the esophagus at the position of the unit or anvil, fixating the stomach tissue to the wall of the esophageal tissue, delivering fixation members such that the fixation members penetrates the tissue of the stomach and the tissue of the wall of the esophagus and engages with the anvil or unit for attaching the stomach and esophageal tissue above the cardia sphincter. Fixating esophagus tissue to stomach tissue may require multiple steps and can in some cases be time consuming. Using the positioning of the anvil and/or unit for delivering fixation members according to this method may be increase accuracy and be less invasive. Thus, this method may for some treatments increase the accuracy of attaching esophagus tissue to stomach tissue at an intended position suited for its purpose.

A method of attaching esophagus to stomach tissue for treating reflux disease, wherein the method comprises the steps of: placing a first elongated member comprising a first part of a unit in the esophagus for engaging in fixating the esophagus tissue to the stomach tissue, and placing a second elongated member spaced apart from the first elongated member comprising a second part of the unit in the abdominal cavity outside of the stomach clamping the esophagus from the outside thereof including stomach tissue, at the position of the first part of the unit placed above the cardia sphincter and below the diaphragm muscle, using the second elongated member, and delivering the fixating members attaching esophagus tissue to stomach tissue. Fixating esophagus tissue to stomach tissue may in some treatments require multiple steps and can in some cases be time consuming. Thus, this method may for some treatments increase the accuracy of delivering the fixating members attaching esophagus tissue to stomach tissue at an intended position suited for its purpose. This can potentially lead to increased rate of successful treatments of reflux.

A method for treating reflux disease, according to another embodiment , wherein the method comprises: placing a first elongated member comprising a first part of a unit in the abdominal cavity for engaging in fixating the esophagus tissue to the stomach tissue, and placing a second elongated member spaced apart from the first elongated member comprising a second part of the unit in the abdominal cavity outside of the stomach clamping the esophagus from the outside thereof including fundus tissue, at the position of the first part of the unit placed above the cardia sphincter and below the diaphragm muscle, using the second elongated member, and delivering fixating members attaching esophagus tissue to stomach tissue attaching and fixating the folded stomach fundus wall including two layers of stomach fundus wall to one layer of esophagus wall fixating member using a fixating member with a depth attaching only one layer of the two clamped layers of esophageal tissue. A method where one or more fixation members attaches two layers of clamped esophagus wall may in some cases expose the patient risks and discomfort. This method can in some cases decrease the risk of attaching two layers of clamped esophagus and thus in some cases for treating reflux disease bring a less invasive, securer and faster treatment method.

A method, according to another embodiment, of creating a pouch in the stomach from the outside thereof, wherein the method comprises the step of pulling the stomach wall from the outside thereof pre shaping a pouch or impression in the stomach wall, clamping the stomach wall using a clamping member to complete the pouch or pre shaping a closure of the impression, and fixating a closure of the pouch or stomach impression to fulfill the pouch creation when the instrument is used in the abdominal cavity. A pouch created in the stomach may in some cases decrease the risk of post-treatment problems, where one example is infection.

According to another embodiment, wherein the step of pulling the stomach tissue comprises the step using a vacuum suction to hold the pouch or impression in place.

According to yet another embodiment, wherein the step of inserting the laparoscopic instrument comprises the step of inserting the laparoscopic instrument through a trocar from the outside of the patient into the abdominal cavity.

According to yet another embodiment, wherein the method comprises the step of delivering at least one fixating member for fixating the stomach tissue without penetrating the mucosa layer of the stomach wall.

According to yet another embodiment, wherein the method further comprises the step of substantially reducing the volume of the stomach cavity.

According to yet another embodiment, wherein the method further comprises the step of clamping four layers of stomach for creating a pouch of stomach wall from the outside of the stomach with a closed space within the pouch, and wherein the method further comprises the step of delivering at least one fixating member for fixating the stomach tissue penetrating only two muscular layers of the stomach wall.

According to yet another embodiment, wherein the method further comprises the step of delivering fixating members penetrating at least two layers of stomach tissue, and wherein the method further comprises the step of placing the fixating members for creating a substantially closed space surrounded by stomach wall, and creating a pouch of stomach wall including the closed space.

The method according to another embodiment, wherein the method comprises the step of fixating a pouch formed as a tobacco pouch presenting a neck part of the pouch being wrinkled back and forth to create the closed space.

The method according to another embodiment, wherein the method comprises the step of fixating a pouch formed as a flat folded pouch with the fixating members placed in a curve to create the closed space.

The method according to another embodiment, wherein the method further comprises the step of predefining the pouch using vacuum suction for sucking the pouch into a predefined shape.

The method according to another embodiment, wherein the method comprises the step of predefining the pouch by clamping and pulling or pushing the stomach wall into a pre fixating pouch shape.

The method according to another embodiment, wherein the step of delivering a fixating member comprises the step of delivering a staple or suture.

The method according to another embodiment, wherein the method further comprises the step of creating a pouch of stomach tissue, through the fixation of two layers of stomach tissue.

The method according to another embodiment, wherein the step of creating the pouch comprises the step of creating the pouch around a volume filling device placed in contact with the stomach tissue.

According to another embodiment, wherein the step if delivering fixating members comprises the step of delivering fixation members penetrating two layers of stomach tissue and one layer of esophagus tissue, for fixating at least one of the two layers of stomach tissue and one layer of esophagus tissue to each other.

According to yet another embodiment, wherein the step if delivering fixating members comprises the step of delivering at least one fixation member penetrating four layers of stomach tissue and one layer of esophagus tissue, for fixating at least three layers of stomach tissue and one layer of esophagus tissue to each other.

According to yet another embodiment, wherein method further comprises the step of inserting a volume filling device into the pouch, after the creation of the pouch.

According to yet another embodiment, wherein the step of inserting a volume filling device comprises the step of inserting a solidifying liquid.

According to yet another embodiment, wherein the instrument comprises a conduit, and wherein the step of inserting the solidifying liquid comprises the step of inserting the conduit into the pouch created in the stomach tissue, and delivering the solidifying fluid into the pouch through the conduit.

According to yet another embodiment, wherein the instrument comprises at least one operable joint, and wherein the step of delivering the solidifying liquid to the pouch comprises delivering the solidifying liquid into a pouch created in the fundus area of the stomach.

According to yet another embodiment, wherein the step if inserting a volume filling device comprises the step of inserting a plurality of volume filling devices.

According to yet another embodiment, wherein the step of inserting a volume filling device comprising a plurality of volume filling devices, further comprises the step of connecting the plurality of volume filling devices to each other to form an interconnected volume filling device, after the plurality of volume filling devices have been inserted into the pouch.

According to yet another embodiment, wherein the instrument further comprises a tubular member adapted to be inserted into the pouch created of the stomach tissue, and wherein the step of delivering the plurality of volume filling devices into the pouch comprises the step of delivering the plurality of volume filling devices through the tubular member.

According to yet another embodiment, wherein the instrument comprises at least one operable joint, and wherein the step of delivering the plurality of volume filling devices through the tubular member comprises the step of delivering the plurality of volume filling devices to the pouch created in the fundus area of the stomach.

According to yet another embodiment, wherein the step of inserting a volume filling device comprises the step of inserting an expandable volume filling device.

According to another embodiment, wherein the method further comprises the step of filling the expandable volume filling device with a solidifying liquid.

According to another embodiment, wherein the method further comprises the step of filling the expandable volume filling device with a plurality of volume filling devices.

According to another embodiment, wherein the step of inserting a volume filling device comprises the step of inserting a combination of: a plurality of volume filling devices, and a solidifying liquid.

According to another embodiment, wherein the step of inserting the volume filling device comprises the step of inserting the volume filling device through an elongated tubular member adapted to enable the volume filling device to pass through the tubular member and into the pouch.

According to yet another embodiment, wherein the step of inserting the volume filling device comprises the step of inserting the tubular member into the pouch created by the stomach tissue, for enabling the insertion of the volume filling device through the tubular member.

According to yet another embodiment, wherein the method further comprises the step of closing the opening created in the pouch.

According to yet another embodiment, wherein the method further comprises the step of pulling, pushing and/or holding the stomach tissue.

According to yet another embodiment, wherein the step pulling, pushing and/or holding the stomach tissue comprises the step of pulling, pushing and/or holding using a device adapted to engage the stomach tissue by penetrating and/or clamping the stomach tissue.

The method according to another embodiment, wherein the step of pulling, pushing and/or holding the stomach tissue comprises the step of pulling, pushing and/or holding the stomach tissue using suction.

The method according to another embodiment, wherein the method comprises one or more of the following operational method steps: introducing the instrument into the throat, passing down the esophagus, placing an anvil or unit for delivery of fixating members in the esophagus between the cardia and the diaphragm level, for engaging in the fixation of the esophagus to the stomach tissue, passing down the esophagus and additionally further down into the stomach, filling the stomach with gas to expand the stomach, sucking fluid from the stomach, looking at a guiding vision when the instrument comprising a camera, engaging the instrument with the stomach, creating and suturing at least one pouch of the stomach wall, filling the at least one pouch with a fluid and/or volume filling device or two or more volume filling devices, deliver a plurality of volume filling devices into the pouch created in the stomach tissue through a tubular member, passing through the stomach wall with the instrument, passing through the stomach wall with the instrument for the placement of a volume filling device on the outside of the stomach wall , passing through the stomach wall with the instrument for the placement of a tube allowing placement of a subcutaneous injection port, placing an subcutaneous injection port, suturing or stapling the stomach wall from the inside thereof to the esophagus, suturing or stapling the stomach wall to stomach wall from the inside of the stomach, engaging the instrument with the esophagus, suturing or stapling one layer of stomach tissue to one layer of esophageal tissue, suturing or stapling two layers of stomach tissue to one layer of esophageal tissue, suturing or stapling three layers of stomach tissue to one layer of esophageal tissue, suturing or stapling four layers of stomach tissue to one layer of esophageal tissue, suturing or stapling one or more layers of stomach tissue to two or more positions on the esophageal tissue, the esophagus having an esophagus center axis, the esophagus further having an inner and outer substantially cylindrical surface extending radially in relation to the esophagus center axis and wherein the stomach tissue is attached to esophageal tissue both at a first point along a first esophagus surface length axis, substantially parallel to the esophagus center axis and at a second point along a second esophagus surface length axis, substantially parallel to the esophagus center axis, at a distance from the first esophagus surface length axis, radially in relation to the esophagus center axis, delivering fixating members by a unit placed on the instrument, penetrating at least one layer of stomach tissue and one layer of esophagus tissue with the fixating members, placing the fixating members above the gastro-esophageal junction for creating a tunnel between the esophagus and stomach above the junction, placing an esophagus part in the esophagus and a stomach part in the stomach, placing the fixating member substantially between the stomach and esophagus part, inserting the instrument into the main stomach cavity through the cardia and adapted to direct the instrument in cranial direction to reach a position of the unit above the junction, allowing the tunnel a substantially unrestricted contraction and release of the cardia closing sphincter muscle placed in the junction, when such a tunnel has been created. Treating reflux based on one or several of the steps above may bring decreased invasiveness and/or increased treatment efficiency and/or increased accuracy of fixation which may be better adapted for its purpose. One or a combination of these steps may further increase the treatment success rate and in some cases lessen the risk of erroneous treatment.

The method according to another embodiment, wherein the method comprises one or more of the following operational method steps: cutting the skin of a patient, creating an opening in the abdominal wall of the patient Introducing the instrument into the abdominal cavity through the opening in the abdominal wall, engaging the instrument with the stomach, pulling down into the stomach wall for creating at least one pre-shaped pouch of the stomach wall, clamping the stomach wall for creating at least one pre-shaped pouch of the stomach wall, suturing or stapling at least one pouch in the stomach wall, filling the at least one pouch with a fluid and/or volume filling device or two or more volume filling devices, deliver a plurality of volume filling devices into the pouch created in the stomach tissue through a tubular member, passing through the stomach wall into the stomach with the instrument, passing through the stomach wall with the instrument for the placement of a volume filling device on the inside of the stomach wall, passing through the stomach wall with the instrument for suturing the stomach wall to the esophagus wall, placing a volume filling device on the outside of the stomach wall, invaginating the volume filling device in the stomach wall placing a subcutaneous injection port, suturing or stapling the stomach wall to stomach wall from the outside of the stomach, suturing or stapling the stomach wall to stomach wall from the outside of the stomach without penetrating the mucosa, suturing or stapling two layers of stomach wall to one or two layers of stomach wall, engaging the instrument with the esophagus, clamping the on both sides of the esophagus for fixating one layer of esophageal wall to stomach tissue, clamping the on both sides of the esophagus and the stomach fundus wall for fixating one layer of esophageal wall to one or two layers of stomach tissue, introducing a tube or a gastroscopic instrument into the esophagus comprising an anvil member or a fixating delivery member involving in the fixation of the esophagus to the stomach, coordinating the position of the anvil member or a fixating delivery member inside the esophagus to the instrument clamping around the esophagus, suturing or stapling one layer of stomach tissue to one layer of esophageal tissue, suturing or stapling two layers of stomach tissue to one layer of esophageal tissue, suturing or stapling three layers of stomach tissue to one layer of esophageal tissue, suturing or stapling four layers of stomach tissue to one layer of esophageal tissue, stapling using staplers of different stapling depths at different positions in a stapler row, stapling stomach to esophagus with one first stapler depth and stapling stomach to stomach with a second smaller stapler depth, stapling a pouch with stomach to stomach sutures in a stapler row, further comprising stapling the esophagus with staplers of a larger depth included as a part of the stapler row, suturing or stapling one or more layers of stomach tissue to two or more positions on the esophageal tissue, the esophagus having an esophagus center axis, the esophagus further having an inner and outer substantially cylindrical surface extending radially in relation to the esophagus center axis and wherein the stomach tissue is attached to esophageal tissue both at a first point along a first esophagus surface length axis, substantially parallel to the esophagus center axis and at a second point along a second esophagus surface length axis, substantially parallel to the esophagus center axis, at a distance from the first esophagus surface length axis, radially in relation to the esophagus center axis, delivering fixating members by a unit placed on the instrument, penetrating at least one layer of stomach tissue and one layer of esophagus tissue with the fixating members, placing the fixating members above the gastro-esophageal junction for creating a tunnel between the esophagus and stomach above the junction, allowing the tunnel a substantially unrestricted contraction and release of the cardia closing sphincter muscle placed in the junction, when such a tunnel has been created, placing an esophagus part in the esophagus and a stomach part in the stomach via an introduction into the stomach cavity, placing the fixating member substantially between the stomach and esophagus part, inserting the instrument into the main stomach cavity below the junction and adapted to direct the instrument in cranial direction to reach a position of the unit above the junction, operating a joint comprised in the instrument, for enabling the instrument to be inserted into the main stomach cavity bending the joint in a direction to reach a position of the part of the unit in the esophagus above the junction. Treating reflux based on one or several of the steps above may bring decreased invasiveness and/or increased treatment efficiency and/or increased accuracy of fixation which may be better adapted for its purpose. One or a combination of these steps may further increase the treatment success rate and in some cases lessen the risk of erroneous treatment.

The method according to any one of the embodiments herein could comprise one or more of the following laparoscopic operational method steps: cutting the skin of a patient introducing a tube through the abdominal wall, filling a fluid or gas into the abdominal cavity, introducing two or more trocars into the abdominal cavity, introducing a camera into the abdominal cavity through one of the trocars, introducing the instrument into the abdominal, cavity through a trocar, engaging the instrument with the stomach, pulling down into the stomach wall for creating at least one pre-shaped pouch of the stomach wall, clamping the stomach wall for creating at least one pre-shaped pouch of the stomach wall, suturing or stapling at least one pouch in the stomach wall, filling the at least one pouch with a fluid and/or a volume filling device or two or more volume filling devices, deliver a plurality of volume filling devices into the pouch created in the stomach tissue through a tubular member, passing through the stomach wall into the stomach with the instrument, passing through the stomach wall with the instrument for the placement of a volume filling device on the inside of the stomach wall, passing through the stomach wall with the instrument for suturing the stomach wall to the esophagus wall, placing a volume filling device on the outside of the stomach wall, invaginating the volume filling device in the stomach wall placing a subcutaneous injection port, suturing or stapling the stomach wall to stomach wall from the outside of the stomach, suturing or stapling two layers of stomach wall to one or two layers of stomach wall, suturing or stapling the stomach wall to stomach wall from the outside of the stomach without penetrating the mucosa, engaging the instrument with the esophagus, clamping the on both sides of the esophagus for fixating one layer of esophageal wall to stomach tissue, clamping the on both sides of the esophagus and the stomach fundus wall for fixating one layer of esophageal wall to one or two layers of stomach tissue, introducing a tube or a gastroscopic instrument into the esophagus comprising an anvil member or a fixating delivery member involving in the fixation of the esophagus to the stomach, coordinating the position of the anvil member or a fixating delivery member inside the esophagus to the instrument clamping around the esophagus, suturing or stapling one layer of stomach tissue to one layer of esophageal tissue, suturing or stapling two layers of stomach tissue to one layer of esophageal tissue, suturing or stapling three layers of stomach tissue to one layer of esophageal tissue, suturing or stapling four layers of stomach tissue to one layer of esophageal tissue, stapling using staplers of different stapling depths at different positions in a stapler row, stapling stomach to esophagus with one first stapler depth and stapling stomach to stomach with a second smaller stapler depth, stapling a pouch with stomach to stomach sutures in a stapler row, further comprising stapling the esophagus with staplers of a larger depth included as a part of the stapler row, suturing or stapling one or more layers of stomach tissue to two or more positions on the esophageal tissue, the esophagus having an esophagus center axis, the esophagus further having an inner and outer substantially cylindrical surface extending radially in relation to the esophagus center axis and wherein the stomach tissue is attached to esophageal tissue both at a first point along a first esophagus surface length axis, substantially parallel to the esophagus center axis and at a second point along a second esophagus surface length axis, substantially parallel to the esophagus center axis, at a distance from the first esophagus surface length axis, radially in relation to the esophagus center axis, delivering fixating members by a unit placed on the instrument, penetrating at least one layer of stomach tissue and one layer of esophagus tissue with the fixating members, placing the fixating members above the gastro-esophageal junction for creating a tunnel between the esophagus and stomach above the junction, allowing the tunnel a substantially unrestricted contraction and release of the cardia closing sphincter muscle placed in the junction, when such a tunnel has been created, placing an esophagus part in the esophagus and a stomach part in the stomach via an introduction into the stomach cavity, placing the fixating member substantially between the stomach and esophagus part, inserting the instrument into the main stomach cavity below the junction and adapted to direct the instrument in cranial direction to reach a position of the unit above the junction, operating a joint comprised in the instrument, for enabling the instrument to be inserted into the main stomach cavity bending the joint in a direction to reach a position of the part of the unit in the esophagus above the junction. Treating reflux based on one or several of the steps above may bring decreased invasiveness and/or increased treatment efficiency and/or increased accuracy of fixation which may be better adapted for its purpose. One or a combination of these steps may further increase the treatment success rate and in some cases lessen the risk of erroneous treatment.

The apparatus that may be used together with the instruments and methods outlined above may comprise many different aspects and embodiments. Different embodiments are outlined below:
The invention relates to an apparatus for treating reflux disease in a human or animal mammal patient comprising an implantable movement restriction device having an outer surface with an elongated shape adapted to be at least partly be invaginated by a stomach wall portion of a patient.

In one embodiment the movement restriction device has, when implanted in a patient, a proximal part and a distal part, and the device is adapted to rest at least partially with the outer surface of its proximal part against the patient's stomach fundus wall, in a position between the patient's diaphragm and the fundus wall, such that movement of the cardiac notch of the patient's stomach towards the patient's diaphragm is restricted, when the movement restriction device is implanted in the patient, to thereby prevent the cardia from sliding through the patient's diaphragm opening into the patient's thorax, so as to maintain the supporting pressure against the patient's cardia sphincter muscle exerted from the patient's abdomen. The device is also adapted to stabilize and hold the proximal part by the distal part adapted to be substantially invaginated in the stomach wall in order to prevent the cardia from sliding through the patient's diaphragm opening into the patient's thorax. The terms "proximal" and "distal" have the usual anatomical meaning.

In the present context proximal and distal refer to parts of the movement restriction in its implanted position. The length of the distal invaginated part of the elongated movement restriction device is sufficiently long to stabilize the proximal part of the elongated movement restriction device to prevent the cardia from sliding through the patient's diaphragm opening into the patient's thorax. The circumference of the distal invaginated part of the elongated movement restriction device is such that it is stabilizing the proximal part of the elongated movement restriction device to prevent the cardia from sliding through the patient's diaphragm opening into the patient's thorax. The proximal part of the movement restriction device has a size of at least 125 mm³ and a circumference of at least 15 mm.

Preferably, the apparatus comprises an implantable first fixation device that secures the proximal part of the movement restriction device in a position that restricts the movement of the cardiac notch of the stomach towards the patient's diaphragm, with the outer surface of the movement restriction device substantially contacting the patient's stomach fundus wall. The first fixation device may include sutures or staples that attach together portions of the fundus stomach wall that enclose the proximal part of the movement restriction device to secure the movement restriction device in said position, i.e., the movement restriction device is at least partly placed in an invaginated space. Thus, by affixing the proximal part of the implantable movement restriction device indirectly in this manner, no suturing between the movement restriction device and tissue is required, which, in turn, further reduces the risk for complications. Keeping the proximal part of the movement restriction device in place in this manner has resulted in an elastic suspension with improved long term properties.

The first fixation device, such as sutures or staples, attach together portions of the fundus stomach wall so at to invaginate the proximal part of the movement restriction device from either inside or outside of the patient's stomach wall.

Alternatively, a tissue growth promoting structure may be sutured to the stomach wall with a relatively large contact surface towards the stomach. The relatively large surface of the structure, such as a net, will allow for in-growth of human tissue for holding the proximal part of the movement restriction device in place over the long term. The tissue growth promoting structure may comprise sutures or staples that attach the net like structure to the fundus stomach wall.

In addition to affixing the proximal part of the movement restriction device to the stomach wall a second fixation device may be employed. The second fixation device can be used to affix the proximal part of movement restriction device in relation to the cardia. For example, the proximal part of the movement restriction device can be affixed in a position above the cardia, between the cardia and the diaphragm muscle, by a second direct or indirect affixation of the proximal part of the movement restriction device via the fundus stomach wall. The second fixation device may secure, indirectly or directly, the proximal part of the movement restriction device to the esophagus close to the patient's angle of His. The second fixation device suitably includes a plurality of sutures or staples that attach the fundus wall and a wall of the patient's esophagus to hold the movement restriction device in said position.

The apparatus may also comprise a third fixation device that secures, indirectly or directly, the proximal part of the movement restriction device to the patient's diaphragm muscle or other muscle tissue. The third fixation device suitably comprises a plurality of sutures or staples that attach the fundus wall and the diaphragm muscle or other muscle tissue to hold the movement restriction device in said position.

The proximal part of the movement restriction device may be adapted to be substantially or completely invaginated by the patient's stomach fundus wall, and be placed either on the inside or outside of the stomach fundus wall.

The proximal part of the movement restriction device may be adapted to be placed on the outside of the patient's stomach wall, such that the stomach cavity is substantially reduced, by a volume substantially exceeding the volume of the movement restriction device.

At least a part of the proximal part of the movement restriction device may be made of a material which is destructible or not destructible by stomach acid.

In an embodiment, the proximal part of the movement restriction device is inflatable and adapted to be inflated with a gel or fluid. A fluid or gel receiving member for receiving fluid to inflate the movement restriction device may be provided.

In one embodiment the apparatus according to the invention further comprise an adjustment device for adjusting at least the proximal part of the movement restriction device. For this purpose, the movement restriction device can comprise a body, the size of which is hydraulically adjustable, and the adjustment device can comprise a hydraulic fluid reservoir that, when implanted in the patient, is connected to the body, and wherein the body's size is non-invasively regulated by manually pressing the hydraulic fluid reservoir so as to adjust the amount of hydraulic fluid supplied to the body and thereby the body's size. The apparatus can further comprise a hydraulic regulation device comprising at least one chamber that, when implanted in the patient, is invaginated in the patient's stomach wall with the body and connected to the body, and wherein the amount of hydraulic fluid contained in the body is non-invasively regulated by distributing fluid between the hydraulic reservoir and the at least one chamber. Preferably, the at least one chamber is, when implanted in the patient, filled with the hydraulic fluid using a pump in the reservoir so as to stretch the fundus wall to create satiety in the patient. Further, the adjustment device further can comprise a reverse servo, wherein a small volume of fluid in the reservoir is compressed with a higher force and the chamber creates a movement of a larger total volume with less force per unit of volume. In one embodiment, the body forms a first chamber, further comprising at least one additional body forming a second chamber smaller than the first chamber, the first and second chambers being in contact with each other, preferably in fluid communication with each other. The hydraulic reservoir is preferably adapted to be placed subcutaneously in the patient and the hydraulic reservoir is preferably adapted to be placed in the patient's abdomen. The hydraulic reservoir may have a wall defining the volume thereof, and the volume of the hydraulic reservoir is regulated by moving a wall portion of the wall of the hydraulic reservoir. The apparatus can comprise a motor for moving the wall portion. The hydraulic regulation device can comprise a pump, and the hydraulic reservoir is regulated by the pump pumping fluid between the hydraulic reservoir and said at least one chamber. A mechanical device can operatively be connected to the hydraulic regulation device to be moved as the hydraulic regulation device is operated. In one embodiment, at least the proximal part of the movement restriction device is mechanically regulated. The apparatus can further comprise a motor for mechanically regulating the movement restriction device.

In one embodiment, the apparatus according further comprises a second body that, when implanted in the patient with the body, fills two volumes, respectively, at two different parts of the patient's the stomach, thereby affecting the patient's reflux. The reflux disease treatment device is adapted to be postoperatively and non-invasively regulated, and adapted to be regulated from time to time such that at a first time one of the filling bodies fills the volume at one of the parts of the stomach and at a second time the other of the filling bodies fills the volume at the other part of the stomach.

In one embodiment, the apparatus according to the invention comprises an adjustment device for adjusting the size and/or shape of the movement restriction device. The size of the movement restriction device can be hydraulically adjustable, and the adjustment device can comprises a hydraulic fluid reservoir that, when implanted in the patient, is connected to the movement restriction device, and the size of the movement restriction device can be non-invasively regulated by moving hydraulic fluid between the hydraulic fluid reservoir and the movement restriction device. The movement restriction device may be seen as a body. The apparatus can further comprise a hydraulic regulation device comprising at least one chamber that, when implanted in the patient, is invaginated in the patient's stomach wall with the body and connected to the body, and wherein the amount of hydraulic fluid contained in the body is non-invasively regulated by distributing fluid between the hydraulic reservoir and the at least one chamber. The at least one chamber can, when implanted in the patient, be filled with the hydraulic fluid using a pump in the reservoir so as to stretch the fundus wall to create satiety in the patient. Further, the adjustment device can comprise a reverse servo comprising three adjustable reservoirs with hydraulic fluid, wherein a small volume of fluid in a first reservoir placed subcutaneously, being part of a first closed system including a second reservoir, is compressed with a high force per area unit for moving a small volume of hydraulic fluid, and wherein the second reservoir affects a larger volume of hydraulic fluid in a third reservoir, the third reservoir being part of a second closed system having larger volume than said first reservoir, thereby creating a movement of a larger total volume of hydraulic fluid with less force per area unit.

In a special embodiment, the movement restoration device as outlined in the previous sections comprises two or more movement restriction device segments, preferably comprising more than three segments, adapted to be assembled to an implantable assembled movement restriction device of a controlled size involving at least the proximal part of the movement restriction device. The assembled movement restriction device is adapted to rest with at least a part of its outer surface against the patient's stomach fundus wall, in a position between the patient's diaphragm and the fundus wall, such that movement of the cardiac notch of the patient's stomach towards the patient's diaphragm is restricted, when the movement restriction device is implanted in the patient, to thereby prevent the cardia from sliding through the patient's diaphragm opening into the patient's thorax, so as to maintain the supporting pressure against the patient's cardia sphincter muscle exerted from the patient's abdomen. The assembled movement restriction device preferably is adapted to disassemble into its segments if it leaves its implanted position of at least partially contacting the stomach fundus wall in a position between the diaphragm and the fundus wall. Preferably, the assembled movement restriction device is adapted to be invaginated in the stomach wall, and to disassemble into its segments if it leaves its implanted stomach position including penetrating the stomach wall to retain a position inside the stomach. The segments are preferably adapted to separately pass through the food passage way, thereby reducing risk of causing obstruction/ileus in the patient's intestine. The movement restriction device segments can be adapted to pass through a trocar, for assembly and implantation of said movement restriction device into the abdominal cavity. The movement restriction device segments can have a flexible outer shape adapted to pass through a trocar. The movement restriction device segments can be adapted to have a shape allowing them to be assembled into said movement restriction device, when implanted. In one embodiment, the movement restriction device segments are hollow with a flexible outer surface. The movement restriction device segments can be adapted to be filled with at least one of a fluid a foam, a gel or a fluid that hardens to a solid material. In one embodiment the movement restriction device segments are solid. It is preferred that the movement restriction device segments are adapted to temporary be holding their assembled position, preferably by the invaginated stomach wall, or alternatively by an adhesive.

For its assembly, the movement restriction device is provided with at least one assembly element that sufficiently fits with at least one assembly element of another segment, so the segments by fitting assembly elements can be assembled into the implantable movement restriction device. Preferably, the segments for this purpose comprise a core part and a plurality of outer parts, and preferably, the at least one assembly element is selected among sufficiently fitting flanges and slits. The core part is adapted to receive and assemble the outer elements into an implantable movement restriction device, and preferably the core part has assembly slits adapted to receive corresponding assembly flanges of the outer parts when assembling the movement restriction device. In one embodiment the slits are distributed around the outer peripheral area of the core part. The outer parts are then provided with flanges sufficiently matching the slits to assemble the device. In another embodiment, the at least one assembly element immobilizes each of the movement restriction device segments to a core part along a first plane, and wherein movement, and wherein the movement restriction device segments and the core part further comprises a second assembly element, which following the assembly of said segments and core part, immobilize each segment and core part along a second plane in an angle to said first plane. For example, the first plane and the second plane can be substantially perpendicular. The second assembly element comprises mating elements, preferably with matching protrusions and recesses provided on the movement restriction device segments and the core part, while the at least one assembly element further comprises protrusions and recesses. Preferably, the at least one assembly element comprises an assembly slit in the core part and an assembly flange in a segment, and wherein a mating element comprises a protrusion in said slit and a recess in said flange; or alternatively, the at least one assembly element comprises an assembly flange in the core part and an assembly slit in a segment, and wherein the a mating element comprises a protrusion in said slit and a recess in said flange.

Other features of the proximal part of the movement restriction device will be described in the following part of description. These features can be combined with any of the following features related to the distal part of the movement restriction device.

In an advantageous embodiment of the apparatus now adapted to also treat obesity, the body is adjustable in size and invaginated in the patient's fundus stomach wall. As a result, the body stretches the patient's stomach fundus wall when the size thereof is increased, thereby creating satiety in a patient also suffering from obesity. At least two implantable adjustable stretching devices may be provided to stretch different parts of the patient's stomach wall, to thereby treat obesity by efficiently affecting the patient's appetite. The two stretching devices are suitably regulated from outside of the patient's body, whereby a first of the stretching devices is regulated at a first time to stretch a first part of the patient's stomach wall and a second of the stretching devices is regulated at a second time to stretch a second part of the patient's stomach wall.

The apparatus of the present invention in any form outlined in the previous sections can be further adapted to treat obesity together with reflux disease as it is common that a patient suffers from both complications. For this purpose the distal part of the movement restriction device is further adapted for obesity treatment. The distal part will have the functionality of a volume filling device

In accordance with a first option, the distal part of the movement restriction device is adapted to be placed inside the stomach with the outer surface of the distal part of resting against the inside of the stomach wall.

In accordance with a second option, the distal part of the movement restriction device is adapted to be placed outside the stomach with the outer surface of the volume filling device resting against the outside of the stomach wall.

Preferably, the distal part of the movement restriction device is adapted to be completely invaginated by the stomach wall of the patient and to be placed inside or outside the stomach wall via a gastroscopic instrument. To this end the distal part of the movement restriction device may comprise an attachment device adapted to co-operate with a gripping instrument. Suitably, the distal part of the movement restriction device is adapted to be non-invasively adjustable postoperatively.

The apparatus may comprise a fixation device, suitably two or more fixation devices, adapted to be involved in the fixation of the distal part of the movement restriction device to the stomach wall. The distal part of the movement restriction device may comprise a holding device adapted to be held by an instrument, suitably two or more holding devices, to simplify the implantation of the device.

At least a part of the distal part of the movement restriction device may be made of a material which is not destructible by stomach acid. The distal part of the movement restriction device may be destructible by acids, for example hydrochloric acid.

In an embodiment, the distal part of the movement restriction device is inflatable to an expanded state and comprises an enclosure wall defining a chamber, the distal part of the movement restriction device is inflated with a gel or fluid supplied into the chamber. At least one tube may be connected to the distal part of the movement restriction device for supplying gel or fluid to the chamber. An injection port connectible with the tube may be provided. Alternatively, the distal part of the movement restriction device may be provided with an inlet port for a fluid or a gel connectible to a gastroscopic instrument, wherein the inlet port comprises a fluid connection adapted to interconnect the inflatable device and the gastroscopic instrument.

The distal part of the movement restriction device has a circumference of at least 30. 50, 80, 120, 150, 180 or 220 mm.

The distal part of the movement restriction device has a volume in the range of 0.0001 to 0.001 m³, or 0.00001 to 0.001 m³, or 0.00001 to 0.0002 m³. The volume of the volume filling device has a volume of less than 0.0002 m³.

The distal part of the movement restriction device may comprise at least two interconnectable portions adapted to be placed inside or outside the stomach as separate portions.

The distal part of the movement restriction device may comprise an elastic material, a bio-compatible material and/or silicone. Suitably, at least one of the distal and proximal parts of the movement restriction device is provided with at least one layer. For example, a metal layer, a Parylene layer, a polytetrafluoroethylene layer or a polyurethane layer. The layers may comprise multiple layers in any order. Suitably, one of the layers may be made of made of metal, silicon or PTFE. The volume filling device may comprise an outer surface layer of silicone, polyurethane, Teflon^{®}, or polytetrafluoroethylene, metal, Parylene, PTFE or a combination thereof. The volume filling device may comprise an inner surface layer of silicone, polyurethane, Teflon^{®}, or polytetrafluoroethylene, metal, Parylene, PTFE or a combination thereof. Other combinations of layers include an inner surface layer of polytetrafluoroethylene and an outer layer of silicone, an inner surface layer of polytetrafluoroethylene, an intermediate layer of silicone, and an outer layer of Parylene, an inner surface layer of polyurethane and an outer layer of silicone, and an inner surface layer of polyurethane, an intermediate layer of silicone, and an outer layer of Parylene.

The distal part of the movement restriction device may comprise a fluid adapted to be transformed into solid state or fixed form. Such a fluid may be liquid polyurethane or isotonic. The fluid may comprises large molecules, such as iodine molecules, to prevent diffusion.

The apparatus may include an implantable stimulation device that sends out stimulation pulses to the cardia muscle, especially the cardia sphincter muscle to stimulate the cardia muscle and thereby further close the cardia to additionally prevent reflux disease. The stimulation device is comprised of at least one conductor and at least one electrode that receives the stimulation pulses and applies them to the cardia sphincter muscle to thereby stimulate the cardia sphincter muscle. The at least one electrode may also be kept in place by the stomach-esophagal sutures or invagination in the stomach wall. The stimulation pulses may be sent as a train of pulses, wherein the pulse train is repeated with a time break in between, the break extending the break between each pulse in the pulse train. Preferably, the stimulation device sends out a number of pulse trains in a row followed by a break longer than that between the pulse trains to let the muscle rest still keeping the cardia sphincter closed The stimulation device may include an electronic circuit and an energy source preferably adapted to incorporate the electronic circuit and energy source.

The stimulation device preferably comprises at least one sensor for sensing a physical parameter of the patient or a functional parameter of the movement restriction device and an internal control unit for controlling the stimulation device.

Normally, the internal control unit controls the stimulation device in response to information from the sensor.

A sensor sensing a contraction wave of the esophagus, or any other parameter correlated to food intake, sends the information to the internal control unit and the internal control unit then ceases the stimulation in response to such information from the sensor.

The stimulation device may, at any time, be controlled by the patient.

This object is also obtained by providing an apparatus, the apparatus comprising an implantable movement restriction device having an elongated shape and having, when implanted in a patient, a proximal and a distal part, wherein the proximal part is adapted to be at least partly invaginated by the patient's stomach fundus wall and having an outer surface that preferably includes a biocompatible material, wherein a substantial part of the outer surface of the proximal part of the movement restriction device is adapted to rest against the stomach wall without injuring the latter in a position between the patient's diaphragm and at least a portion of the lower part of the invaginated stomach fundus wall, such that movement of the cardiac notch of the patient's stomach towards the patient's diaphragm is restricted, when the movement restriction device is invaginated, to thereby prevent the cardia from sliding through the patient's diaphragm opening into the patient's thorax, so as to maintain the supporting pressure against the patient's cardia sphincter muscle exerted from the patient's abdomen. The distal part of the movement restriction device stabilizes and holds the proximal part and is adapted by substantially invaginated in the stomach wall. The fundus stomach wall is more easily movable and thereby stabilized by the distal part being invaginated in the stomach wall at least partly below the fundus stomach wall. The proximal part of the movement restriction device has a size of at least 125 mm³ and a circumference of at least 15 mm, further comprises an implantable stimulation device adapted to engage with the cardia sphincter of a patient, and a control device for controlling the stimulation device to stimulate the cardia sphincter, wherein the stimulation of the cardia sphincter is made with energy pulses to increase sphincter tonus so that the cardia closes and said control device is operable by the patient in that it can be set out of operation, wherein the control device is further operable by the patient to set the stimulation device into operation, in which operational state the stimulation device continuously alternates at a time when the patient does not swallow between an operation mode, in which the cardia sphincter is stimulated with said energy pulses, and a rest mode, in which the cardia sphincter is not stimulated.

### Movement restriction device

The movement restriction device of the apparatus will be described. It is to be understood that in the present context all features, functionalities or adaptations described with the movement restriction device in this section are related to its proximal part, even if this is not explicitly mentioned. However, all features, embodiments, or part of embodiments as well as any method described in this application may, where applicable, be used for both the proximal or distal part of the device.

The apparatus comprises an implantable movement restriction device having an outer surface that includes a biocompatible material, wherein the movement restriction device is adapted to rest with at least a part of its outer surface against the patient's stomach fundus wall, in a position between the patient's diaphragm and the fundus wall, such that movement of the cardiac notch of the patient's stomach towards the patient's diaphragm is restricted, an apparatus for treating Gastro Esophageal Reflux Disease is obtained. The movement restriction device has a size of at least 125 mm³ and a circumference of at least 15 mm and restricts movement of the cardiac notch of the patient's stomach towards the patient's diaphragm thereby preventing the cardia from sliding through the patient's diaphragm opening into the patient's thorax, maintaining the supporting pressure against the patient's cardia sphincter muscle exerted from the patient's abdomen. Fixation device are adapted to secure the movement restriction device in said position.

By adapting the outer surface of the implanted movement restriction device to rest against the wall of the fundus, there is a minimal risk of complications, such as migration of damage to tissue, because the fundus is less fragile than the esophagus.

In a first embodiment of the invention, the fixation device comprises sutures or staples that attach together portions of the fundus stomach wall that enclose the movement restriction device to secure the movement restriction device in said position. I.e., the movement restriction device is at least partly placed in an invaginated space. Thus, by affixing the implantable movement restriction device indirectly in this manner, no suturing between the movement restriction device and tissue is required, which, in turn, further reduces the risk for complications. Keeping the movement restriction device in place in this manner has resulted in an elastic suspension with improved long term properties.

The fixation device, such as sutures or staples, may attach together portions of the fundus stomach wall so at to substantially or completely invaginate the movement restriction device from either inside or outside of the patient's stomach wall. Where the movement restriction device is placed on the outside of the patient's stomach wall, the movement restriction device is invaginated by the fundus stomach wall such that the stomach cavity is substantially reduced, by a volume substantially exceeding the volume of the movement restriction device.

In a another embodiment of the invention, the fixation device comprises an implantable first fixation device that attach the movement restriction device in said position to the fundus wall, a second fixation device that secures, indirectly or directly, the movement restriction device to the esophagus close to the patient's angle of His, and a third fixation device that secures, indirectly or directly, the movement restriction device to the patient's diaphragm muscle or associated muscles. Any of the first, second and third fixation devices may be comprised of a plurality of sutures or staples. The first fixation device may comprise a tissue growth promoting structure for long term attachment of the movement restriction device to the stomach wall. The tissue growth promoting structure may be sutured to the stomach wall with a relatively large contact surface towards the stomach. The relatively large surface of the structure, such as a net, will allow for in-growth of human tissue for holding the movement restriction device in place over the long term. The tissue growth promoting structure may comprise sutures or staples that attach the net like structure to the fundus stomach wall.

In addition to invaginating the movement restriction device in accordance with the first embodiment of the invention, the second fixation device can be used to secure, indirectly or directly, the movement restriction device to the esophagus close to the patient's angle of His, and the third fixation device may be used to secure, indirectly or directly, the movement restriction device to the patient's diaphragm muscle or other muscle tissue.

At least a part of the movement restriction device may be made of a material which is destructible or not destructible by stomach acid.

The movement restriction device may be inflatable and adapted to be inflated with a gel or fluid. A fluid or gel receiving member for receiving fluid to inflate the movement restriction device may be provided.

The movement restriction device may include a homogenous material and may be a solid body.

The movement restriction device may include an enclosure wall defining a chamber.

The movement restriction device may have a rigid, elastic or flexible outer wall. Where the outer wall is rigid, it is rigid enough to maintain non-deformed when subject to forces created by stomach movements. Where the movement restriction device is invaginated, in accordance with the first embodiment described above, the movement restriction device preferably comprises a body adapted to be at least partly invaginated by the patient's stomach fundus wall and having an outer surface that includes a biocompatible material. A substantial part of the outer surface of the body is adapted to rest against the stomach wall in said position between the patient's diaphragm and the portion of the lower part of the invaginated stomach fundus wall. Suitably, the body is made of a material softer than 25 or 15 Shore.

In accordance with a first general design of the body, the body has a maximum circumference as seen in a plane perpendicular to an axis through the body. The circumferences of the body as seen in other planes perpendicular to said axis are equal to the maximum circumference or decrease as seen along said axis in the direction from the maximum circumference. For example, the body may be substantially egg shaped, spherically shaped, or substantially shaped like an egg with an indented middle section or like a bent egg.

In accordance with a second general design of the body, the circumference of the body as seen in a plane perpendicular to an axis through the body increases and decreases at least two times as the plane is displaced along said axis, or decreases and increases at least one time as the plane is displaced along said axis. For example, the body may be substantially shaped like a kidney.

Preferably, the body is dimensioned with a size larger than the intestinal outlet from the stomach. The body may have a smallest outer diameter of 30 or 40mm or larger and may have a smallest outer circumference of 150, 110, 90, 70, 50 or 30 mm.

Suitably, the body has rounded contours without too sharp edges that would be damaging to the patient's stomach wall, and has a generally smooth outer surface for resting against the fundus wall.

The body is implantable either inside or outside of the patient's stomach and is adapted to be attached to the patient's stomach wall by surgery. The body may be changeable to assume a slender form having a smaller diameter than that of a trocar for laparoscopic use, whereby the body when changed to said slender form can be pushed or pulled through the trocar. The body may include a flexible outer wall defining a chamber filled with a fluid, such as a gel, allowing the body to pass through such a trocar. Alternatively, the body may include an elastic compressible material, allowing the body to pass through a trocar.

The body may be hollow and include at least two separate pieces adapted to be inserted into the hollow body, and further adapted to be put together to one unitary piece inside the body, thereby allowing the body to pass through a trocar for laparoscopic use. Alternatively, the body may include an outer wall and a hollow compressed inner part, for being filled with a fluid or gel after insertion into the patient's body.

The body may include a chamber with an injection port, wherein the chamber of the body is filled with a fluid through the injection port.

### Stimulation device

The stimulation device of the apparatus will now be described.

The control device is operable by the patient to control the stimulation device to continuously alternate between an operation mode, in which the cardia sphincter is stimulated with energy pulses, and a rest mode, in which the cardia sphincter is not stimulated. (The term "patient" includes an animal or a human being.) The continuous alternation between the operation and rest modes gives the advantage that the cardia sphincter is able to "recover" during the rest modes and as a result be more sensitive during the operation modes. Another advantage is that the energy consumption of the new apparatus will be considerably lower as compared with the above-discussed prior continuous stimulation system. In addition, since the control device is operable by the patient he or she may choose when the apparatus should be in operation. For example, for some patients it may be sufficient to keep the apparatus temporarily "on" when the patient feels reflux troubles, such as at night when the patient is lying, others may need to have the apparatus all the time "on", except when the patient eats.

In accordance with a preferred embodiment of the invention, the apparatus comprises a source of energy, wherein the control device controls the source of energy to release energy for use in connection with the power of the stimulation device, when the stimulation device is implanted. As a result, the apparatus of the invention provides a simple and effective control of the energy supplied to implanted components of the apparatus, which ensures an extended and reliable functionality of the apparatus, possibly for the rest of the patient's life and at least many years.

In the preferred embodiment, the control device may be controllable from outside the patient's body to control the stimulation device to vary the intensity of the stimulation of the cardia sphincter over time. More specifically, the control device may be adapted to control the stimulation device to change the intensity of the stimulation of the cardia sphincter so that the cardia sphincter tonus is changed.

Preferably, the source of energy comprises an electric source of energy and the control device is adapted to control the electric source of energy to deliver electric pulses to the stimulation device. An implantable switch for switching the delivery of electric pulses from the electric source of energy may be provided. The switch may be manually operable by the patient, or, alternatively, the control device may comprise a wireless remote control operable by the patient to control the switch.

Where the stimulation device stimulates the cardia sphincter with electric pulses there may be a problem of providing a voltage intensity strong enough to achieve the desired electric stimulation of the cardia sphincter. This is so because the intensity of the electric stimulation might fade over time, due to increasing electric resistance caused by the formation of fibrosis where electric conductors engage the cardia sphincter. This problem is solved by a main embodiment of the present invention, in which the stimulation device comprises electric conductors for engaging the cardia sphincter, the electric source of energy is adapted to provide a current through the electric conductors, and the control device is adapted to control the electric source of energy to release electric energy such that the intensity of the current through the electric conductors amounts to a predetermined value. As a result, decreasing current intensity caused by the formation of fibrosis where the conductors engage the cardia sphincter can be compensated for. Thus, if the current through the conductors decreases the control device automatically controls the electric source of energy to release more electric energy to restore the desired current intensity.

Advantageously, the control device is adapted to control the electric source of energy to release energy in the form of an alternating current. The inventor has found that unlike an alternating current a direct current could cause electrolysis in the cardia sphincter. Such electrolysis could injure the cardia sphincter.

All of the above embodiments may be combined with at least one implantable sensor for sensing at least one physical parameter of the patient, wherein the control device may control the stimulation device in response to signals from the sensor. In particular, the sensor may sense as the physical parameter the contraction wave in the esophagus caused by the patient swallowing food. In this case the stimulation device is adapted to cease the stimulation of the cardia sphincter in response to the sensor sensing the contraction wave in the patient's esophagus.

As an alternative, the sensor may comprise a pressure sensor for directly or indirectly sensing the pressure in the esophagus. The expression "indirectly sensing the pressure in the esophagus" should be understood to encompass the cases where the sensor senses the pressure against the stimulation device or human tissue of the patient.

The control device may comprise an internal control unit, preferably including a microprocessor, to be implanted in the patient for controlling the stimulation device. The internal control unit may suitably directly control the stimulation device in response to signals from the sensor. In response to signals from the sensor, for example pressure, the patient's position, the contraction wave in the patient's esophagus or any other important physical parameter, the internal control unit may send information thereon to outside the patient's body. The control unit may also automatically control the stimulation device in response to signals from the sensor. For example, the control unit may control the stimulation device to efficiently stimulate the cardia sphincter, such that the cardia for certain is completely closed in response to the sensor sensing that the patient is lying.

The control device may also, or alternatively, comprise an external control unit outside the patient's body, wherein the internal control unit is programmable by the external control unit, for example for controlling the stimulation device over time. Alternatively, the internal control unit may control the stimulation device over time in accordance with an activity schedule program, which may be adapted to the patient's needs.

The external control unit may also, suitably directly, control the stimulation device in response to signals from the sensor. The external control unit may store information on the physical parameter sensed by the sensor and may be manually operated to control the stimulation device based on the stored information. In addition, there may be at least one implantable sender for sending information on the physical parameter sensed by the sensor.

A great advantage is that the patient is enabled to keep the cardia completely closed by means of the stimulation device by using the control device whenever he likes during the day. This advantage should not be underestimated, because in case the patient would need to vomit it would be very difficult for him to do so if he were unable to immediately stop the stimulation of the cardia.

Conveniently, the external control unit may load the internal control unit with data in accordance with a loading mode only authorized for a doctor. For specialized controls of the stimulation device, such as electric power, electric pulse frequency etc, the external control unit may control the internal control unit in accordance with a doctor mode only authorized for the doctor. For simple controls of the stimulation device, such as "on" and "off", the external control unit may control the internal control unit in accordance with a patient mode permitted for the patient. Thus, by using the external control unit in accordance with different modes it is possible to have certain functions of the stimulation device controlled by the patient and other more advanced functions controlled by the doctor, which enables a flexible post-operation treatment of the patient.

The control device may be adapted to control the source of energy to release energy, for instance to intermittently release energy in the form of a train of energy pulses, for direct use in connection with the power of the stimulation device. In accordance with a suitable embodiment the control device controls the source of energy to release electric energy, and the apparatus further comprises an implantable capacitor for producing the train of energy pulses from the released energy. In this case the term "direct" is used to mean, on one hand, that the released energy is used while it is being released by the control device, on the other hand, that the released energy may be somewhat delayed, in the order of seconds, by for instance an energy stabilizer before being used in connection with the power of the stimulation device.

Further below is described further embodiments related to energy supply and control. All these embodiments may be used for all the different applicable embodiments in this application not only for the stimulation device.

In accordance with an embodiment of the invention, the apparatus comprises implantable electrical components including at least one, or only one single voltage level guard and a capacitor or accumulator, wherein the charge and discharge of the capacitor or accumulator is controlled by use of the voltage level guard.

In one embodiment, the source of energy is external to the patient's body and the control device controls the source of energy to release wireless energy. An energy storage device, preferably an electric accumulator, may be implanted in the patient for storing the wireless energy released from the external source of energy. The electric accumulator may comprise at least one capacitor or at least one rechargeable battery, or a combination of at least one capacitor and at least one rechargeable battery. Alternatively, a battery may be implanted in the patient for supplying electric energy to implanted electric energy consuming components of the apparatus, in addition to the supply of wireless energy. Where the control device comprises an implantable control unit the electronic circuit thereof and the stimulation device may be directly powered with transformed wireless energy, or energy from either the implanted energy storage device or battery.

In one embodiment the wireless energy is directly used for the power of the stimulation device, i.e. the stimulation device is powered as the wireless energy is released from the external source of energy by the control device. In this case the term "directly" is used to mean, on one hand, that the stimulation device is promptly powered by using the released energy whiteout first storing the latter, on the other hand, that the released energy may be somewhat delayed, in the order of seconds, by for instance an energy stabilizer before being used for the power of the stimulation device. Both the wireless energy may be used to create direct kinetic energy the wireless field affecting an the apparatus directly or by using an energy transforming device, transforming the wireless energy to electric energy which may be used to power any energy consuming parts of the apparatus directly during wireless energy transmission or indirect after charging an accumulator. As a result, a very simple control of the stimulation device is achieved and there are only a few implanted components of the apparatus. For example, there is no implanted source of energy, such as a battery, nor any implanted complicated signal control system. This gives the advantage that the apparatus will be extremely reliable.

In one embodiment, the source of energy comprises an implantable internal source of energy. Thus, when the internal source of energy is implanted in a patient the control device controls it from outside the patient's body to release energy. This solution is advantageous for sophisticated embodiments of the apparatus that have a relatively high consumption of energy that cannot be satisfied by direct supply of wireless energy. The internal source of energy preferably comprises an electric source of energy, such as an accumulator or a battery. Alternatively, the control device may be adapted to release wireless energy from the internal source of energy and to control the stimulation device to stimulate the patient's cardia sphincter with the released wireless energy. The wireless energy may comprise radiant energy or sound energy, such as ultrasound energy.

In one embodiment of the invention, the apparatus comprises a switch implanted in the patient for directly or indirectly switching the power of the stimulation device and an internal electric source of energy, such as a battery, implanted in the patient for supplying electric energy for the power of the stimulation device, wherein the switch directly or indirectly affects the supply of electric energy from the internal electric source of energy. This solution is advantageous for embodiments of the apparatus that have a relatively high consumption of energy that cannot be met by direct supply of wireless energy.

In one embodiment of the invention, the switch switches between an "off" mode, in which the internal electric source of energy is not in use, and an "on" mode, in which the internal electric source of energy supplies electric energy for the power of the stimulation device. In this case, the switch is conveniently operated by the wireless energy released from the external source of energy to switch between the "on" and "off" modes. The control device, preferably comprising a wireless remote control, may control the external source of energy to release the wireless energy. The advantage of this embodiment is that the lifetime of the implanted electric source of energy, such as a battery, can be significantly prolonged, since the implanted source of energy does not supply energy when the switch is in its off mode.

In one embodiment, the control device comprises a wireless remote control for controlling the internal electric source of energy. In this case, the switch is operable by the wireless energy from the external source of energy to switch between an "off" mode, in which the internal electric source of energy and remote control are not in use, and a "standby" mode, in which the remote control is permitted to control the internal electric source of energy to supply electric energy for the power of the stimulation device.

In one embodiment, the apparatus further comprises an energy transforming device to be implanted in the patient for transforming the wireless energy into storable energy, and an implantable energy storage device for storing the storable energy, wherein the switch is operable by energy from the implanted energy storage device to switch between an "off" mode, in which the internal electric source of energy is not in use, and an "on" mode, in which the internal electric source of energy supplies electric energy for the power of the stimulation device. In this case, the control device suitably comprises a wireless remote control for controlling the energy storage device to operate the switch.

An external data communicator may be provided outside the patient's body and an internal data communicator to be implanted in the patient may be provided for communicating with the external data communicator. The internal data communicator may feed data related to the patient, or related to the stimulation device, back to the external data communicator. Alternatively or in combination, the external data communicator may feed data to the internal data communicator. The internal data communicator may suitably feed data related to at least one physical signal of the patient.

Suitably, an implantable stabilizer, such as a capacitor, a rechargeable accumulator or the like, may be provided for stabilizing the electric energy released by the control device. In addition, the control device may control the source of energy to release energy for a determined time period or in a determined number of energy pulses.

All of the above embodiments are preferably remote controlled. Thus, the control device advantageously comprises a wireless remote control transmitting at least one wireless control signal for controlling the stimulation device. With such a remote control it will be possible to adapt the function of the apparatus to the patient's need in a daily basis, which is beneficial with respect to the treatment of the patient.

The wireless remote control may be capable of obtaining information on the condition of the stimulation device and of controlling the stimulation device in response to the information. Also, the remote control may be capable of sending information related to the stimulation device from inside the patient's body to the outside thereof.

In a particular embodiment of the invention, the wireless remote control comprises at least one external signal transmitter or transceiver and at least one internal signal receiver or transceiver implantable in the patient. In another particular embodiment of the invention, the wireless remote control comprises at least one external signal receiver or transceiver and at least one internal signal transmitter or transceiver implantable in the patient.

The remote control may transmit a carrier signal for carrying the control signal, wherein the carrier signal is frequency, amplitude or frequency and amplitude modulated and is digital, analogue or digital and analogue. Also the control signal used with the carrier signal may be frequency, amplitude or frequency and amplitude modulated.

The control signal may comprise a wave signal, for example, a sound wave signal, such as an ultrasound wave signal, an electromagnetic wave signal, such as an infrared light signal, a visible light signal, an ultra violet light signal, a laser signal, a micro wave signal, a radio wave signal, an x-ray radiation signal, or a gamma radiation signal. Where applicable, two or more of the above signals may be combined.

The control signal may be digital or analogue, and may comprise an electric or magnetic field. Suitably, the wireless remote control may transmit an electromagnetic carrier wave signal for carrying the digital or analogue control signal. For example, use of an analogue carrier wave signal carrying a digital control signal would give safe communication. The control signal may be transmitted in pulses by the wireless remote control.

The control device may be activated in a manual or non-manual manner to control the source of energy to release energy.

In the above-presented embodiments of the invention the released energy may comprise electric energy and an implantable capacitor having a capacity less than 0.1 µF may be provided for producing the above-mentioned train of energy pulses.

Generally, the wireless energy comprises a signal.

The apparatus may further comprise an implantable energy transforming device for transforming wireless energy, for example in the form of sound waves, directly or indirectly into electric energy, for the power of the stimulation device. More specifically, the energy transforming device may comprise a capacitor adapted to produce electric pulses from the transformed electric energy.

Generally, the stimulation device advantageously is embedded in a soft or gel-like material, such as a silicone material having hardness less than 20 Shore.

The stimulation device may comprise a band for application around the cardia, wherein the band has electric conductors for contacting the cardia sphincter. The electric conductors may comprise hooks to secure the conductors on the cardia.

The present invention also provides a system for treating heartburn and reflux disease, comprising an implantable stimulation device adapted to stimulate the cardia sphincter of a patient to increase the sphincter tonus, and a control device that controls the stimulation device to continuously alternate between an operation mode, in which the cardia sphincter is stimulated with energy pulses, and a rest mode, in which the cardia sphincter is not stimulated. The energy pulses may comprise electric pulses. The stimulation device may comprise electric conductors for engaging the cardia sphincter, and an electric source of energy may be adapted to provide a current through the electric conductors to form the electric pulses. Advantageously, the control device may control the electric source of energy to release the electric energy such that the current through the electric conductors amounts to a predetermined value.

All the above described various components may be combined in the different embodiments where applicable. Also the various functions described in connection with the above embodiments of the invention may be used in different applications, where applicable.

All the various ways of transferring energy and controlling the energy presented in the present specification may be practiced by using all of the various components and solutions described.

The present invention also provides methods for treating heartburn and reflux disease.

Accordingly, in accordance with a first alternative method, there is provided a method of treating heartburn and reflux disease, comprising the steps of:
implanting an stimulation device in a patient, so that the stimulation device engages the cardia, and
controlling the stimulation device to continuously alternate between an operation mode, in which the cardia sphincter is stimulated with energy pulses to increase the sphincter tonus, so that the cardia completely closes, and a rest mode, in which the cardia sphincter is not stimulated.

The first alternative method may also be performed laparoscopically. Thus, there may be provided a laparoscopic method of treating heartburn and reflux disease, comprising the steps of:
laparosopically implanting an stimulation device in a patient, so that the stimulation device engages the cardia, and
controlling the stimulation device to continuously alternate between an operation mode, in which the cardia sphincter is stimulated with energy pulses to increase the sphincter tonus, so that the cardia completely closes, and a rest mode, in which the cardia sphincter is not stimulated.

In accordance with a second alternative method, there is provided a method of treating a patient having heartburn and reflux disease, comprising:
(a) Surgically implanting in the patient an electric stimulation device engaging the cardia.
(b) Providing a source of energy external to the patient's body.
(c) Controlling the external source of energy from outside the patient's body to release wireless energy. And
(d) using the released wireless energy in connection with the powering of the stimulation device.

The second alternative method may further comprise implanting an energy transforming device, controlling the external source of energy to release wireless energy, and transforming the wireless energy by the energy transforming device into energy different from the wireless energy for use in connection with the power of the stimulation device. This method may further comprise implanting a stabilizer in the patient for stabilizing the energy transformed by the energy-transforming device.

There is also provided a method of treating heartburn and reflux disease, comprising the steps of: implanting a stimulation device in a patient to engage the cardia sphincter, providing a control device for controlling the stimulation device to stimulate the cardia sphincter to increase the sphincter tonus, so that the cardia completely closes, and permitting the patient to operate the control device to vary the intensity of
In one embodiment there is provided an apparatus where the stimulation of the cardia sphincter is made with energy pulses to increase the sphincter tonus so that the cardia completely closes and said control device is operable by the patient in that it can be set out of operation, wherein the control device is further operable by the patient to set the stimulation device into operation, in which operational state the stimulation device continuously alternates between an operation mode, in which the cardia sphincter is stimulated with said energy pulses, and a rest mode, in which the cardia sphincter is not stimulated, wherein the apparatus further comprises at least one implantable sensor for sensing at least one physical parameter of the patient, wherein the control device is adapted to control the stimulation device to cease the continuous alternation between the operation mode and the rest mode and to put the stimulation device in the rest mode in response to the sensor sensing the physical parameter of the patient.

In one embodiment there is provided an apparatus where the stimulation of the cardia sphincter is made with energy pulses to increase the sphincter tonus so that the cardia completely closes and said control device is operable by the patient in that it can be set out of operation, wherein the control device is further operable by the patient to set the stimulation device into operation, in which operational state the stimulation device continuously alternates between an operation mode, in which the cardia sphincter is stimulated with said energy pulses, and a rest mode, in which the cardia sphincter is not stimulated, wherein the apparatus further comprises at least one implantable sensor for sensing as a physical parameter of the patient at least the contraction wave in the esophagus caused by the patient swallowing food, wherein the control device is adapted to control the stimulation device to cease the continuous alternation between the operation mode and the rest mode and to put the stimulation device in the rest mode in response to the sensor sensing the contraction wave in the patient's esophagus.

### Combination with obesity treatment

The various embodiments can be combined with various methods for treating obesity. In particular two embodiments, one comprising a stretching device and one comprising a volume filling device, will be described below.

### Stretching device for treating obesity

Please note that all embodiment or part of embodiments or methods may be used where applicable for all the different embodiments in this application.

In addition the various embodiments the apparatus for treating reflux can be combined with a device for the treatment of obesity that that is based on the realization that by creating a stretching effect of the stomach wall a feeling of satiety is created. By means of providing an apparatus with a stretching device stretching part of the stomach wall, a simpler, safer and long term working apparatus is provided.

The expression "powered" should be understood as energized with everything without manual force, preferably electric energy. In other words, the adjustment device is operated in a non-manual manner. The expression "non-manual manner" should be understood to mean that the adjustment device is not operated by manually touching subcutaneously implanted components of the apparatus or not manipulated by touching the skin of the patient. Thus, as opposed to prior practice when treating anal incontinence, the adjustment device of the invention is not operated by manual forces, such as by manually compressing a fluid containing balloon implanted in the scrotum or in the region of labia major. Of course, manual manipulation of an implanted reservoir or other mechanical or hydraulic solutions may also be used as well as manual manipulation of a subcutaneous start button or the like for activating the powered operation device everything is permitted within the scope of the present invention.

Alternatively, or preferably in combination with a powered operation device, the servo means may be used, which enables for example a motor to run with high speed and low force and with for example a gear box to decrease the speed and increase the force or torque. The servo means may comprise hydraulic means, electric control means, magnetic means, or mechanical means, which may be activated by manual manipulating means and/or remote control. Using a servo system will save the use of force when adjusting the adjustment device, which may be of importance in many applications.

The term "servo means" encompasses the normal definition of a servo mechanism, i.e. an automatic device that controls large amounts of power by means of very small amounts of power, but may alternatively or additionally encompass the definition of a mechanism that transfers a weak force acting on a moving element having a long stroke into a strong force acting on another moving element having a short stroke. The servo means may comprise a motor, preferably an electric motor, which may be reversible.

Alternatively, or preferably in combination with a manual manipulation, a reversed servo means may be used, which enables for example a the patients hand to use a higher force to with for example manipulate a hydraulic reservoir to move a small amount of fluid with strong force to control a larger movement of fluid. The reversed servo means may comprise hydraulic means, electric control means, magnetic means, or mechanical means, which may be activated by manual manipulating means and/or remote controlled. Using a reversed servo system will save the use of stroke when adjusting the adjustment device, which may be of importance in many applications.

The term "reversed servo means" encompasses the definition of an device that is controlled with a higher force and a small stroke i.e. for example movement of a small amount of fluid with a high force controls a larger amount of fluid moving by means of very smaller force, but may alternatively or additionally encompass the definition of a mechanism that transfers a strong force acting on a moving element having a short stroke into a small force acting on another moving element having a long stroke. The reversed servo means is preferably used when manual control of the device through intact skin is possible.

In general, two points on the stomach wall should be moved in relation to each other and away from each other to cause distension of a small part of the stomach wall, thereby causing satiety. This could be done in many different ways. One way is to expand an invaginated device invaginated in the stomach wall. Another way is to move two fixation points on the stomach wall. Of course first and second positions may be sutured or fixated to the stomach wall in many possible ways and the invention covers all possibilities to distend the stomach wall by moving two portions of the stomach wall away from each other and thereby a first fixation of the device being moved in relation to a second fixation, at least two positions on the stomach wall. However, the soft suspended connection to the stomach wall achieved by invaginating at least one adapted part of the device is preferred, where fibrotic stomach to stomach tissue helps to give a long term stable position.

Any kind of mechanical construction may be used. Any mechanical construction driven mechanically or hydraulically or any pneumatic construction may be used. Any motor or any pump or moving material changing form when powered may be used to achieve the simple goal of stretching a part of the stomach wall by moving at least two portions of the stomach wall away from each other.

Any kind of hydraulic operation may be used. It will be appreciated that instead of hydraulic operation, pneumatic operation can be used, wherein air instead of hydraulic fluid is moved between a reservoir and a chamber formed by the stretching device. Preferably the reservoir has a locking position to keep it in the desired position if it is handled by the patient. To compress the reservoir it preferably stays compressed and releases after pressing again.

Any kind of hydraulic solution may be used for the stretching device. The hydraulic solution may be driven by both mechanically and powered with any motor or pump as well as manual.

Of course just expanding an in-vaginated part of the stomach also stretches away the stomach wall which also may be achieved both mechanically, hydraulically, pneumatically and both being powered with a motor or pump or by manual force.

### Volume filling device for treating obesity

In addition the various embodiments the apparatus for treating reflux can be adapted to additionally treat obesity that is based on volume filling capacity of the distal part of elongated movement restriction device in the stomach that creates satiety. In the present context, when volume filling device and its features, functionality and adaptations are discussed, it refers to the distal part of the elongated movement restriction device. Please note that, any feature, embodiment, part of embodiment or method described herein may where applicable be used for both the distal or proximal part of the movement restriction device.

The following embodiment is based on the realization that by invaginating a volume filling device (here represented by the distal part of the movement restriction device) by the stomach wall of the patient, this inflatable object is protected from the stomach acids and will thus remain functioning for a very long time.

In order to provide a movement restriction device which is more stable in the proximal part, this part can be made of a different configuration from the distal part. Thus, in an embodiment the proximal part, which is fixed to a position above the cardia area, comprises a larger ball shaped part or segment, while the distal part comprises a plurality of small movement restriction device segments. This embodiment combines the advantages of a stable proximal part used for preventing reflux with a more adaptable distal part used as a volume filling device for treating obesity. Generally, the proximal and distal parts can have different configurations and contents, independent of each other. This content can be a mixture of solid and fluid content, such as friction enhancing or reducing fluid.

Small volume filling devices and volume filling device segments could be used interchangeable.

In one embodiment of the apparatus, at least one of the volume filling device segments has at least one flat surface. Preferably, the volume filling device segments has the shape of a polyhedron, preferably one of the following shapes: tetrahedron, hexahedron, octahedron, dodecahedron and icosahedrons.

In one embodiment of the apparatus, a friction enhancing material is provided. This increases the friction between adjacent volume filling device segments, thereby stabilizing the volume filling device. This friction enhancing material is preferably a glue or an adhesive. Alternatively, at least one of the volume filling device segments has a surface with a rugged texture.

In one embodiment of the apparatus, at least one of the volume filling device segments has spherical shape. Alternatively, it has at least one flat surface.

In an alternative embodiment, the apparatus comprises a fluid for reducing the friction between adjacent volume filling device segments. The volume filling device can thereby more easily adapt its shape to the movements of the patient's body.

The apparatus may in one embodiment comprise a friction reducing material on the outer surface of the volume filing device segments. This friction reducing material may be a fluid reducing the friction between adjacent volume filling device segments.

The apparatus may comprise an expandable second volume filling device segment for enclosing two or more first volume filling device segments different from the second volume filling device segment, wherein the second volume filling device segment and the first volume filling device segments together form the volume filling device. In one alternative, the second volume filling device segment comprises a friction reducing material on an inner surface thereof, the friction reducing material being in contact with the first volume filling device segments, when implanted.

The second volume filling device segment may be adapted to be filled with a fluid to allow mutual movement between adjacent first volume filling device segments so that the shape of the volume filling device adapts to stomach wall movements, when said volume filling device is invaginated in a stomach wall. It is then preferred that at least a wall portion of the second volume filling device segment is flexible or stretchable.

The fluid provided in the volume filling device may be isotonic or hypertonic.

The volume filling device segments may be adapted to be inserted into a pouch formed by part of a stomach wall of the patient. The volume filling device segments may be adapted to be filled, directly or indirectly, into the pouch formed by part of a stomach wall of the patient via a tubular instrument.

In one embodiment, the volume filling device comprises a solidifying liquid.

This liquid or fluid may be supplied to the pouch by means of a conduit.

In one embodiment, the plurality of volume filling device segments are adapted to be interconnected to form the volume filling device, after said plurality of volume filling device segments have been inserted into a human or artificial pouch.

The apparatus, wherein the volume filling device segments adapted to be assembled to an implantable volume filling device.

According to one embodiment of the invention, an apparatus to treat obesity and reflux of a patient having a stomach with a food cavity is provided, the apparatus comprising at least one volume filling device adapted to be at least substantially invaginated by a stomach wall portion of the patient, wherein the volume filling device is adapted to be placed on the outside of the stomach wall, so that the volume of the food cavity is reduced in size by a volume substantially exceeding the volume of the volume filling device, wherein the surface of the volume filling device comprises a biocompatible material, wherein a substantial part of the surface of the volume filling device is adapted to rest against the outside of the stomach wall, and wherein the volume filling device has a maximum circumference of at least 30 mm.

By invaginating the volume filling device by a stomach wall portion of the patient on the outside of the stomach wall, the volume filling device is protected from the stomach acids, thereby providing a device that will last for a long time.

The volume filling device is adapted to be placed with the outer surface of the volume filling device resting against the stomach wall, such that the volume of the food cavity is reduced in size by a volume substantially exceeding the volume of the volume filling device. The volume filling device has a maximum circumference of at least 30 millimeters. Accordingly, the apparatus of the present invention is well suited for treating obesity of an obese patient, as well as reflux disease of the same patient. This is advantageous, because reflux disease is a very common condition among human beings suffering from obesity.

In accordance with a first option, the volume filling device is adapted to be placed inside the stomach with the outer surface of the volume filling device resting against the inside of the stomach wall.

In accordance with a second option, the volume filling device is adapted to be placed outside the stomach with the outer surface of the volume filling device resting against the outside of the stomach wall.

Preferably, the volume filling device is adapted to be completely invaginated by the stomach wall of the patient and to be placed inside or outside the stomach wall via a gastroscopic instrument. To this end the volume filling device may comprise an attachment device adapted to co-operate with a gripping instrument. Suitably, the volume filling device is adapted to be non-invasively adjustable postoperatively.

The apparatus may comprise a fixation device, suitably two or more fixation devices, adapted to be involved in the fixation of the volume filling device to the stomach wall. The volume filling device may comprise a holding device adapted to be held by an instrument, suitably two or more holding devices, to simplify the implantation of the device.

At least a part of the volume filling device may be made of a material which is not destructible by stomach acid. The volume filling device may be destructible by acids, for example hydrochloric acid.

In an embodiment, the volume filling device is inflatable to an expanded state and comprises an enclosure wall defining a chamber, wherein the volume filling device is inflated with a gel or fluid supplied into the chamber. At least one tube may be connected to the volume filling device for supplying gel or fluid to the chamber. An injection port connectible with the tube may be provided. Alternatively, the volume filling member may be provided with an inlet port for a fluid or a gel connectible to a gastroscopic instrument, wherein the inlet port comprises a fluid connection adapted to interconnect the inflatable device and the gastroscopic instrument.

The volume filling device may include a homogenous material, such as gel having a Shore value of less than 15. The device may also be a solid body.

The volume filling device may comprise a rigid, elastic or flexible outer surface. Where the outer surface is rigid, it is rigid enough to maintain non-deformed when subject to forces created by stomach movements. The volume filling device may comprise a flexible non-elastic material.

In accordance with a first general design of the volume filling device, the device has a maximum circumference as seen in a plane perpendicular to an axis through the device. The circumferences of the device as seen in other planes perpendicular to said axis are equal to the maximum circumference or decrease as seen along said axis in the direction from the maximum circumference. For example, the device may be substantially egg shaped, spherically shaped, or substantially shaped like an egg with an indented middle section or like a bent egg.

In accordance with a second general design of the device, the circumference of the device as seen in a plane perpendicular to an axis through the device increases and decreases at least two times as the plane is displaced along said axis, or decreases and increases at least one time as the plane is displaced along said axis. For example, the device may be substantially shaped like a kidney.

The volume filling device has an elongated, rounded, bent and/or curved shape.

The volume filling device has a circumference of at least 120, 150, 180 or 220 mm.

The volume filling device has a volume in the range of 0.0001 to 0.001 m³, or 0.00001 to 0.001 m³, or 0.00001 to 0.0002 m³. The volume of the volume filling device has a volume of less than 0.0002 m³.

The volume filling device may comprise at least two interconnectable portions adapted to be placed inside or outside the stomach as separate portions.

The volume filling device may comprise an elastic material, a bio-compatible material and/or silicone.

Suitably, the volume filling device is provided with a coating, for example a Parylene coating, a polytetrafluoroethylene coating or a polyurethane coating. The coating may be a multi-layer coating. The volume filling device may comprise an outer surface layer of polyurethane, Teflon^{®}, or PTFE, or a combination thereof.

The volume filling device may comprise a fluid adapted to be transformed into solid state or fixed form. Such a fluid may be liquid polyurethane or isotonic. The fluid may comprises large molecules, such as iodine molecules, to prevent diffusion.

The volume filling device may have a maximum circumference of at least 50 millimeters, preferably at least 80 millimeters. Suitably, the volume filling device is deformable to a maximum diameter, so as to be insertable into a laparoscopic trocar.

Preferably, the volume filling device is adapted to be kept in place by stomach-to-stomach sutures or staples to invaginate the device in the stomach wall. Advantageously, the volume filling device has varying circumference to better be kept in place invaginated in the stomach wall of the patient. The stomach-to-stomach sutures or staples may be provided with fixation portions exhibiting a structure adapted to be in contact with the stomach wall to promote growth in of human tissue to secure long term placement of the volume filling device attached to the stomach wall. The structure may comprise a net like structure.

In embodiment of the invention, the apparatus also comprises a stretching device placed outside the stomach wall and adapted to stretch a portion of the stomach wall, thereby affecting the patient's appetite. When the volume filling device is inflatable, the apparatus may comprise a fluid connection interconnecting the stretching device and the volume filling device.

In an embodiment, the apparatus comprises a stretching device comprising at least one operable stretching device implantable in an obese patient and adapted to stretch a portion of the patient's stomach wall and an operation device for operating the stretching device when implanted to stretch the stomach wall portion such that satiety is created.

In an embodiment, the apparatus comprises at least one operable stretching device implantable in the patient and adapted to stretch a portion of the patient's stomach wall, and an implantable control unit for automatically controlling the operable stretching device, when the control unit and stretching device are implanted, to stretch the stomach wall portion in connection with the patient eating such that satiety is created.

In an embodiment, the apparatus comprises a stretching device comprising at least one operable stretching device implantable in an obese patient and adapted to stretch a portion of the patient's stomach wall, wherein said stretching device comprising an expandable stretching reservoir and an operation device for operating the stretching device when implanted to stretch the stomach wall portion, wherein the at least the distal part of the movement restriction device is inflatable and in fluid connection with said stretching reservoir, wherein said operation device comprises a pump for pumping fluid between said main reservoir and said stretching reservoir to stretch said stomach wall portion such that satiety is created. A control device may be provided for controlling said stretching device including said pump. The control device may comprise a wireless remote control adapted to control the stretching device from the outside of the patient's body, or an implantable control unit for controlling said stretching device. Alternatively, the control device may comprise a subcutaneously placed switch or reservoir adapted to control the stretching device from the outside of the patient's body. A sensor or sensing device to be implanted in the patient body may be provided, wherein the implantable control unit is adapted to control the stretching device from the inside of the patient's body using information from said a sensor or sensing device, adapted to sense, direct or indirect, the food intake of the patient.

In an embodiment, the distal part of the movement restriction device comprises a main volume filling reservoir, a stretching device comprising at least one operable stretching device implantable in an obese patient and adapted to stretch a portion of the patient's stomach wall, wherein said stretching device comprising an expandable reservoir, adapted to be invaginated in the stomach wall at the upper part of the stomach, higher up than the inflatable main volume filling device when the patient is standing, wherein the volume filling device is inflatable and in fluid connection with said stretching reservoir, wherein normal contractions of the stomach wall, related to food intake, cause fluid to flow from said invaginated main volume filling reservoir lower placed onto the stomach wall adapted to cause said stretching reservoir to stretch said stomach wall portion such that satiety is created. The fluid connection between the main volume filling reservoir and the stretching reservoir comprises a non-return valve. The fluid connection between the main volume filling reservoir and the stretching reservoir comprises a release function adapted to release the volume in the stretching reservoir back to the main volume filling reservoir. Said release function may comprise a fluid return connection of a substantially smaller area than said fluid connection, to slowly release back fluid to said main volume filling device reservoir from the stretching reservoir to release said stretching of the stomach wall portion. A further manual control device comprising a subcutaneously placed reservoir adapted to control the stretching device from the outside of the patient's body may be provided to further affect the stretching device to stretch the stomach wall portion.

In an embodiment, the a main volume filling reservoir adapted to be inflatable may be provided, wherein the distal part of the movement restriction device further comprises an expandable structure, adapted to expand, when the device is invaginated in the stomach wall, wherein said structure comprising a bellow adapted to take into account the fibrosis surrounding the device when implanted, such that the movement of the bellow is substantially unaffected of fibrosis.

In an embodiment, the apparatus comprises a stretching device comprising at least one operable stretching device implantable in an obese patient and adapted to stretch a portion of the patient's stomach wall and wherein the stretching device comprising a expandable structure, adapted to expand and stretch the stomach wall portion, when the device is invaginated in the stomach wall, wherein said structure comprising a special bellow adapted to take into account the fibrosis surrounding the device when implanted, such that the movement of the bellow is substantially unaffected of said fibrosis. An operation device for operating the stretching device may be provided to stretch the stomach wall portion such that satiety is created. The apparatus may comprise an implantable control unit for automatically controlling the operable stretching device, when the control unit and stretching device are implanted, to stretch the stomach wall portion in connection with the patient eating such that satiety is created.

In an embodiment, the apparatus comprises a stretching device comprising at least one operable stretching device implantable in an obese patient and adapted to stretch a portion of the patient's stomach wall such that satiety is created. The control device may comprise a wireless remote control adapted to control the stretching device from the outside of the patient's body or an implantable control unit for controlling said stretching device. Alternatively, said control device may comprise a subcutaneously placed switch or reservoir adapted to control the stretching device from the outside of the patient's body. A sensor or sensing device adapted to be implanted in the patient body may be provided, wherein the implantable control unit is adapted to control the stretching device from the inside of the patient's body using information from said sensor or sensing device, adapted to sense, direct or indirect, the food intake of the patient.

In an embodiment, the apparatus is adapted to treat reflux disease. To this end, it further comprises an implantable movement restriction device having an elongated shape with a proximal part and a distal part, the proximal part being adapted to be at least partly invaginated by the patient's stomach fundus wall and having an outer surface that includes a biocompatible material. A substantial part of the outer surface of the proximal part of the movement restriction device is adapted to rest against the stomach wall without injuring the latter in a position between the patient's diaphragm and at least a portion of the lower part of the invaginated stomach fundus wall, such that movement of the cardiac notch of the patient's stomach towards the patient's diaphragm is restricted, when the proximal part of the movement restriction device is invaginated, to thereby prevent the cardia from sliding through the patient's diaphragm opening into the patient's thorax, so as to maintain the supporting pressure against the patient's cardia sphincter muscle exerted from the patient's abdomen,. The proximal part of the movement restriction device has a size of at least 125 mm3 and a circumference of at least 15 mm. The distal part stabilizes and holds the proximal part and is adapted to be substantially invaginated in the stomach wall.

In another embodiment, the apparatus is adapted to treat reflux disease. To this end, it further comprises an implantable movement restriction device having an elongated shape with a proximal part and a distal part and having an outer surface including a biocompatible material. The proximal part of the movement restriction device is adapted to rest with at least a part of its outer surface against the patient's stomach fundus wall, in a position between the patient's diaphragm and the fundus wall, such that movement of the cardiac notch of the patient's stomach towards the patient's diaphragm is restricted, when the movement restriction device is implanted in the patient, to thereby prevent the cardia from sliding through the patient's diaphragm opening into the patient's thorax, so as to maintain the supporting pressure against the patient's cardia sphincter muscle exerted from the patient's abdomen, wherein the proximal part of the movement restriction device having a size of at least 125 mm³ and a circumference of at least 15 mm. An affixation device adapted to secure the proximal part of the movement restriction device in said position, when the movement restriction device is implanted. The distal part stabilizes and holds the proximal part and is adapted to be substantially invaginated in the stomach wall.

In another embodiment, the apparatus is adapted to treat reflux disease. To this end, it further comprises an implantable movement restriction device having an elongated shape with a proximal part and a distal part and having an outer surface that includes a biocompatible material, the proximal part being adapted to be at least partly invaginated by the patient's stomach fundus wall., A substantial part of the outer surface of the proximal part of the movement restriction device is adapted to rest against the stomach wall without injuring the latter in a position between the patient's diaphragm and at least a portion of the lower part of the invaginated stomach fundus wall, such that movement of the cardiac notch of the patient's stomach towards the patient's diaphragm is restricted, when the movement restriction device is invaginated, to thereby prevent the cardia from sliding through the patient's diaphragm opening into the patient's thorax, so as to maintain the supporting pressure against the patient's cardia sphincter muscle exerted from the patient's abdomen, the movement restriction device having a size of at least 125 mm3 and a circumference of at least 15 mm, further comprising a stretching device comprising at least one operable stretching device implantable in the obese patient and adapted to stretch a portion of the patient's stomach wall such that satiety is created. The distal part stabilizes and holds the proximal part and is adapted to be substantially invaginated in the stomach wall.

In another embodiment, the apparatus is adapted to treat reflux disease. To this end, it further comprises an implantable movement restriction device having an elongated shape with a proximal part and a distal part and having an outer surface including a biocompatible material. The proximal part of the movement restriction device is adapted to rest with at least a part of its outer surface against the patient's stomach fundus wall, in a position between the patient's diaphragm and the fundus wall, such that movement of the cardiac notch of the patient's stomach towards the patient's diaphragm is restricted, when the movement restriction device is implanted in the patient, to thereby prevent the cardia from sliding through the patient's diaphragm opening into the patient's thorax, so as to maintain the supporting pressure against the patient's cardia sphincter muscle exerted from the patient's abdomen, wherein the movement restriction device having a size of at least 125 mm³ and a circumference of at least 15 mm, and a fixation device adapted to secure the movement restriction device in said position, when the movement restriction device is implanted. The distal part stabilizes and holds the proximal part and is adapted to be substantially invaginated in the stomach wall. The apparatus further comprises a stretching device comprising at least one operable stretching device implantable in the obese patient and adapted to stretch a portion of the patient's stomach wall such that satiety is created.

In an embodiment, the apparatus further comprises a stretching device comprising three or more mechanical parts engaged with different parts of the stomach wall, one part each, wherein said engagement includes suturing or stapling to the stomach wall or invaginating the mechanical parts in the stomach wall part with stomach to stomach sutures, wherein the three or more mechanical parts are adapted to move in relation to each other adapted to stretch three different wall portions, the stretching device further adapted to having said wall portions stretched independently from each other both regarding force used for stretching the stomach wall portion as well as, time periods the stretching is applied, and when the stretching is applied.

In an embodiment, the apparatus further comprises a stretching device comprising two or more hydraulic parts engaged with different parts of the stomach wall, one part each, wherein said engagement includes suturing or stapling to hydraulic part to the stomach wall or invaginating the hydraulic parts in the stomach wall part, with stomach to stomach sutures, wherein the two or more hydraulic parts are adapted to move in relation to each other adapted to stretch three different wall portions, the stretching device further adapted to having said wall portions stretched independently from each other both regarding force used for stretching the stomach wall portion as well as, time periods the stretching is applied, and when the stretching is applied.

In an embodiment, the apparatus further comprises a stretching device is engaged with a part of the stomach wall, including suturing or stapling the stretching device to the stomach wall or invaginating the stretching device in the stomach wall part, with stomach to stomach sutures, wherein the stretching device is further adapted to stretch a stomach wall portion controlling force used for stretching the stomach wall portion as well as, time periods the stretching is applied, and when the stretching is applied.

In an embodiment, the apparatus further comprises a stretching device comprising two parts engaged with different parts of the stomach wall, one part each, wherein said engagement includes suturing or stapling the parts to the stomach wall or invaginating the parts in the stomach wall part, with stomach to stomach sutures, wherein the stretching device further adapted to have different wall portions stretched independently from each other controlling force used for stretching the stomach wall portion as well as, time periods the stretching is applied, and when the stretching is applied.

In an embodiment, the apparatus further comprises an external control unit for controlling the distal part of the movement restriction device from the outside of the patient's body. The external control unit may comprise a wireless remote control adapted to control the device from the outside of the patient's body. Alternatively, the external control unit may comprise a subcutaneously placed switch or reservoir adapted to control the device from the outside of the patient's body.

In an embodiment, the apparatus further comprises a sensor or sensing device adapted to be implanted in the patient body, wherein the implantable control unit is adapted to control the device from the inside of the patient's body using information from said a sensor or sensing device, adapted to sense, direct or indirect, the food intake of the patient.

In accordance with another aspect of the present invention, there is provided an apparatus for treating a reflux disease and obesity of an obese patient having a stomach with a food cavity, the apparatus comprises an implantable movement restriction device having a proximal part and a distal part, wherein the distal part is adapted to be at least substantially invaginated by a stomach wall portion of the patient and having an outer surface that includes a biocompatible material, wherein the distal part of the movement restriction device is adapted to be placed inside the stomach with the outer surface of the volume filling device resting against the inside of the stomach wall, such that the volume of the food cavity is reduced in size by a volume substantially exceeding the volume of the volume filling device. The distal part of the movement restriction device has a maximum circumference of at least 30 millimeters.

It should be noted that any embodiment or part of embodiment or feature or method or associated system or part of system described herein may be combined in any combination.

### Alternative embodiments for volume filling devices treating obesity

In general terms, the present invention relates to an apparatus for treating obesity in a human or animal mammal patient comprising two or more volume filling device segments adapted to be assembled to an implantable volume filling device of a controlled size. The assembled volume filling combination device is adapted to be at least substantially be invaginated by a stomach wall portion of a patient, wherein said assembled volume filling device is adapted to be placed with the outer surface of the device resting against the stomach wall, such that the volume of the food cavity is reduced in size by a volume substantially exceeding the volume of the assembled volume filling device, when the assembled volume filling device is invaginated in the stomach wall. The assembled volume filling device is adapted to disassemble into its volume filling device segments if the assembled device leaves its implanted invaginated stomach position and inadvertently penetrates the stomach wall to become located inside the stomach including penetrating the stomach wall to retain a position inside the stomach, wherein said segments are adapted to separately pass through the food, thereby reducing risk for causing obstruction/ileus in the patients intestine. Preferably, the assembled volume filling device has a maximum circumference of at least 15 millimeters, and more preferably of at least 30 millimeters. Preferably, the volume filling device segments has at least part of an outer surface including a biocompatible material. By invaginating an assembled volume filling device by the stomach wall on the outside thereof this device is protected from the stomach acids and will thus remain functioning for a very long time.

According to one alternative, assembled the volume filling device is adapted to be placed inside the stomach with the outer surface of the volume filling device resting against the inside of the stomach wall, such that the volume of the food cavity is reduced in size by a volume exceeding the volume of the volume filling device. The volume filling device is preferably adapted to be placed inside the stomach with a gastroscope.

According to another alternative, assembled the volume filling device is adapted to be placed on the outside of the stomach wall with the outer surface of the volume filling device resting against the outside of the stomach wall, such that the volume of the food cavity is reduced in size by a volume substantially exceeding the volume of the volume filling device. Preferably, the assembled volume filling device is adapted to be completely invaginated by the stomach wall of the patient and to be placed outside the stomach wall via a gastroscopic instrument.

To this end the volume filling device segments may comprise an attachment device adapted to co-operate with a gripping instrument. Suitably, the assembled volume filling device is adapted to be non-invasively adjustable postoperatively.

The assembled volume filling device preferably is adapted to disassemble into its segments if it leaves its placed with the outer surface of the device resting against the stomach wall, i.e. its implanted at last partially invaginated position. The segments are preferably adapted to separately pass through the food passage way, thereby reducing risk of causing obstruction/ileus in the patient's intestine. The volume filling device segments can be adapted to pass through a trocar, for assembly and implantation of said volume filling device into the abdominal cavity. The volume filling device segments can have a flexible outer shape adapted to pass through a trocar. The volume filling device segments can be adapted to have a shape allowing them to be assembled into the device when implanted. In embodiment, at least one of the volume filling device segments have flexible outer surface. In one embodiment, at least one of the volume filling device segments comprises a rigid outer surface. In one embodiment, at least one of the volume filling device segments is hollow with a flexible outer surface. In one embodiment at least one of the volume filling device segments comprises an enclosure wall defining a chamber. At least one of the volume filling device segments can be adapted to be filled with at least one of a fluid a foam, a gel or a fluid that hardens to a solid material. In one embodiment the volume filling device segments comprises a homogenous and/or solid material, for example a solid body. In one embodiment at least of said segments comprises a flexible, non-elastic material. In one embodiment at least of said segments comprises a inflatable chamber and at least one tube connected thereto for supplementation of fluid to the chamber. It is preferred that the volume filling device segments are adapted to temporary be holding their assembled position, preferably by the invaginated stomach wall, or alternatively by an adhesive.

For its assembly, the volume filling device is provided with at least one assembly element that sufficiently fits with at least one assembly element of another segment, so the segments by fitting assembly elements can be assembled into the implantable volume filling device. Preferably, the segments for this purpose comprise a core part and a plurality of outer parts, and preferably, the at least one assembly element is selected among sufficiently fitting flanges and slits. The core part is adapted to receive and assemble the outer elements into an implantable volume filling device, and preferably the core part has assembly slits adapted to receive corresponding assembly flanges of the outer parts when assembling the volume filling device. In one embodiment the slits are distributed around the outer peripheral area of the core part. The outer parts are then provided with flanges sufficiently matching the slits to assemble the device. In another embodiment, the at least one assembly element immobilizes each of the volume filling device segments to a core part along a first plane, and wherein movement, and wherein the volume filling device segments and the core part further comprises a second assembly element, which following the assembly of said segments and core part, immobilize each segment and core part along a second plane in an angle to said first plane. For example, the first plane and the second plane can be substantially perpendicular. The second assembly element comprises mating elements, preferably with matching protrusions and recesses provided on the volume filling device segments and the core part, while the at least one assembly element further comprises protrusions and recesses. Preferably, the at least one assembly element comprises an assembly slit in the core part and an assembly flange in a segment, and wherein a mating element comprises a protrusion in said slit and a recess in said flange; or alternatively, the at least one assembly element comprises an assembly flange in the core part and an assembly slit in a segment, and wherein the a mating element comprises a protrusion in said slit and a recess in said flange.

In one particular embodiment, the apparatus preferably further comprises a guiding device, operable for assembling the volume filling device segments to an implantable volume filling device. Preferably, the guiding device is an operation wire operably connected to the segments.

The operation wire can be made of a material that is biodegradable in contact with the body fluid in the abdominal cavity so as to facilitate disassembly of the volume filling device into its segments. In order to assist with assembly procedure, each segment can be provided with at least one assembly element that sufficiently fits with at least one assembly element of another segment, so the segments by fitting assembly elements can be assembled into the implantable volume filling device. In one embodiment the segments comprise a core part and a plurality of outer parts and in one embodiment wherein the assembly elements are selected among sufficiently fitting flanges and slits. The core part preferably is adapted to receive and assemble the outer elements into an implantable volume filling device. In one embodiment the core part has assembly slits adapted to receive corresponding assembly flanges of the outer parts when assembling the volume filling device. Preferably the slits are distributed around the outer peripheral area of the core part. The slits and flanges may be designed to have loose fit adapted keep the segments together as a volume filling device at its implanted located, but assist with disassemble the device if it inadvertently leaves such a position, for example to the stomach cavity. In such event the degradation of the guiding device will also assist with disassembling the volume filling device into segments which are designed not cause any obstructions or in any other form damage the patient.

In order to assemble the segments, the operation wire is connected to the core part and to the outer parts so the outer parts can be sequentially assembled to the core part so as to assemble the volume filling device. For this purpose, the operation wire preferably is connected to the assembly flanges of the outer part and preferably, the core part is provided with at least one operation channel for receiving the operation wire. Preferably, each outer part is connected to two operation channels by the operation wire. In one embodiment, a first operation channel has a first orifice in an end surface of the core part and second orifice in a first slit of the core part, so when displacing the operation wire received in said first operation channel in a direction from said end surface, a first outer part is assembled to said core part. A second operation channel has two orifices in a second slit of the core part, so when displacing the operation wire connected to the first operation cannel in a directed from the end surface, a second outer part is assembled to said core part. Preferably, the guiding wire protrudes from the first channel orifice so it can be operated on with an instrument to displace the guiding wire and a first outer element so its assembly flange fits with its designated first assembly slit on the core element, and in a predetermined sequence in the same manner displacing the remaining outer elements so as to assemble the implantable volume filling device. The segments can comprise three or more outer parts assembled to designated slits of the core part with the guiding wire through operation channels having orifices in each designated slit of said core part. In one embodiment the volume filling device comprises one core part and four outer parts. However other ways of designing the segments within the present concept is feasible according to the skilled person. The so assembled volume filling device can retain a generally spherical form, but as will be described later other shapes and additional function elements are made part of the present invention.

The apparatus may comprise a fixation device, suitably two or more fixation devices, adapted to be involved in the fixation of the volume filling device to the stomach wall. The volume filling device including at least one of its segments may comprise a holding device adapted to be held by an instrument, suitably two or more holding devices, to simplify the implantation of the device.

At least a part of the assembled volume filling device may be made of a material which is not destructible by stomach acid. The volume filling device may be destructible by acids, for example hydrochloric acid.

In an embodiment, the volume filling device including at least one of its segments is inflatable to an expanded state and comprises an enclosure wall defining a chamber, wherein the volume filling device is inflated with a gel or fluid supplied into the chamber. At least one tube may be connected to the volume filling device for supplying gel or fluid to the chamber. An injection port connectible with the tube may be provided. Alternatively, the volume filling member may be provided with an inlet port for a fluid or a gel connectible to a gastroscopic instrument, wherein the inlet port comprises a fluid connection adapted to interconnect the inflatable device and the gastroscopic instrument.

The volume filling device may include a homogenous material, such as gel having a shore value of less than 15. The device may also be a solid body.

The volume filling device including at least one of its segments may comprise a rigid, elastic or flexible outer surface. Where the outer surface is rigid, it is rigid enough to maintain non-deformed when subject to forces created by stomach movements. The volume filling device may comprise a flexible non-elastic material.

In accordance with a first general design of the volume filling device, the device has a maximum circumference as seen in a plane perpendicular to an axis through the device. The circumferences of the device as seen in other planes perpendicular to said axis are equal to the maximum circumference or decrease as seen along said axis in the direction from the maximum circumference. For example, the device may be substantially egg shaped, spherically shaped, or substantially shaped like an egg with an indented middle section or like a bent egg.

In accordance with a second general design of the device, the circumference of the device as seen in a plane perpendicular to an axis through the device increases and decreases at least two times as the plane is displaced along said axis, or decreases and increases at least one time as the plane is displaced along said axis. For example, the device may be substantially shaped like a kidney.

The assembled volume filling device in yet another embodiment has a circumference as seen in a plane perpendicular to an axis through the body, and wherein the circumference constantly increases or remains constant when moving along said axis from a first end point of said axis to a intermediate point with a maximum, and the circumference constantly decreases or remains constant when moving from said intermediate point to a second end point of said axis.

The assembled volume filling device in yet another embodiment has a circumference as seen in a plane perpendicular to an axis through the body, and wherein the circumference constantly increases or remains constant when moving along said axis from a first end point of said axis to a first intermediate point with a first maximum, the circumference constantly decreases or remains constant when moving from said first intermediate point to a second intermediate point with a first minimum, the circumference constantly increases or remains constant when moving along said axis from said second intermediate point of said axis to a third intermediate point with a second maximum, and the circumference constantly decreases or remains constant when moving from said third intermediate point to a second end point of said axis.

### Further embodiments for treating obesity with volume filling devices:

The object of the present invention to provide obesity treatment apparatus, system with improved long term properties.

This object and others are obtained by an apparatus described in the appended claims. Thus, by providing an apparatus comprising at least one volume filling device adapted to be at least substantially invaginated by a stomach wall portion of the patient, and
wherein the volume filling device is adapted to be placed outside of the stomach wall with the outer surface of the volume filling device resting against the outside of the stomach wall, such that the volume of the food cavity is reduced in size by a volume substantially exceeding the volume of the volume filling device, whereby an apparatus for treating obesity is obtained.

The present invention is based on the realization that by invaginating a volume filling device by the stomach wall on the outside thereof this device is protected from the stomach acids and will thus remain functioning for a very long time.

In an embodiment, an apparatus for treating obesity of an obese patient having a stomach with a food cavity, comprises at least one volume filling device adapted to be at least substantially invaginated by a stomach wall portion of the patient, and
wherein the at least one volume filling device is adapted to be placed on the outside of the stomach wall with the outer surface of the at least one volume filling device resting against the outside of the stomach wall, such that the volume of the food cavity is reduced in size by a volume substantially exceeding the volume of the at least one volume filling device.

In an embodiment, an apparatus for treating obesity of an obese patient having a stomach with a food cavity, comprises two or more volume filling devices, wherein the outer surface of at least one volume filling device rests against the outside of the stomach wall via the outer surface of at least another volume filling device.

In an embodiment" at least one of the volume filling devices has a surface with a rugged texture or a flat surface.

In an embodiment" at least one of the volume filling devices has one of the following shapes: polyhedron, tetrahedron, hexahedron, octa hedron, dodecahedron, icosahedrons, and spherical.

In an embodiment,, the apparatus comprises a friction enhancing material.

In an embodiment,, the friction enhancing material is any of the following: a glue and an adhesive.

In an embodiment,, the apparatus comprises a friction reducing material on the outer surface of the volume filling devices.

In an embodiment,, the friction reducing material is a fluid reducing the friction between adjacent volume filling devices.

In an embodiment,, the apparatus comprises a combined volume filling device, said combined volume filling device comprising two or more volume filling devices.

In an embodiment,, the combined volume filling device further comprises a volume filling device receptacle for receiving and enclosing two or more volume filling devices.

In an embodiment,, the volume filling device receptacle comprises a friction reducing material on an inner surface thereof thereby reducing friction between said inner surface and said volume filling devices, when implanted.

In an embodiment,, the expandable volume filling device receptacle is adapted to be filled with a fluid to allow mutual movement between adjacent volume filling devices so that the combined shape of the combined volume filling device adapts to stomach wall movements, when said combined volume filling device is invaginated in a stomach wall.

In an embodiment" at least a wall portion of said volume filling device receptacle is flexible, stretchable, expandable, or elastic.

In an embodiment" said fluid is isotonic or hypertonic.

In an embodiment,, the volume filling devices are adapted to be directly or indirectly filled into the pouch formed by part of a stomach wall of a patient via a tubular instrument.

In an embodiment,, the volume filling devices comprise a solidifying liquid.

In an embodiment" said apparatus comprises a conduit adapted to deliver said solidifying liquid into said pouch trough said conduit.

In an embodiment" said plurality of volume filling devices are adapted to be interconnected after said volume filling devices have been inserted into a human or artificial pouch, or are adapted to be assembled to an implantable volume filling device.

In an embodiment" said plurality of volume filling devices are adapted to form an implantable volume filling device of a controlled size.

In an embodiment, the volume filling devices are adapted to be inserted into a pouch formed by part of a stomach wall.

In an embodiment" a combined volume filling device comprises two or more volume filling devices according to any of the preceding claims, where the combined volume filling device has a maximum circumference of at least 30 millimeters, wherein said volume filling device has a volume of less than 0.0002 m³

### Additional embodiments:

The assembled volume filling device and/or one or more of the volume filling device segments may have an elongated, rounded, bent and/or curved shape.

The assembled volume filling device has a circumference of at least 30, 50, 80 120, 150, 180 or 220 mm. The volume filling device may in all embodiments described herein also be combined with other volume filling devices in such a case the individual volume filling device or volume filling device segment may be really small with a circumference between 0,01 mm - 10 mm or larger. Any type of fluid may also be used.

The assembled volume filling device has a volume in the range of 0.0001 to 0.001 m³, or 0.00001 to 0.001 m³, or 0.00001 to 0.0002 m³. The volume of the volume filling device has in yet another embodiment a volume of less than 0.0002 m³. The individual volume filling device or volume filling device segment may be really small with a volume between 0,01 mm3 - 10mm3 or larger. Any type of fluid may also be used.

The volume filling device may comprise at least two interconnectable portions adapted to be placed inside the stomach as separate portions.

The volume filling device including at least one of its segments may comprise an elastic material, a bio-compatible material and/or silicone.

Suitably, the volume filling device is provided with at least one layer. For example, a metal layer, a Parylene layer, a polytetrafluoroethylene layer or a polyurethane layer. The layers may comprise multiple layers in any order. Suitably, one of the layers may be made of made of metal, silicon or PTFE. The volume filling device may comprise an outer surface layer of silicone, polyurethane, Teflon^{®}, or polytetrafluoroethylene, metal, Parylene, PTFE or a combination thereof. The volume filling device may comprise an inner surface layer of silicone, polyurethane, Teflon^{®}, or polytetrafluoroethylene, metal, Parylene, PTFE or a combination thereof. Other combinations include an inner surface layer of polytetrafluoroethylene and an outer layer of silicone, an inner surface layer of polytetrafluoroethylene, an intermediate layer of silicone, and an outer layer of Parylene, an inner surface layer of polyurethane and an outer layer of silicone, and an inner surface layer of polyurethane, an intermediate layer of silicone, and an outer layer of Parylene.

The volume filling device including at least one of its segments may comprise a fluid adapted to be transformed into solid state or fixed form. Such a fluid may be liquid polyurethane or isotonic. The fluid may comprises large molecules, such as iodine molecules, to prevent diffusion.

The volume filling device may have a maximum circumference of at least 50 millimeters, preferably at least 80 millimeters. Suitably, the volume filling device is deformable to a maximum diameter; so as to be insertable into a laparoscopic trocar.

Preferably, the assembled volume filling device is adapted to be kept in place by stomach-to-stomach sutures or staples to invaginate the device in the stomach wall. Advantageously, the volume filling device has varying circumference to better be kept in place invaginated in the stomach wall of the patient. The stomach-to-stomach sutures or staples may be provided with fixation portions exhibiting a structure adapted to be in contact with the stomach wall to promote growth in of human tissue to secure long term placement of the volume filling device attached to the stomach wall. The structure may comprise a net like structure.

In embodiment of the invention, the apparatus comprises a stretching device placed outside the stomach wall and adapted to stretch a portion of the stomach wall, thereby affecting the patient's appetite. Where the volume filling device including at least one of its segments is inflatable, the apparatus may comprise a fluid connection interconnecting the stretching device and the volume filling device.

In an embodiment, the apparatus comprises at least one operable stretching device implantable in an obese patient and adapted to stretch a portion of the patient's stomach wall and an operation device for operating the stretching device when implanted to stretch the stomach wall portion such that satiety is created.

In an embodiment, the apparatus comprises at least one operable stretching device implantable in the patient and adapted to stretch a portion of the patient's stomach wall, and an implantable control unit for automatically controlling the operable stretching device, when the control unit and stretching device are implanted, to stretch the stomach wall portion in connection with the patient eating such that satiety is created.

In an embodiment, the apparatus comprises at least one operable stretching device implantable in an obese patient and adapted to stretch a portion of the patient's stomach wall, wherein said stretching device comprising an expandable stretching reservoir and an operation device for operating the stretching device when implanted to stretch the stomach wall portion, wherein the volume filling device is inflatable and in fluid connection with said stretching reservoir, wherein said operation device comprises a pump for pumping fluid between said main reservoir and said stretching reservoir to stretch said stomach wall portion such that satiety is created. A control device may be provided for controlling said stretching device including said pump. The control device may comprise a wireless remote control adapted to control the stretching device from the outside of the patient's body, or an implantable control unit for controlling said stretching device. Alternatively, the control device may comprise a subcutaneously placed switch or reservoir adapted to control the stretching device from the outside of the patient's body. A sensor or sensing device to be implanted in the patient body may be provided, wherein the implantable control unit is adapted to control the stretching device from the inside of the patient's body using information from said a sensor or sensing device, adapted to sense, direct or indirect, the food intake of the patient.

In an embodiment, the assembled volume filling device comprises a main volume filling reservoir, a stretching device comprising at least one operable stretching device implantable in an obese patient and adapted to stretch a portion of the patient's stomach wall, wherein said stretching device comprising an expandable reservoir, adapted to be invaginated in the stomach wall at the upper part of the stomach, higher up than the inflatable main volume filling device when the patient is standing, wherein the volume filling device is inflatable and in fluid connection with said stretching reservoir, wherein normal contractions of the stomach wall, related to food intake, cause fluid to flow from said invaginated main volume filling reservoir lower placed onto the stomach wall adapted to cause said stretching reservoir to stretch said stomach wall portion such that satiety is created. The fluid connection between the main volume filling device reservoir and the stretching reservoir comprises a non-return valve. The fluid connection between the main volume filling device reservoir and the stretching reservoir comprises a release function adapted to release the volume in the stretching reservoir back to the main volume filling device reservoir. Said release function may comprise a fluid return connection of a substantially smaller area than said fluid connection, to slowly release back fluid to said main volume filling device reservoir from the stretching reservoir to release said stretching of the stomach wall portion. A further manual control device comprising a subcutaneously placed reservoir adapted to control the stretching device from the outside of the patient's body may be provided to further affect the stretching device to stretch the stomach wall portion.

In an embodiment, the a main volume filling device reservoir adapted to be inflatable may be provided, wherein the volume filling device further comprises an expandable structure, adapted to expand, when the device is invaginated in the stomach wall, wherein said structure comprising a bellow adapted to take into account the fibrosis surrounding the device when implanted, such that the movement of the bellow is substantially un-affected of said fibrosis.

In an embodiment, the apparatus comprises a stretching device comprising at least one operable stretching device implantable in an obese patient and adapted to stretch a portion of the patient's stomach wall and wherein the stretching device comprising a expandable structure, adapted to expand and stretch the stomach wall portion, when the device is invaginated in the stomach wall, wherein said structure comprising a special bellow adapted to take into account the fibrosis surrounding the device when implanted, such that the movement of the bellow is substantially un-affected of said fibrosis. An operation device for operating the stretching device may be provided to stretch the stomach wall portion such that satiety is created. The apparatus may comprise an implantable control unit for automatically controlling the operable stretching device, when the control unit and stretching device are implanted, to stretch the stomach wall portion in connection with the patient eating such that satiety is created.

In an embodiment, the apparatus comprises a stretching device comprising at least one operable stretching device implantable in an obese patient and adapted to stretch a portion of the patient's stomach wall such that satiety is created. The control device may comprise a wireless remote control adapted to control the stretching device from the outside of the patient's body or an implantable control unit for controlling said stretching device. Alternatively, said control device may comprise a subcutaneously placed switch or reservoir adapted to control the stretching device from the outside of the patient's body. A sensor or sensing device adapted to be implanted in the patient body may be provided, wherein the implantable control unit is adapted to control the stretching device from the inside of the patient's body using information from said sensor or sensing device, adapted to sense, direct or indirect, the food intake of the patient.

In an embodiment, the apparatus further comprises a stretching device comprising three or more mechanical parts engaged with different parts of the stomach wall, one part each, wherein said engagement includes suturing or stapling to the stomach wall or invaginating the mechanical parts in the stomach wall part with stomach to stomach sutures, wherein the three or more mechanical parts are adapted to move in relation to each other adapted to stretch three different wall portions, the stretching device further adapted to having said wall portions stretched independently from each other both regarding force used for stretching the stomach wall portion as well as, time periods the stretching is applied, and when the stretching is applied.

In an embodiment, the apparatus further comprises a stretching device comprising two or more hydraulic parts engaged with different parts of the stomach wall, one part each, wherein said engagement includes suturing or stapling to hydraulic part to the stomach wall or invaginating the hydraulic parts in the stomach wall part, with stomach to stomach sutures, wherein the two or more hydraulic parts are adapted to move in relation to each other adapted to stretch three different wall portions, the stretching device further adapted to having said wall portions stretched independently from each other both regarding force used for stretching the stomach wall portion as well as, time periods the stretching is applied, and when the stretching is applied.

In an embodiment, the apparatus further comprises a stretching device is engaged with a part of the stomach wall, including suturing or stapling the stretching device to the stomach wall or invaginating the stretching device in the stomach wall part, with stomach to stomach sutures, wherein the stretching device is further adapted to stretch a stomach wall portion controlling force used for stretching the stomach wall portion as well as, time periods the stretching is applied, and when the stretching is applied.

In an embodiment, the apparatus further comprises a stretching device comprising two parts engaged with different parts of the stomach wall, one part each, wherein said engagement includes suturing or stapling the parts to the stomach wall or invaginating the parts in the stomach wall part, with stomach to stomach sutures, wherein the stretching device further adapted to have different wall portions stretched independently from each other controlling force used for stretching the stomach wall portion as well as, time periods the stretching is applied, and when the stretching is applied.

In an embodiment, the apparatus is further adapted to treat reflux disease. To this end, it further comprises an implantable volume filling device adapted to be at least partly invaginated by the patient's stomach fundus wall and having an outer surface that includes a biocompatible material, wherein a substantial part of the outer surface of the volume filling device is adapted to rest against the stomach wall without injuring the latter in a position between the patient's diaphragm and at least a portion of the lower part of the invaginated stomach fundus wall, such that movement of the cardiac notch of the patient's stomach towards the patient's diaphragm is restricted, when the volume filling device is invaginated, to thereby prevent the cardia from sliding through the patient's diaphragm opening into the patient's thorax, so as to maintain the supporting pressure against the patient's cardia sphincter muscle exerted from the patient's abdomen, the volume filling device having a size of at least 125 mm3 and a circumference of at least 15 mm.

In another embodiment, the apparatus is further adapted to treat reflux disease. To this end, it further comprises an implantable volume filling device having an outer surface including a biocompatible material, wherein the movement restriction device is adapted to rest with at least a part of its outer surface against the patient's stomach fundus wall, in a position between the patient's diaphragm and the fundus wall, such that movement of the cardiac notch of the patient's stomach towards the patient's diaphragm is restricted, when the movement restriction device is implanted in the patient, to thereby prevent the cardia from sliding through the patient's diaphragm opening into the patient's thorax, so as to maintain the supporting pressure against the patient's cardia sphincter muscle exerted from the patient's abdomen, wherein the volume filling device having a size of at least 125 mm3 and a circumference of at least 15 mm, and an a fixation device adapted to secure the volume filling device in said position, when the volume filling device is implanted.

In another embodiment, the apparatus is further adapted to treat reflux disease. To this end, it further comprises an implantable movement restriction device adapted to be at least partly invaginated by the patient's stomach fundus wall and having an outer surface that includes a biocompatible material, wherein a substantial part of the outer surface of the movement restriction device is adapted to rest against the stomach wall without injuring the latter in a position between the patient's diaphragm and at least a portion of the lower part of the invaginated stomach fundus wall, such that movement of the cardiac notch of the patient's stomach towards the patient's diaphragm is restricted, when the movement restriction device is invaginated, to thereby prevent the cardia from sliding through the patient's diaphragm opening into the patient's thorax, so as to maintain the supporting pressure against the patient's cardia sphincter muscle exerted from the patient's abdomen, the movement restriction device having a size of at least 125 mm3 and a circumference of at least 15 mm, further comprising a stretching device comprising at least one operable stretching device implantable in the obese patient and adapted to stretch a portion of the patient's stomach wall such that satiety is created.

In another embodiment, the apparatus is further adapted to treat reflux disease. To this end, it further comprises an implantable movement restriction device having an outer surface including a biocompatible material, wherein the movement restriction device is adapted to rest with at least a part of its outer surface against the patient's stomach fundus wall, in a position between the patient's diaphragm and the fundus wall, such that movement of the cardiac notch of the patient's stomach towards the patient's diaphragm is restricted, when the movement restriction device is implanted in the patient, to thereby prevent the cardia from sliding through the patient's diaphragm opening into the patient's thorax, so as to maintain the supporting pressure against the patient's cardia sphincter muscle exerted from the patient's abdomen, wherein the movement restriction device having a size of at least 125 mm³ and a circumference of at least 15 mm, and a fixation device adapted to secure the movement restriction device in said position, when the movement restriction device is implanted, further comprising a stretching device comprising at least one operable stretching device implantable in the obese patient and adapted to stretch a portion of the patient's stomach wall such that satiety is created.

In an embodiment, the apparatus further comprises an external control unit for controlling the volume filling device from the outside of the patient's body. The external control unit may comprise a wireless remote control adapted to control the device from the outside of the patient's body. Alternatively, the external control unit may comprise a subcutaneously placed switch or reservoir adapted to control the device from the outside of the patient's body.

In an embodiment, the apparatus further comprises a sensor or sensing device adapted to be implanted in the patient body, wherein the implantable control unit is adapted to control the device from the inside of the patient's body using information from said a sensor or sensing device, adapted to sense, direct or indirect, the food intake of the patient.

In accordance with another aspect of the present invention, there is provided an apparatus for treating obesity of an obese patient having a stomach with a food cavity, the apparatus comprising at least one volume filling device adapted to be at least substantially invaginated by a stomach wall portion of the patient and having an outer surface that includes a biocompatible material, wherein the volume filling device is adapted to be placed inside the stomach with the outer surface of the volume filling device resting against the inside of the stomach wall, such that the volume of the food cavity is reduced in size by a volume substantially exceeding the volume of the volume filling device, the volume filling device having a maximum circumference of at least 30 millimeters.

In a preferred embodiment, the apparatus comprises at least one switch implantable in the patient for manually and non-invasively controlling the volume filling device.

In another preferred embodiment, the apparatus comprises a wireless remote control for non-invasively controlling the volume filling device.

In a preferred embodiment, the apparatus comprises a hydraulic operation device for operating volume filling device.

In one embodiment, the apparatus comprises comprising a motor or a pump for operating the volume filling device.

In one embodiment the apparatus comprises an adjustment device for adjusting the size and/or shape of the volume filling device including at least one of its segments. The size of the volume filling device can be hydraulically adjustable and the adjustment device comprises a hydraulic fluid reservoir that, when implanted in the patient, is connected to at least one of the volume filling device segments, and wherein the size of the volume filling device is non-invasively regulated by moving hydraulic fluid from the reservoir to at least one volume filling device segment, thereby adjusting the size of at least one segment of the volume filling device. The apparatus can further comprise hydraulic regulation device comprising at least one chamber that, when implanted in the patient, is invaginated in the patient's stomach wall with the volume filling devices and in being connection therewith, and wherein the amount of hydraulic fluid contained in at least one of the volume filling device segments is non-invasively regulated by distributing fluid between the hydraulic reservoir and the at least one chamber. Preferably, the at least one chamber, when implanted in the patient, is filled with the hydraulic fluid using a pump in the reservoir so as to stretch the fundus wall to create satiety in the patient. The adjustment device can further comprise a reverse servo comprising three adjustable reservoirs with hydraulic fluid, wherein a small volume of fluid in a first reservoir placed subcutaneously, being part of a first closed system including a second reservoir, is compressed with a high force per area unit for moving a small volume of hydraulic fluid, and wherein the second reservoir affects a larger volume of hydraulic fluid in a third reservoir, the third reservoir being part of a second closed system having larger volume than said first reservoir, thereby creating a movement of a larger total volume of hydraulic fluid with less force per area unit. The apparatus of the discussed embodiment can comprise a wireless remote control, wherein the volume filling device, when implanted in the patient, is non-invasively regulated by the wireless remote control. The apparatus of the discussed embodiment can further comprise an energy source that powers the adjustable volume filling device when implanted in a patient. The energy source preferably comprises an internal energy source implantable in the patient. The energy source can also comprise an external energy source transmitting wireless energy. The internal energy source, when implanted in the patient can be chargeable by the wireless energy transmitted by the external energy source. The wireless remote control can comprise at least one external signal transmitter and receiver, further comprising an internal signal receiver and transmitter implantable in the patient for receiving signals transmitted by the external signal transmitter and sending feedback signals back to the remote control. The wireless control signal can comprise an electric or magnetic field, or a combined electric and magnetic field.

In one embodiment, the apparatus comprises a wireless energy transmitter for non-invasively energizing any part of the apparatus in need of energy supplementation. The energy transmitter preferably transmits energy by at least one wireless energy signal. Preferably, the wireless energy comprises a wave signal or a field, or the wireless energy signal can comprise an electric or magnetic field, or a combined electric and magnetic field. The wave signal preferably is selected from the group consisting of: a sound wave signal, an ultrasound wave signal, an electromagnetic wave signal, an infrared light signal, a visible light signal, an ultra violet light signal, a laser light signal, a micro wave signal, a radio wave signal, an x-ray radiation signal and a gamma radiation signal. The apparatus of this embodiment can further comprise an implantable accumulator and an energy transforming device transforming wireless energy to electric energy, wherein the electric energy is used at least partly to charge the accumulator or to run any energy consuming part of the apparatus direct from the energy transforming device.

In one embodiment, the apparatus comprises a sensor sensing a parameter, a functional parameter or a physical parameter of the patient. The functional parameter is correlated to a wireless transfer of energy for charging an internal energy source implantable in the patient. The apparatus can further comprise a feedback device that, when implanted in the patient, sends feedback information from inside the patient's body to the outside thereof, the feedback information being related to the functional parameter. The apparatus can also comprise an implantable internal control unit for controlling the volume filling device in response to the sensor sensing the functional parameter. The sensor for sensing the physical parameter is a pressure sensor or a motility sensor. An implantable internal control unit can control o the volume filling device of the apparatus in response to the sensor sensing the physical parameter.

In one embodiment, the apparatus comprises an operation device for operating the volume filling device in order to control its size and/or shape. For this purpose, the operation device can comprise a motor or a pump.

In one embodiment of the apparatus, the volume filling device is adapted to further receive wireless energy, wherein the wireless energy is used to power the operation device to create kinetic energy for the operation of the volume filling device. The wireless energy can for example be used to directly power the operation device to create kinetic energy for the operation of the volume filling device, as the wireless energy is being transmitted by the energy-transmission device. The volume filling deice may also be adapted to receive energy from an energy transforming device directly during wireless energy transfer or from an energy accumulator, being rechargeable by the wireless energy and energy transforming device The wireless energy, preferably comprises a wave signal that is selected from the group consisting of: a sound wave signal, an ultrasound wave signal, an electromagnetic wave signal, an infrared light signal, a visible light signal, an ultra violet light signal, a laser light signal, a micro wave signal, a radio wave signal, an x-ray radiation signal and a gamma radiation signal. The wireless energy signal can also comprise an electric or magnetic field, or a combined electric and magnetic field.

In one embodiment of the apparatus it further comprises implantable electrical components including at least one voltage level guard, or at least one constant current guard.

In general terms any applicable feature or embodiment or part of embodiment or method described herein are, when applicable, valid for both the assembled volume filling device as well as for the volume filling device segments.

It is understood that a skilled person is in the position of combining steps, changing the order of steps, and combining elements of the different embodiments of the invention without inventive effort, and without departing from the scope of the invention as defined in the description and claims.

### Operational Method

An operational method to be combined in any way using any apparatus, part of apparatus or system or part of system or any claimed embodiment described anywhere in this document.

A method or part of method to be used in any combination and using any apparatus or part of apparatus or any feature in any combination where the following method steps is applicable , wherein said method may comprise one or more of the following operational method steps:
- introducing said instrument into the throat,
- passing down the esophagus,
- placing an anvil or unit for delivery of fixating members in the esophagus between the cardia and the diaphragm level, for engaging in the fixation of the esophagus to the stomach tissue,
- passing down the esophagus and additionally further down into the stomach,
- filling the stomach with gas to expand the stomach,
- sucking fluid from the stomach,
- looking at a guiding vision when said instrument comprising a camera,
- engaging the instrument with the stomach,
- creating and suturing at least one pouch of the stomach wall,
- filling said at least one pouch with a fluid and/or volume filling device or two or more volume filling devices,
- deliver a plurality of volume filling devices into said pouch created in the stomach tissue through a tubular member,
- passing through the stomach wall with said instrument,
- passing through the stomach wall with said instrument for the placement of a volume filling device on the outside of the stomach wall ,
- passing through the stomach wall with said instrument for the placement of a tube allowing placement of a subcutaneous injection port,
- placing an subcutaneous injection port,
- suturing or stapling the stomach wall from the inside thereof to the esophagus,
- suturing or stapling the stomach wall to stomach wall from the inside of the stomach,
- engaging the instrument with the esophagus,
- suturing or stapling one layer of stomach tissue to one layer of esophageal tissue,
- suturing or stapling two layers of stomach tissue to one layer of esophageal tissue,
- suturing or stapling three layers of stomach tissue to one layer of esophageal tissue,
- suturing or stapling four layers of stomach tissue to one layer of esophageal tissue,
- suturing or stapling one or more layers of stomach tissue to two or more positions on the esophageal tissue, the esophagus having an esophagus center axis, the esophagus further having an inner and outer substantially cylindrical surface extending radially in relation to the esophagus center axis and wherein the stomach tissue is attached to esophageal tissue both at a first point along a first esophagus surface length axis, substantially parallel to said esophagus center axis and at a second point along a second esophagus surface length axis, substantially parallel to said esophagus center axis, at a distance from said first esophagus surface length axis, radially in relation to said esophagus center axis,
- delivering fixating members by a unit placed on said instrument,
- penetrating at least one layer of stomach tissue and one layer of esophagus tissue with said fixating members,
- placing said fixating members above the gastro-esophageal junction for creating a tunnel between the esophagus and stomach above said junction,
- placing an esophagus part in the esophagus and a stomach part in the stomach,
- placing the fixating member substantially between the stomach and esophagus part,
- inserting said instrument into the main stomach cavity through the cardia and adapted to direct the instrument in cranial direction to reach a position of said unit above said junction,
- allowing the tunnel a substantially unrestricted contraction and release of the cardia closing sphincter muscle placed in said junction, when such a tunnel has been created.

A method or part of method to be used in any combination and using any apparatus or part of apparatus or any feature in any combination where the following method steps is applicable , wherein said method comprises one or more of the following operational method steps:
- cutting the skin of a patient
- creating an opening in the abdominal wall of the patient
- Introducing said instrument into the abdominal cavity through said opening in the abdominal wall,
- engaging the instrument with the stomach,
- pulling down into the stomach wall for creating at least one pre-shaped pouch of the stomach wall,
- clamping the stomach wall for creating at least one pre-shaped pouch of the stomach wall,
- suturing or stapling at least one pouch in the stomach wall,
- filling said at least one pouch with a fluid and/or volume filling device or two or more volume filling devices,
- deliver a plurality of volume filling devices into said pouch created in the stomach tissue through a tubular member,
- passing through the stomach wall into the stomach with said instrument,
- passing through the stomach wall with said instrument for the placement of a volume filling device on the inside of the stomach wall,
- passing through the stomach wall with said instrument for suturing the stomach wall to the esophagus wall,
- placing a volume filling device on the outside of the stomach wall,
- invaginating said volume filling device in the stomach wall
- placing a subcutaneous injection port,
- suturing or stapling the stomach wall to stomach wall from the outside of the stomach,
- suturing or stapling the stomach wall to stomach wall from the outside of the stomach without penetrating the mucosa,
- suturing or stapling two layers of stomach wall to one or two layers of stomach wall,
- engaging the instrument with the esophagus,
- clamping the on both sides of the esophagus for fixating one layer of esophageal wall to stomach tissue,
- clamping the on both sides of the esophagus and the stomach fundus wall for fixating one layer of esophageal wall to one or two layers of stomach tissue,
- introducing a tube or a gastroscopic instrument into the esophagus comprising an anvil member or a fixating delivery member involving in the fixation of the esophagus to the stomach,
- coordinating the position of the anvil member or a fixating delivery member inside the esophagus to said instrument clamping around the esophagus,
- suturing or stapling one layer of stomach tissue to one layer of esophageal tissue,
- suturing or stapling two layers of stomach tissue to one layer of esophageal tissue,
- suturing or stapling three layers of stomach tissue to one layer of esophageal tissue,
- suturing or stapling four layers of stomach tissue to one layer of esophageal tissue,
- stapling using staplers of different stapling depths at different positions in a stapler row,
- stapling stomach to esophagus with one first stapler depth and stapling stomach to stomach with a second smaller stapler depth,
- stapling a pouch with stomach to stomach sutures in a stapler row, further comprising stapling the esophagus with staplers of a larger depth included as a part of said stapler row,
- suturing or stapling one or more layers of stomach tissue to two or more positions on the esophageal tissue, the esophagus having an esophagus center axis, the esophagus further having an inner and outer substantially cylindrical surface extending radially in relation to the esophagus center axis and wherein the stomach tissue is attached to esophageal tissue both at a first point along a first esophagus surface length axis, substantially parallel to said esophagus center axis and at a second point along a second esophagus surface length axis, substantially parallel to said esophagus center axis, at a distance from said first esophagus surface length axis, radially in relation to said esophagus center axis,
- delivering fixating members by a unit placed on said instrument,
- penetrating at least one layer of stomach tissue and one layer of esophagus tissue with said fixating members,
- placing said fixating members above the gastro-esophageal junction for creating a tunnel between the esophagus and stomach above said junction,
- allowing the tunnel a substantially unrestricted contraction and release of the cardia closing sphincter muscle placed in said junction, when such a tunnel has been created,
- placing an esophagus part in the esophagus and a stomach part in the stomach via an introduction into the stomach cavity,
- placing the fixating member substantially between the stomach and esophagus part,
- inserting said instrument into the main stomach cavity below said junction and adapted to direct the instrument in cranial direction to reach a position of said unit above said junction,
- operating a joint comprised in said instrument, for enabling said instrument to be inserted into the main stomach cavity bending said joint in a direction to reach a position of said part of the unit in the esophagus above said junction.

A method or part of method to be used in any combination and using any apparatus or part of apparatus or any feature in any combination where the following method steps is applicable, wherein said method comprises one or more of the following laparoscopic operational method steps:
- cutting the skin of a patient
- introducing a tube through the abdominal wall,
- filling a fluid or gas into the abdominal cavity,
- introducing two or more trocars into the abdominal cavity,
- introducing a camera into the abdominal cavity through one of the trocars,
- introducing said instrument into the abdominal cavity through a trocar,
- engaging the instrument with the stomach,
- pulling down into the stomach wall for creating at least one pre-shaped pouch of the stomach wall,
- clamping the stomach wall for creating at least one pre-shaped pouch of the stomach wall,
- suturing or stapling at least one pouch in the stomach wall,
- filling said at least one pouch with a fluid and/or a volume filling device or two or more volume filling devices,
- deliver a plurality of volume filling devices into said pouch created in the stomach tissue through a tubular member,
- passing through the stomach wall into the stomach with said instrument,
- passing through the stomach wall with said instrument for the placement of a volume filling device on the inside of the stomach wall,
- passing through the stomach wall with said instrument for suturing the stomach wall to the esophagus wall,
- placing a volume filling device on the outside of the stomach wall,
- invaginating said volume filling device in the stomach wall
- placing a subcutaneous injection port,
- suturing or stapling the stomach wall to stomach wall from the outside of the stomach,
- suturing or stapling two layers of stomach wall to one or two layers of stomach wall,
- suturing or stapling the stomach wall to stomach wall from the outside of the stomach without penetrating the mucosa,
- engaging the instrument with the esophagus,
- clamping the on both sides of the esophagus for fixating one layer of esophageal wall to stomach tissue,
- clamping the on both sides of the esophagus and the stomach fundus wall for fixating one layer of esophageal wall to one or two layers of stomach tissue,
- introducing a tube or a gastroscopic instrument into the esophagus comprising an anvil member or a fixating delivery member involving in the fixation of the esophagus to the stomach,
- coordinating the position of the anvil member or a fixating delivery member inside the esophagus to said instrument clamping around the esophagus,
- suturing or stapling one layer of stomach tissue to one layer of esophageal tissue,
- suturing or stapling two layers of stomach tissue to one layer of esophageal tissue,
- suturing or stapling three layers of stomach tissue to one layer of esophageal tissue,
- suturing or stapling four layers of stomach tissue to one layer of esophageal tissue,
- stapling using staplers of different stapling depths at different positions in a stapler row,
- stapling stomach to esophagus with one first stapler depth and stapling stomach to stomach with a second smaller stapler depth,
- stapling a pouch with stomach to stomach sutures in a stapler row, further comprising stapling the esophagus with staplers of a larger depth included as a part of said stapler row,
- suturing or stapling one or more layers of stomach tissue to two or more positions on the esophageal tissue, the esophagus having an esophagus center axis, the esophagus further having an inner and outer substantially cylindrical surface extending radially in relation to the esophagus center axis and wherein the stomach tissue is attached to esophageal tissue both at a first point along a first esophagus surface length axis, substantially parallel to said esophagus center axis and at a second point along a second esophagus surface length axis, substantially parallel to said esophagus center axis, at a distance from said first esophagus surface length axis, radially in relation to said esophagus center axis,
- delivering fixating members by a unit placed on said instrument,
- penetrating at least one layer of stomach tissue and one layer of esophagus tissue with said fixating members,
- placing said fixating members above the gastro-esophageal junction for creating a tunnel between the esophagus and stomach above said junction,
- allowing the tunnel a substantially unrestricted contraction and release of the cardia closing sphincter muscle placed in said junction, when such a tunnel has been created,
- placing an esophagus part in the esophagus and a stomach part in the stomach via an introduction into the stomach cavity,
- placing the fixating member substantially between the stomach and esophagus part,
- inserting said instrument into the main stomach cavity below said junction and adapted to direct the instrument in cranial direction to reach a position of said unit above said junction,
- operating a joint comprised in said instrument, for enabling said instrument to be inserted into the main stomach cavity bending said joint in a direction to reach a position of said part of the unit in the esophagus above said junction.

Please note that any embodiment or part of embodiment as well as any method or part of method or any apparatus or part of apparatus or any feature or part of feature or any system or part of system or any figure or part of figure could be combined in any applicable way. All examples herein should be seen as part of the general description and therefore possible to combine in any way in general terms.

### Brief description of drawings

The invention is now described, by way of example, with reference to the accompanying drawings, in which:
Fig. 1 is an overall view of a patient showing the outlines of the stomach.
Fig. 2 is an overall view of a stomach showing the outlines of the cardia.
Figs. 3-5 show the method for restoring the relocation of the cardia and the fundus in a patient suffering from reflux disease.
Figs. 6 - 7c show a first embodiment for fixating stomach tissue to esophagus.
Figs. 7d - 8c show an embodiment for fixating a plurality of stomach tissue layers to esophagus and creating a pouch.
Figs. 9a - 9e illustrate embodiments adapted for invaginating a volume filling devices in a tissue wall.
Figs. 10a-10f show embodiments of a method and an instrument with vacuum ports adapted for invaginating a volume filling device in a tissue wall.
Figs. 11a-11j and 12 a- 12h show embodiments adapted for invaginating a volume filling devices in a tissue wall.
Figs. 13a - 13e show an embodiment adapted to suck stomach tissue into a chamber creating a pouch.
Figs. 14 and 15 show an embodiment of an instrument comprising two operable joints introduced through the throat adapted to pull stomach tissue into a lumen.
Figs. 16 and 17 show the instrument from 14 and 15 further adapted for placing and fastening stomach tissue to esophagus and creating a pouch.
Figs. 18 and 19 show an embodiment of an instrument comprising two operable joints for sucking stomach tissue into a lumen of the instrument.
Figs. 20 and 21 show the instrument comprising two operable joints of 14 -19 and 22 introducing a volume filling device in a pouch created of stomach tissue.
Fig. 22 illustrates a stapling port with a releasable wire.
Fig 23 illustrates a stapling port with a releasable wire.
Fig 24 and 25 shows an embodiment of an instrument introduced from the stomach cavity with one part in esophagus and another part pulling stomach tissue into a lumen.
Fig. 26 and 27 shows an embodiment of an instrument introduced from the stomach cavity with one part in esophagus and another part sucking stomach tissue into a lumen.
Fig. 28 and 29 shows an instrument of 24, 25, 26 and 27 introducing a volume filling device in a pouch of stomach tissue
Fig. 30 shows an instrument of 24, 25, 26 and 27 adapted to fixate stomach tissue to esophagus and creating a pouch.
Fig. 31 illustrates a stapling port with a releasable wire.
Fig 32 and 33 shows an embodiment of an abdominal method and instrument adapted to form a pouch of fundus tissue.
Fig. 34 and 35 illustrates the instrument of 32 and 33 and a method for securing and fixate the opening of the pouch to esophagus.
Fig. 36 and 37 shows an embodiment of an abdominal method and instrument of creating a pouch of fundus tissue.
Fig. 38 and 39 shows the instrument of 34-37 introducing a volume filling device in the pouch of fundus tissue.
Fig. 40 shows another embodiment of a hinged laparoscopic instrument comprising a closed three part head.
Fig 41. Illustrates the instrument of fig 41 introduced to the stomach through esophagus and shows an open three part head where two parts is positioned in the stomach and one in the esophagus.
Fig 42 shows the instrument of fig. 41 from a cranial-caudal view.
Fig. 43 shows another embodiment of a hinged laparoscopic instrument comprising a closed three part head.
Fig. 44 shows the instrument of fig. 43, introduced to the stomach through the stomach wall, with an open three part head with two parts positioned the stomach and one in esophagus.
Fig. 45 shows the instrument of fig. 44 from a cranial-caudal view.
Fig. 46 shows another embodiment of a hinged laparoscopic instrument comprising a closed three part head.
Fig. 47 illustrates the instrument of fig 46 abdominally positioned and adapted to fixate stomach tissue to esophagus.
Fig. 48a illustrates the instrument of fig. 47 from a caudal-cranial view.
Fig. 48b shows an alternative embodiment of the instrument.
Fig. 48c - 51 shows an embodiment of a laparoscopic instrument with two fixation units and one anvil.
Fig 52 - 53 shows an embodiment of a luminal instrument introducing a volume filling device through the wall into the pouch.
Fig 54, 55 a-b and 56 shows a method and instrument for introducing a plurality of volume filling devices though the wall into the pouch.
Fig 57 shows one embodiment of a polyhedral volume filling device.
Fig. 58 - 60 shows a method and instrument adapted to introduce a fluid or a solidifying fluid through the wall into a pouch.
Fig. 61-63 shows an embodiment and a method for introduction and assembly of an interconnected volume filling device segments.
Fig. 64 shows an assembled volume filling device of fig 61.
Fig. 65a shows the insertion of an expandable member.
Fig. 65b shows an expandable member filled with a solidifying fluid.
Fig. 66 shows an expandable member filled with a plurality of volume filling devices.
Fig 67 shows a plurality of volume filling devices in a solidifying fluid invaginated in a tissue pouch.
Fig. 68 shows an invaginated balloon filled with a plurality of volume filling devices and a solidifying fluid.
Fig. 69a-c shows an invaginated movement restriction device and/or combined volume filling device in section filled with a plurality of volume filling devices or volume filling device segments.

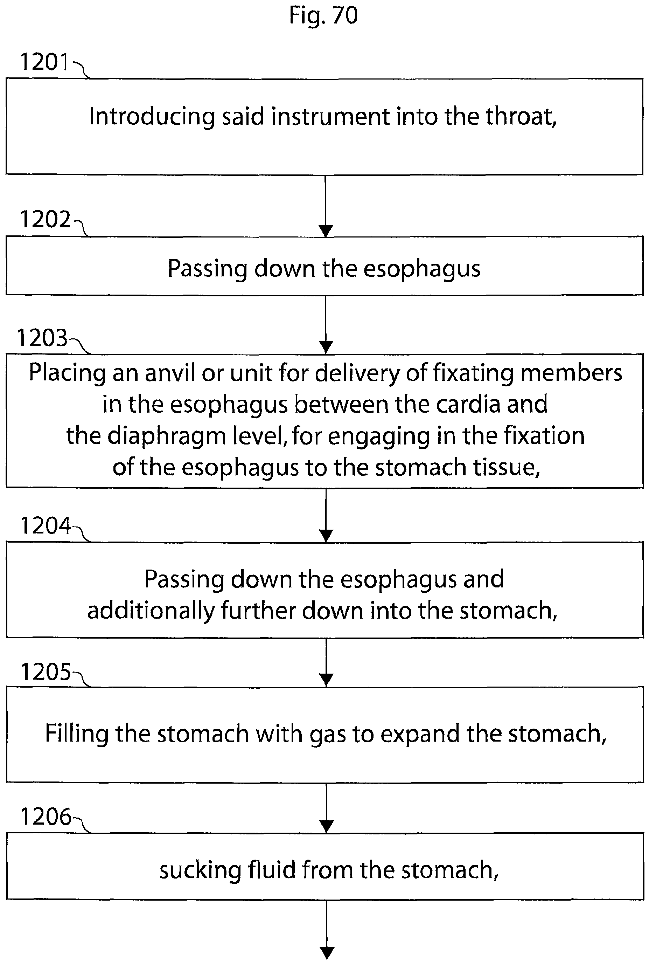

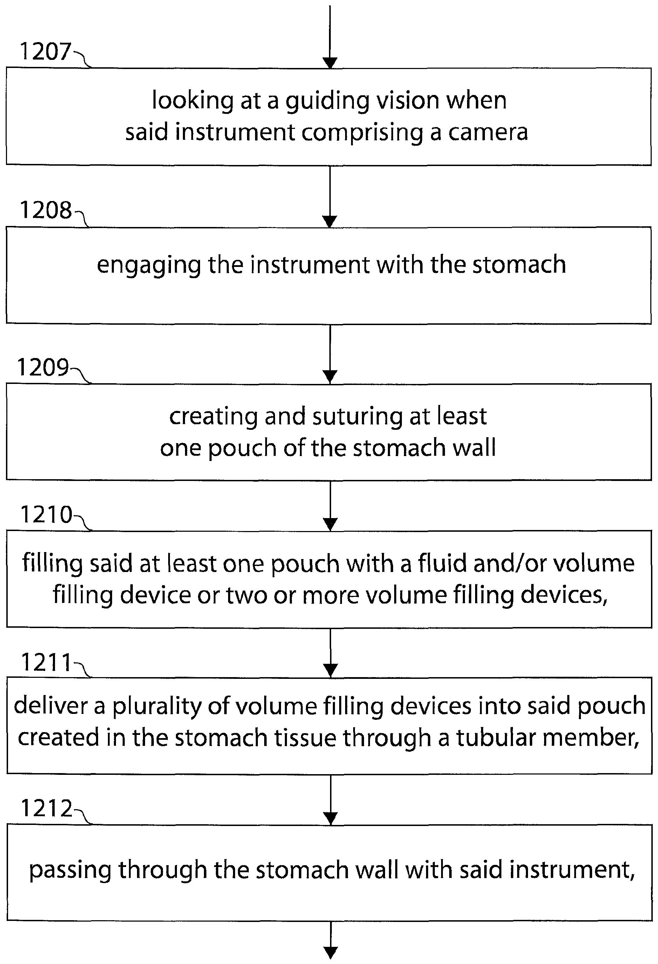

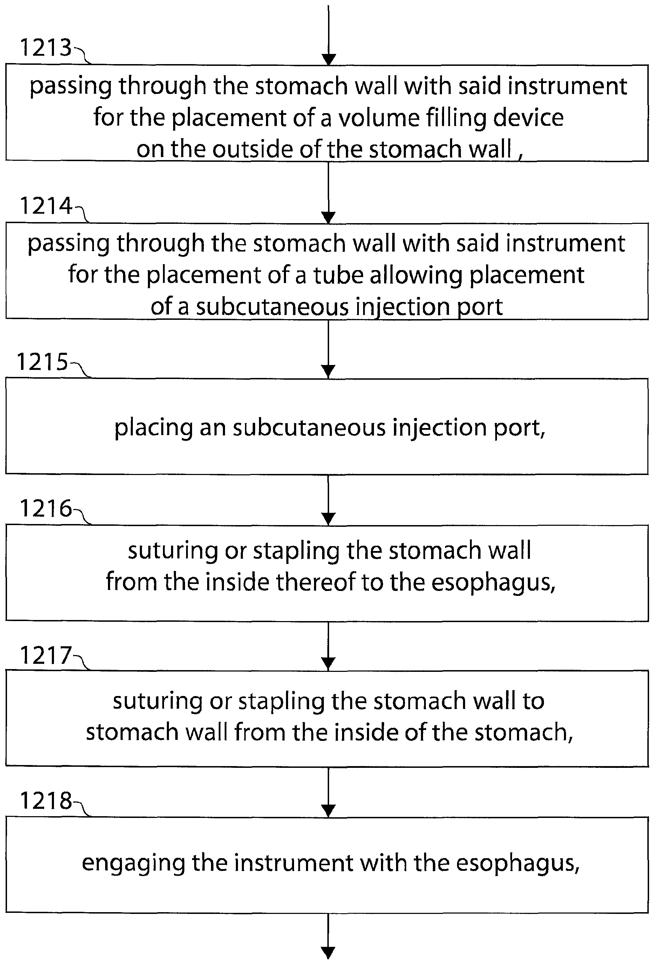

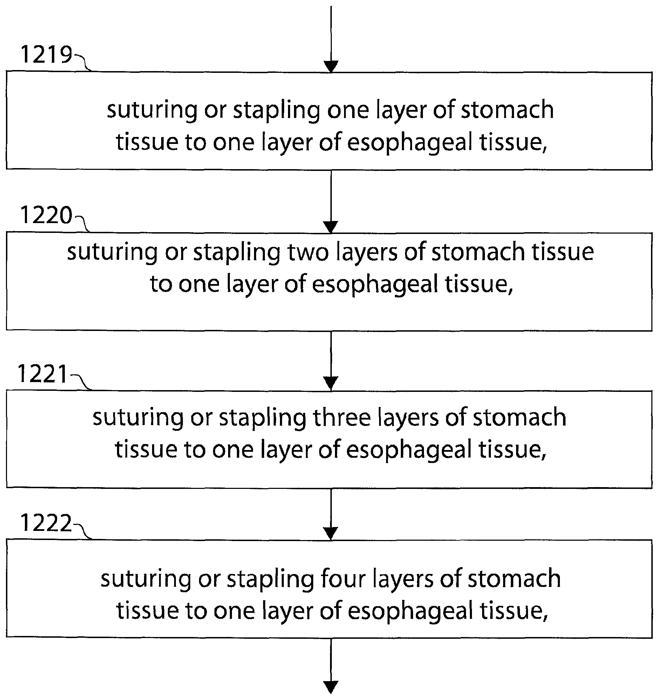

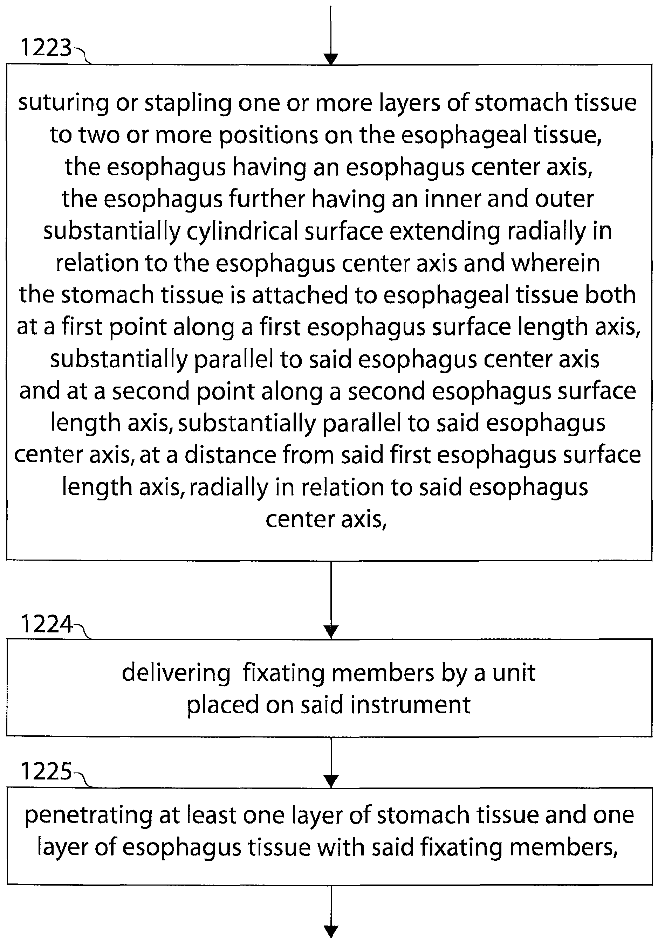

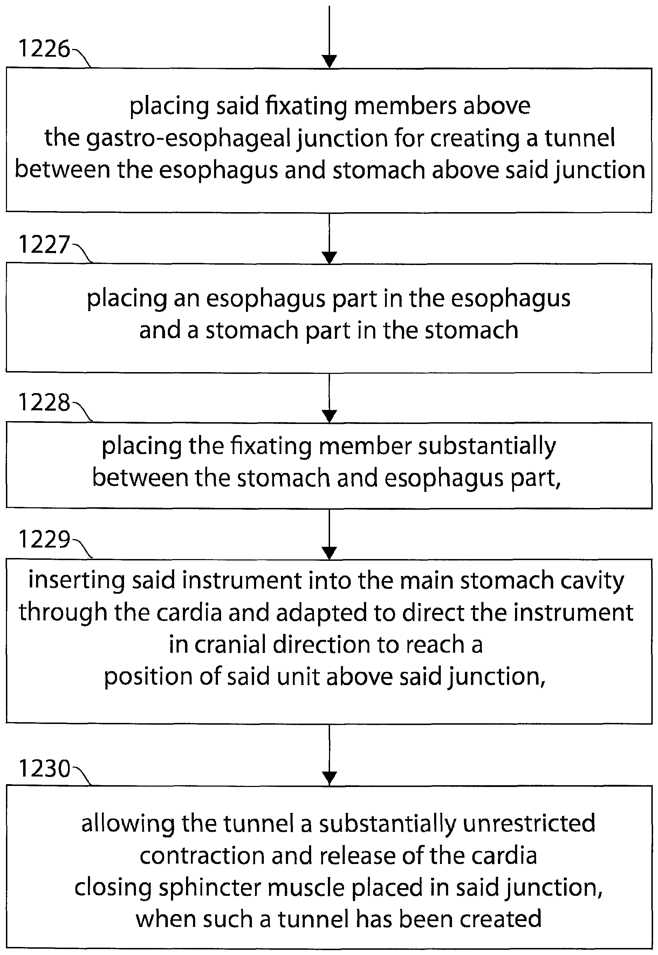

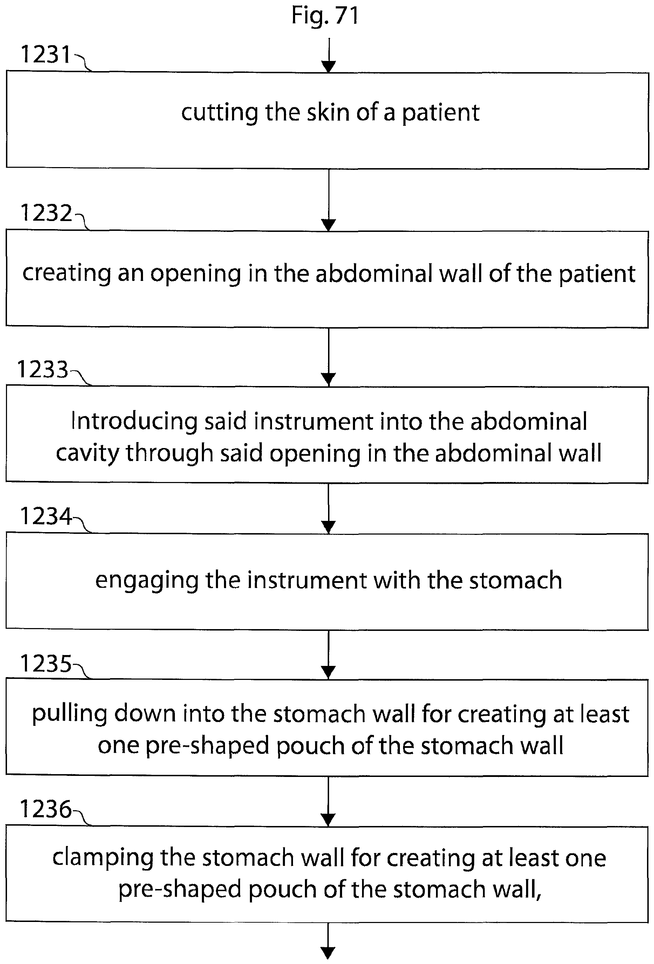

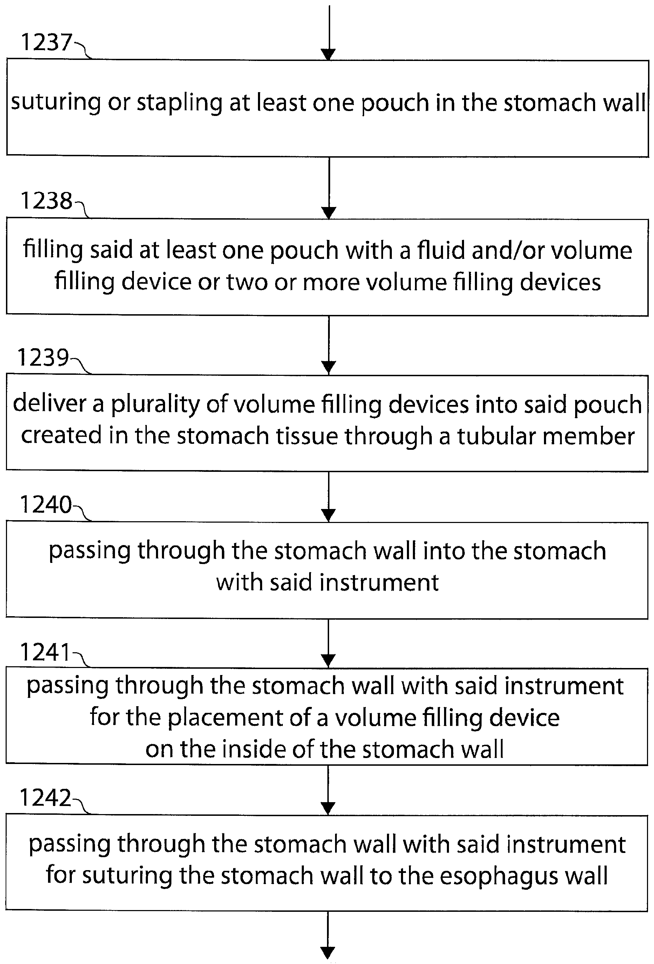

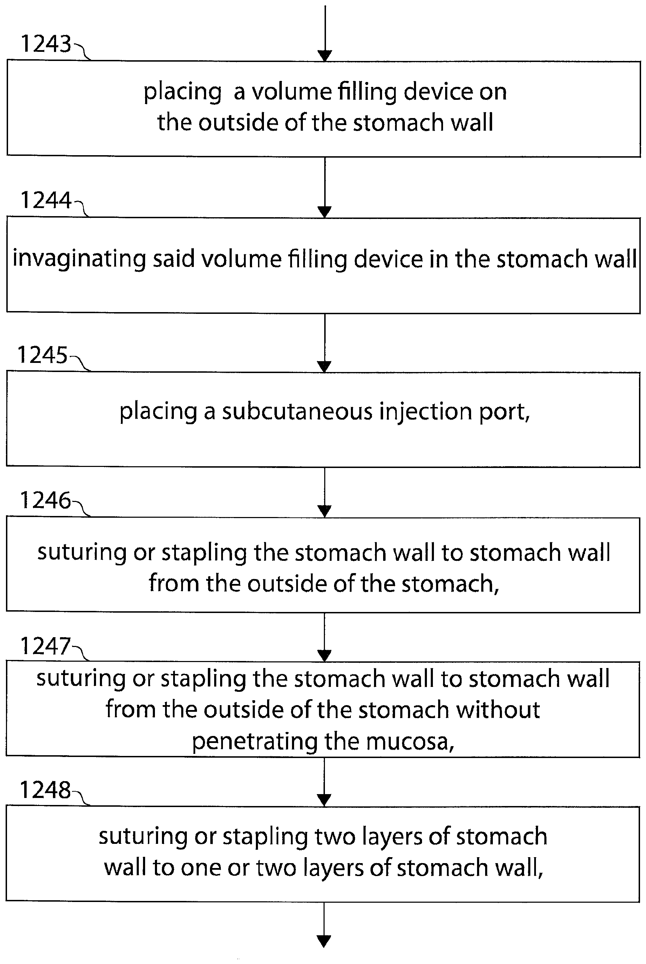

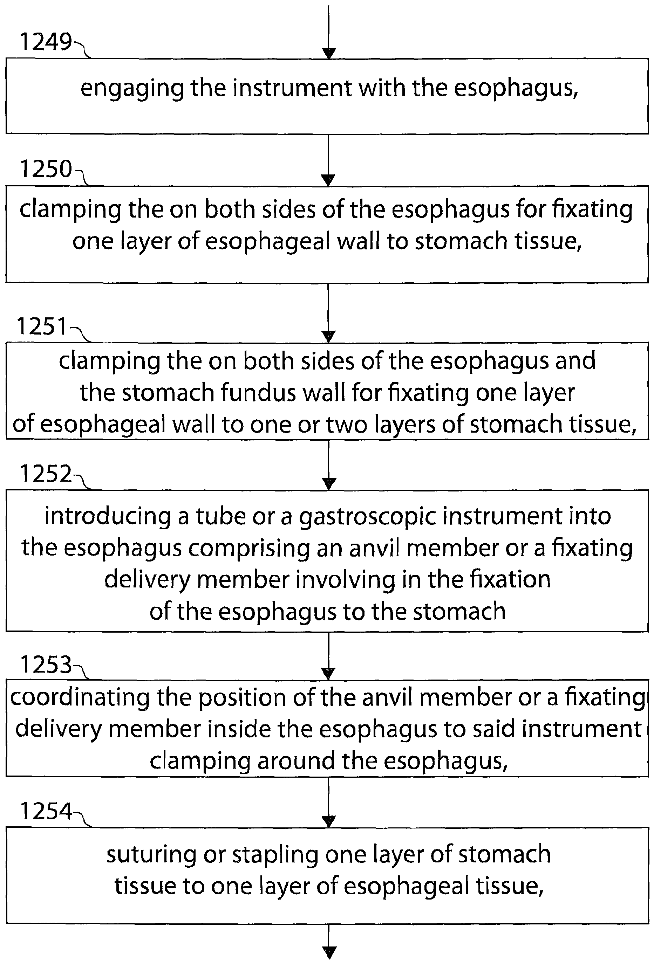

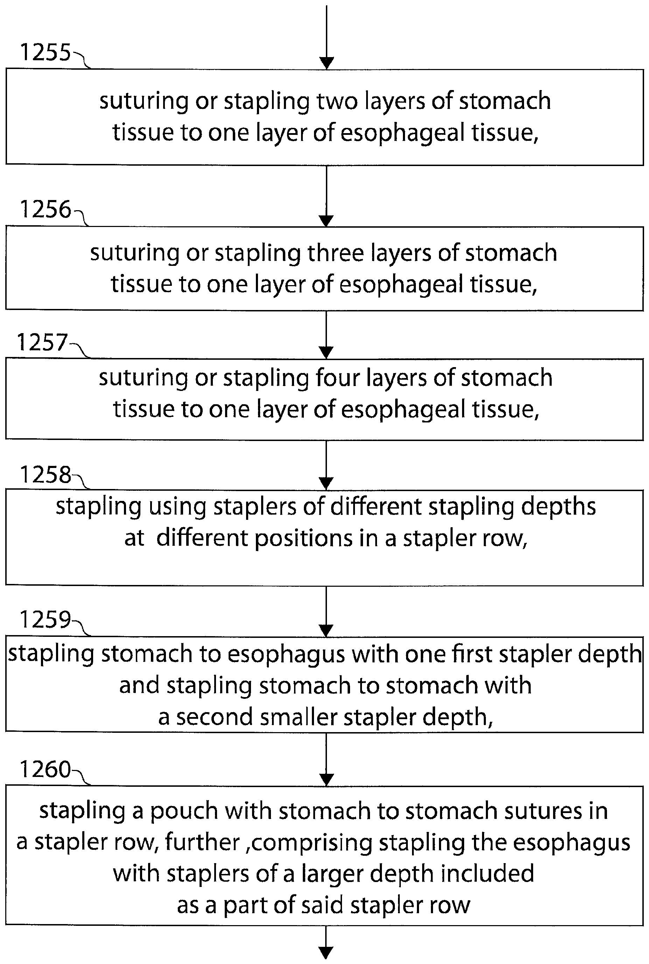

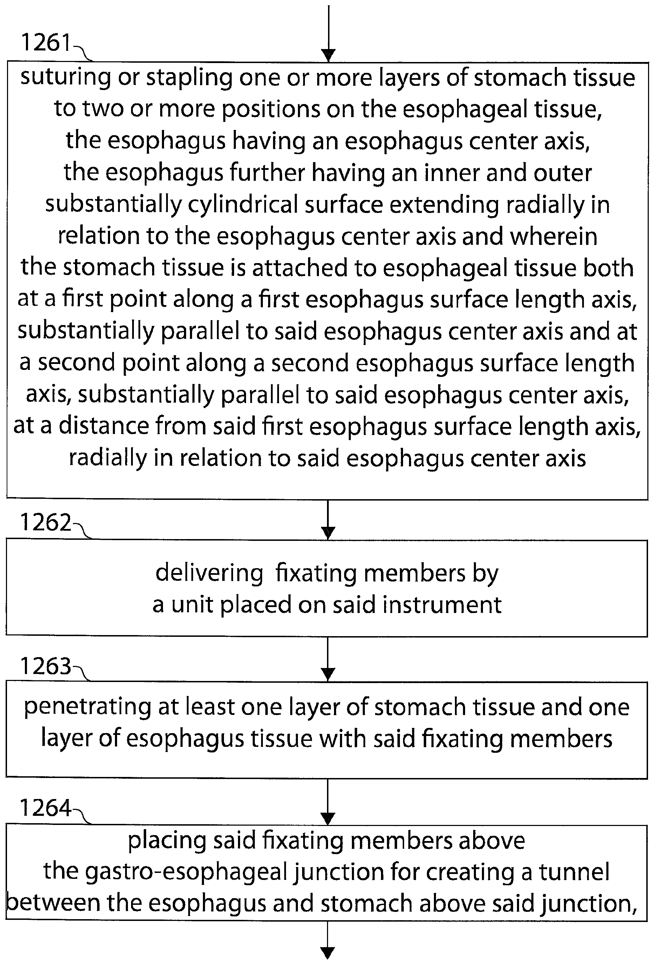

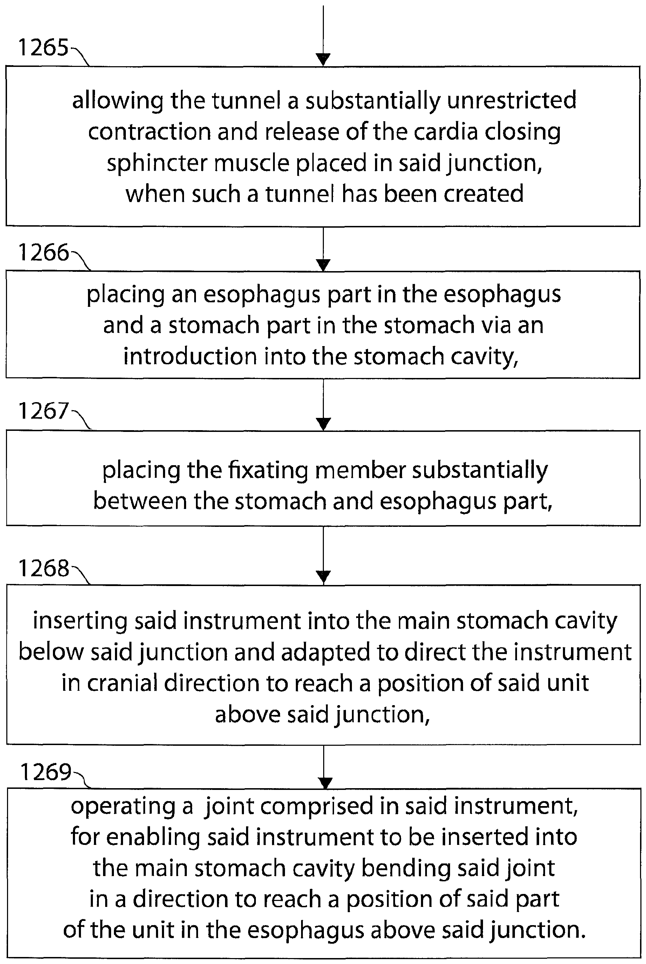

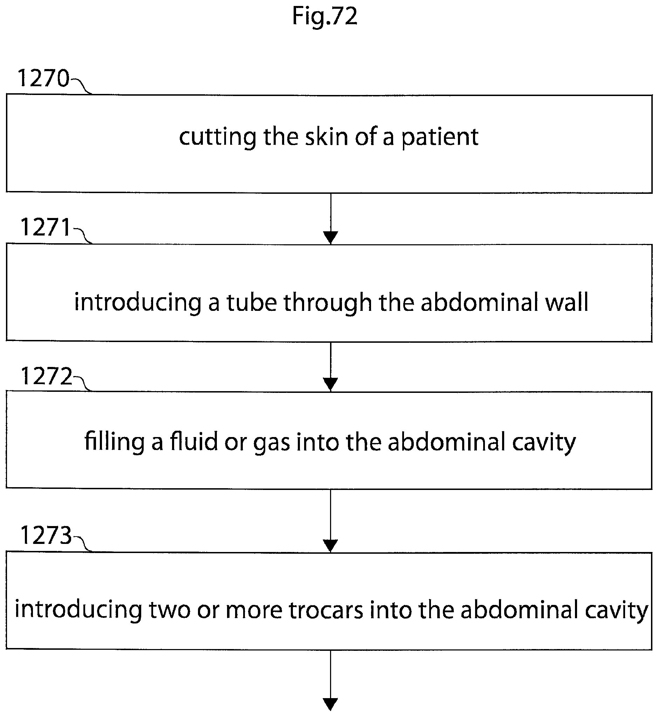

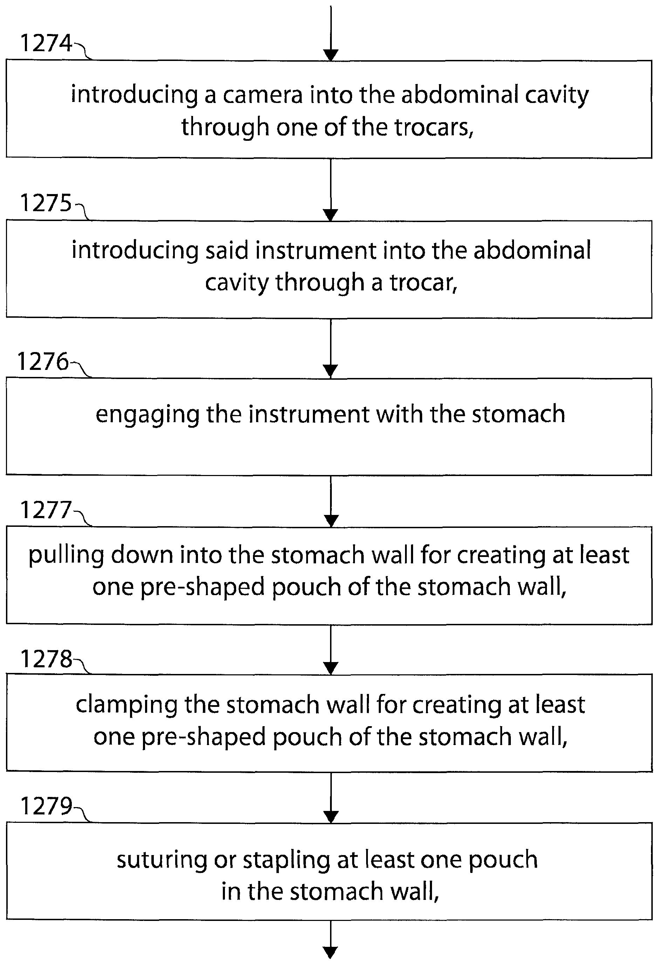

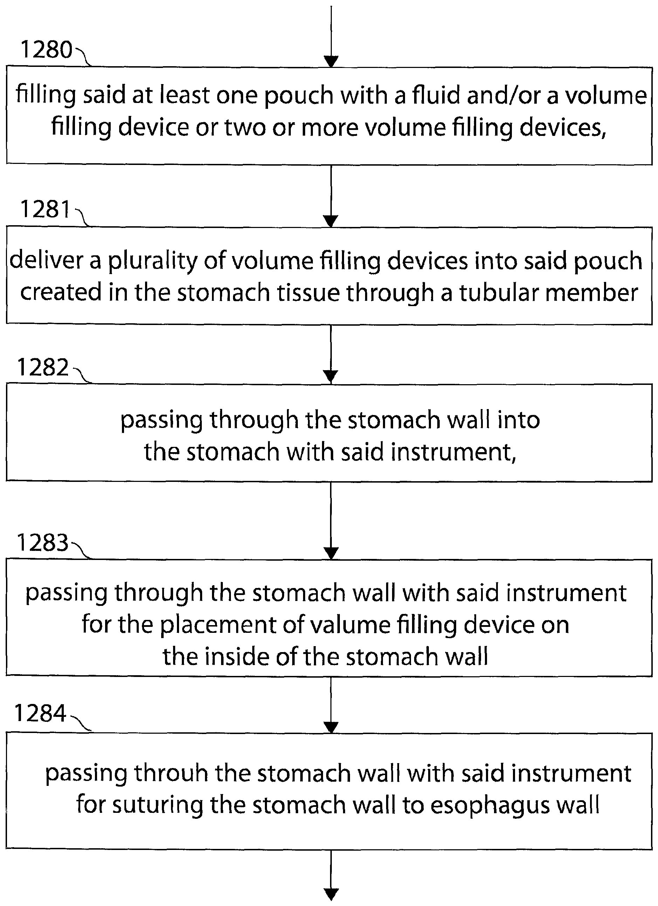

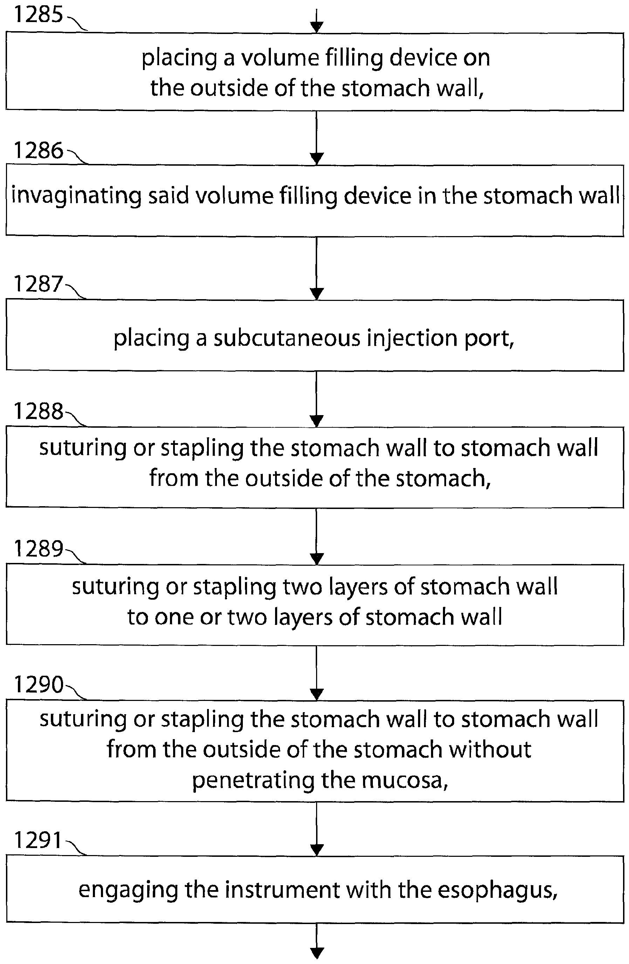

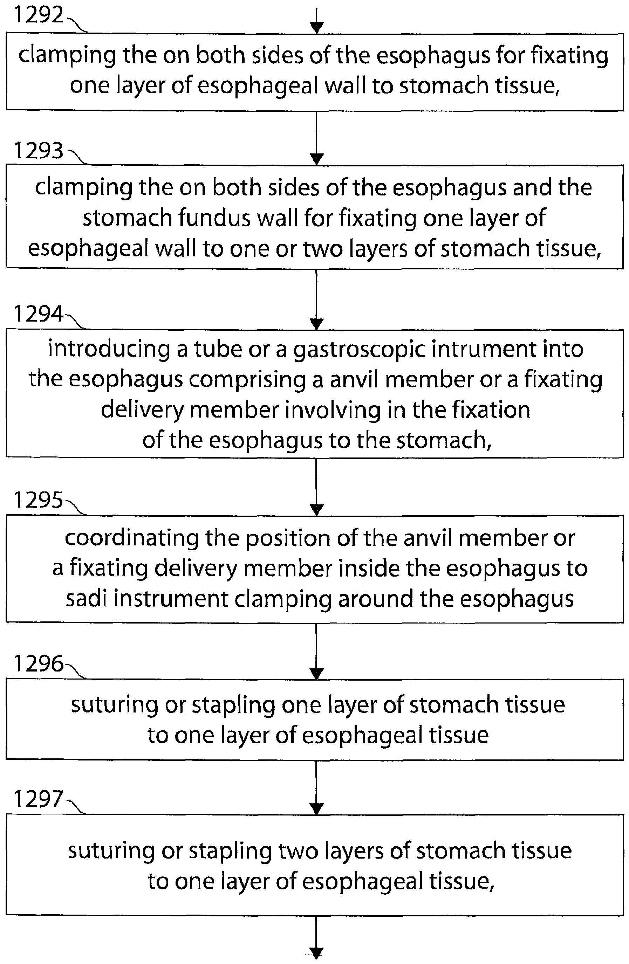

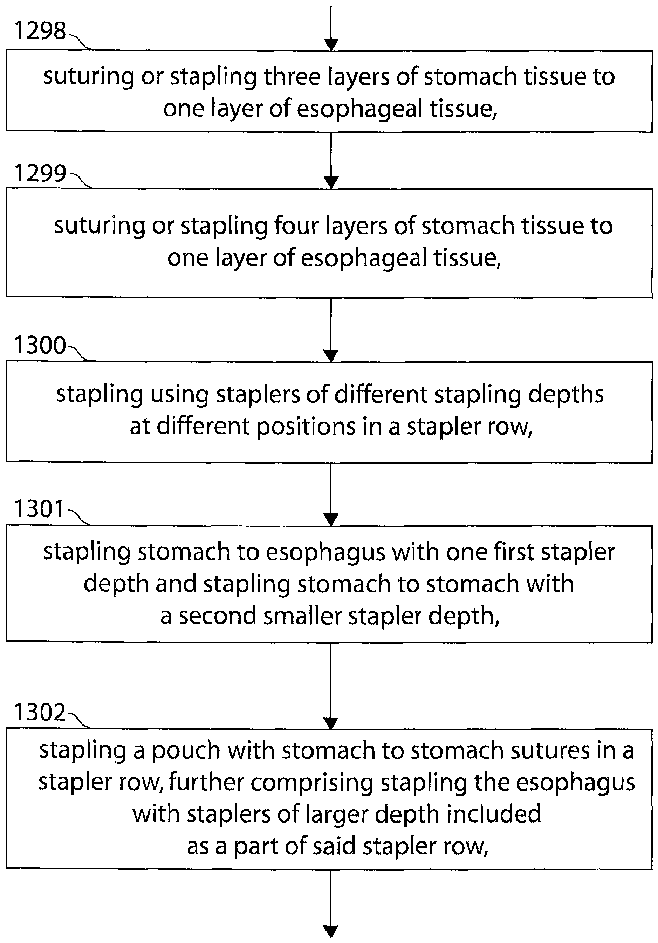

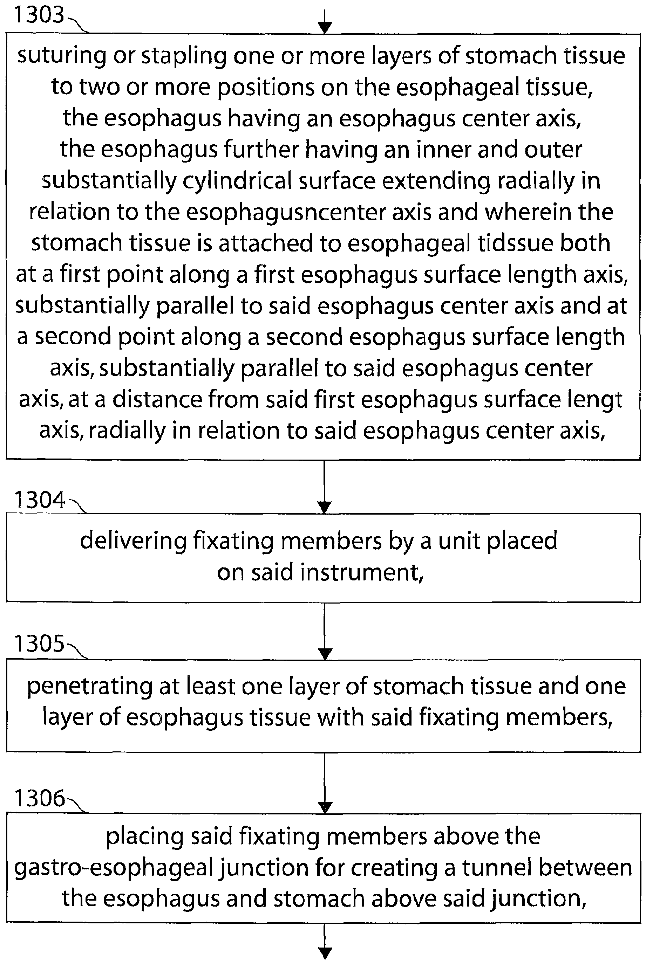

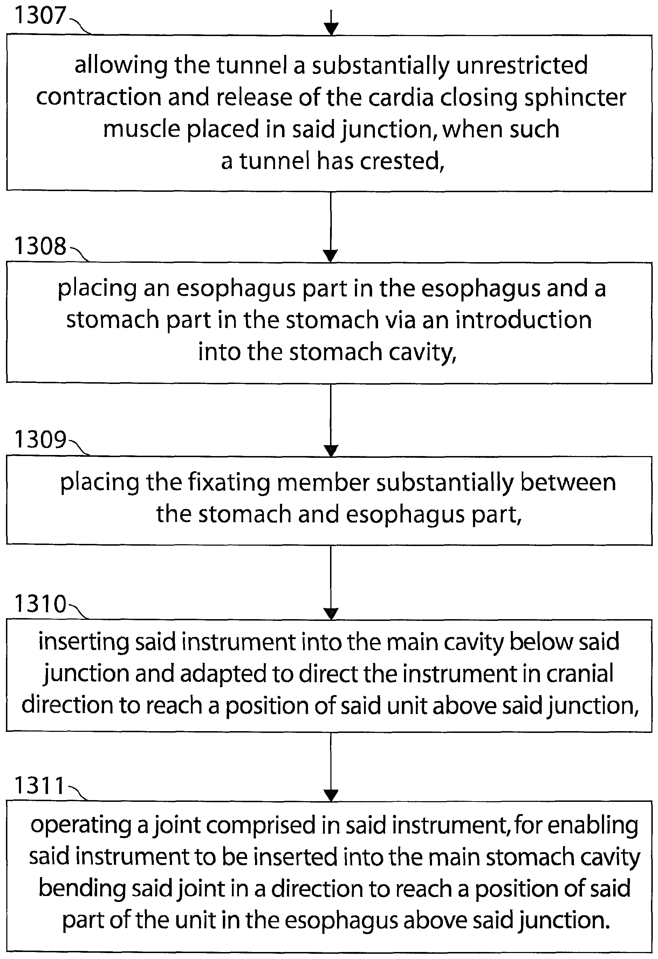

Figs. 70-72 show a flowchart describing a method or part of method.

### Detailed description

Embodiments of the instrument and method will now be described in further detail. Reference numerals designate the same parts throughout the description. The scope of the patent should not be limited by the embodiments disclosed, but rather by the appended claims. All of the embodiments disclosed could be combined unless doing so is clearly contradictory.

The terms cardia sphincter, cardia muscle and lower esophageal sphincter is to be understood as the sphincter in the esophagus hindering stomach content and stomach acid from reaching the esophagus.

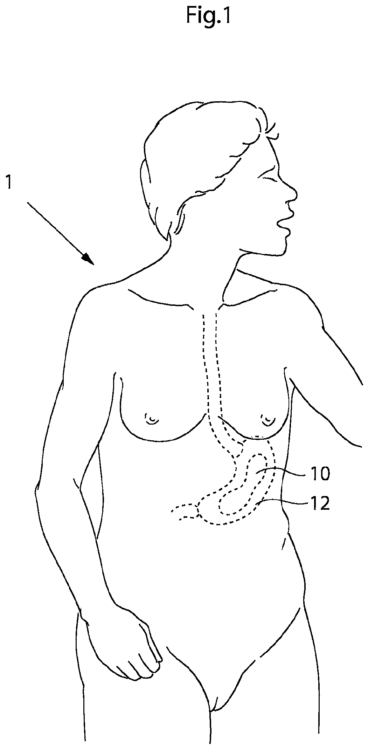

Fig. 1 shows a human patent 1, whom is being treated for reflux disease or obesity by means of a volume filling device 10. The function and the operation of the volume filling device will be described and explained in detail in the following description.

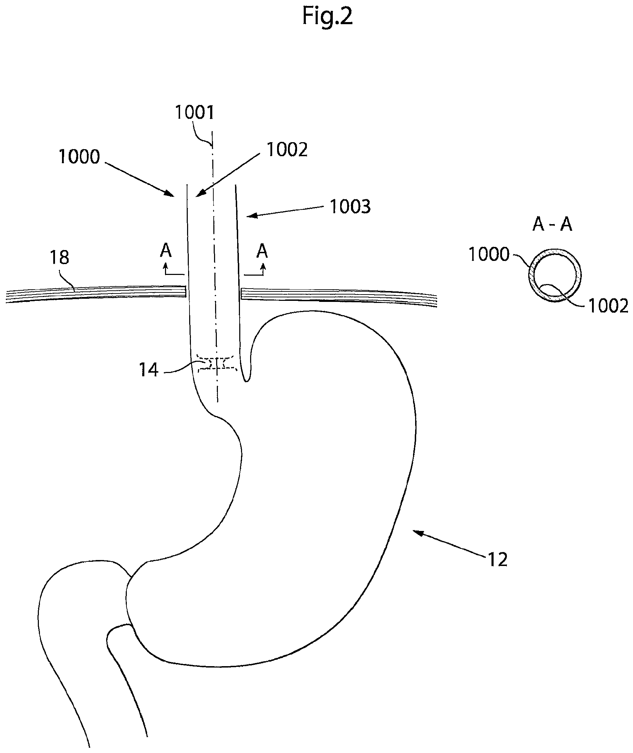

Fig. 2 shows the stomach region of a patient comprising the esophagus 1000 which passes through the thoracic diaphragm 18 which further supports the cardia sphincter, 14 which in turn prevents the stomach content and stomach acid from reaching the area of the esophagus. The esophagus 1000 being a substantially tube shaped tissue leading to the stomach 12 comprising stomach tissue, the esophagus having an esophagus center axis 1001, being substantially aligned with a cranial-caudal axis of the patient in and having a substantially circular circumference substantially aligned with a horizontal plane of the erect patient. The esophagus 1000 further has an inner 1002 and outer 1003 substantially cylindrical surface extending radially in relation to the esophagus center axis 1001.

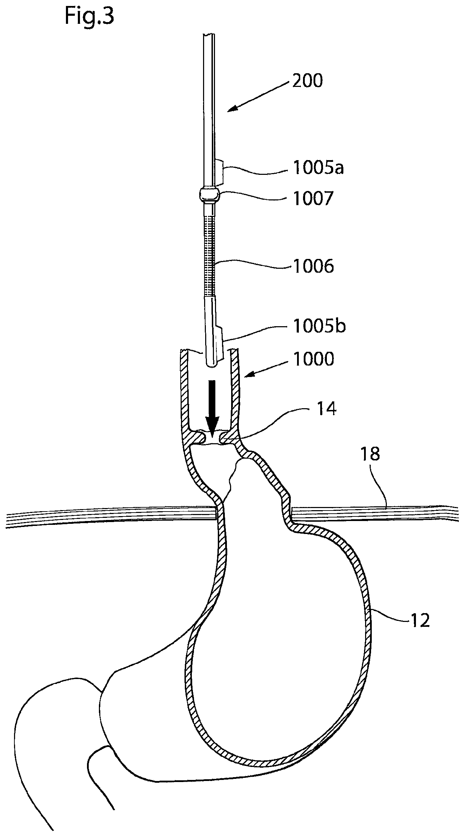

Fig. 3 shows the stomach region of a patient with reflux disease, wherein the cardia sphincter has slid up through the foramen in the thoracic diaphragm 18 and thus is no longer supported by the thoracic diaphragm 18. Reflux disease makes the stomach content and stomach acid entering the esophagus and creating a burning sensation in the esophagus tissue, which in long term exposure could affect the cell structure of the inner layer of esophagus tissue transforming said structure from a squamous epithelium to a gland epithelium, a transformation which increases the risk of adenocarcinoma. The esophagus connects to the stomach at the gastroesophageal junction 1008 which normally is placed below the thoracic diaphragm 18. Fig. 3 further shows an instrument 200 adapted to deliver at least one fixating member, such as sutures or staples, such that said at least one fixating member engages the stomach. The instrument comprises a unit 1005 comprising a first 1005a and second 1005b part. The first 1005a part is a part adapted to deliver fixating members, and the second part 1005b is an anvil, or the other way around. The fixating members are adapted to penetrate at least one layer of stomach 12 tissue and one layer of esophagus 1000 tissue, or two layers of stomach 12 and engage the anvil which alters the fixating member to a state in which the fixating member is hindered from penetrating the stomach tissue. The unit could be a unit adapted to deliver fixating members in the form of staples or a sutures or another type of fixating member comprising a first and second state, wherein said fixating member is adapted to, in the first state, penetrate tissue, and in the second state, hinder the fixating member from penetrating tissue. The fixating member could be adapted to automatically change from the first to the second state after the fixating member has penetrated the tissue, e.g. by means of the fixating member comprising a hinged and/or spring loaded part. The instrument shown in fig. 3 further shows a cardia contacting portion 1007 which is adapted to engage the cardia sphincter 14, and a bendable portion 1006 for positioning the portion of the instrument comprising the second part of the unit 1005b.

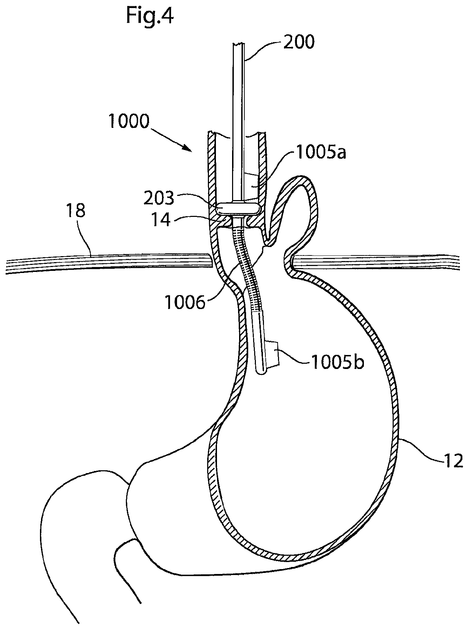

Fig. 4 shows the instrument disclosed in fig. 4 when the cardia engaging portion 203 engages the cardia muscle 14 and presses the esophagus 1000 through the foramen in the thoracic diaphragm 18 for restoring the cardia sphincter 14 to its normal position below the thoracic diaphragm 18. In fig. 4 a portion of the stomach has slid up through the thoracic diaphragm 18 creating a hiatal hernia. Fig. 4 further shows the bendable part 1006 starting to bend for positioning the second part 1005b of the unit in a position such that fixating members can be delivered from the first part of the unit 1005a to the second part of the unit 1005b, or the other way around, for fixating stomach 12 tissue to esophagus 1000 tissue.

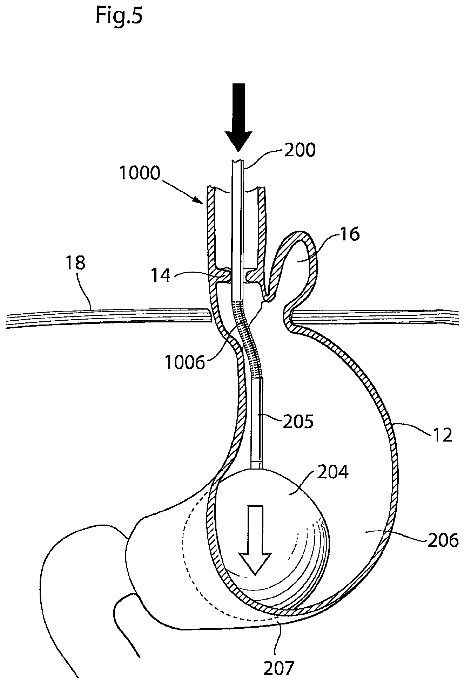

Fig. 5 shows an alternative embodiment of the instrument 200, wherein the instrument comprises an expandable member 204 adapted to engage the bottom of the stomach for restoring the cardia sphincter 14 to its normal position, supported by the thoracic diaphragm 18. In the embodiment of fig. 5 the instrument 200 comprises a bendable portion 1006 for positioning the expandable member 204 in an optimal position in relation to the stomach 12.

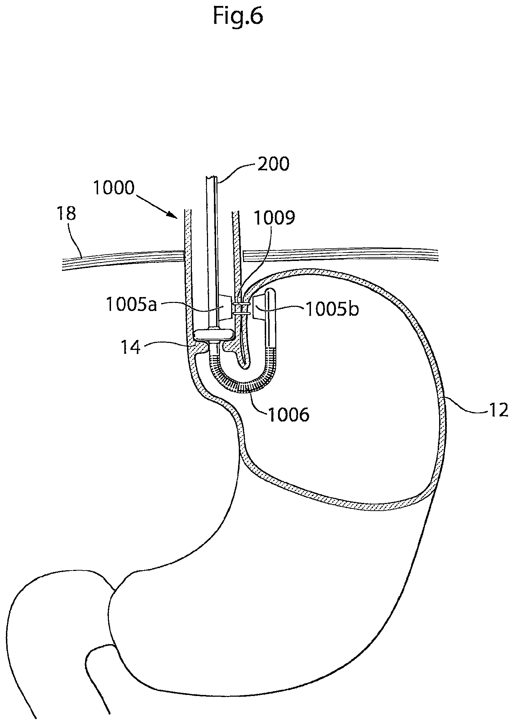

Fig. 6 shows the instrument of fig 4 when the instrument 200 is positioned for delivering fixating members 1009 from the first part 1005a of the unit, to the second part of the unit 1005b. The bendable portion 1006 has been bended for positioning the second part 1005b of the unit in a position enabling the fixating members to pass from the first part 1005a of the unit to the second part 1005b of the unit. Fig. 6 shows the instrument after the fixating members 1009 have been delivered and thus fixates the esophagus 1000 tissue to the stomach 12 tissue, restraining the esophagus and preventing the cardia sphincter 14 portion of the esophagus from sliding through the foramen in the thoracic diaphragm 18.

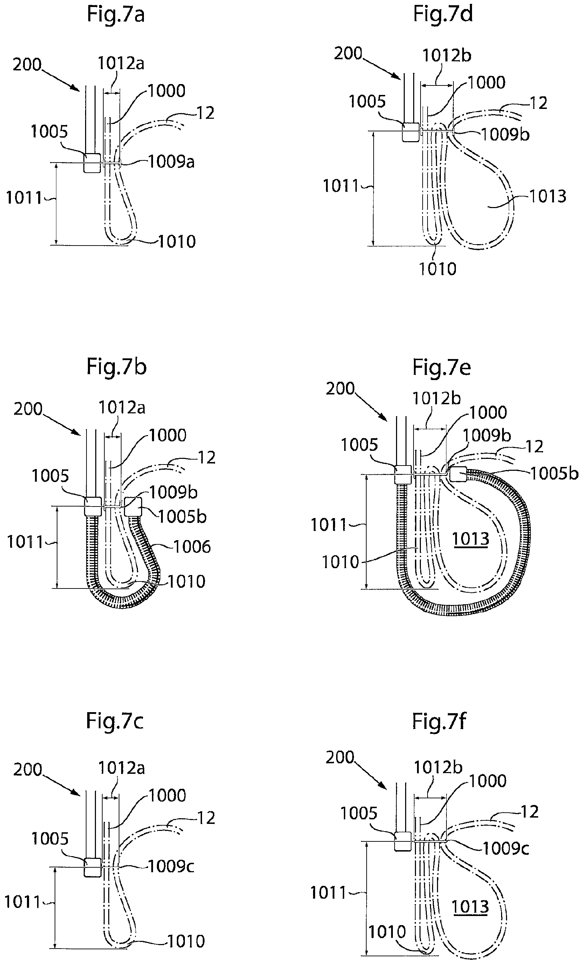

Figs. 7a - 7f describe a gastroscopic approach of using the instrument for treating reflux disease and/or obesity.

Fig. 7a shows the stomach 12 and esophagus 1000 in section when a fixating member 1009a has been delivered such that said fixating member 1009a fixates the esophagus 1000 tissue to the stomach 12 tissue using a unit 1005 mounted onto the instrument 200. For achieving the desired effect of restraining the esophagus on the abdominal side of the thoracic diaphragm 18, the fixating member 1009a has been placed at a distance 1011 from the angle of His 1010. The distance 1011 is according to one embodiment more than 1 cm, and according to another embodiment more than 2 cm, and according to another embodiment more than 3 cm, and according to another embodiment more than 4 cm, and according to another embodiment more than 5 cm. The fixating member 1009a is a fixating member comprising a first and second state, wherein said fixating member 1009a is adapted to, in said first state, penetrate tissue, and in said second state, hinder the fixating member from penetrating tissue. The fixating member 1009a could be altered from the first to the second state either automatically, after the fixating member has penetrated the tissue, or by means of a fixation member altering device, which could be a part of the instrument 200. The fixating member 1009a has a length or depth 1012a being adapted for fixating one layer of stomach 12 tissue and one layer of esophagus 1000 tissue to each other. The depth is according to one embodiment more than 1mm, and according to another embodiment more than 2 mm, and according to another embodiment more than 3 mm, and according to another embodiment more than 4 mm, and according to another embodiment more than 5 mm.

Fig. 7b shows the stomach 12 and esophagus 1000 in section when a fixating member 1009b has been delivered such that said fixating member 1009b fixates the esophagus 1000 tissue to the stomach 12 tissue using a unit 1005 comprising a first part 1005a and a second part 1005b. In the embodiment shown in fig. 7b the first part of the unit 1005s delivers the fixating member 1009b and the second part 1005b is an anvil bending the fixating member 1009b such that said fixating member 1009b is placed in a second state in which the fixating member 1009b is hindered from penetrating tissue. For achieving the desired effect of restraining the esophagus on the abdominal side of the thoracic diaphragm 18, the fixating member 1009b has been placed at a distance 1011 from the angle of His 1010. The distance 1011 is according to one embodiment more than 1 cm, and according to another embodiment more than 2 cm, and according to another embodiment more than 3 cm, and according to another embodiment more than 4 cm, and according to another embodiment more than 5 cm. For enabling the instrument 200 to place the fixating member at the distance from the angle of His as described, the instrument comprises a bendable portion 1006 such that the instrument could go around the angle of His and up to the fundus region of the stomach for fixating fundus tissue to the esophagus 1000. The fixating member 1009b has a length or depth 1012a being adapted for fixating one layer of stomach 12 tissue and one layer of esophagus 1000 tissue to each other. The depth is according to one embodiment more than 1 mm, and according to another embodiment more than 2 mm, and according to another embodiment more than 3 mm, and according to another embodiment more than 4 mm, and according to another embodiment more than 5 mm. According to one embodiment the fixating member 1009b is a staple.

Fig. 7c shows an embodiment similar to the embodiment of fig. 7a with the difference that the unit 1005 of fig. 7c is adapted to deliver a fixating member 1009c in form of a suture 1009c penetrating the esophagus 1000 tissue and the stomach 12 tissue and fixating the esophagus 1000 tissue to the stomach 12 tissue.

Fig. 7d shows the stomach 12 and esophagus 1000 in section when a fixating member 1009a has been delivered such that said fixating member 1009a fixates the esophagus 1000 tissue to three layers of stomach 12 tissue using a unit 1005 mounted onto the instrument 200. For achieving the desired effect of restraining the esophagus on the abdominal side of the thoracic diaphragm 18, the fixating member 1009a has been placed at a distance 1011 from the angle of His 1010. The distance 1011 is according to one embodiment more than 1 cm, and according to another embodiment more than 2 cm, and according to another embodiment more than 3 cm, and according to another embodiment more than 4 cm, and according to another embodiment more than 5 cm. The fixating member 1009a is a fixating member comprising a first and second state, wherein said fixating member 1009a is adapted to, in said first state, penetrate tissue, and in said second state, hinder the fixating member from penetrating tissue. The fixating member 1009a could be altered from the first to the second state either automatically after the fixating member has penetrated the tissue, or by means of a fixation member altering device, which could be a part of the instrument 200. The fixating member 1009a has a length or depth 1012b being adapted for fixating three layers of stomach 12 tissue and one layer of esophagus 1000 tissue to each other. The fixation of two of the three layers of stomach 12 tissue creating a pouch 1013 of stomach 12 tissue which is adapted to receive a volume filling device for further hindering the esophagus 1000 from sliding through the foramen in the thoracic diaphragm. The depth of the fixating member is according to one embodiment more than 2mm, and according to another embodiment more than 4 mm, and according to another embodiment more than 6 mm, and according to another embodiment more than 8 mm, and according to another embodiment more than 10 mm.

Fig. 7e shows the stomach 12 and esophagus 1000 in section when a fixating member 1009b has been delivered such that said fixating member 1009b fixates the esophagus 1000 tissue to three layers of stomach 12 tissue using a unit 1005 comprising a first part 1005a and a second part 1005b. In the embodiment shown in fig. 7b the first part of the unit 1005s delivers the fixating member 1009b and the second part 1005b is an anvil bending the fixating member 1009b such that said fixating member 1009b is placed in a second state in which the fixating member 1009b is hindered from penetrating tissue. For achieving the desired effect of restraining the esophagus on the abdominal side of the thoracic diaphragm, the fixating member 1009b has been placed at a distance 1011 from the angle of His 1010. The distance 1011 is according to one embodiment more than 1 cm, and according to another embodiment more than 2 cm, and according to another embodiment more than 3 cm, and according to another embodiment more than 4 cm, and according to another embodiment more than 5 cm. For enabling the instrument 200 to place the fixating member at the distance from the angle of His as described, the instrument comprises a bendable portion 1006 such that the instrument could go around the angle of His and up to the fundus region of the stomach for fixating fundus tissue to the esophagus 1000. The fixating member 1009b has a length or depth 1012b being adapted for fixating three layers of stomach 12 tissue and one layer of esophagus 1000 tissue to each other. The fixation of two of the three layers of stomach 12 tissue creating a pouch 1013 of stomach 12 tissue which is adapted to receive a volume filling device for further hindering the esophagus 1000 from sliding through the foramen in the thoracic diaphragm. The depth is according to one embodiment more than 2mm, and according to another embodiment more than 4 mm, and according to another embodiment more than 6 mm, and according to another embodiment more than 8 mm, and according to another embodiment more than 10 mm. According to one embodiment the fixating member 1009b is a staple.

Fig. 7f shows an embodiment similar to the embodiment of fig. 7e with the difference that the unit 1005 of fig. 7f is adapted to deliver a fixating member 1009c in form of a suture 1009c penetrating the esophagus 1000 tissue and three layers of stomach 12 tissue and fixating the esophagus 1000 tissue to the stomach 12 tissue and creating a pouch 1013 of stomach tissue.

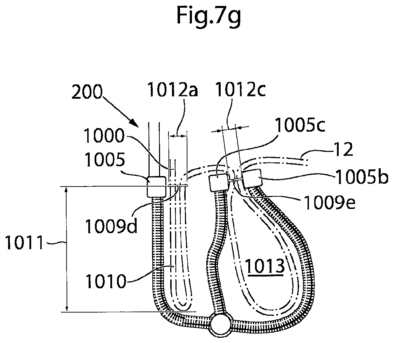

Fig. 7g is in many ways similar to fig 7e, however with the instrument introduced through the throat the pouch is sutured with a separate suture 1009e and the stomach is sutured to the esophagus with a separate suture 1009d. The same instrument may be adapted to suture both positions. The instrument may include either one or more anvil members and/ or one or more units for delivering fixating members. As shown an anvil member and fixating delivering member 1005 b and 1005c may be involved in suturing the pouch separate but they may also suture both positions excluding 1005. Of course many alternative embodiments could solve theses embodiment. The instrument may also be introduced into the stomach from the abdominal cavity.

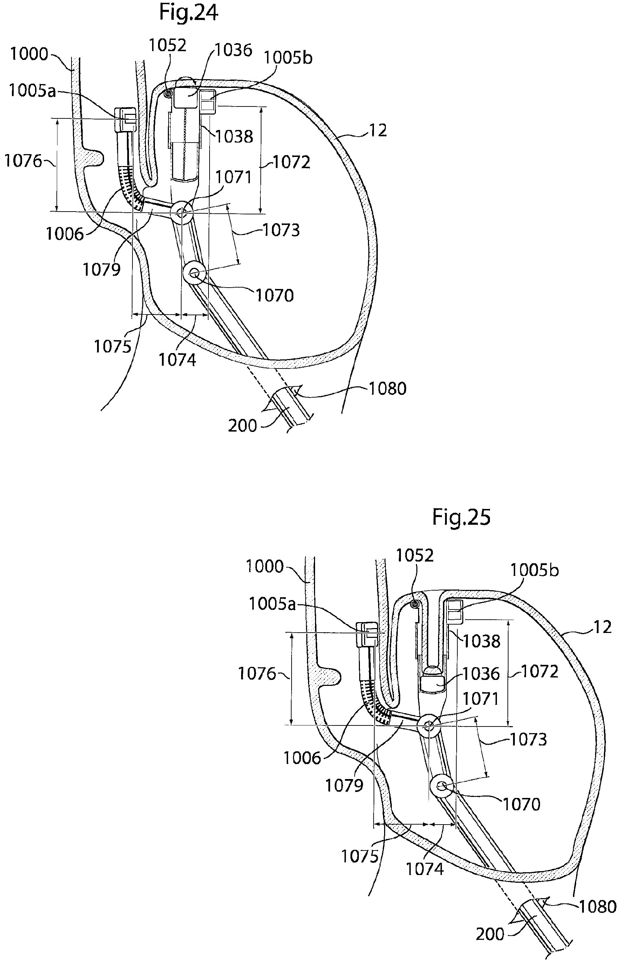

The gastroscopic methods/instruments disclosed with reference to figs. 7a - 7f could further be laparoscopic or surgical methods/instruments, according to the principle disclosed with reference to figs. 24 - 29.

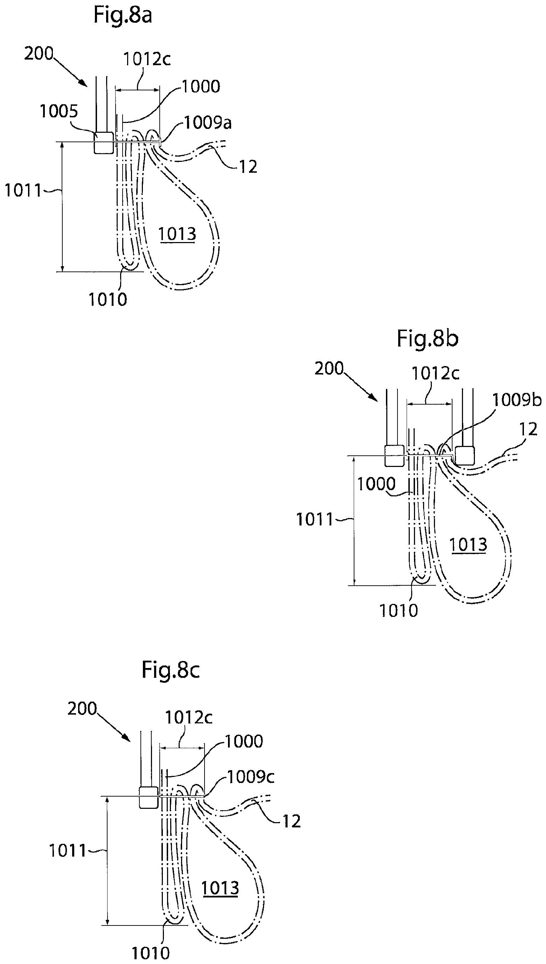

Figs. 8a - 8c describe a laparoscopic/gastroscopic and/or surgical approach of using the instrument for treating reflux disease and/or obesity.

Fig. 8a shows the stomach 12 and esophagus 1000 in section when a fixating member 1009a has been delivered such that said fixating member 1009a fixates the esophagus 1000 tissue to four layers of stomach 12 tissue using a unit 1005 mounted onto the instrument 200. For achieving the desired effect of restraining the esophagus on the abdominal side of the thoracic diaphragm, the fixating member 1009a has been placed at a distance 1011 from the angle of His 1010. The distance 1011 is according to one embodiment more than 1 cm, and according to another embodiment more than 2 cm, and according to another embodiment more than 3 cm, and according to another embodiment more than 4 cm, and according to another embodiment more than 5 cm. The fixating member 1009a is a fixating member comprising a first and second state, wherein said fixating member 1009a is adapted to, in said first state, penetrate tissue, and in said second state, hinder the fixating member from penetrating tissue. The fixating member 1009a could be altered from the first to the second state either automatically after the fixating member has penetrated the tissue, or by means of a fixation member altering device, which could be a part of the instrument 200. The fixating member 1009a has a length or depth 1012c being adapted for fixating four layers of stomach 12 tissue and one layer of esophagus 1000 tissue to each other, and thus reaching from the inside of the esophagus to the outside of the stomach 12. The fixation of two of the four layers of stomach 12 tissue creating a pouch 1013 of stomach 12 tissue which is adapted to receive a volume filling device for further hindering the esophagus 1000 from sliding through the foramen in the thoracic diaphragm. The depth 1012c is according to one embodiment more than 3mm, and according to another embodiment more than 5 mm, and according to another embodiment more than 7 mm, and according to another embodiment more than 9 mm, and according to another embodiment more than 11 mm.

Fig. 8b shows the stomach 12 and esophagus 1000 in section when a fixating member 1009b has been delivered such that said fixating member 1009b fixates the esophagus 1000 tissue to four layers of stomach 12 tissue using a unit 1005 comprising a first part 1005a placed in the esophagus using a gastroscopic approach, and a second part 1005b placed in the abdominal cavity using a surgical or laparoscopic approach. In the embodiment shown in fig. 8b the first part 1005a of the unit 1005 delivers the fixating member 1009b and the second part 1005b is an anvil bending the fixating member 1009b such that said fixating member 1009b is placed in a second state in which the fixating member 1009b is hindered from penetrating tissue, however it is equally conceivable throughout the different embodiments that the anvil part is placed in the esophagus and the part delivering fixating members is placed in the abdominal cavity. For achieving the desired effect of restraining the esophagus on the abdominal side of the thoracic diaphragm 18, the fixating member 1009b has been placed at a distance 1011 from the angle of His 1010. The distance 1011 is according to one embodiment more than 1 cm, and according to another embodiment more than 2 cm, and according to another embodiment more than 3 cm, and according to another embodiment more than 4 cm, and according to another embodiment more than 5 cm. The fixating member 1009a has a length or depth 1012c being adapted for fixating four layers of stomach 12 tissue and one layer of esophagus 1000 tissue to each other, and thus reaching from the inside of the esophagus to the outside of the stomach 12. The fixation of two of the four layers of stomach 12 tissue creating a pouch 1013 of stomach 12 tissue which is adapted to receive a volume filling device for further hindering the esophagus 1000 from sliding through the foramen in the thoracic diaphragm. The depth 1012c is according to one embodiment more than 3mm, and according to another embodiment more than 5 mm, and according to another embodiment more than 7 mm, and according to another embodiment more than 9 mm, and according to another embodiment more than 11 mm. According to one embodiment the fixating member 1009b is a staple.

Fig. 8c shows an embodiment similar to the embodiment of fig. 8a with the difference that the unit 1005 of fig. 8c is adapted to deliver a fixating member 1009c in form of a suture 1009c penetrating the esophagus 1000 tissue and four layers of stomach 12 tissue and fixating the esophagus 1000 tissue to the stomach 12 tissue and creating a pouch 1013 of stomach 12 tissue.

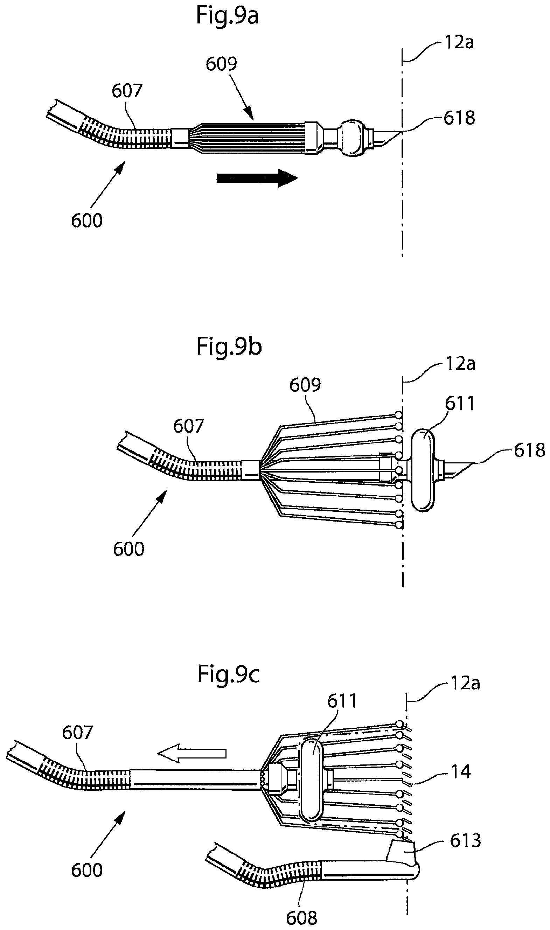

Figs. 9a - e shows a gastroscopic and/or laparoscopic and/or surgical instrument and method for invaginating a volume filling device 10 in the stomach wall 12a of the patient by creating a pouch of stomach wall 12a material in which the volume filling device 10 is placed. The instrument, generally designated 600, comprises an elongated member 607 having a proximal end and a distal end, the elongated member 607 having a diameter less than that of the patient's esophagus and being flexible through a bendable portion 1006 such as to allow introduction of the flexible elongated member 607 with its distal end first through the patient's throat, esophagus and into the stomach 12 to the stomach wall 12a, alternatively through an incision in the abdomen or through a trocar placed in the abdominal region and penetrating in to the abdominal cavity.

The stomach penetration device or cutter 618 is provided on the elongated member 607 at the distal en thereof for penetrating the stomach wall 12a so as to create a hole in the stomach wall 12a, to allow introduction of the elongated member 607 through the hole. The stomach penetration device 618 could be adapted to be operable for retracting said stomach penetration device 615 after the stomach fundus wall 12a has been penetrated, for not further damaging tissue within the body. The instrument further comprises a special holding device 609 provided on the elongated member 607 on the proximal side to the penetration device 618.

The elongated member further comprises an expandable member 611 which is adapted to be expanded after the elongated member has penetrated the stomach wall 12a and thereby assist in the creation of a cavity or pouch adapted to hold the volume filling device 10. The expandable member 611 may comprise an expandable circular balloon provided circumferentially around the distal end portion of the flexible elongated member 607.

The method steps when invaginating the volume filling device 10 will now be described in detail. After the instrument 600 has been inserted into the stomach 12, the stomach penetration device 618 is placed into contact with the stomach wall 12a, see Fig. 9a. The stomach penetration device or cutter 618 is then brought to create the hole in the stomach wall 12a, whereafter at least the expandable member 611 is brought through the hole in the stomach wall 12a. The special holding device 609 is in this step brought to a holding state wherein it expands radially so as to form an essentially circular abutment surface to the stomach wall 12a, see Fig. 9b. In this way, the insertion of the stomach penetration device 618 and the expandable member 611 through the hole in the stomach wall is limited to the position shown in Fig. 9b.

The expandable member 611 is then expanded. In the case the expandable member comprises a balloon or the like, air or other fluid is injected into it.

The part of the elongated member 607 comprising the expandable member 611 is then retracted in the proximal direction, as indicated by the arrow in Fig. 9c, thereby pulling the stomach wall 612 into a basket or cup like structure created by the special holding device 609.

A suturing or stapling device 608, also comprises a bendable portion 1006, is further provided, either as a device connected to the elongated member 607 or as a separate instrument. The suturing or stapling member comprises a suturing or stapling end 613 which is adapted to close the cavity or pouch 1013 by means of stomach to stomach sutures or staples 14.

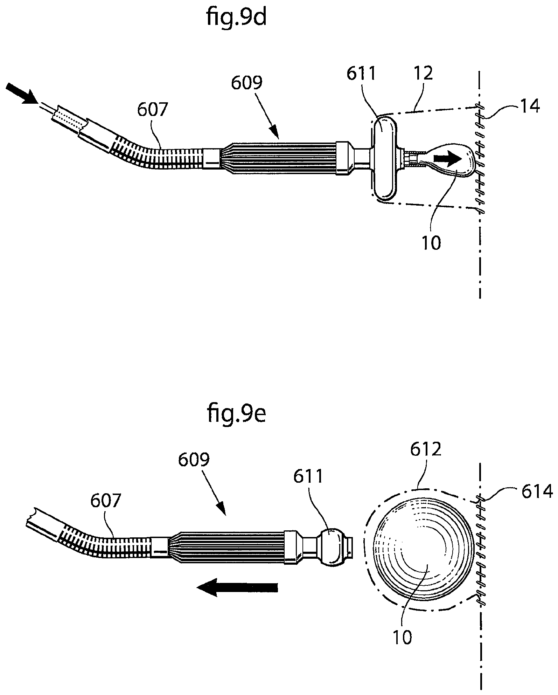

In a further step, illustrated in Fig. 9d, an expandable volume filling device 10 is placed in its deflated state in the cup like structure. The volume filling device 10 is then inflated to its inflated or expanded state, see Fig. 9e. This inflation of the volume filling device 10 can be accomplished by injecting a fluid or a gel into the deflated volume filling device 10. It can also be accomplished by injecting a material which is allowed to cure, thus being a solidifying material, thereby forming a solid device 10. Thus, the volume filling device 10 shown in Figs. 9d and 9e can illustrate either a balloon-like device 10 which is subsequently filled with fluid or gel or alternatively a material which is simply injected into the cup like structure formed by the stomach wall 12a.

The fluid which is used to fill the volume filling device 10 could be any suitable fluid suitable to fill the expandable device 10, such as a salt solution. In another embodiment, when this fluid is a fluid which is adapted to be transformed into solid state, the fluid could be liquid polyurethane.

In order to minimize or entirely eliminate leakage, the fluid is isotonic, i.e., it has the same osmolarity as human body fluids. Another way of preventing diffusion is to provide a fluid which comprises large molecules, such as iodine molecules.

The stomach-to-stomach sutures or staples are preferably provided with fixation portions exhibiting a structure, such as a net like structure, adapted to be in contact with the stomach wall to promote growth in of human tissue to secure the long term placement of the volume filling device attached to the stomach wall.

After the expandable device 10 has been inflated, partly or fully, the instrument 600 is retracted from the hole 12b, which is subsequently closed in some suitable way, e.g. by means of a closing member delivering sutures or staples. The instrument is then removed from the stomach 600 and the expandable device 10 in its inflated or expanded state is invaginated by a stomach wall portion of the patient on the outside of the stomach wall.

During one or more of the above described steps, the stomach may be inflated with gas, preferably by means of the gastroscopic instrument.

The volume filling device 10 described above with reference to Figs. 9a - 9e has been described as an expandable volume filling device. It will be appreciated that is also can be an elastic volume filling device with an elasticity allowing compression so as to be inserted into a gastroscopic instrument and which expands to an expanded state after leaving the instrument.

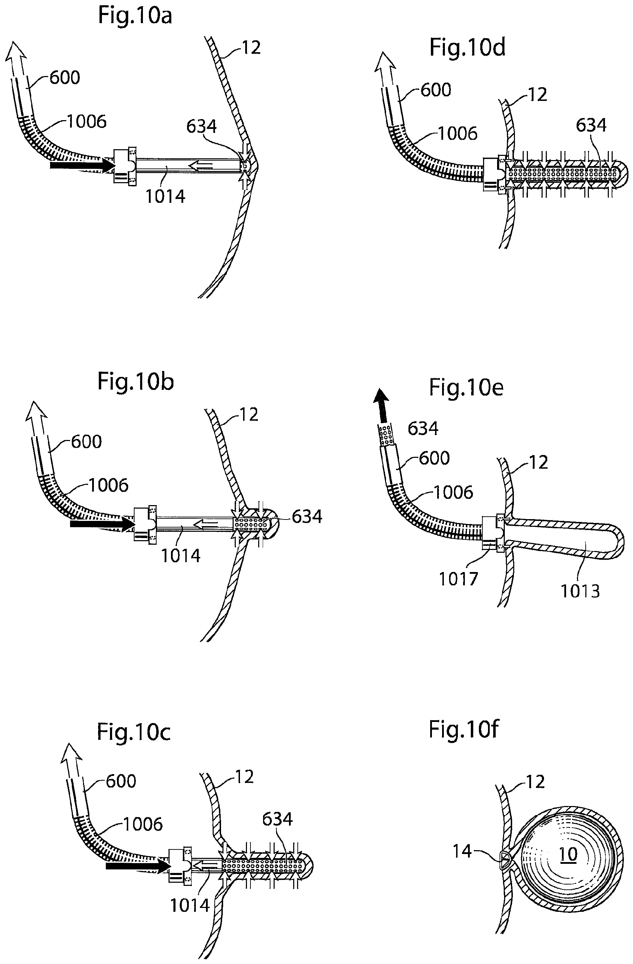

Fig. 10a shows an instrument 600 adapted to create a pouch in the stomach wall 12 using a sucking portion 634 using vacuum to connect to the stomach wall 12. In fig. 10a the tip of the suction portion 634 is pressed against the stomach wall 12 forming a recess therein. When the suction portion 634 is further pressed against the stomach wall 12, see Fig. 10b, a larger recess is formed and at the same time a sleeve 1014 is retracted exposing new vacuum holes adapted to connect with the stomach wall. The part of the stomach wall 12 that forms the recess will, due to the suction effect, adhere to the suction portion 634 of the instrument 600. As the suction portion 634 is further pressed into the stomach wall 12, see Fig. 10c, a deeper recess will be formed until the entire suction portion 634 is embedded in the recess, see Fig. 10d and the sleeve 1014 is fully retracted.

The rim of the recess will at this stage be fixated by means of fixation elements 14, such as sutures or staples delivered by a unit 1017 adapted to deliver fixation members 14. The suction portion 634 is thereafter removed, as seen in fig. 10e and a volume filling device 10 is inserted into the pouch created, see fig. 10f. The volume filling device 10 could be a compressed elastic volume filling device 10 which is filled and expanded to its final shape, see Fig. 10f.

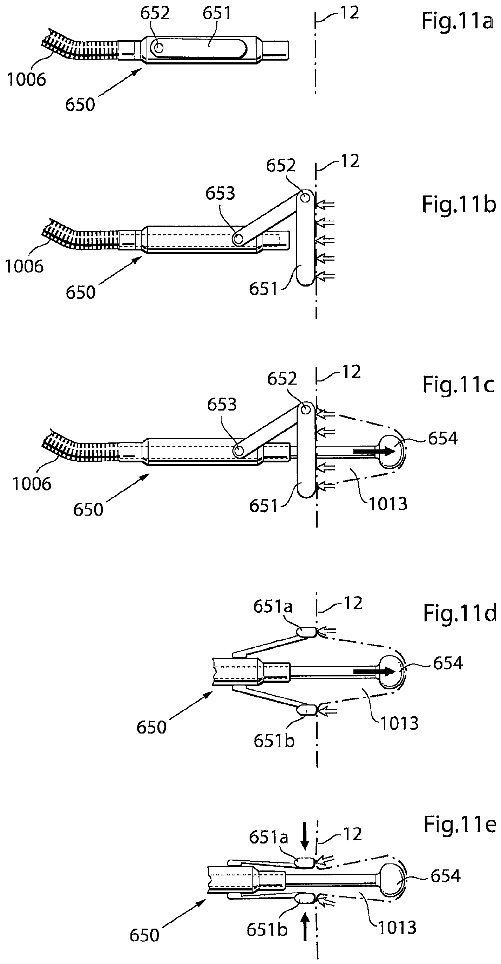

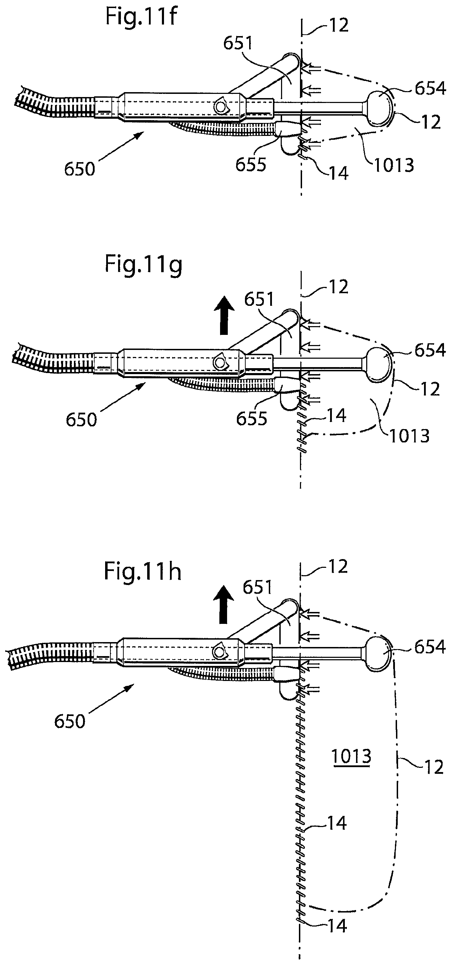

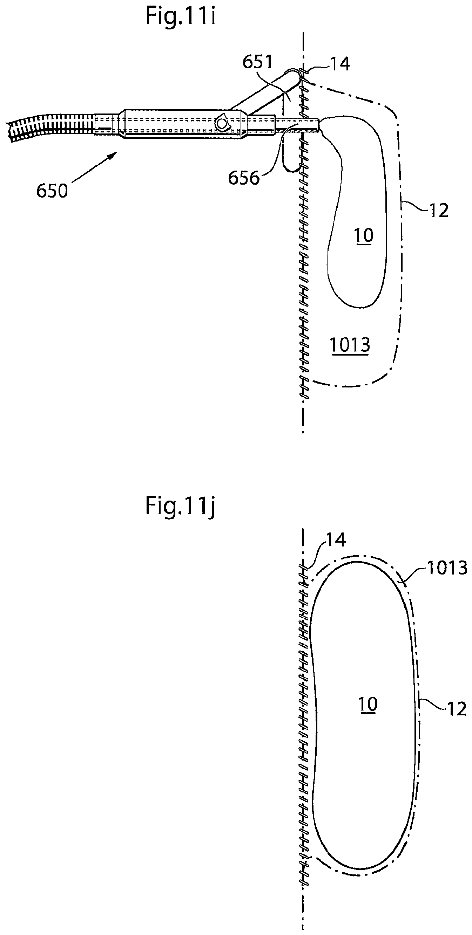

Figs. 11a-j show an instrument for use in a method of placing a volume filling device 10 in the stomach wall 12 of a patient. The instrument is adapted to be inserted through a narrow tube shaped object such as a gastroscope, used in an intraluminar procedure, or a laparoscopic trocar used in a laparoscopic procedure. The instrument comprises an elongated member 650 which is adapted to be flexible by means of the construction comprising a bendable portion 1006, a construction comprising multiple ring shaped members, however it is equally conceivable that said elongated member 650 is adapted to be flexible by means of said elongated member 650 being made of a flexible or adjustable material or by means of at least one joint. The elongated member 650 is inserted into the body and placed in proximity to the stomach wall 12 of the patient, from the outside or inside thereof. The elongated member 650 has a special holding device 651 adapted to hold the stomach by means of mechanical grabbing members or vacuum. The special holding device 651 comprises a first joint 652 and a second joint 653, which enable the special holding device 651 be operable in relation to the elongated member 650 and thereby place the part of the holding device 651 comprising the mechanical grabbing members or vacuum elements into contact with the stomach wall 12 of the patient. Fig. 11b shows the special holding device 651 when placed in contact with the stomach wall 12 of the human patient, after which the special holding member 651 connects to the stomach wall 12, for holding the stomach wall 12. Fig. 11c shows the instrument when the step of advancing a pushing rod 654 from the elongated member 650 is performed. The pushing rod 654 pushes the stomach wall 12 to create a cavity or pouch thereof. Fig. 11d shows the instrument turned 90 degrees in relation to figs. 11a-c. This view shows the special holding members 651a,b operably attached to two sides of the elongated member 650 and being in contact with the stomach wall 12, holding the stomach wall 12 as the pushing rod 654 pushes to create a cavity or pouch. When the pushing rod 654 has pushed the stomach wall 12 to a desired position the special holding devices 651a,b moves towards the pushing rod 654 and thereby closes the cavity or pouch 1013.

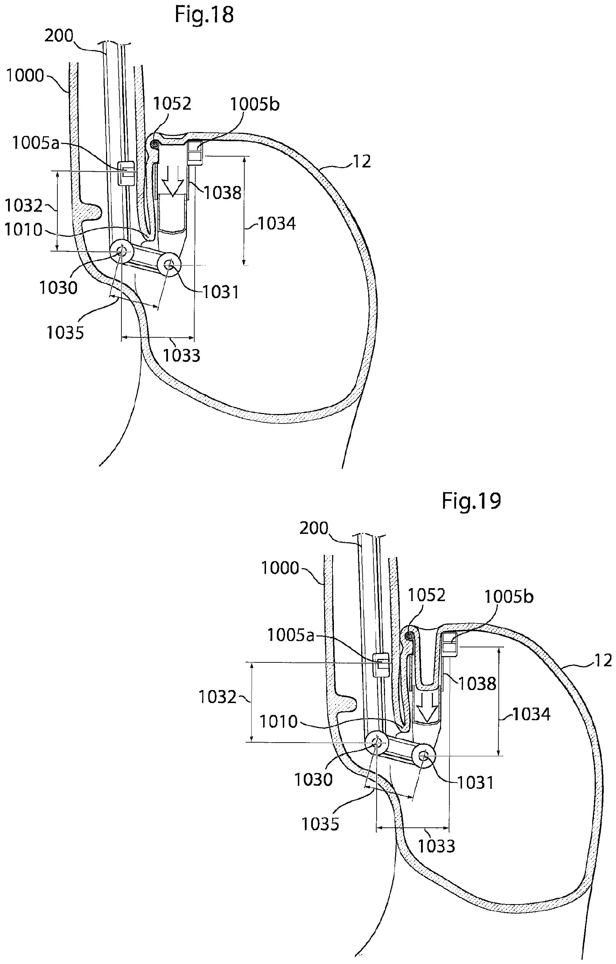

After the cavity or pouch 1013 has been created it needs to be sealed. Fig. 11f shows the advancement of a suturing or stapling device 655 from the elongated member 650. The suturing or stapling device 655 is positioned in connection with the stomach wall after which the suturing or stapling device commences with the suturing or stapling of the stomach wall 12, creating a seal of stomach to stomach sutures or staples 14. The instrument is moved along the stomach wall 12 of the patient and thereby a cavity or pouch 1013 is created and sealed using the instrument, as shown in fig. 19g and 19h. When a cavity or pouch 1013 or desired size has been created and sealed an inserting member 656 is advanced from the elongated member 650. The inserting member 656 is adapted to insert a volume filling device 10 being expandable, as described earlier in this application. After the inserting member 656 has been positioned in the cavity or pouch 1013, as shown in fig. 11i, the volume filling device 10 is inserted through the inserting member 656 and into the cavity or pouch 1013 by means of a pressurized fluid or gas, or a mechanical advancement member pushing said expandable volume filling device 10 into the cavity or pouch 1013. The insertion member then inflates the expandable volume filling device 10 with a fluid or gas and seals of the final section of the pouch 1013 using stomach to stomach sutures or staples 14. The embodiment described explains the process of inserting an expandable volume filling device, however it is equally conceivable that the volume filling device 10 is expandable by means of the volume filling device 10 being made of an elastic material.

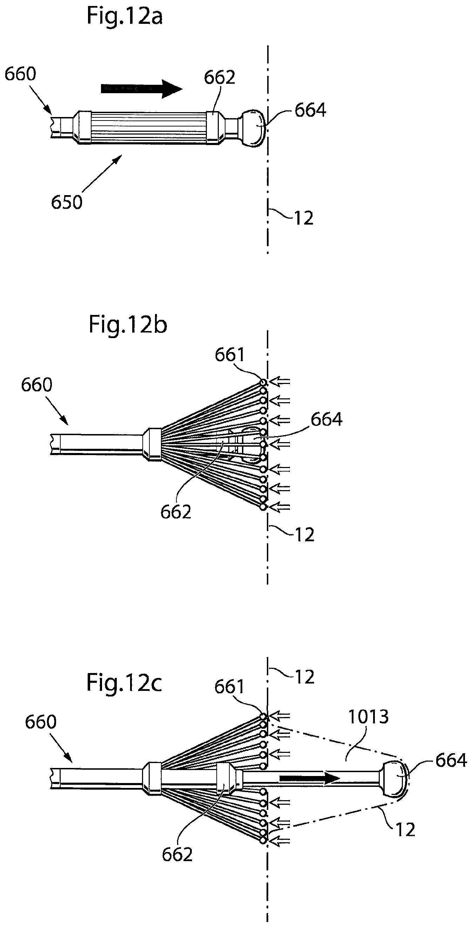

Figs. 12a - f shows an instrument for use in a method of placing a volume filling device 10 in the stomach wall 12 of a patient. The instrument is adapted to be inserted through a narrow tube shaped object such as a gastroscope, used in an intraluminar procedure, or a laparoscopic trocar used in a laparoscopic procedure. The instrument comprises an elongated member 660 which is adapted to be flexible by means of a construction comprising multiple ring shaped members, however it is equally conceivable that said elongated member 660 is adapted to be flexible by means of said elongated member 660 being made of a flexible or adjustable material, or by means of at least one joint. The elongated member 660 is inserted into the body and placed in proximity to the stomach wall 12 of the patient, from the outside or inside thereof. The elongated member 660 has multiple special holding devices 661 adapted to hold the stomach by means of mechanical grabbing members or vacuum. The special holding devices 661 are locked in a position alongside the elongated member 660 by means of a locking ring 662. The special holding devices 661 are made of a flexible material end pre-bent to expand into a funnel-shaped device when said locking ring 662 is removed. The special holding device in its funnel shaped expandable state is shown in fig. 12b. Fig. 12b further shows the special holding device 661 when placed in contact with the stomach wall 12 of the human patient, after which the special holding member 661 connects to the stomach wall 12, for holding the stomach wall 12. Fig. 12c shows the instrument when the step of advancing a pushing rod 664 from the elongated member 660 is performed. The pushing rod 664 pushes the stomach wall 12 to create a cavity or pouch 1013 thereof. When the pushing rod 664 has pushed the stomach wall 12 to a desired position the special holding devices 661 moves towards the pushing rod 664 and thereby closes the cavity or pouch 1013.

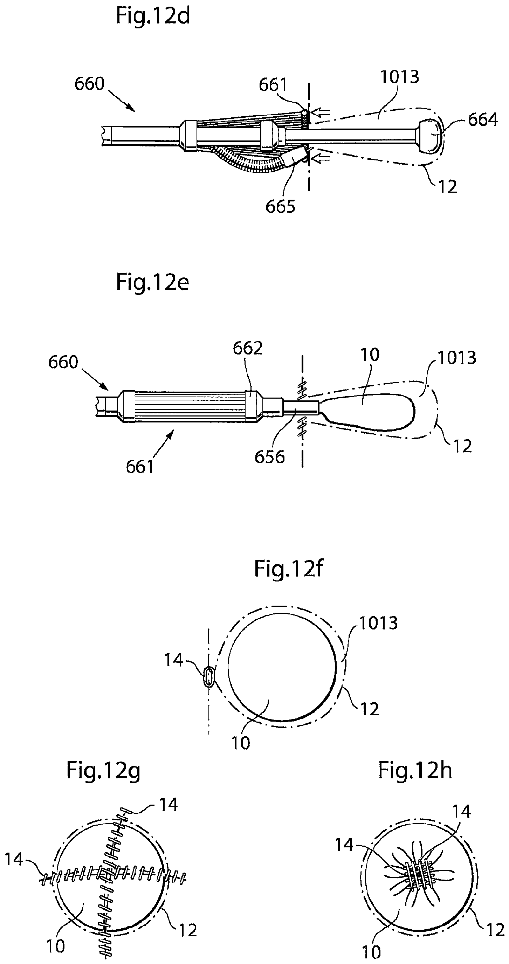

After the cavity or pouch 1013 has been created it needs to be sealed. Fig. 12d shows the advancement of a suturing or stapling device 665 from the elongated member 660. The suturing or stapling device 665 is positioned in connection with the stomach wall 12 after which the suturing or stapling device 665 commences with the suturing or stapling of the stomach wall 12, creating a seal of stomach to stomach sutures or staples 14. Thereafter an inserting member 666 is advanced from the elongated member 660 and the special holding devices 661 are retracted. The inserting member 666 is adapted to insert a volume filling device 10 being expandable, as described earlier in this application. After the inserting member 666 has been positioned in the cavity or pouch 1013 the volume filling device 10 is inserted through the inserting member 666 and into the cavity or pouch 1013 by means of a pressurized fluid or gas, or a mechanical advancement member pushing said expandable volume filling device 10 into the cavity or pouch. The insertion member 656 then inflates the expandable volume filling device with a fluid or gas and seals of the final section of the pouch 1013 using stomach to stomach sutures or staples 14. The embodiment described explains the process of inserting an expandable volume filling device 10, however it is equally conceivable that the volume filling device 10 is expandable by means of the volume filling device 10 being made of an elastic material. Fig. 12 f shows the volume filling device 10 as invaginated in the stomach wall 12, in a cavity or pouch 1013 sealed with stomach to stomach sutures or staples 14.

Fig. 12g shows an alternative way of closing the pouch created in the stomach wall 12 using sutures or staples 14 placed in two directions intersecting each other.

Fig. 12h shows an alternative way of closing the pouch created in the stomach wall 12 using sutures or staples 14 placed in two directions intersecting each other and creating a tobacco pouch like pouch having the edges of the opening being stomach 12 folded back and forth and fixated with fixating members 14 such as sutures or staples intersecting each other, and in the embodiment shown in fig. 12e intersecting each other perpendicularly.

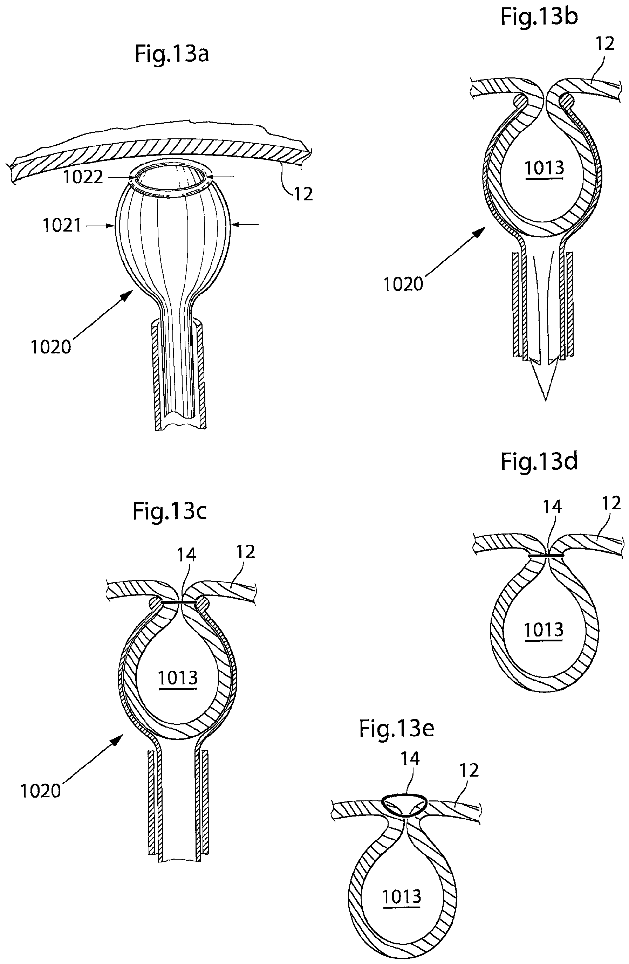

Figs. 13a - e shows an instrument comprising a stomach tissue 12 pulling device 1020 adapted to pull the stomach tissue 12 into an opening 1022 of a luminal space 1021 inside of said instrument. The pulling of the tissue is according to the embodiment shown in figs. 13a - e performed by means of vacuum, however, in other embodiments (not shown) the pulling is performed using pulling members mechanically engaging the stomach 12, either through penetrating the stomach tissue 12 or clamping the stomach tissue.

Fig. 13b shows the state when the stomach tissue 12 has been placed in the luminal space 1021 of the instrument 1020 and a pouch 1013 is created of stomach tissue 12. A first cross-section of the luminal space 1021 is larger than an area of a second parallel cross-section placed in the opening 1022 of the luminal space, placed more distally in the luminal space or distally in the instrument.

Fig. 13c shows the placing of a fixating member 14 in form of a suture of staple closing the pouch created in the stomach 12.

Fig. 13d shows the pouch created in the stomach after the instrument has been removed.

Fig. 13e shows an alternative way of closing the pouch 1013 created in the stomach 12 after the instrument has been removed, the alternative way comprises placing fixating members 14, such as sutures, not penetrating the mucosa layer, thus not entering the stomach.

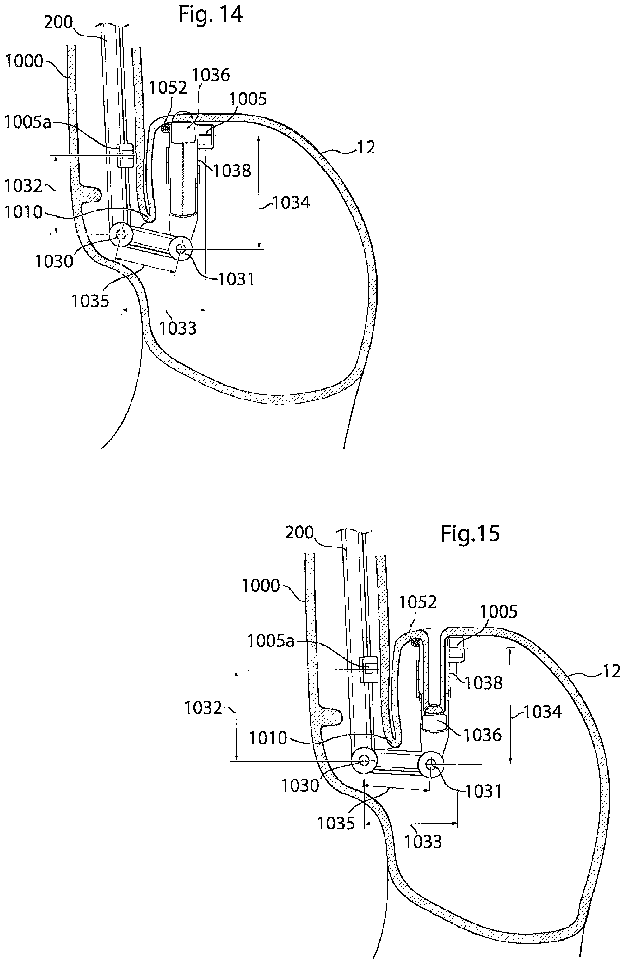

Fig. 14 - 23 shows an embodiment of an instrument and method for creating a pouch in the stomach 12 and fixating the esophagus 1000 to the stomach tissue 12.

Fig. 14 shows the instrument 200 as a gastroscopic instrument 200 which has been inserted into the throat of a patient. The instrument having a unit 1005 mounted thereon having a first and second part 1005a,b adapted to engage fixating members. For enabling the instrument to go around the angle of His 1010 and reach the fundus area of the stomach the instruments have two operable joints 1030 and 1031. The first operable joint 1030 is placed at a distance 1032 from the first part of the unit 1005a. The distance 1032 is according to one embodiment more than 1 cm, and according to another embodiment more than 2 cm, and according to another embodiment more than 3 cm, and according to another embodiment more than 4 cm, and according to another embodiment more than 5 cm. A portion of the instrument is placed between the first 1030 and second 1031 joint and is a portion having a length 1035. The length 1035 is according to one embodiment more than 1 cm, and according to another embodiment more than 2 cm, and according to another embodiment more than 3 cm, and according to another embodiment more than 4 cm, and according to another embodiment more than 5 cm. The second unit 1005b is then placed at a distance 1034 from the second joint 1031 for placing the second unit 1005b in a position such that the first and second 1005a,b units can deliver fixating members between themselves and engaging the first and second unit 1005a,b. The distance 1034 is according to one embodiment more than 1 cm, and according to another embodiment more than 2 cm, and according to another embodiment more than 3 cm, and according to another embodiment more than 4 cm, and according to another embodiment more than 5 cm. The instrument 200 comprises a stomach tissue penetrating member 1036 adapted to grab a hold of the stomach 12 for pulling the stomach 12. The most distal portion of the instrument 200 comprises a telescopic portion 1038 which could be used for adapting the position of the second unit 1005b in relation to the first unit 1005a, and later be used to assist in the insertion of at least one volume filling device into the pouch created in the stomach tissue 12.

Fig. 15 shows the instrument and method step when the stomach tissue has been pulled into a luminal apace of the distal portion of the instrument, thus creating a pouch of the stomach tissue 12.

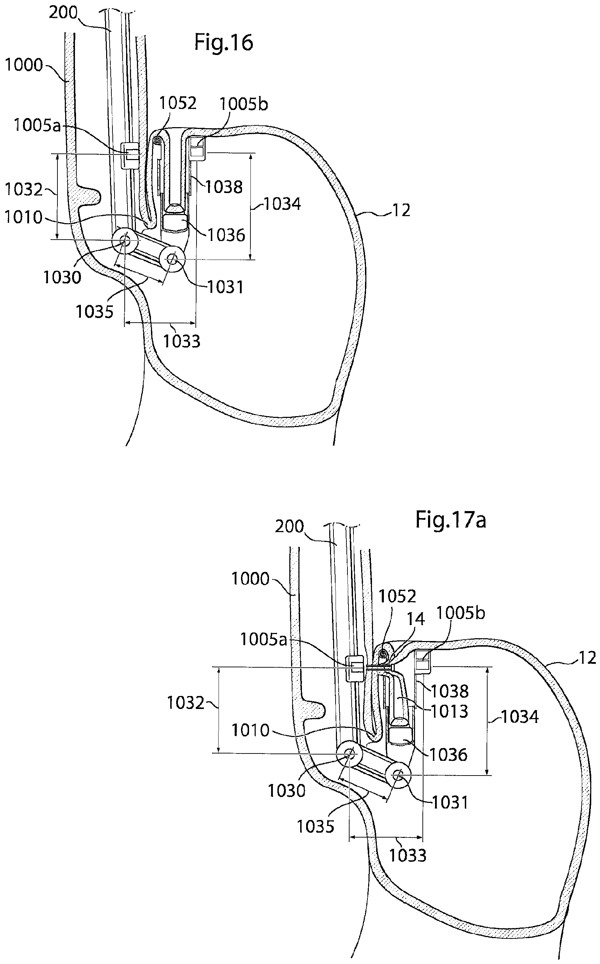

Fig. 16 shows the instrument when the instrument is in the state in which fixation members can be delivered either from the first or second part of the unit 1005a,b. The part of the unit not delivering fixating members is an anvil member engaging the fixating member and assisting in the fixation thereof.

Fig 17a shows the instrument when the fixation member 14 has been delivered such that the fixation member penetrates three layers of stomach tissue and one layer of esophagus tissue thus creating a pouch 1013 by the two layers of stomach tissue being fixated to each other. The creation of a pouch 1013 adapted to be filled with a volume filling device and the fixation of the stomach tissue 12 to the esophagus tissue restrains the esophagus 1000 in the abdominal cavity and thus assists in the treating of reflux disease.

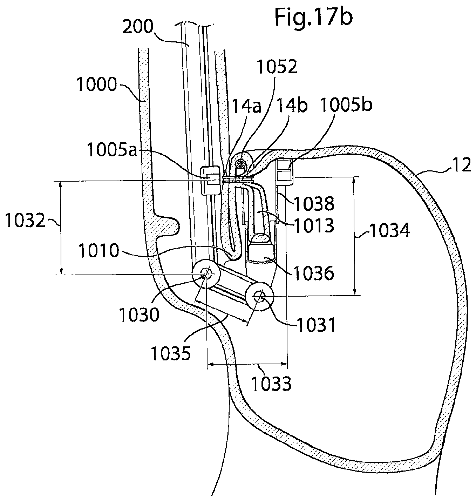

Fig. 17b shows an alternative embodiment, when separate fixating members 14a,b have been placed such that the first fixating member 14a fixates two layers of stomach 12 to each other, and the second fixating member 14b fixates one layer of stomach tissue 12 with one layer of esophagus tissue 1000.

Fig. 18 and 19 shows an alternative embodiment of the instrument 200, in which the instrument is adapted to pull the stomach 12 using suction or vacuum. Except for the difference in the stomach pulling member, fig. 18 and 19 discloses the instrument and method steps of fig. 14 and fig. 15 being steps that are performed before the delivering of fixating members for finalizing the pouch 1013.

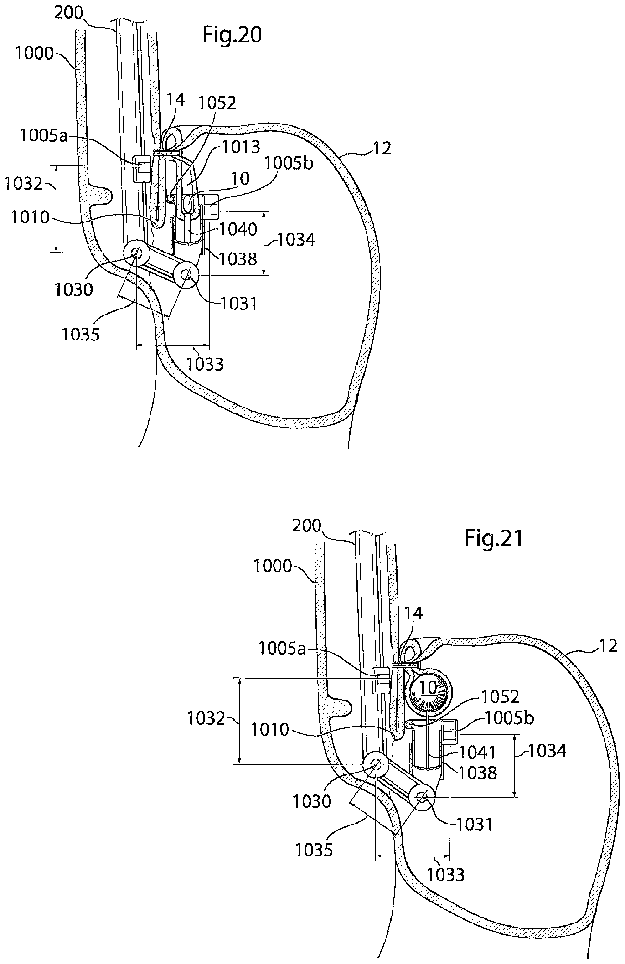

Fig. 20 shows the instrument after the steps of creating the pouch 1013 have been concluded and the step of delivering an expandable volume filling device 10 starts. The volume filling device 10 is delivered through a tubular insertion device 1040 placed in the instrument and being adapted to enter the pouch 1013 created in the stomach 12.

Fig. 21 shows the instrument after the step of inserting the expandable volume filling device 10 has been performed and the step of filling the volume filling device 10 with a fluid for expanding the volume filling device 10 is performed. The fluid is transported through a conduit 1041 placed in the instrument and entering the pouch 1013 from below.

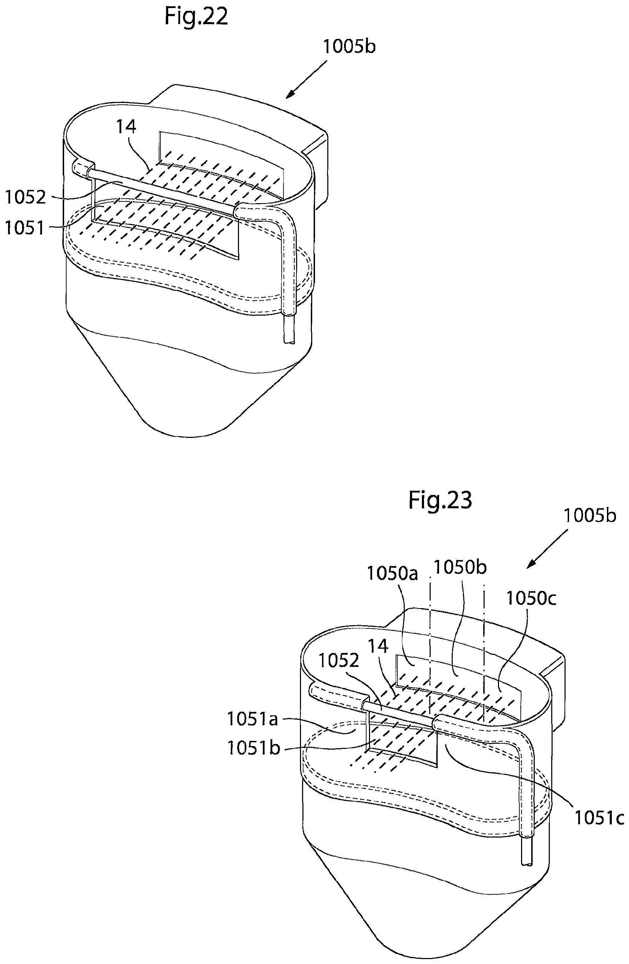

Fig. 22 shows a first embodiment of the distal portion of the instrument disclosed in the previous figures. The distal portion comprises the opening to the luminal space in the instrument adapted to receive stomach tissue 12. The second unit 1005b is mounted on the distal part and is, according to the embodiment shown in fig. 23 a unit adapted to deliver fixating members for fixating two layers of stomach tissue 12 to each other and/or layers of stomach tissue 12 to the esophagus. The unit 1005 is adapted to deliver fixating members adapted to enter through a recess 1051 in the distal portion of the instrument fur further passing though the esophagus 1000 for fixating layers of stomach 12 to the esophagus 1000. The distal portion further comprises a retractable holding portion 1052 adapted to hold the stomach tissue 12 in position when delivering the fixating members, and then be retracted to enable the removal of the instrument from the area of the stomach 12.

Fig. 23 shows a second embodiment of the distal portion of the instrument disclosed in the previous figures. The distal portion comprises the opening to the luminal space in the instrument adapted to receive stomach tissue 12. The second unit 1005b is mounted on the distal part and is, according to the embodiment shown in fig. 23 a unit adapted to deliver fixating members for fixating two layers of stomach tissue 12 to each other and/or layers of stomach tissue 12 to the esophagus. The unit 1005b delivering fixating members is divided into three different portions 1050a, 1050b and 1050c, wherein the first and third portion 1050a and 1050c are adapted to deliver fixating members adapted to engage anvils 1051a and 1051c located on the opposite side of the luminal space for fixating at least two layers of stomach tissue 12 to each other. The second portion 1050b of the unit is adapted to deliver fixating members of longer length or depth which are adapted to enter through a recess 1051b in the distal portion of the instrument fur further passing though the esophagus 1000 for fixating layers of stomach 12 to the esophagus 1000. The distal portion further comprises a retractable holding portion 1052 adapted to hold the stomach tissue 12 in position when delivering the fixating members, and then be retracted to enable the removal of the instrument from the area of the stomach 12.

Fig. 24 discloses an embodiment of the instrument and a method of using said instrument, wherein the method is a surgical or laparoscopic method. The instrument penetrates the stomach wall through an incision 1080 in the stomach wall 12 performed from outside of the human patient and this way enters the stomach 12 cavity for operating of the stomach 12 from the inside thereof. The instrument comprises two joints 1070 and 1071 for positioning the instrument such that the instrument can operate and perform required steps on the stomach 12. The first operable joint 1070 is placed at a distance 1073 from the second operable joint 1071. The distance 1073 is according to one embodiment more than 1 cm, and according to another embodiment more than 2 cm, and according to another embodiment more than 3 cm, and according to another embodiment more than 4 cm, and according to another embodiment more than 5 cm. The second unit 1005b is placed at a distance 1072 from the second joint 1071 for placing the second unit 1005b in a position such that the first and second 1005a,b units can deliver fixating members between themselves and engaging the first and second unit 1005a,b. The distance 1072 is according to one embodiment more than 1 cm, and according to another embodiment more than 2 cm, and according to another embodiment more than 3 cm, and according to another embodiment more than 4 cm, and according to another embodiment more than 5 cm. The instrument 200 comprises a stomach tissue penetrating member 1036 adapted to grab a hold of the stomach 12 for pulling the stomach 12. The most distal portion of the instrument 200 comprises a telescopic portion 1038 which could be used for adapting the position of the second unit 1005b in relation to the first unit 1005a, and later be used to assist in the insertion of at least one volume filling device into the pouch created in the stomach tissue 12. The first part of the unit is placed on a portion 1079 of the instrument comprising a bendable portion and being adapted to enter into the esophagus from below. In the embodiment disclosed with reference to fig. 24 the part of the unit delivering fixating members are placed in the second part 1005b of the unit, however it is equally conceivable that it is the other way around.

Fig. 25 shows the instrument and method step when the stomach tissue has been pulled into a luminal apace of the distal portion of the instrument, thus creating a pouch of the stomach tissue 12.

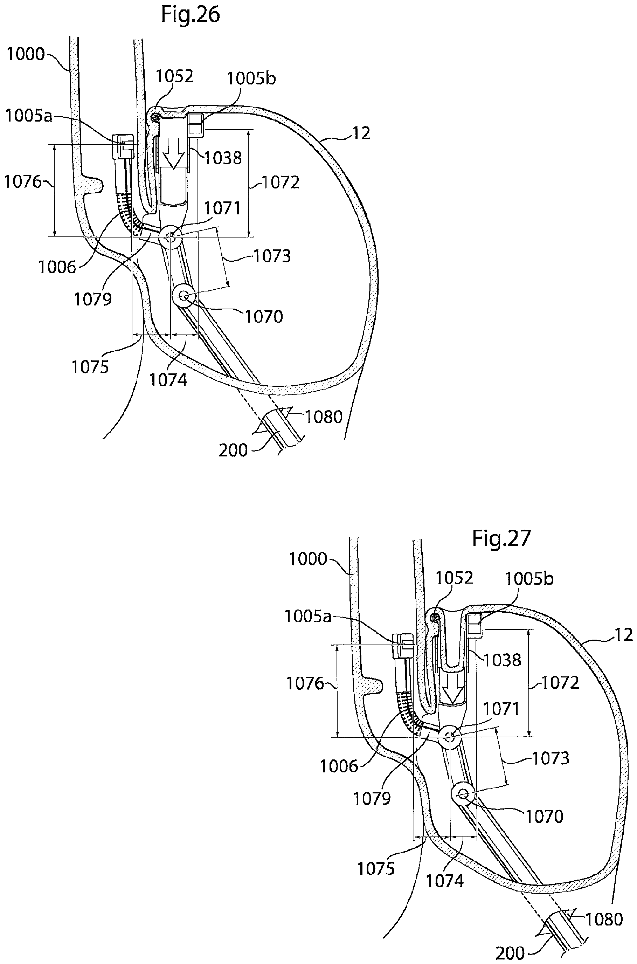

Fig. 26 and 27 shows an alternative embodiment of the instrument 200, in which the instrument is adapted to pull the stomach 12 using suction or vacuum. Except for the difference in the stomach pulling member, fig. 26 and 27 discloses the instrument and method steps of fig. 24 and fig. 25 being steps that are performed before the delivering of fixating members for finalizing the pouch 1013.

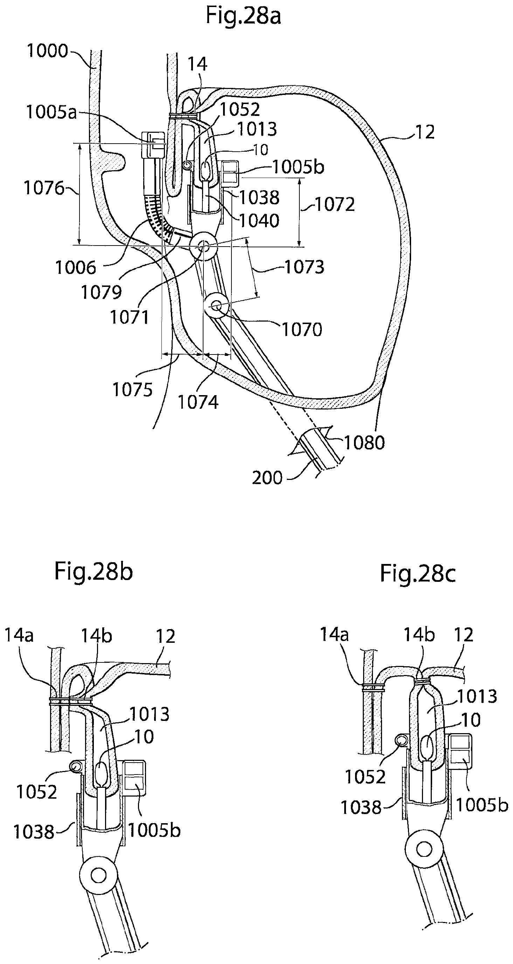

Fig. 28a shows the instrument after the steps of creating the pouch 1013 have been concluded and the step of delivering an expandable volume filling device 10 starts. The volume filling device 10 is delivered through a tubular insertion device 1040 placed in the instrument and being adapted to enter the pouch 1013 created in the stomach 12.

Fig. 28b and 28c shows an alternative embodiment, suturing from the inside of the stomach either as per fig 28a with the instrument introduced into the stomach from the abdominal cavity or alternatively through the throat into the stomach cavity, when separate fixating members 14a,b have been placed such that the first fixating member 14b fixates two layers of stomach 12 to each other, and the second fixating member 14a fixates one layer of stomach tissue 12 with one layer of esophagus tissue 1000.

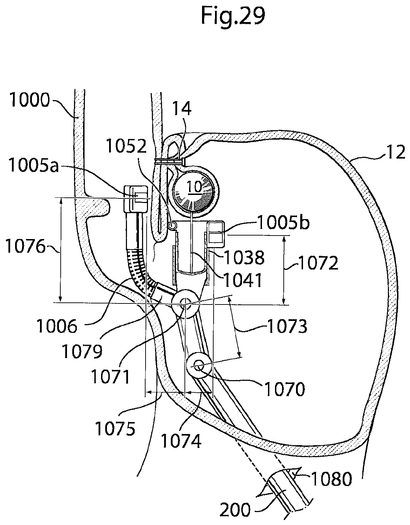

Fig. 29 shows the instrument after the step of inserting the expandable volume filling device 10 has been performed and the step of filling the volume filling device 10 with a fluid for expanding the volume filling device 10 is performed. The fluid is transported through a conduit 1041 placed in the instrument and entering the pouch 1013 from below.

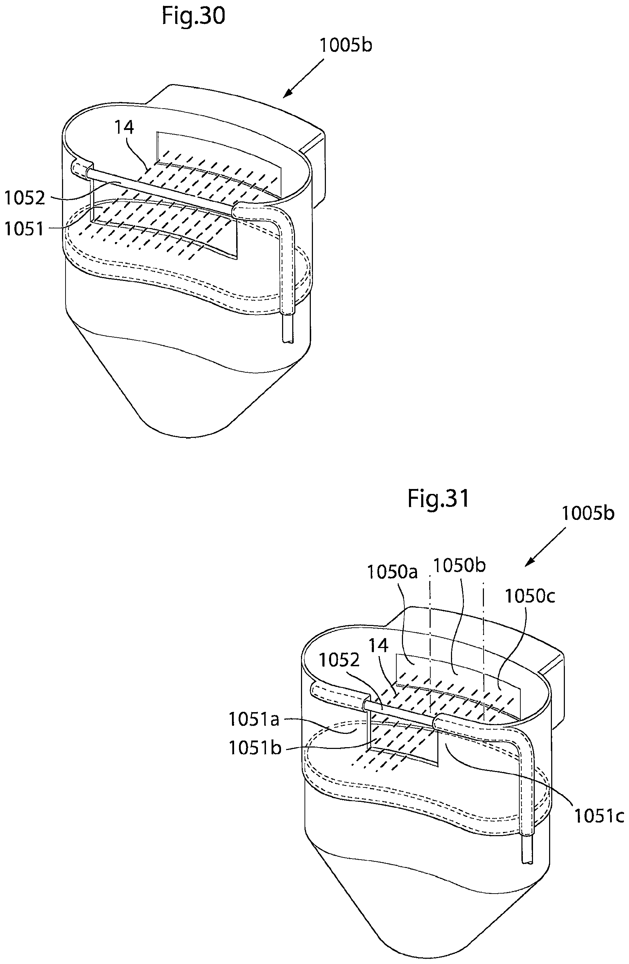

Fig. 30 shows a first embodiment of the distal portion of the instrument disclosed in the previous figures. The distal portion comprises the opening to the luminal space in the instrument adapted to receive stomach tissue 12. The second unit 1005b is mounted on the distal part and is, according to the embodiment shown in fig. 23 a unit adapted to deliver fixating members for fixating two layers of stomach tissue 12 to each other and/or layers of stomach tissue 12 to the esophagus. The unit 1005 is adapted to deliver fixating members adapted to enter through a recess 1051 in the distal portion of the instrument fur further passing though the esophagus 1000 for fixating layers of stomach 12 to the esophagus 1000. The distal portion further comprises a retractable holding portion 1052 adapted to hold the stomach tissue 12 in position when delivering the fixating members, and then be retracted to enable the removal of the instrument from the area of the stomach 12.

Fig. 31 shows a second embodiment of the distal portion of the instrument disclosed in the previous figures. The distal portion comprises the opening to the luminal space in the instrument adapted to receive stomach tissue 12. The second unit 1005b is mounted on the distal part and is, according to the embodiment shown in fig. 31 a unit adapted to deliver fixating members for fixating two layers of stomach tissue 12 to each other and/or layers of stomach tissue 12 to the esophagus. The unit 1005b delivering fixating members is divided into three different portions 1050a, 1050b and 1050c, wherein the first and third portion 1050a and 1050c are adapted to deliver fixating members adapted to engage anvils 1051a and 1051c located on the opposite side of the luminal space for fixating at least two layers of stomach tissue 12 to each other. The second portion 1050b of the unit is adapted to deliver fixating members of longer length or depth which are adapted to enter through a recess 1051b in the distal portion of the instrument fur further passing though the esophagus 1000 for fixating layers of stomach 12 to the esophagus 1000. The distal portion further comprises a retractable holding portion 1052 adapted to hold the stomach tissue 12 in position when delivering the fixating members, and then be retracted to enable the removal of the instrument from the area of the stomach 12.

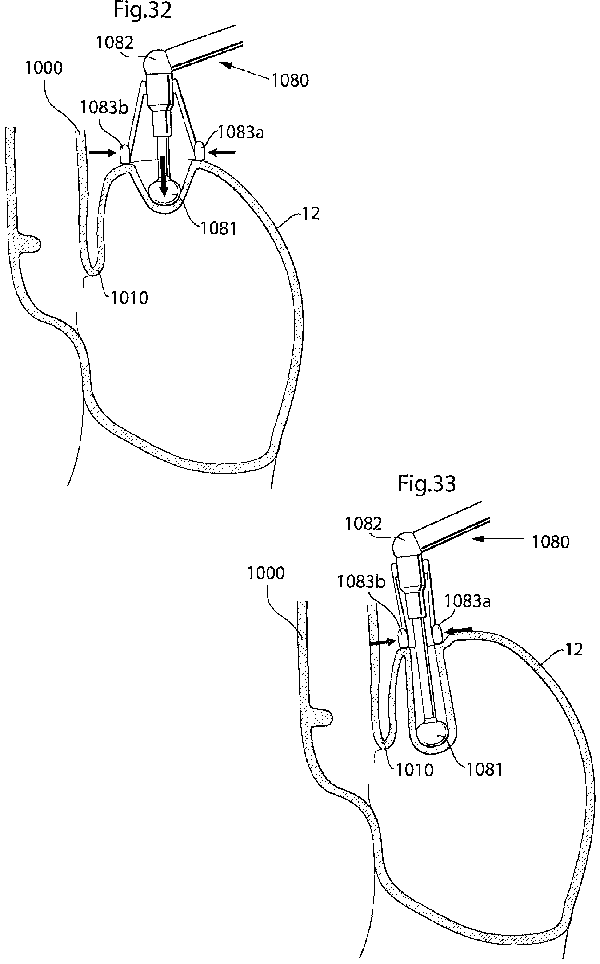

Fig. 32 shows an embodiment of the instrument in which the instrument is a surgical or laparoscopic instrument adapted to be used in a surgical or laparoscopic method to operate on the stomach from the outside thereof. The instrument 1080 is adapted to be inserted into the abdominal area through an incision or a trocar placed in the abdominal region penetrating the abdominal wall. The instrument comprises an operable joint 1082 for positioning the instrument in relation to the stomach 12. The instrument further comprises operable stomach holding members 1083a,b adapted to hold the stomach 12 while a stomach pushing member 1081 pushes the stomach 12 creating a recess in the stomach 12.

Fig. 33 shows the instrument when the stomach pushing member 1081 has pushed the stomach 12 further crating a larger recess or cavity in the stomach 12. When the stomach pushing member 1081 pushes the stomach 12 the two operable stomach holding members 1083a,b are moved closer together for allowing stomach tissue to create the recess.

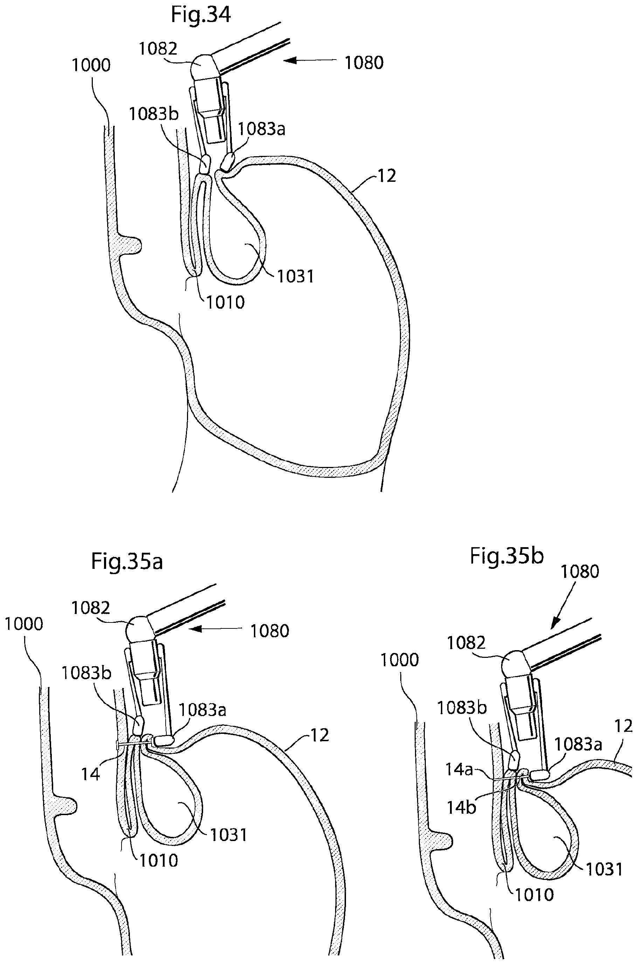

Fig. 34 shows the instrument after the recess has been made and the stomach pushing member has been retracted leaving a pouch 1013 created in the stomach wall 12. The stomach holding members 1083a,b are further adapted to deliver at least one fixating member for fixating layers of stomach tissue 12 to each other and/or fixating layers of stomach tissue to the esophagus 1000, and in the state shown in fig. 34 the stomach holding members 1083a,b are being positioned to deliver the at least one fixating member.

Fig. 35a shows the instrument when the fixating member 14 has been delivered fixating four layers of stomach tissue 12 and one layer of esophagus tissue 1000 to each other.

Fig. 35b shows the instrument in an embodiment identical to the embodiment of fig. 35a, however in the embodiment of fig. 35b the fixating member is a first 14a and second 14b fixating member, wherein the first fixating member is adapted to fixate three layers of stomach tissue 12 to each other thus creating a pouch 1013 of stomach tissue 12. The second fixating member 14b is adapted to fixate the esophagus to at least one layer of stomach tissue.

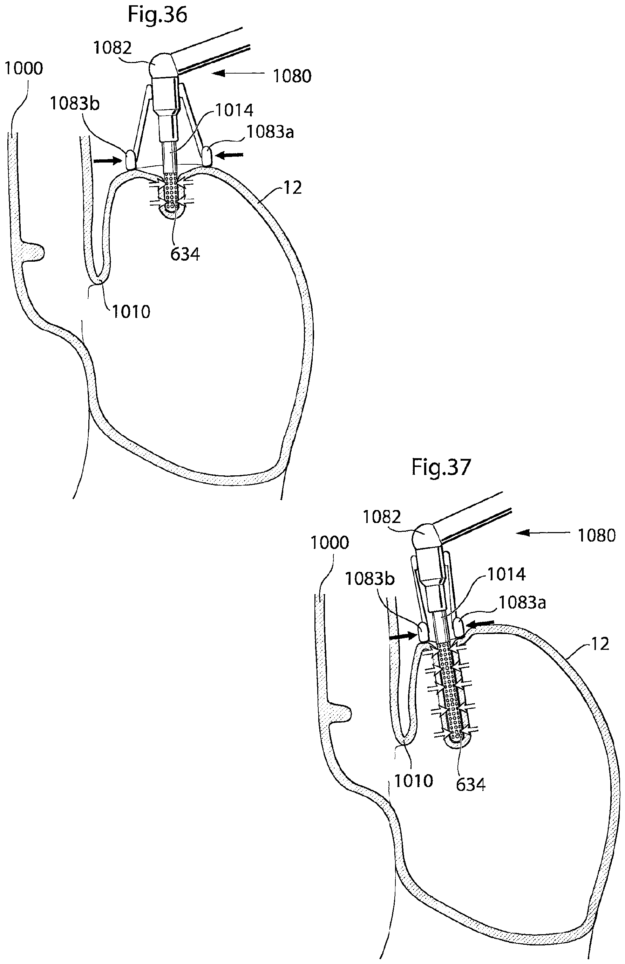

Fig. 36 and 37 shows an instrument performing the same steps as the instrument disclosed with reference to figs. 32 and 33 with the difference that in the embodiment of figs. 36 and 37 a stomach pushing member using vacuum is used. The stomach pushing member having a sucking portion adapted to connect to the stomach 12. The stomach pushing member comprising the sucking portion 634 is advanced from a sleeve and pushed into the stomach for creating the recess or cavity in the stomach. The vacuum instrument is further disclosed with reference to figs. 10a - 10e.

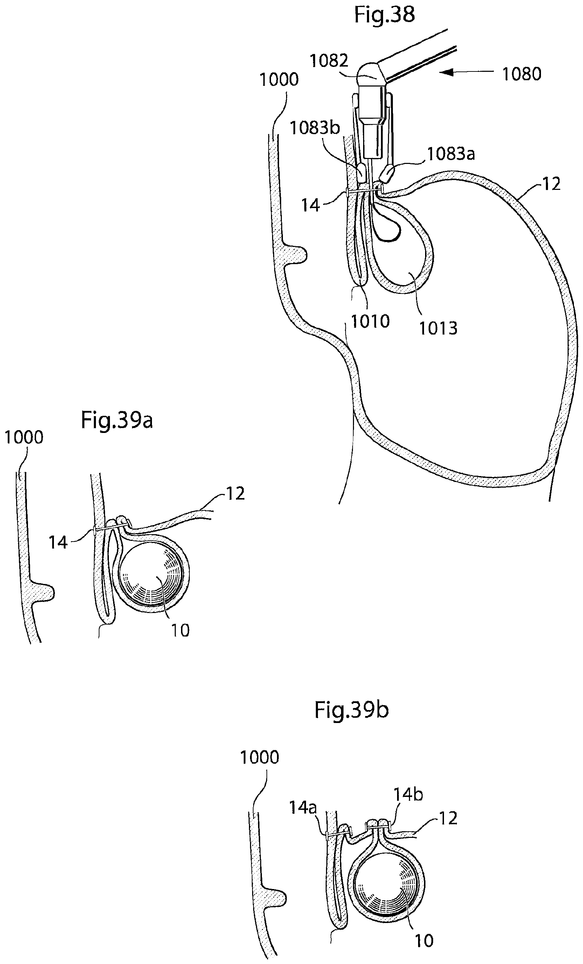

Fig. 38 shows the instrument after the steps of creating the pouch 1013 have been concluded and the step of delivering an expandable volume filling device 10 starts. The volume filling device 10 is delivered through a tubular insertion device 1040 placed in the instrument and being adapted to enter the pouch 1013 created in the stomach 12.

Fig. 39 shows the stomach when the expandable volume filling device has been expanded and fixating member fixates layers of stomach wall 12 to each other and the stomach 12 tissue to the esophagus 1000. Fig. 39a shows the movement restriction device inserted into the pouch.

Fig 39b shows an alternative route of suturing, wherein suture 14a sutures the double layer stomach to the esophagus from the outside of the stomach and suture 14b sutures four layers of stomach to create a pouch.

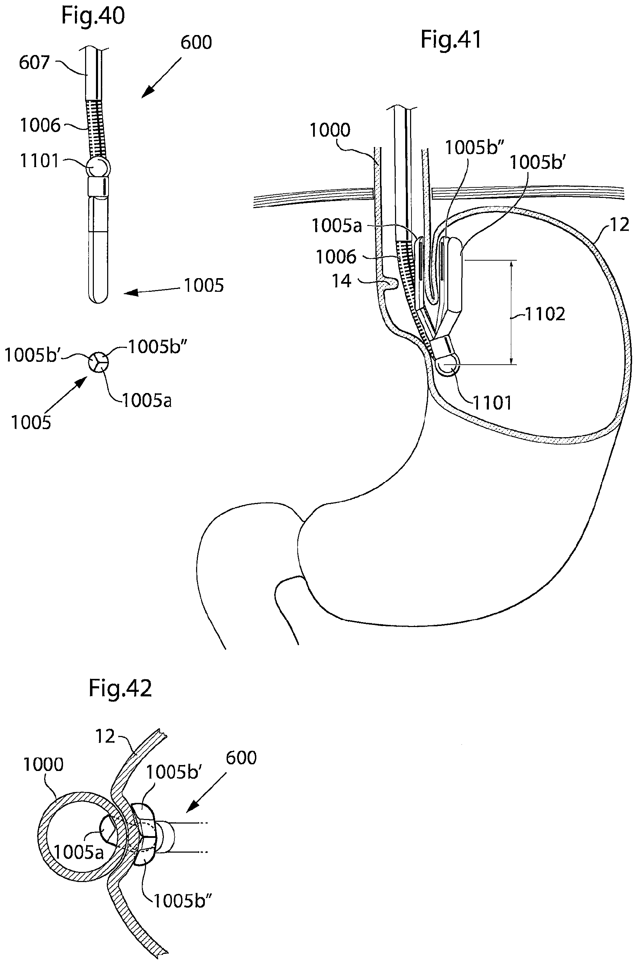

Fig.40 shows a gastroscopic and/or laparoscopic and/or surgical instrument 600 comprising an elongated member 607 having a proximal end and a distal end. The elongated member 607 having a diameter less than that of the patient's esophagus and being flexible through a bendable portion 1006 such as to allow introduction of the flexible elongated member 607 with its distal end first through the patient's throat, esophagus and into the stomach 12, alternatively through an incision in the abdomen or through a trocar placed in the abdominal region and penetrating in to the abdominal cavity. The instrument 600 is further provided with a fixating part 1005 hingedly arranged in an operable joint 1101 to a bendable part 1006 of said instrument 600. Fig. 40 further shows the fixating part 1005, divided in a first part1005a, a second part 1005b' and a third part 1005b". The fixating parts 1005a,b',b" are generally collapsed in a closed position when introduced into the stomach gastroscopically and/or laparoscopically and/or surgically. The fixating parts 1005a,b',b" further comprises fixating means (not shown) arranged at the surfaces adjacent and facing the respective fixation parts 1005a,b',b".

Fig. 41 shows the instrument of fig. 40 placed in the stomach 12 through esophagus 1000 for fixating stomach 12 tissue to esophagus 1000. The operable joint 1101 has been placed at a distance 1102 form a position above cardia 14. For enabling of the instrument 600 to position the hinged member 1101 at said distance 1 102 the instrument 600 comprises a bendable portion 1006. Fig 41 further shows the fixating part 1005 rotated to a second position around said operable joint 1101 and divided such that a first fixating part 1005a placed in esophagus 1000 and a second fixating part 1005b' and a third fixating part ,b" placed at their respective position in the stomach 12. The fixating parts are equipped such that a first fixating part 1005a is an anvil and 1005,b',b" is units for delivering of fixating members, however it is equally conceivable that 1005b' or 1005b" comprise anvil and the other two, respectively, comprise units for delivering of fixating members. The stomach 12 tissue is fixated by said fixating part 1005 to esophagus 100, thus hindering the esophagus 1000 from sliding through the foramen in the thoracic diaphragm 18.

Fig. 42 is a section view of esophagus 1000 and adjacent stomach 12 tissue where an instrument according to fig. 40 has been placed with a first fixating part 1005a in esophagus and second and a third fixating part 1005b',b" such that each fixating part 1005a,b',b" place stomach 12 tissue and esophagus 1000 tissue at a position for delivering fixating members. The fixating parts are equipped such that a first fixating part 1005a is an anvil and 1005,b',b" is units for delivering of fixating members, however it is equally conceivable that 1005b' or 1005b" comprise anvil and the other two, respectively, comprise units for delivering of fixating members.

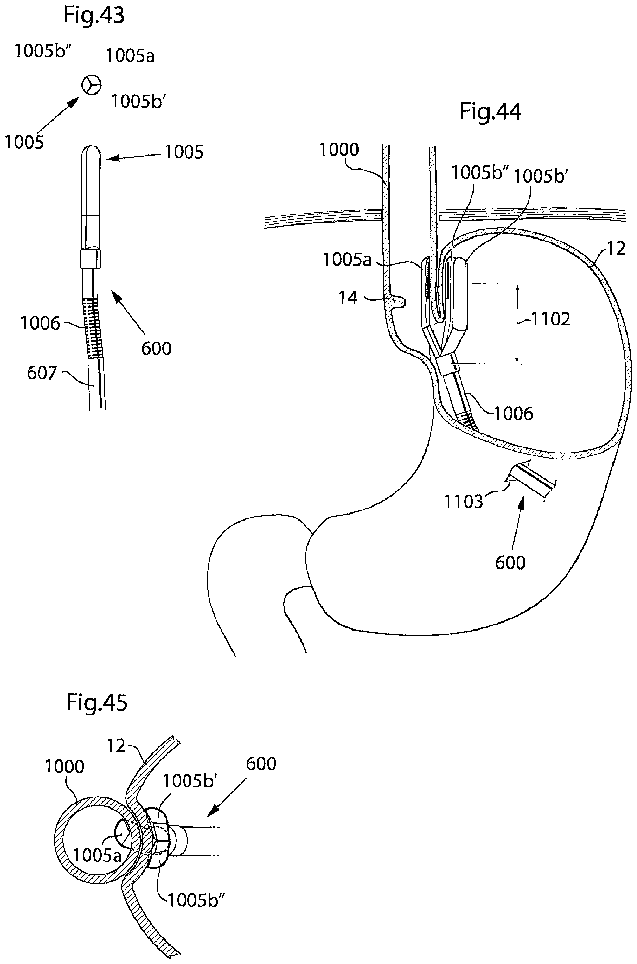

Fig. 43 shows a gastroscopic and/or laparoscopic and/or surgical instrument 600 comprise an elongated member 607 having a proximal end and a distal end. The elongated member 607 having a diameter less than that of the patient's esophagus and being flexible through a bendable portion 1006 such as to allow introduction of the flexible elongated member 607 with its distal end first through the patient's throat, esophagus and into the stomach 12, alternatively through an incision in the abdomen or through a trocar placed in the abdominal region and penetrating in to the abdominal cavity. The instrument further comprises a bendable member 1006 to enable positioning of a fixating part 1005. Said fixating part 1005 is divided in a first part1005a, a second part 1005b' and a third part 1005b". The fixating part 1005a,b',b" is generally collapsed in a closed position when introduced gastroscopically and/or laparoscopically and/or surgically The fixating parts 1005a,b',b" further comprises fixating means (not shown) arranged at the surfaces adjacent and facing the respective fixation parts 1005 a, b', b".

Fig. 44 shows the instrument 600 of fig. 43 introduced through the stomach 12 wall. The instrument 600 has been placed such that the fixating member delivery units of the fixating part 1005 are positioned at a distance 1102 above cardia 14. The fixating part 1005 is further placed such that a first fixating part 1005a is placed in esophagus, a second fixating part 1005b' and a third fixating part 1005b" is placed in a corresponding position in the stomach 12. Said first fixating part 1005a comprise an anvil and 1005b',b" comprise units for delivering of fixating members, however it is equally conceivable that 1005b' or 1005b" is an anvil and the other two, respectively, is units for delivering of fixating members. The instrument 600 further comprise a bendable member 1006 enabling the placing of the fixating member 1005 after the instrument 600 is introduced through the stomach wall 1103, by penetrating abdomen or through a trocar placed in the abdominal region and penetrating in to the abdominal cavity and. Fig. 45 shows a section view of esophagus 1000 and a part of the stomach 12 when fixating parts 1005a, b', b" of the instrument 600 of fig.43 has been place in accordance with fig.44.

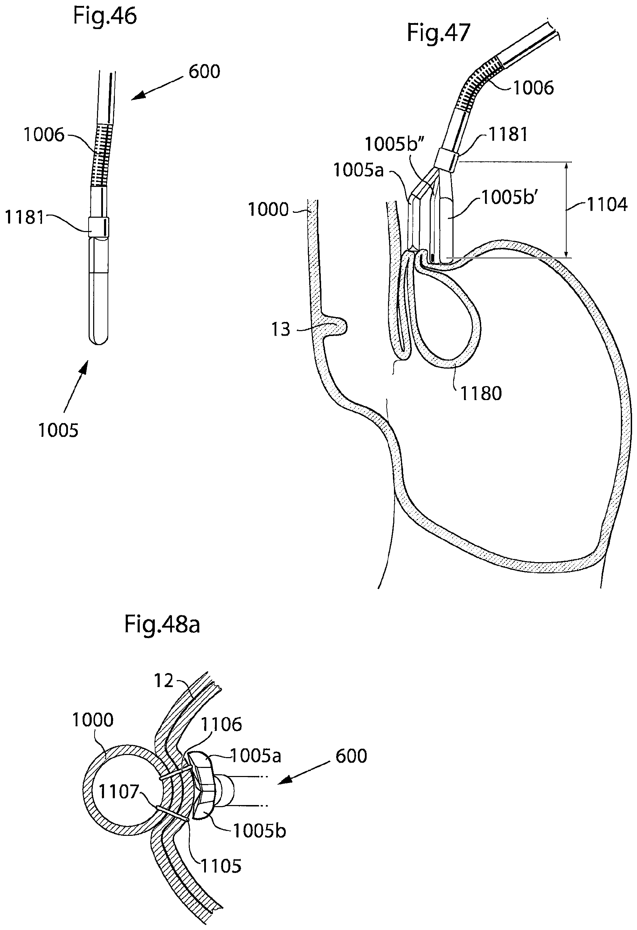

Fig. 46 shows a gastroscopic and/or laparoscopic and/or surgical instrument 600 comprises an elongated member having a proximal end and a distal end. The elongated member having a diameter less than that of the patient's esophagus and being flexible through a bendable portion 1006 such as to allow introduction of the flexible elongated member with its distal end first through the patient's throat, esophagus and into the stomach 12, alternatively through an incision in the abdomen or through a trocar placed in the abdominal region and penetrating in to the abdominal cavity. The instrument further comprises a bendable member 1006 to enable positioning of a fixating part 1005. Said fixating part 1005 is divided in a first part1005a, a second part 1005b' and a third part 1005b". The fixating parts 1005a,b',b" are generally collapsed in a closed position when introduced into the stomach 12 cavity gastroscopically and/or laparoscopically and/or surgically. The fixating parts 1005a,b',b" further comprises fixating means (not shown) arranged at the surfaces adjacent and facing the respective fixation parts 1005a,b',b".

Fig. 47 shows the instrument of 46 in an abdominal embodiment, used in an abdominal method. The instrument creates a poach 1180 fixating two folds, each comprising two layers of stomach 12, to one layer of esophagus 1000 and creating a pouch 1180 of said two folds. The distal ends of the fixating parts 1005a,b',b" is further equipped with holding devices to place and fold stomach tissue 12 adjacent to esophagus delivering a fixating member through said four layers of stomach 12 tissue and esophagus 1000 at a position a distance 1104 from a attachment point 1181 and at a position above cardia 14.

Fig. 48a shows a section view of esophagus 1000 and two layers of stomach 12 tissue. Where the instrument 600 of sig. 46 is placed abdominally in accordance with fig. 47 to fixate said two layers of stomach 12 to esophagus 1000, and thereby creating a poach 1180 in accordance with figure 47. The fixating parts 1005a,b comprises units delivery for fixating members 1 106,1 107. Said fixating members 1106, 1107 are delivered through two layers of stomach 12 tissue and one layer of esophagus 1000. In one embodiment, the fixating members 1106, 1107 are sutures. In another embodiment, fixating members 1106, 1107 are staples.

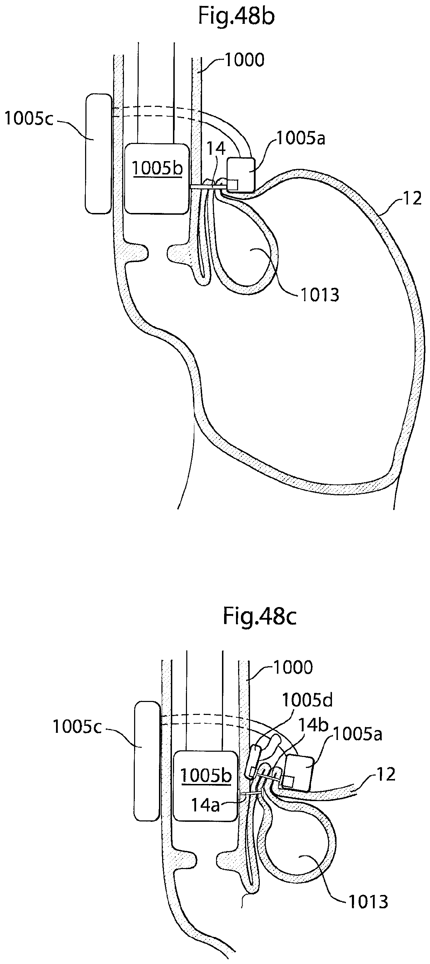

Fig. 48b shows an alternative embodiment of an instrument creating a pouch 1013 in the stomach 12 and fixating stomach tissue 12 to the esophagus 1000. The instrument comprises an abdominal portion adapted to engage the esophagus and stomach from the outside thereof. The instrument may clamp the esophagus and a folded thus double stomach wall. The abdominal part comprises a first part 1005a of a unit for delivering fixating members 14 and a third part 1005c adapted to act as a dolly when delivering the fixating members. The instrument further comprises an esophagus part adapted to be inserted into the esophagus through the throat of the patient. The esophagus part comprises a second part 1005b acting as an anvil member for the fixating member 14 delivered from the first part 1005a. This embodiment could be combined with an instrument creating a pouch 1013 according to any of the embodiments herein.

As shown in 48c the pouch could be sutured separate clamping only the four layers of stomach wall after having sutured the esophagus to the folded stomach double wall. This folded stomach wall will thereafter be used as one side of the pouch wall. In slide 48c, 1005 d is used as an anvil member or fixating device delivery member. This could be the same as 1005c and moved over to create the pouch or 1005b may be used instead although the suture should not pass the esophagus. The sutures of different length are marked as 14a suturing also the esophageal wall and 14b suturing the pouch. I all figures 48 it is as well as in the other cases where applicable possible to use a laparoscopic instrument adapted to pass through a trocar in the abdominal wall or using open surgery.

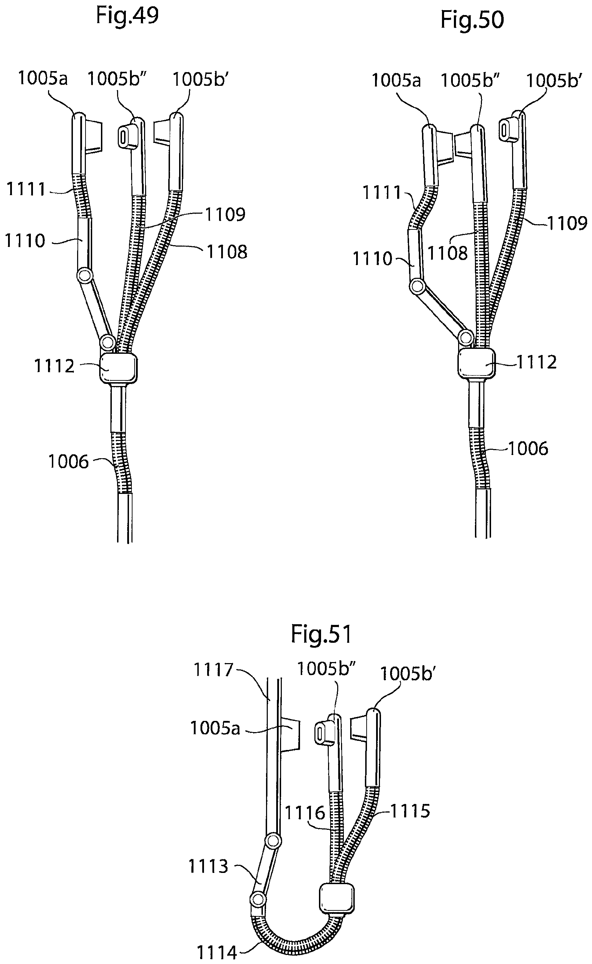

Fig. 49 shows an instrument with an elongated member comprising bendable portion 1006 arranged to a base member 1112, further comprising a first elongated member 1 108, a second elongated members 1109 and a third double hinged elongated member 1111 by means of two operable joints. Wherein a first and second elongated member are arranged at a respectively proximal end to said base member 1112. The third double hinged elongated member 1111 having a proximal end arranged to the base member 1112 such that a first hinge is arranged proximally adjacent to said base member 1112 and a second hinge at a distance from said first hinge. Each elongated member further comprise a fixating part 1005a, 1005b', 1005b" arranged at a distal end. In one embodiment, a first fixating part 1005a comprise an anvil and a second and third fixating part 1005b', 1005b" comprise fixating delivery units for delivery of fixating members. However, it is equally conceivable in another embodiment that a second fixating part 1005b' or a third fixating part 1005b" comprise an anvil and the other two fixating parts, respectively, comprise units for delivering of fixating members. Fig. 50 shows the instrument of fig 49 wherein the double hinged elongated member is placed in a second position, placing a first fixating part 1005a in a position facing a third fixating part 1005b" for deployment of fixating members.

Fig. 51 shows another embodiment of an instrument comprising a first part 1117 of the instrument with a proximal and a distal end arranged to a double hinged member 1113, wherein a second hinge is arranged to a bendable portion 1114 enabling a u-shape. A first elongated member 1116 and a second elongated member 1115 extending from said bendable portion 1114. Said first elongated member 1116 arranged with a fixating part 1005b' and said second elongated member 1115 arranged with a fixating part 1005b". Said fixating parts 1005b',1005b" are arranged to be substantially aligned with a radial axis from the first part 1117 through the third fixating part 1005a. Said third fixating part 1005a is further arranged at the first part 1117 a distance from a first hinge in form of an operable joint. In one embodiment, said third fixating part 1005a is stationary arranged to the first part 1117. In a second embodiment, said is the third fixating part 1005a movable parallel to the center axis of the first part 1117.

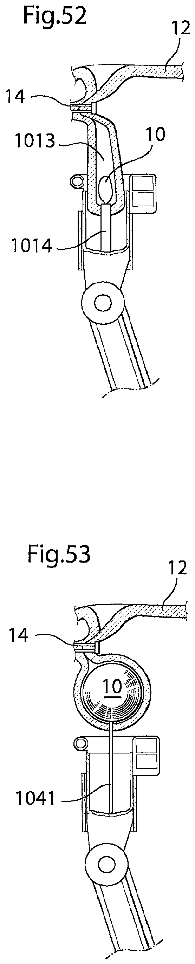

Fig. 52 shows the step of inserting an expandable member into the pouch 1013 created in the stomach in further detail, without the step of fixating the esophagus to the stomach. The steps of creating the pouch 1013 have been concluded and the step of delivering an expandable volume filling device 10 starts. The volume filling device 10 is delivered through a tubular insertion device 1040 placed in the instrument and being adapted to enter the pouch 1013 created in the stomach 12.

Fig. 53 shows the instrument after the step of inserting the expandable volume filling device 10 has been performed and the step of filling the volume filling device 10 with a fluid for expanding the volume filling device 10 is performed. The fluid is transported through a conduit 1041 placed in the instrument and entering the pouch 1013 from below.

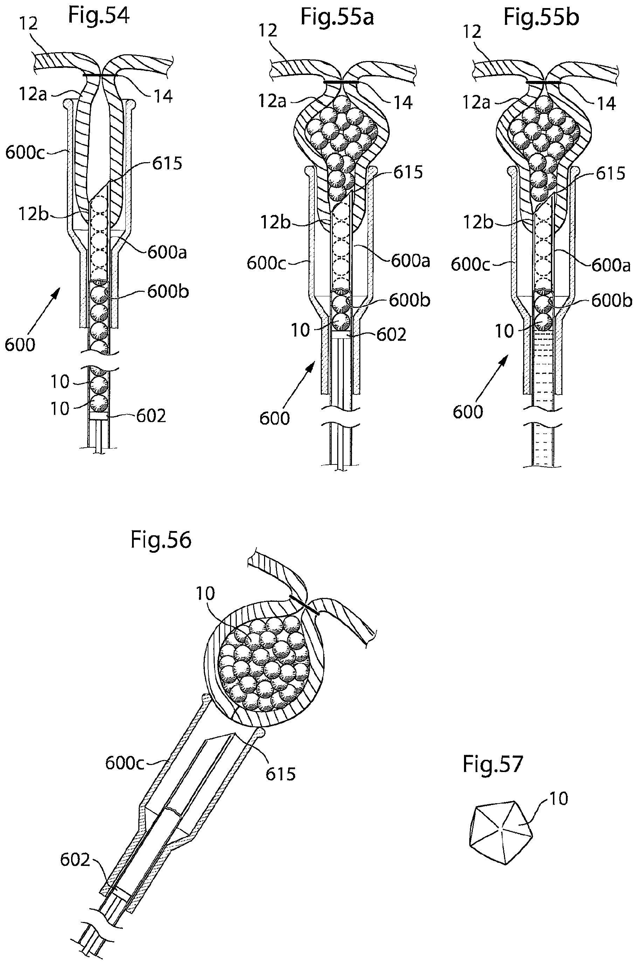

It is evident from the general description and the appended claims that many of other ways designing the volume filling device is possible without departing from this concept. One such way is to let a plurality of relatively small volume filling device segments form a volume filling device, which will now be described with reference to Figs. 54 - 57*.*

This method of injecting or inserting a plurality of volume filling devices or volume filling device segments into a pouch is similar to the one described above, after a pouch has been created in the stomach wall. Thus, Fig. 54 illustrates a stomach wall portion 12a after sutures or staples 14 have been applied to create a pouch in the stomach wall. The pouch can be provided by using the method described above.

Volume filling devices 10 are inserted or injected into the pouch by means of a gastroscopic or laparoscopic, tube-like instrument 600. The instrument comprises an outer sleeve and an inner sleeve, which can be displaced longitudinally relatively to the outer sleeve. The inner sleeve is provided with a cutter in the form of a cutting edge 615 at the distal end thereof. This cutting edge can be used for cutting a hole in the stomach wall, as will be explained in detail in the following.

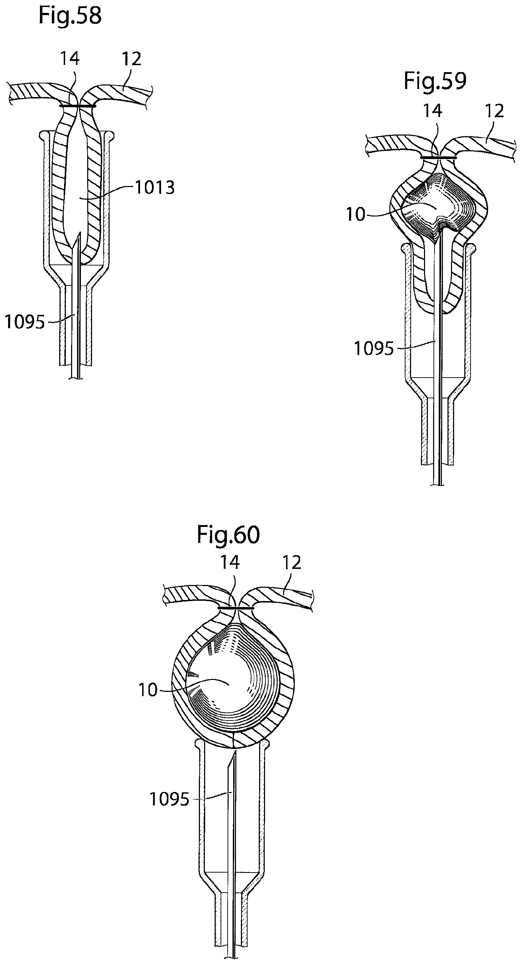

When the instrument reaches a stomach wall, the inner sleeve is brought forward from its position in the outer sleeve and into contact with the stomach wall 12a. The cutting edge 615 of the inner sleeve then cuts a hole in the stomach wall so as to allow subsequent insertion of the volume filling devices 10 into and through this hole, see Fig. 55a. In order to push the volume filling device through the hole, a piston 602 may be provided in the instrument. Thus, the instrument further comprises a piston 602 adapted for pushing a plurality of volume filling devices 10 out from a position in the inner sleeve, this position being shown in Fig. 60, to a position outside of the inner sleeve, this being shown in Fig. 55a.

In order to protect the deflated volume filling device 10 from the cutting edge 615 of the inner sleeve, a further protective sleeve (not shown) can be provided around the volume filling device.

The tube-like instrument 600 is in the shown embodiment provided with a cup-shaped extension keeping the pouch in place during the insertion of the volume filling device segments 10 into the pouch. By gradually withdrawing the tube-like instrument 600 during this process, see Fig. 55a, the pouch can be filled with volume filling devices 10 in a controlled way.

After the pouch has been filled with volume filling devices to a desired degree, see Fig. 56, the hole 12b cut in the stomach wall 12a by means of the tube-shaped instrument 600 is permanently closed by means of suturing or stapling, for example.

In the embodiment shown in Figs. 54 - 57, the tube-like instrument 600 comprises a piston 602 adapted to push or displace the volume filling devices 10 along the sleeve 600b. Alternatively, pressure exerted by fluid can be used to push or displace the volume filling device segments 10, as shown in fig. 55b.

The volume filling devices 10 can take many different shapes. In the embodiments shown, they are essentially spherical. However, in alternative embodiments, they exhibit one or a plurality of flat or essentially flat surfaces. Preferably, they then take the shape of polyhedrons, such as tetrahedrons, hexahedrons, octahedrons, dodecahedrons or icosahedrons, i.e., regular polyhedrons with four, six, eight, twelve, and twenty flat surfaces, respectively. An example of volume filling device segments is shown in Fig. 57.

Fig. 58 shows the insertion of conduit for injecting a solidifying fluid into the pouch1013, which step is shown in fig. 59. Fig. 60 shows the solidifying volume filling device when cured and in a solid or semi-solid state, the conduit is retracted and the hole in the pouch is preferably closed by means of sutures or staples. The step of injecting a solidifying fluid could be performed as a step in any of the gastroscopic, laparoscopic or surgical method above.

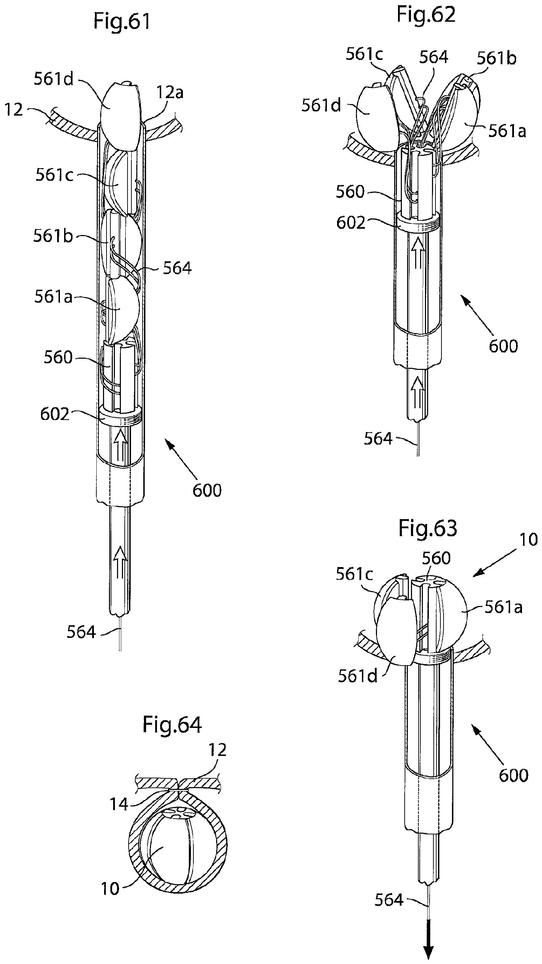

A method of injecting or inserting a plurality of volume filling devices into a pouch formed by a part of a stomach wall will now be described with reference to Figs. 61 - 64. A tube-like instrument, generally designated 600, comprises a sleeve 600a having a cross-sectional diameter and shape so as to allow the passage of the core part 560 and the four outer parts 561a-561d. A piston 602 is provided to displace the volume filling device segments through the sleeve 600a and into a space, wherein the volume filling device segments are to be assembled into or form an interconnected volume filling device. As shown in Fig. 61, in this example the instrument 600 is used to insert or inject the volume filling device segments through a hole 12a in a stomach wall portion 12 of a patient.

As shown in Fig. 62, the guiding wire 564 forces the outer parts 561a-d to take a respective position like petals to allow the core part 560 to take a position allowing the outer parts 561a-d and the core part 560 to assemble into an essentially spherical volume filling device 10. By pulling the guiding wire 564, see Fig. 63, the outer parts 561a-d are moved into engagement with the core part 560, being kept in position by means of the interlocking flanges 563a-d.

After being fully assembled, see Fig. 64, the essentially spherical volume filling device 10 is invaginated in part of the stomach wall 12 by means of sutures or staples 14. The limited space of the pouch formed by the stomach wall prevents the volume filling device 10 from falling apart, even after the biodegradable guiding wire 564 has been degraded. However, in the event that volume filling device 10 comes loose, such as by the sutures or staples 14 breaking, the volume filling device 10 falls apart so that the different segments, each being smaller than the assembled volume filling device 10, can pass through the gastrointestinal system.

The volume filling devices can additionally be kept in their mutual relationship by means of additional measures. For example, the surfaces of the volume filling device segments can be provided with friction enhancing agent or material to minimize slipping of the volume filling device segments. The friction enhancing agent can be some kind of glue or the like. Alternatively or additionally, the surface or surfaces of the volume filling device segments can be given a rugged texture to increase the friction between adjacent volume filling device segments.

As mentioned above, the volume filling device 10 may be inflated with a gel or fluid supplied into a chamber defined by the volume filling device 10, see Fig. 62.

Insertion or injection of a plurality of volume filling device segments 10 into a natural pouch provided by a stomach wall portion 12 has been described above with reference to Figs. 54 - 63. However, in an alternative embodiment of an apparatus for treating obesity, the volume filling device segments can also comprise a volume filling device segment for collecting two or more other volume filling device segments, which are of a different kind. Thus, the apparatus comprises an expandable second volume filling device segment 10' for collecting two or more first volume filling device segments 10 different from the second volume filling device segment, wherein the second volume filling device segment and the first volume filling device segments together form the volume filling device. It is preferred that the second volume filling device segment is flexible or stretchable.

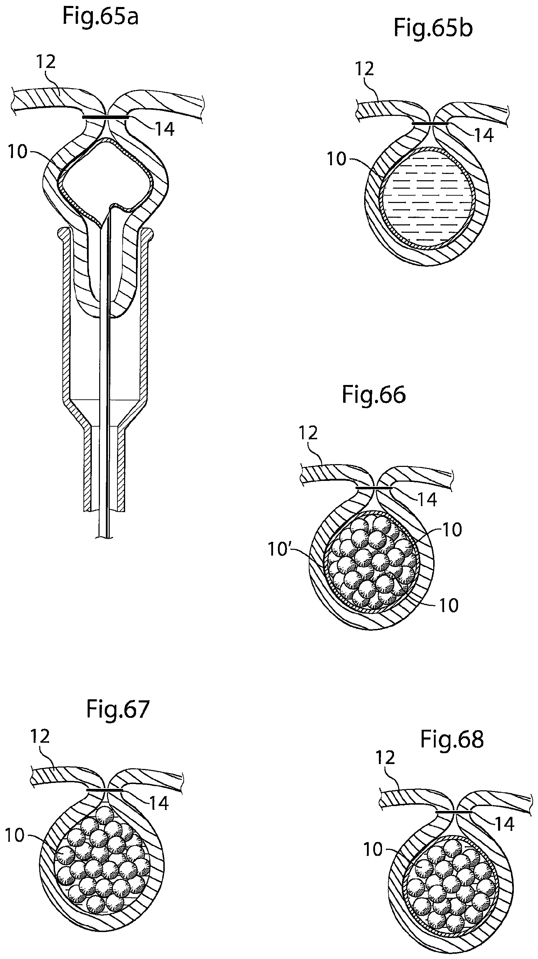

Fig. 65a shows the insertion of an expandable volume filling device into the pouch created of stomach tissue 12.

Fig. 65b shows the expandable volume filling device after the expandable volume filling device has been injected with a fluid, such as a solidifying fluid.

Fig. 66 shows an embodiment when a plurality of volume filling device is inserted into the expandable volume filling device.

In an alternative embodiment, to allow reshaping of the volume filling device, there may be a friction reducing fluid to reduce the friction between adjacent volume filling device segments. In Fig. 67, it is shown how a plurality of volume filling device segments 10 are provided in a pouch created by part of a stomach wall 12 and a fluid has been injected into this pouch, which allows mutual movement between adjacent first volume filling device segments so that the shape of the volume filling device adapts to stomach wall movements, when said volume filling device is invaginated in a stomach wall.

The embodiments of Fig. 66 and Fig. 67 may be combined, so that an outer volume filling device segment 10' encloses both a plurality of different, in this case spherical, volume filling device segments 10 as well as a fluid, which reduces the friction between the different volume filling device segments.

The fluid described above may, instead of reducing the friction between adjacent volume filling device segments, enhance the friction between adjacent volume filling device segments, thus making the volume filling device more stable. The friction enhancing material may also be a glue or an adhesive, i.e., a solidifying liquid.

Volume filling device segments adapted to be used in a plurality to form a volume filling device have been described above. It will be appreciated that these volume filling device segments can be given the same properties as the volume filling devices described earlier regarding materials, properties etc.

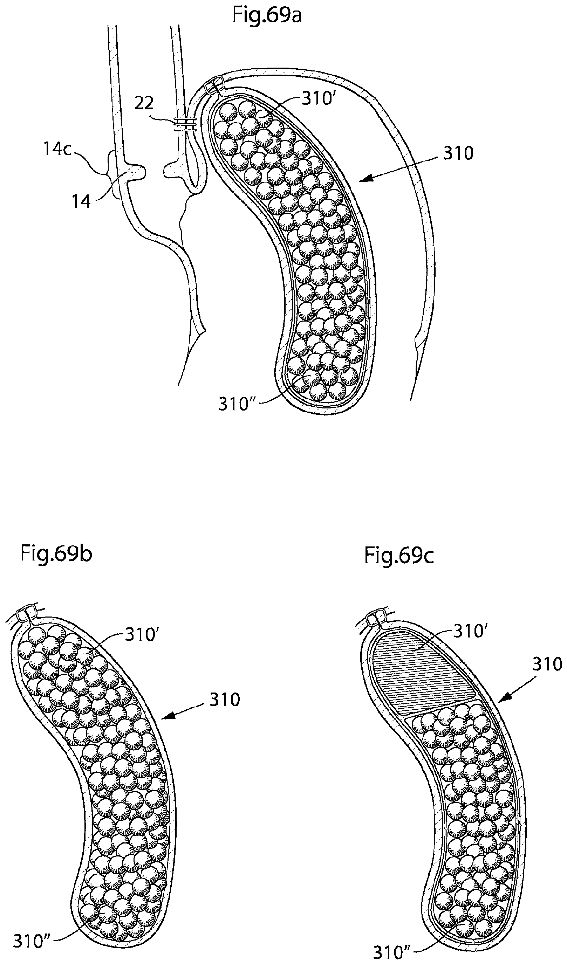

Fig. 69a-c shows an invaginated movement restriction device and/or combined volume filling device in section filled with a plurality of volume filling devices. The multiple volume filling devices or the multiple volume filling device segments may as in fig 69b be filled directly into a pouch created by stomach wall or may as in fig 69a be filled into a collecting hollow preferable flexible volume filling device. The shown embodiment in fig 69c includes a proximal part, a movement restriction device 310' for treating reflux disease preventing the cardia sphincter sliding up into the thorax and a distal part 310", a volume filling device that may have double function: first, stabilizing the movement restriction device being invaginated in the stomach wall and second, acting as a volume filling device for treating obesity and reducing stomach cavity volume.

In this preferred embodiment, the apparatus for treating both reflux disease and obesity comprises two or more movement restriction device segments adapted to form the movement restriction device 310. In this embodiment, there are a plurality of spherical movement restriction device segments in the form of small balls which are contained in a movement restriction device segment in the form of an outer layer or shell, which preferably is elastic or flexible. In this way, the outer layer can be inserted into the stomach as a separate part, which subsequently is filled with a plurality of small, preferably spherical or polyhedral movement restriction device segments. This method will be explained below with reference to Figs. 59a-c.

By providing a movement restriction device 310 with a plurality of movement restriction device segments, the movement restriction device 310 easily adapts to the movement of the stomach into which it is invaginated.

In an alternative embodiment, the small movement restriction device segments are inserted or injected into a pouch previously created by part of the stomach wall of the patient without any collecting outer layer or shell. This embodiment is illustrated in Fig. 11b and corresponds to the method described below with reference to Fig. 59a-c.

In order to provide a movement restriction device which is more stable in the proximal part 310', this part can be made of a different configuration from the distal part 310". Thus, in an embodiment shown in Fig. 11c, the proximal part 310', which is fixed to a position above the cardia area 14c, comprises a larger ball shaped part or segment, while the distal part 310" comprises a plurality of small movement restriction device segments. This embodiment combines the advantages of a stable proximal part 310' used for preventing reflux with a more adaptable distal part 310" used as a volume filling device for treating obesity. Generally, the proximal and distal parts can have different configurations and contents, independent of each other. This content can be a mixture of solid and fluid content, such as friction enhancing or reducing fluid.

Small volume filling devices and volume filling device segments could be used interchangeable.

In one embodiment of the apparatus, at least one of the volume filling device segments has at least one flat surface. Preferably, the volume filling device segments has the shape of a polyhedron, preferably one of the following shapes: tetrahedron, hexahedron, octahedron, dodecahedron and icosahedrons.

In one embodiment of the apparatus, a friction enhancing material is provided. This increases the friction between adjacent volume filling device segments, thereby stabilizing the volume filling device. This friction enhancing material is preferably a glue or an adhesive. Alternatively, at least one of the volume filling device segments has a surface with a rugged texture.

In one embodiment of the apparatus, at least one of the volume filling device segments has spherical shape. Alternatively, it has at least one flat surface.

In an alternative embodiment, the apparatus comprises a fluid for reducing the friction between adjacent volume filling device segments. The volume filling device can thereby more easily adapt its shape to the movements of the patient's body.

The apparatus may in one embodiment comprise a friction reducing material on the outer surface of the volume filing device segments. This friction reducing material may be a fluid reducing the friction between adjacent volume filling device segments.

The apparatus may comprise an expandable second volume filling device segment for enclosing two or more first volume filling device segments different from the second volume filling device segment, wherein the second volume filling device segment and the first volume filling device segments together form the volume filling device. In one alternative, the second volume filling device segment comprises a friction reducing material on an inner surface thereof, the friction reducing material being in contact with the first volume filling device segments, when implanted.

The second volume filling device segment may be adapted to be filled with a fluid to allow mutual movement between adjacent first volume filling device segments so that the shape of the volume filling device adapts to stomach wall movements, when said volume filling device is invaginated in a stomach wall. It is then preferred that at least a wall portion of the second volume filling device segment is flexible or stretchable.

The fluid provided in the volume filling device may be isotonic or hypertonic.

The volume filling device segments may be adapted to be inserted into a pouch formed by part of a stomach wall of the patient. The volume filling device segments may be adapted to be filled, directly or indirectly, into the pouch formed by part of a stomach wall of the patient via a tubular instrument.

In one embodiment, the volume filling device comprises a solidifying liquid.

This liquid or fluid may be supplied to the pouch by means of a conduit.

In one embodiment, the plurality of volume filling device segments are adapted to be interconnected to form the volume filling device, after said plurality of volume filling device segments have been inserted into a human or artificial pouch.

The apparatus, wherein the volume filling device segments adapted to be assembled to an implantable volume filling device.

The volume filling device segments are preferably adapted to form an implantable volume filling device of a controlled size.

Please note that any embodiment or part of embodiment as well as any method or part of method or any apparatus or part of apparatus or any feature or part of feature or any system or part of system could be combined in any applicable way. All examples herein should be seen as part of the general description and therefore possible to combine in any way in general terms.

## Claims

1. An endoscopic instrument (200) adapted to engage the stomach tissue and/or the esophagus tissue of a patient from the inside and/or outside thereof, the esophagus (1000) being a substantially tube shaped tissue leading to the stomach (12) comprising stomach tissue, the esophagus (1000) having an esophagus center axis, being substantially aligned with a cranial-caudal axis of the patient and having a substantially circular circumference substantially aligned with a horizontal plane of the patient, the esophagus (1000) further having an inner and outer substantially cylindrical surface extending radially in relation to the esophagus center axis, wherein said instrument (200) is adapted to deliver at least one fixating member (14, 14a,b), such that said at least one fixating member (14, 14a,b) engages at least the stomach tissue, the endoscopic instrument (200) comprising a first and a second operable joint (1030, 1031) configured to allow said instrument (200) to go around the angle of His (1010) and reach the fundus area of the stomach (12)
**characterized in that**
said instrument (200) comprises:
a first and a second part (1005a,b) adapted to engage said at least one fixating member (14, 14a,b); the first and second parts (1005a,b) being separated by at least the first and second operable joints (1030, 1031) such that the first and second parts can deliver said at least one fixating member (14, 14a,b) between themselves and such that esophagus tissue and stomach tissue is placed between the first and second parts (1005a,b) when positioned to engage said at least one fixating member (14,14a,b); and
a pulling member configured to pull or grab stomach tissue and hold said stomach tissue in position for delivery of said at least one fixating member (14, 14a,b);
wherein at least one of the first and second parts (1005a,b) is configured to deliver said at least one fixating member (14, 14a,b) adapted to penetrate at least two layers of stomach tissue, or at least one layer of stomach tissue and one layer of esophagus tissue, thereby creating a pouch (1013) of stomach tissue, and the other of the first and second parts (1005a,b) is configured to function as an anvil member to assist in the fixation of said at least one fixating member (14, 14a,b).

2. The endoscopic instrument (200) according to claim 1, wherein said at least one of the first and second parts (1005a,b) is adapted to deliver said at least one fixating member (14, 14a,b) in form of a staple or a suture.

3. The endoscopic instrument (200) according to claim 2, wherein said at least one of the first and second parts (1005a,b) is adapted to deliver said at least one fixating member (14, 14a,b) comprising a first and second state, wherein said at least one fixating member (14, 14a,b) is adapted to, in said first state, penetrate tissue, and in said second state, hinder said at least one fixating member (14, 14a,b) from penetrating tissue.

4. The endoscopic instrument (200) according to claim 1, wherein said anvil member is adapted to be positioned within the esophagus.

5. The endoscopic instrument (200) according to claim 1, wherein said anvil member is adapted to be positioned within the stomach (12).

6. The endoscopic instrument (200) according to claim 1, wherein said instrument (200) is adapted to create the pouch around a volume filling device placed in contact with the stomach tissue.

7. The endoscopic instrument (200) according to claim 1, wherein said instrument (200) is further adapted to insert a volume filling device in the pouch (1013), after the creation of the pouch (1013).

8. The endoscopic instrument (200) according to claim 1, wherein said at least one of the first and second parts (1005a,b) is adapted to place said at least one fixating member (14, 14a,b) above the gastroesophageal junction.

9. The endoscopic instrument (200) according to claim 1, further comprising a retractable member (1052) configured to hold said stomach tissue in position for delivery of said at least one fixating member (14, 14a,b), and to then be retracted to enable removal of the instrument from the stomach (12).

10. The endoscopic instrument (200) according to claim 1, wherein the pulling member comprises at least one tissue penetrating member.

11. The endoscopic instrument (200) according to claim 1, wherein the pulling member is configured to pull the stomach tissue using suction or vacuum.

## Patentansprüche

1. Endoskopisches Instrument (200), das dazu angepasst ist, das Magengewebe und oder das Speiseröhrengewebe eines Patienten von innen und/oder von außen in Eingriff zu nehmen, wobei die Speiseröhre (1000) im Wesentlichen ein röhrenförmiges Gewebe ist, das zum Magen (12) führt, der Magengewebe umfasst, wobei die Speiseröhre (1000) eine Speiseröhrenmittelachse hat, die im Wesentlichen auf eine kraniokaudale Achse des Patienten ausgerichtet ist und einen im Wesentlichen kreisförmigen Umfang hat, der im Wesentlichen auf eine horizontale Ebene des Patienten ausgerichtet ist, wobei die Speiseröhre (1000) ferner eine im Wesentlichen zylindrische Innen- und Außenfläche hat, die sich in Bezug auf die Speiseröhrenmittelachse radial erstreckt, wobei das Instrument (200) dazu angepasst ist, mindestens ein Befestigungselement (14, 14a,b) zuzuführen, sodass das mindestens eine Befestigungselement (14, 14a,b) mindestens das Magengewebe in Eingriff nimmt, wobei das endoskopische Instrument (200) ein erstes und ein zweites bedienbares Gelenk (1030, 1031) umfasst, das dazu ausgelegt ist, zu ermöglichen, dass das Instrument (200) den His-Winkel (1010) umrundet und den Fundusbereich des Magens (12) erreicht,
**dadurch gekennzeichnet, dass**
das Instrument (200) umfasst:
ein erstes und ein zweites Teil (1005a,b), die dazu angepasst sind, das mindestens eine Befestigungselement (14, 14a,b) in Eingriff zu nehmen;
wobei das erste und das zweite Teil (1005a,b) durch mindestens das erste und das zweite bedienbare Gelenk (1030, 1031) derart getrennt werden, dass das erste und das zweite Teil das mindestens eine Befestigungselement (14, 14a,b) zwischen sich zuführen und derart, dass das Speiseröhrengewebe und das Magengewebe zwischen dem ersten und dem zweiten Teil (1005a,b) platziert werden, wenn positioniert, um das mindestens eine Befestigungselement (14, 14a,b) in Eingriff zu nehmen; und
ein Zugelement, das dazu ausgelegt ist, Magengewebe zu ziehen oder zu greifen und das Magengewebe zur Zuführung des mindestens einen Befestigungselements (14, 14a,b) in Position zu halten;
wobei mindestens eins des ersten und des zweiten Teils (1005a,b) dazu ausgelegt ist, das mindestens eine Befestigungselement (14, 14a,b) zuzuführen, das dazu ausgelegt ist, in mindestens zwei Lagen von Magengewebe einzudringen, oder in mindestens eine Lage Magengewebe und eine Lage Speiseröhrengewebe und dadurch eine Tasche (1013) aus Magengewebe zu erzeugen, und das andere des ersten und des zweiten Teils (1005a,b) dazu ausgelegt ist, als ein Ambosselement zu fungieren, um die Befestigung des mindestens einen Befestigungselements (14, 14a,b) zu unterstützen.

2. Endoskopisches Instrument (200) nach Anspruch 1, wobei das mindestens eine des ersten und des zweiten Teils (1005a,b) dazu angepasst ist, mindestens ein Befestigungselement (14, 14a,b) in Form einer Klammer oder einer Naht zuzuführen.

3. Endoskopisches Instrument (200) nach Anspruch 2, wobei das mindestens eine des ersten und des zweiten Teils (1005a,b) dazu angepasst ist, das mindestens eine Befestigungselement (14, 14a,b), das einen ersten und einen zweiten Zustand umfasst, zuzuführen, wobei das mindestens eine Befestigungselement (14, 14a,b) dazu angepasst ist, in dem ersten Zustand, in Gewebe einzudringen, und in dem zweiten Zustand, das mindestens eine Befestigungselement (14, 14a,b) daran zu hindern, in Gewebe einzudringen.

4. Endoskopisches Instrument (200) nach Anspruch 1, wobei das Ambosselement dazu angepasst ist, in der Speiseröhre positioniert zu werden.

5. Endoskopisches Instrument (200) nach Anspruch 1, wobei das Ambosselement dazu angepasst ist, im Magen (12) positioniert zu werden.

6. Endoskopisches Instrument (200) nach Anspruch 1, wobei das Instrument (200) dazu angepasst ist, die Tasche um eine Volumenfüllvorrichtung zu bilden, die in Kontakt mit dem Magengewebe platziert ist.

7. Endoskopisches Instrument (200) nach Anspruch 1, wobei das Instrument (200) ferner dazu angepasst ist, nach der Bildung der Tasche (1013) eine Volumenfüllvorrichtung in die Tasche (1013) einzusetzen.

8. Endoskopisches Instrument (200) nach Anspruch 1, wobei das mindestens eine des ersten und des zweiten Teils (1005a,b) dazu angepasst ist, das mindestens eine Befestigungselement (14, 14a,b) über dem gastroösophagealen Übergang zu platzieren.

9. Endoskopisches Instrument (200) nach Anspruch 1, ferner umfassend ein zurückziehbares Element (1052), das dazu ausgelegt ist, das Magengewebe zur Zufuhr des mindestens einen Befestigungselements (14, 14a,b) in Position zu halten, und dann zurückgezogen zu werden, um das Entfernen des Instruments aus dem Magen (12) zu ermöglichen.

10. Endoskopisches Instrument (200) nach Anspruch 1, wobei das Zugelement mindestens ein Gewebeeindringelement umfasst.

11. Endoskopisches Instrument (200) nach Anspruch 1, wobei das Zugelement dazu ausgelegt ist, das Magengewebe mithilfe von Ansaugen oder Vakuum zu ziehen.

## Revendications

1. Instrument endoscopique (200) adapté pour venir en prise avec le tissu stomacal et/ou le tissu œsophagien d'un patient depuis l'intérieur et/ou l'extérieur de celui-ci, l'œsophage (1000) étant un tissu en forme de tube menant à l'estomac (12) et comprenant du tissu stomacal, l'œsophage (1000) ayant un axe central d'œsophage, qui est sensiblement aligné avec un axe crânio-caudal du patient et ayant une circonférence sensiblement circulaire sensiblement alignée avec un plan horizontal du patient, l'œsophage (1000) ayant en outre une surface sensiblement cylindrique intérieure et extérieure s'étendant radialement par rapport à l'axe central de l'œsophage, ledit instrument (200) étant adapté pour poser au moins un élément de fixation (14, 14a,b), de telle sorte que ledit au moins un élément de fixation (14, 14a,b) vient en prise avec au moins le tissu stomacal, l'instrument endoscopique (200) comprenant une première et une seconde articulation actionnable (1030, 1031) configurées pour permettre audit instrument (200) de contourner l'angle de His (1010) et d'atteindre la zone du fundus de l'estomac (12),
**caractérisé en ce que**
ledit instrument (200) comprend :
une première et une deuxième parties (1005a,b) adaptées pour venir en prise avec ledit au moins un élément de fixation (14, 14a,b) ;
les première et deuxième parties (1005a,b) étant séparées par au moins les première et deuxième articulations mobiles (1030, 1031) de sorte que les première et deuxième parties peuvent poser ledit au moins un élément de fixation (14, 14a,b) entre elles et de sorte que le tissu œsophagien et le tissu stomacal sont placés entre les première et deuxième parties (1005a,b) lorsqu'elles sont positionnées pour venir en prise avec ledit au moins un élément de fixation (14, 14a,b) ; et
un élément de traction configuré pour tirer ou saisir le tissu stomacal et maintenir ledit tissu stomacal en position pour la pose dudit au moins un élément de fixation (14, 14a,b) ;
au moins une des première et deuxième parties (1005a,b) étant configurée pour poser ledit au moins un élément de fixation (14, 14a,b) adapté pour pénétrer au moins deux couches de tissu stomacal, ou au moins une couche de tissu stomacal et une couche de tissu œsophagien, créant ainsi une poche (1013) de tissu stomacal, et l'autre des première et deuxième parties (1005a,b) étant configurée pour fonctionner comme un élément d'enclume pour aider à la fixation dudit au moins un élément de fixation (14, 14a,b).

2. Instrument endoscopique (200) selon la revendication 1, ladite au moins une des première et deuxième parties (1005a,b) étant adaptée pour poser ledit au moins un élément de fixation (14, 14a,b) sous la forme d'une agrafe ou d'une suture.

3. Instrument endoscopique (200) selon la revendication 2, ladite au moins une des première et deuxième parties (1005a,b) étant adaptée pour poser ledit au moins un élément de fixation (14, 14a,b) comprenant des premier et deuxième états, ledit au moins un élément de fixation (14, 14a,b) étant adapté pour, dans ledit premier état, pénétrer dans les tissus, et dans ledit deuxième état, empêcher ledit au moins un élément de fixation (14, 14a,b) de pénétrer dans les tissus.

4. Instrument endoscopique (200) selon la revendication 1, ledit élément d'enclume étant adapté pour être positionné à l'intérieur de l'œsophage.

5. Instrument endoscopique (200) selon la revendication 1, ledit élément d'enclume étant adapté pour être positionné à l'intérieur de l'estomac (12).

6. Instrument endoscopique (200) selon la revendication 1, ledit instrument (200) étant adapté pour créer la poche autour d'un dispositif de remplissage de volume placé au contact du tissu stomacal.

7. Instrument endoscopique (200) selon la revendication 1, ledit instrument (200) étant en outre adapté pour insérer un dispositif de remplissage de volume dans la poche (1013), après la création de la poche (1013).

8. Instrument endoscopique (200) selon la revendication 1, ladite au moins une des première et deuxième parties (1005a,b) étant adaptée pour placer ledit au moins un élément de fixation (14, 14a,b) au-dessus de la jonction gastro-œsophagienne.

9. Instrument endoscopique (200) selon la revendication 1, comprenant en outre un élément rétractable (1052) configuré pour maintenir ledit tissu stomacal en position pour la pose dudit au moins un élément de fixation (14, 14a,b), et pour être ensuite rétracté afin de permettre le retrait de l'instrument de l'estomac (12).

10. Instrument endoscopique (200) selon la revendication 1, l'élément de traction comprenant au moins un élément de pénétration des tissus.

11. Instrument endoscopique (200) selon la revendication 1, l'élément de traction étant configuré pour tirer le tissu stomacal à l'aide d'une aspiration ou d'un vide.
